(19) Europäisches Patentamt

European Patent Office

Office européen des brevets

(11) EP 4 748 945 A2

(12) EUROPEAN PATENT APPLICATION

(43) Date of publication:
27.05.2026 Bulletin 2026/22

(51) International Patent Classification (IPC):
C12Q 1/6851 (2018.01)

(21) Application number: 26166218.3

(22) Date of filing: 07.10.2021

(52) Cooperative Patent Classification (CPC):
(C-Sets available)
C12Q 1/6848; C12Q 1/6809; C12Q 1/6851 (Cont.)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 08.10.2020 GB 202015943

(62) Document number(s) of the earlier application(s) in accordance with Art. 76 EPC:
21794924.7 / 4 225 938

(71) Applicant: Imperial College Innovations Limited
London SW7 2AZ (GB)

(72) Inventors:
• Goertz, John
London, SW7 2AZ (GB)
• Stevens, Molly
London, SW7 2AZ (GB)

(74) Representative: Potter Clarkson
Chapel Quarter
Mount Street
Nottingham NG1 6HQ (GB)

Remarks:
This application was filed on 19-03-2026 as a divisional application to the application mentioned under INID code 62.

(54) METHODS

(57) The invention relates to simplified means of using biological predictive relationships, in some instances reducing the determination of complex gene networks and relative expression patterns to a single reading.

Figure 1 (part 1 of 2)

A

Denaturation (96°C)

Primer annealing (55°C)

Primer extension (72°C)

Repeat 25-35X

Result after 1 cycle: # of DNA molecules doubled

EP 4 748 945 A2

# Figure 1 (part 2 of 2)

B

Polymerization — Forward primer — F ... Q — Reverse primer

Strand displacement

Cleavage

Polymerization completed

(52) Cooperative Patent Classification (CPC): (Cont.)

C-Sets
**C12Q 1/6809, C12Q 2537/143, C12Q 2545/107;**
**C12Q 1/6848, C12Q 2537/143, C12Q 2545/107;**
**C12Q 1/6851, C12Q 2537/143, C12Q 2545/107**

## Description

### Background

Biological systems are incredibly complex, and are governed largely by fluctuations in the expression levels of a multitude of genes. Such differential expression reflects the way those cells interact with others and react to our world. The expression levels of all genes at a particular time point, or in a particular environmental situation, can represent one particular "state". Gene expression levels can change very rapidly, and so therefore can the "state" of a particular biological system, for example a cell or tissue or organ. Determining the "state", i.e. the relative expression of a number of genes at a particular point has clear utility in diagnostics, prognostics and in for example industrial biotechnology, since it is important to know whether a particular biological system is behaving as expected/desired.

Genes do not act in isolation, but as part of complex networks. Because there are so many interacting genes and separate gene networks, fully determining the state of a biological system, such as a cell, is itself highly complex. Although it is now possible to relatively routinely analyse the expression level of all genes within a biological system, for example via RNA-seq, this is not cost nor time effective, both in terms of the sequencing and the subsequent bioinformatics, particularly since only a subset of genes are likely relevant to predict or classify whether a biological system is in a particular state or is in a different particular state, or is exhibiting a particular activity, for example a high protein production state. Determining such complex relationships require pattern recognition, rather than simple algebraic thresholds.

The premise that particular gene networks can be approximated to relatively discrete units underlies much of modern diagnostics, and once a predictive relationship or differential gene regulation signature has been identified that utilises information from a small (or relatively small) subset of the total transcriptome, an assessment of the entire transcriptome of each test sample is not necessary to diagnose the sample as being in a particular state or not. For example, there are a large number of instances where gene expression data, for example transcriptome data, has been obtained from two or more different types of sample and has been analysed, using bioinformatics including machine learning, to identify particular subsets of genes/mRNAs that are under or overexpressed, and to different levels, between the two sample types. The identification of such diagnostic or predictive expression patterns has been used in for example cancer diagnostics, cancer prognostics, diagnosis of tuberculosis and sepsis, as well as veterinary uses such as diagnosing bovine tuberculosis and mastitis, and prediction of response to therapy.

The same types of diagnostic and predictive relationships, decision surface or differential gene regulation signatures based on the relative gene expression of a given set of genes can be used in cell and tissue engineering. For example, often, the goal of "regenerative medicine" is to guide stem cells to differentiate into a specific terminal cell type, or to shift the activity of differentiated cells towards one task or another. Through gene expression profiling, and specifically the idea of "molecular time", it is possible to determine "How differentiated are the cells? How polarized are the cells?". In addition, the field of synthetic biology presents a unique challenge. In a population of cells with highly engineered gene pathways, or several such populations cooperating towards a given task, the bioprocess engineer requires a means of determining whether the system is behaving the way it was designed to.

In the simplest instance, such a predictive relationship, decision surface or differential gene regulation signature can involve the assessment of the presence or absence of expression from a single gene. For example, the presence of mRNA from gene A in a sample predicts that the sample is in a state A (for example "has disease A") and the absence of mRNA from gene A predicts that the sample is in a state B (for example "does not have disease A" i.e. has a different disease or has no disease).

However, most disease states, or other states such as particular regulatory states (for example states in which gene regulation occurs within tolerance windows defined by engineers for quality control) that may be relevant for the bioproduction of various compounds, can only be accurately predicted or diagnosed using the expression data from a larger number of genes. The requirement for the assessment of expression data from a larger number of genes means that even once the predictive relationship or differential gene regulation signature has been determined (e.g. from the analysis of a larger set of expression data to identify those "markers" that can be used to predict a particular state), specialised equipment and skilled bioinformaticians are required to analyse the diagnostic/predictive expression data and form the prediction/diagnosis. For example, the use of techniques such as microarrays, RNA-seq, Nanostring to determine the expression levels of a number of genes requires the use of a range of probes for example with a range of labels, each requiring separate determination. Current methods of determining the diagnosis/prediction of a particular state therefore requires extensive data handling and statistics post sample preparation and post obtaining the actual expression data, and are not suitable for, for example, point of care diagnostic situations.

It would be beneficial to simplify the use and output of a predictive relationship or differential gene regulation signature such that the end-user can perform simple assays that give one, or a low number, of outputs which is typically directly predictive of one of two or more particular states, for example "has disease" or "does not have disease", and which does not require input from statisticians or complicated equipment.

The present invention solves at least the above-mentioned problems with the prior art methods of using predictive relationships or differential gene regulation signatures generated from biological data.

### *Summary of invention*

The inventors of the present invention have developed methods and components that can be used to significantly reduce the complexity of converting the pre-determined predictive relationship, decision surface or differential target oligonucleotide pattern (such as a gene regulation signature between gene expression pattern and a particular state) into a useful diagnostic or predictive result.

The methods described herein use the molecules of the assay themselves to reflect the complex math and artificial intelligence currently used to analyse the standard target oligonucleotide pattern (for example expression data) that is routinely obtained in, for example, medical diagnostics.

The methods disclosed are easy to use, with no requirement for particularly specialist instrumentation, and sample preparation is standard. Once the necessary components have been optimised through routine procedures, actually putting the methods into practice for example in diagnostics/prognostics is very simple and requires in some embodiments a simple multiplex PCR amplification reaction and the reading of two fluorophores. This is in contrast to the present methods that require for example amplification of a number of RNA species using multiple fluorophores, determining the amount of each fluorophore, and subsequently feeding those data into a complicated bioinformatics system that compares the relative levels of each RNA species to determine the "state". For example, if a predictive relationship, decision surface or differential target oligonucleotide pattern (such as a differential gene regulation signature) is based on the relative expression of 10 genes, currently either the expression level of each gene needs to be determined separately, so that the same fluorophore may be used; or 10 different fluorophores need to be used so that the amplification method can be multiplexed. Accordingly, in either case, at least 10 different readings are needed. A key advantage of the present invention is that it reduces the number of readings down, in some cases to a single reading of two different fluorophores (or of all fluorophores used), in a single tube.

The results produced by the methods of the invention are easy to obtain, are clear and can be interpreted by the laboratory researcher, the fermentation specialist and the bedside clinician.

The methods are typically centred around nucleic acid amplification, which the skilled person will understand is highly routine and can be performed with minimal equipment.

In addition, many of the prior art methods reduce the complex networks and predictive relationships, decision surfaces or target oligonucleotide pattern (such as a differential gene regulation signatures) to simple linear relationships, i.e. for example more expression from one gene predicts a certain state, more expression of a different gene predicts a different state. Such a reductionist approach does not accurately reflect biological systems and does not adequately capture and reflect the predictive relationships or differential gene regulation signatures that are capable of being identified and generated, for example through the use of AI.

For example, an AI system may determine that if the expression of gene A is above an arbitrary expression threshold of 10 and the expression of gene B is below a threshold of 5, and the expression of gene C is above a threshold of 7, then the sample is in a particular state, e.g. State A; whereas if the expression of gene A is above a threshold of 10 and the expression of gene B is above a threshold of 10 and the expression of C is below a threshold of 7 then the sample is in a different particular state, State B.

It will be clear that a larger number of different "states" can be determined and predicted based simply on the expression levels of three genes. Whether or not these different states represent clinically useful or biotechnologically useful states will be determined by the samples that the AI system is trained on. In any event, it is possible to see that expression of gene A above a threshold of 10 (i.e. "more" expression of gene A) does not simply reflect a single state. It is the relative expression levels of each of the genes in the particular network, or that have been identified as being part of the predictive network, that are important.

The methods of the present invention are able to capture this complex interdependent relationship and condense it down to a single output which tells the user whether the sample is in, or is likely to be, State A or State B; or is in State A and not in State B or State C, for example.

The methods of the present invention can be termed Competitive Amplification Networks (CANs). The methods adapt RNA/DNA amplification technologies such as PCR to the recognition of complex gene expression patterns. As the name implies, the reaction is engineered with competitive interactions that translate the information provided by a given gene transcript or a set of transcripts into the relative probability of state A versus state B. In some embodiments which utilise fluorophore labelled probes, these probabilities combine to provide an overall diagnosis represented by two colours: interpretation is as simple as checking which colour is brighter. The networks are scalable to encompass a large number of genes without a significant increase in cost or operational complexity. Finally, these networks can be engineered to perform complex, nonlinear operations on multiple targets simultaneously. This technology provides a platform for engineering application-specific kits for disease diagnosis, therapeutics monitoring, regenerative medicine research, and quality control of bioprocess manufacturing.

***Detailed description of the invention***

Accordingly, the invention provides a method of translating the relative abundance of (or presence or absence of) at least two oligonucleotides, for example the relative expression of at least two genes, or presence or absence of at least two mutations, into the relative probability of a particular state, for example the relative probability of State A versus State B.

The invention also provides a method of combining the relative abundance of at least two oligonucleotides, for example the relative expression of at least two genes, or presence or absence of at least two mutations, into a single value.

The invention also provides:

a method of translating the relative abundance of at least two oligonucleotides, for example the relative expression of at least two genes, or presence or absence of at least two mutations, in a sample into the relative probability of a particular state.

a method of detecting the relative abundance of at least three oligonucleotides, for example the relative expression of at least three genes, or presence or absence of at least three mutations, in a sample using only two fluorophore labelled probes.

a method of combining the relative abundance of at least two oligonucleotides, for example the relative expression of at least two genes, or presence or absence of at least two mutations, in a sample into a single value.

a method of converting the predictive relationship, decision surface or differential target oligonucleotide pattern such as a differential gene regulation signature provided by the relative abundance of at least two oligonucleotides or the presence or absence of at least two mutations, in a sample into a single value.

a method of mimicking statistical information with a competitive amplification network.

Arriving at the "probability of a particular state" and the "predictive relationship", "decision surface", or "differential target oligonucleotide pattern" or "differential gene regulation signature" and the "statistical information" is within the means of the skilled person. Such information is typically obtained from microarray data or RNAseq data, for instance, followed by bioinformatics to produce a relationship between two or more markers that can be used to predict the probability of for example state A versus state B. Many examples of such predictive panels exist, see for example: (1) Warsinske, H.; Vashisht, R.; Khatri, P. Host-Response-Based Gene Signatures for Tuberculosis Diagnosis: A Systematic Comparison of 16 Signatures. PLOS Medicine 2019, 16 (4), e1002786. https://doi.org/10.1371/journal.pmed.1002786.

(2) Sweeney, T. E.; Wong, H. R.; Khatri, P. Robust Classification of Bacterial and Viral Infections via Integrated Host Gene Expression Diagnostics. Science Translational Medicine 2016, 8 (346), 346ra91-346ra91. https://doi.org/10.1126/scitranslmed.aaf7165. (3) Cardoso, F.; van't Veer, L. J.; Bogaerts, J.; Slaets, L.; Viale, G.; Delaloge, S.; Pierga, J.-Y.; Brain, E.; Causeret, S.; DeLorenzi, M.; Glas, A. M.; Golfinopoulos, V.; Goulioti, T.; Knox, S.; Matos, E.; Meulemans, B.; Neijenhuis, P. A.; Nitz, U.; Passalacqua, R.; Ravdin, P.; Rubio, I. T.; Saghatchian, M.; Smilde, T. J.; Sotiriou, C.; Stork, L.; Straehle, C.; Thomas, G.; Thompson, A. M.; van der Hoeven, J. M.; Vuylsteke, P.; Bernards, R.; Tryfonidis, K.; Rutgers, E.; Piccart, M. 70-Gene Signature as an Aid to Treatment Decisions in Early-Stage Breast Cancer. New England Journal of Medicine 2016, 375 (8), 717-729. https://doi.org/10.1056/NEJMoa1602253. (4) Zaas, A. K.; Aziz, H.; Lucas, J.; Perfect, J. R.; Ginsburg, G. S. Blood Gene Expression Signatures Predict Invasive Candidiasis. Science Translational Medicine 2010, 2 (21), 21ra17-21ra17. https://doi.org/10.1126/scitranslmed.3000715.

By a predictive relationship, we include the meaning of any statistical classification technique that can be visualized as "decision surface" where each input dimension represents the concentration of a particular target sequence and each output dimension represents a different class. For example, the input domain could consist of two genes and the output domain two classes, healthy and sick. The "decision surface" is then a two-dimensional surface where a given point represents the concentration of the two gene transcripts and the height of the surface at that point corresponds to the probability of being sick if a patient's two genes are expressed at those respective levels. In another example, the input domain could consist of 10 distinct mutations observed in circulating tumour DNA (ctDNA) of a post-surgical prostate cancer patient and the output domain could consist of three categories: no recurrence, mild recurrence, and aggressive recurrence, each of which recommends to the physician a different course of action. The decision surface in this case is (more or less) a 10-dimensional cube, where each point translates a particular combination of mutation concentrations to a relative probability of the three categories, perhaps visualized with color as the relative intensities of the red, green, and blue components of an image.

While a 10-dimensional tricolored cube is difficult to visualize, arriving at such a representation would be routine for a biostatistician, bioinformatician, mathematician, statistician, or data scientist. The expert would begin with a dataset containing the measured concentrations of many potential targets, such as expression of various genes or mutational profile of post-surgical ctDNA, from many individuals, where each individual is known to belong to a different category (e.g., healthy/sick or no/mild/aggressive recurrence). The expert would then apply any of several classification algorithms to arrive at the decision surface, including but not limited to logistic regression, Gaussian process classification, artificial neural network classification, decision trees, random forests, naïve bayes, support vector machines, or nearest neighbours.

Alternatively, the decision surface may be constructed in a more manual, principled manner. For instance, the bioproduction engineer may know the optimal expression level and respective tolerance for each of several genes expressed by their engineered organism or population of organisms. For quality control and process-monitoring purposes, the engineer may wish to know if any of those genes is outside that tolerance window. In this instance, the decision surface could be represented as a multidimensional Gaussian distribution that extends from -1 to +1 in the output domain. Each dimension, as specified above, would represent the concentration of the particular gene transcript, and the marginal Gaussian distribution along that dimension would have its mean (peak) at that gene's ideal concentration and its standard deviation (width) correspond to the respective tolerance window. The competitive amplification network implementation of such a decision surface would exhibit one fluorescent color if all transcripts are at or near their ideal, and another if any transcript is too far beyond its tolerance window.

Another such principled decision surface could arise from personalized surveillance of circulating tumour DNA for the purposes of monitoring a post-surgical prostate cancer patient for early signs of relapse (Coombes et al Clinical Cancer Research 2019 25: DOI: 10.1158/1078-0432.CCR-18-3663). The target mutations of interest would be identified at the time of surgery by comparing the genome of the tumour to that of the patient's healthy tissue. The expert would then select a threshold concentration so that if any of the mutations are observed in the ctDNA above this threshold, the expert would conclude that the cancer has relapsed. The marginal decision surface for a given mutation in this case would consist of a transition from 0 in the absence of the mutation to +1 at that threshold concentration.

Having obtained a decision surface, or probabilistic relationship between targets of interest and classification, the expert would then design a competitive amplification network which approximates this relationship. A given signal (fluorophore color, such as FAM, or band intensity on a lateral flow strip) is designated arbitrarily as corresponding to the positive direction of the output and a second signal (such as HEX) is designated as the negative direction. The difference between the intensities of these two colors thus corresponds to the "height" of the decision surface. Alternatively, should the output domain consist of more than two categories, an appropriate number of signals can be chosen so that certain pairwise differences between them correspond to the probability of different output categories.

Having translated the output domain of the decision surface into the relative intensity of various signals, the expert would choose the architecture of the network. This architecture consists of determining how many synthetic competitors to include, how many primers to include, which oligonucleotide strands share which primers, and which strands are targeted by which probes. For each architecture, then, there are numerous combinations of amplification parameters for each oligo in the system. Choosing among architectures and parameter values would be done by simulating the surface produced by a numerous different architectures each at numerous different parameter values (see section "Simulating competitive amplification") to identify the architecture and combination of parameter values that resemble the pre-determined decision surface. There are many ways known to the art of performing this optimization task, including Evolutionary Algorithms and Simulated Annealing for the choice between architectures as well as Gradient Descent, Stochastic Gradient Descent, or Quasi-Newton methods for identifying ideal parameter combinations. Finally, the expert would design target and synthetic competitor oligonucleotides which exhibit the parameters identified here and share primers according to the selected architecture (see section "Testing and predicting competitor amplification behavior"). Further explanation is provided in the Examples below.

Each of these methods involves the amplification of one or more target polynucleotides in such a way so that the amount of each product that indicates a first state can be cumulatively quantified, and each product that indicates a second state can be cumulatively quantified. Combining these two readings produces a single overall reading that indicates whether the sample is more likely to be in a first state or a second state, i.e. regardless of the number of genes under investigation, the difference between the total green intensity and the total orange intensity (for example), integrates the information from the whole system. For example, in one embodiment all products that are associated with a first state are labelled with a first fluorophore and all products that are labelled with a second state are labelled with a second fluorophore. Provided that the relative contribution of each product to the overall predictive relationship or differential gene regulation signature is taken into account, summing the cumulative quantifications of each state produces an accurate and predictive value. The competitive polynucleotides of the invention and that are used in the methods described herein are engineered, designed or tuned to reflect this predictive relationship or differential gene regulation signature.

Accordingly, the invention provides:

a method of translating the relative abundance of at least two oligonucleotides, for example the relative expression of at least two genes, or presence or absence of at least two mutations, in a sample into the relative probability of a particular state;

a method of detecting the relative abundance of at least three oligonucleotides, for example the relative expression of at least three genes, or presence or absence of at least three mutations, in a sample using only two fluorophore labelled probes;

a method of combining the relative abundance of at least two oligonucleotides, for example the relative expression of at least two genes, or presence or absence of at least two mutations, in a sample into a single value;

a method of converting the predictive relationship, decision surface or differential target oligonucleotide pattern such as a differential gene regulation signature provided by the relative abundance of at least two oligonucleotides or the presence or absence of at least two mutations, in a sample into a single value;

a method of mimicking statistical information with a competitive amplification network; and

a method of reducing complex gene expression patterns in a sample to a single value,

wherein the method comprises the step of amplifying one or more target polynucleotides in a sample.

The method of amplifying one or more target polynucleotides in a sample as described herein is itself provided by the invention.

Theoretically, every target molecule in solution should be replicated every cycle until these primers are used up, but, crucially to CAN design principles, i.e. the methods disclosed herein, perfect doubling is actually difficult to achieve. It is the tuned competitor polynucleotides that comprise the appropriate features that allows a single output to reflect a complex network of expression levels.

Target sequence characteristics such as GC content influence the proportion of molecules that are replicated each cycle and these features are deliberately built into the competitor polynucleotides used herein so that the target polynucleotide(s) is amplified with the appropriate efficiency where the efficiency is tailored to mimic the contribution of that particular target in the overall predictive relationship or differential gene regulation signature.

For example, in a hypothetical scenario where increased expression of two genes is predictive of disease:

a particular gene (G1) is associated with "disease" if expressed at an arbitrary level of greater than 12, and is associated with "non-disease" at an expression level of less than 12, and G1 is weakly predictive; and
a second gene (G2) is associated with disease if the expression level is greater than 6, and is associated with non-disease when the expression level is less than 6, and G2 is strongly predictive.

If the expression of G1 and G2 is simply obtained and added together, without taking into account any individual predictive power, then a sample with a G1 expression level of 10 (predicting "non-disease") and a G2 expression level of 7 (predicting "disease") would have an overall expression level of "disease predicting genes" of 17; whereas a sample with a G1 expression level of 1 (predicting "non-disease") and a G2 expression level of 10 would only have an overall expression level of "disease predicting genes" of 11. On the face of it, without taking the individual predictive power into account, then the first sample would appear to be more likely to be diseased than the second sample. However, when we take into account that G1 is only weakly predictive but G2 is strongly predictive, the actual prediction of disease may be much more likely for the second sample.

Accordingly, it is not enough to simply amplify all "disease associated genes" and add up the amount of product. However, adding up the amount of product is a simple means to obtain a cumulative and accurate prediction based on a number of expression level inputs. The inventors have managed to incorporate the individual predictive power into competitive polynucleotides, so that the relative amount of a target versus a corresponding competitor polynucleotide indicates the predictive power.

For example, taking the above hypothetical example, in one example:
G1 is amplified along with a corresponding competitor polynucleotide that strongly competes with the natural G1 target i.e. the competitor has a higher amplification efficiency that the natural target G1. In this way, the high expression of G1 in the first sample (which has an equivalent of 10 target molecules) is converted into a lower actual G1 target product (e.g. 3), which reflects the lower predictive power of G1. The G1 target product may then be probed with a green labelled probe, and the G1 competitor product may be probed with an orange labelled probe. Following amplification and detection, the amount of green label may be less than the orange label (for example green reading of 3 and an orange reading of 7). G2 on the other hand, due to the high predictive power, may be amplified in the presence of a corresponding competitor polynucleotide that is very difficult to amplify, so that more of the G2 natural target is amplified than the G2 competitor polynucleotide. In the case of the first sample which had an equivalent of 7 G2 targets, the amount of G2 target produced may be 6 and the amount of G2 competitor produced may be 1. The G2 target may be probed with a green labelled probe (i.e. 6 green) and the G2 competitor may be probed with an orange labelled probe (i.e. 1 orange). Adding the results from G1 and G2 together this example would provide a green reading (i.e. disease) of 9 and an orange reading of 8.

For sample 2 which had effectively 1 G1 target molecule and 10 G2 target molecules, G1 may produce a green reading of 0.5 and an orange reading of 1; and G2 may produce a green reading of 9 and an orange reading of 2, with a cumulative reading of 9.5 green versus 3 orange.

It will be understood by the skilled person that in some situations an increased expression of one gene and a repressed expression of a different gene may be indicative of a particular state, for example a diseased state.

The predictive relationship or differential gene regulation signature derived from the original data set(s) (e.g. microarray data, RNAseq data) will provide a threshold of how "green" the overall cumulative fluorescence needs to be to result in a diagnosis of "state A" (i.e. "disease").

If the targets were amplified in a 1:1 manner, then sample 1 would have an overall green reading of 17 and sample 2 would have a reading of 11 which does not accurately reflect how likely the samples are to be in that particular state, e.g. a diseased state.

Although the above is discussed in the context of relative gene expression, the skilled person will understand and appreciate that the same premise is true of situations in which the presence or absence of various mutations is indicative of a particular disease state, such as cancer, or the relative abundance of non-coding RNAs (so, strictly not "gene expression" in the context of protein coding genes, but transcription in general).

Accordingly, as discussed above, where reference is made to relative gene expression, this should be read as also applying to combinations of mutations, or relative transcription and production of for example non-coding RNAs.

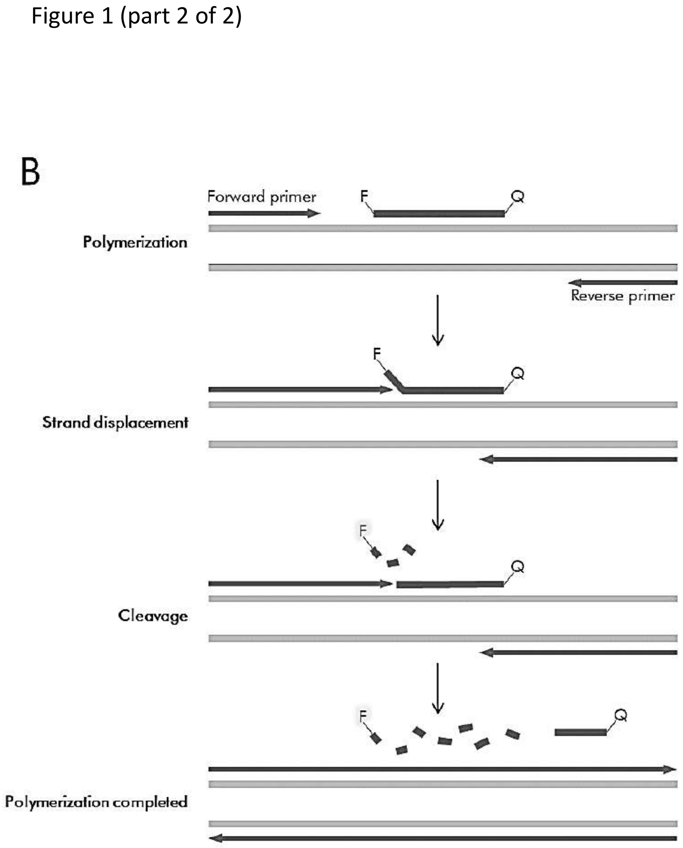

Amplification progression can be monitored in real-time by inclusion of a fluorescently labelled probe oligonucleotide specific to a region of the target product or competitor product between the primer-binding sites (see Figure 1B for an example). For one type of probe, when the appropriate primer is extended, the polymerase degrades the probe into (more or less) individual nucleotides, liberating the fluorophore from the quencher and producing a fluorescent signal. The resulting curve can be modelled as a density-limited exponential growth process:

$$\frac{dF}{dt} = rF\left(1 - \frac{F}{K}\right), \qquad \frac{dK}{dt} = m,$$

[equation (1)]

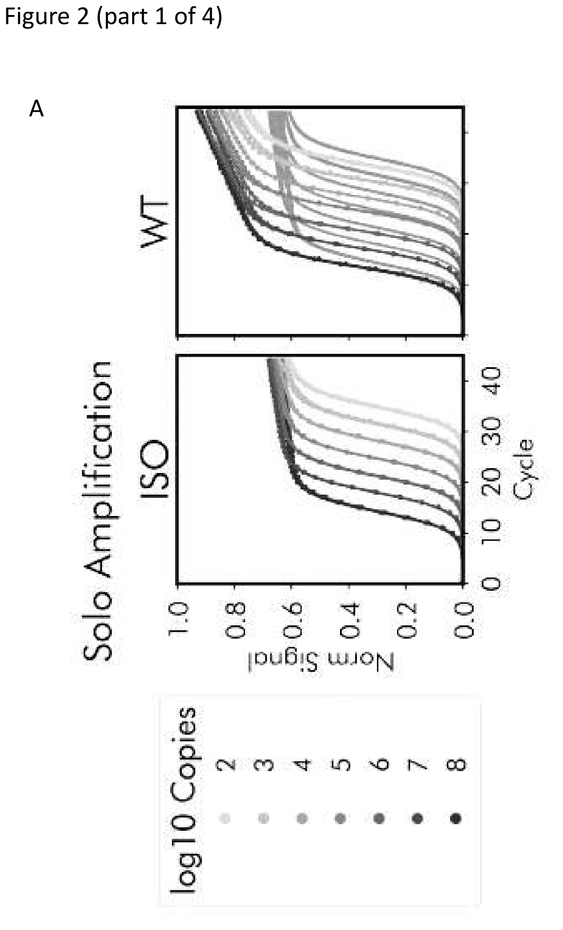

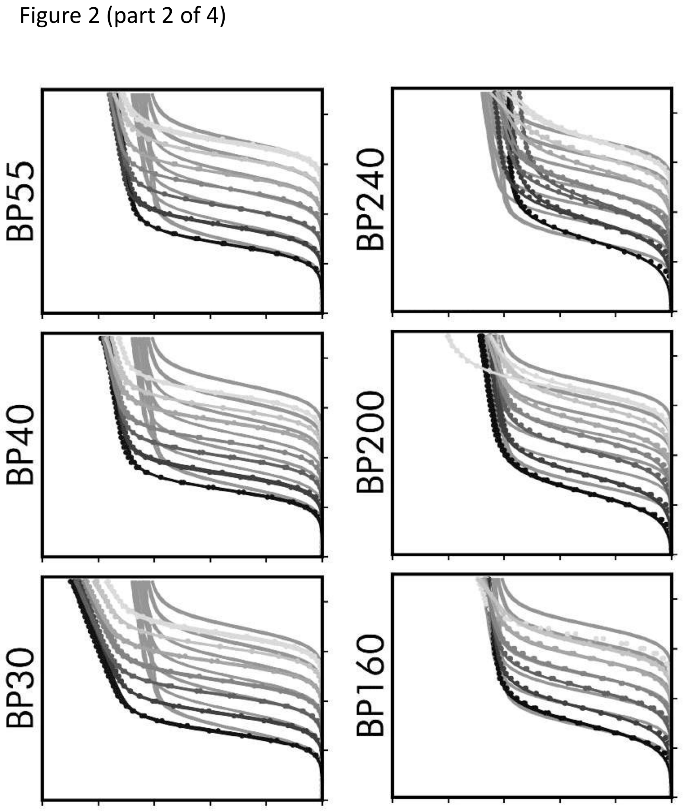

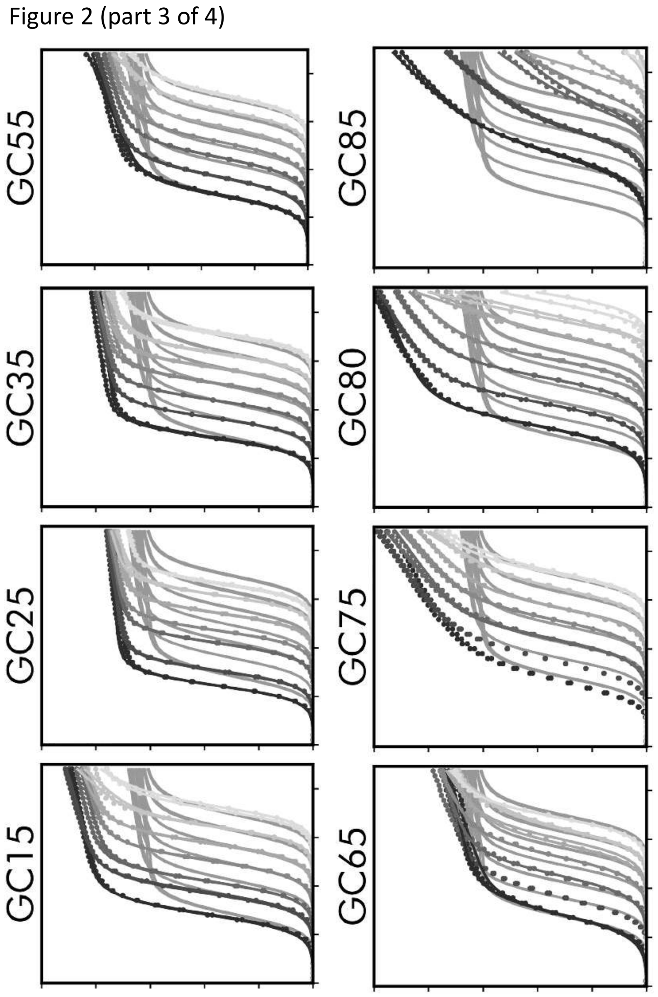

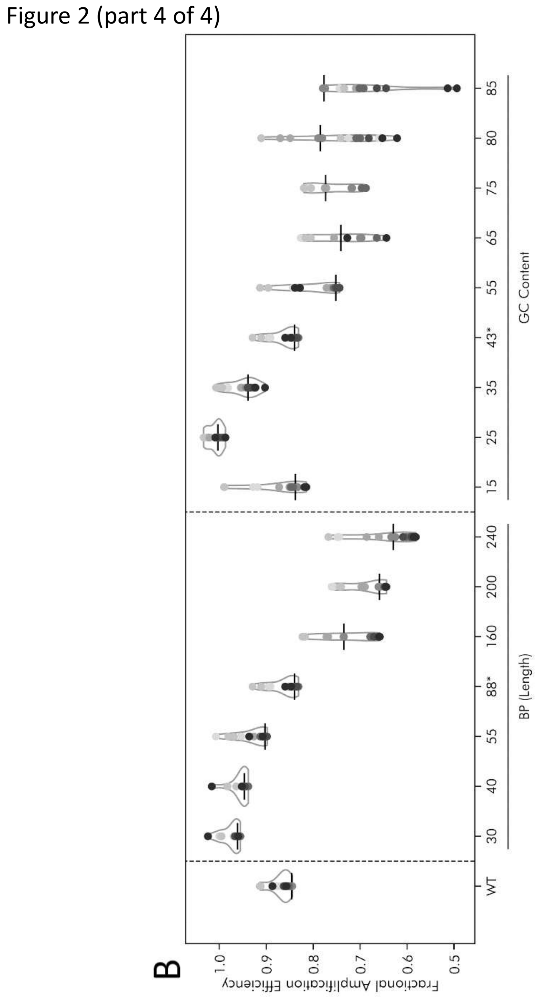

where F is the fluorescence intensity, r is the exponential growth rate (base $e$), $K$ is the signal plateau, and m is the drift of this plateau. The key component here is the $r$, which, when expressed in base 2, represents the fraction of (probe-bound) target strands which replicate each cycle. The r can be changed by altering the sequence of the target between the primer regions, as demonstrated in Figure 2.

Note that, for the most part, all reactions with a given target have the same fluorescence intensity at the end, regardless of the starting quantity of the target. The endpoint of the reaction gives you minimal information about the sample, a drawback remedied by engineering competition into PCR as according to the present invention.

The amplification is a "competitive" amplification that involves the use of a competitor polynucleotide that has been "tuned" to have particular features that are described herein. The skilled person will appreciate that prior art methods of competitive PCR are typically used for target nucleic acid quantification and the competitive polynucleotide used is designed to be as close in sequence to the target as possible, to avoid any discrepancies in amplification efficiency. The amount of target product is compared to the amount of competitor product, typically using gel electrophoresis, and from this the amount of starting target material can be quantified.

In contrast, the present invention specifically requires that the competitor polynucleotide be designed to have a sequence that intentionally results in a particular difference in amplification efficiency between amplification of the target and amplification of the competitor.

In one embodiment then, the invention provides a method of amplifying one or more target polynucleotides in a sample, wherein the method comprises:

providing:

a) a sample comprising polynucleotides
b) a first tuned competitor polynucleotide
c) at least a first primer wherein at least the first primer is capable of hybridising to:

a first target polynucleotide; and
the first tuned competitor polynucleotide; and

initiating a primer extension reaction such that the target polynucleotide (if present in the sample) and the first tuned competitor polynucleotide are amplified,
wherein amplification results in a first target product and a first tuned competitor product.

For the avoidance of doubt, the methods of the present invention are different to "toe-hold" methods in which a "toe hold" primer is initially bound to a shorter "protector" strand, so this protector and the target compete for binding to the target. In this case, the "protector" isn't amplified (it's shorter than the primer.

Also, to be clear, the first tuned competitor polynucleotide is a polynucleotide that has been specifically designed, or "tuned" to have particular properties and has been intentionally introduced into the amplification reaction. A competitor polynucleotide as described herein is considered to be distinct from, for example, other polynucleotides that just happen to also be present in the sample. For example, a competitor polynucleotide according to the invention is not simply another piece of genomic DNA that may compete for hybridisation to the primers, resulting in unwanted background amplification. In one embodiment then, the competitor polynucleotides described herein at intentionally amplified. In one embodiment the competitor polynucleotides described herein are not naturally present in the sample.

It is also important to note that the present method is distinct from prior art methods of competitive amplification whereby the competitor oligonucleotide is designed to intentionally have similar amplification kinetic properties to the target polynucleotide. Such methods are using the art to estimate the concentration of the target polynucleotide, for example where a known amount of competitor polynucleotide is included in the amplification reaction. It is imperative in such methods that the rate of amplification of the competitor mirrors that of the target. It will be clear to the skilled person that this is not the case for the present invention. The present invention requires the tuned competitor oligonucleotide to have different amplification kinetics to the respective target polynucleotide so that the rate of relative amplification of the target and competitor result in products that match the predictive relationship, decision surface or differential target oligonucleotide pattern such as a differential gene regulation signature that is indicative of one of at least two states.

Accordingly in one embodiment the competitor polynucleotide does not have the same or does not have substantially similar amplification kinetics to the respective target polynucleotide.

The present methods are also distinct to methods such as 16s nested PCR which first amplifies a genetic sequence common to most bacteria (a ribosomal subunit) before amplifying or sequencing species-specific sub-regions (Yu et al PLoS One 2015 10: e0132253). A similar approach is used to probe VDJ recombination in human B cells (Koning et al British Journal of Haematology 2016 178: 983-968. In both cases competition occurs, though only among natural sequences. Accordingly, in one embodiment the method is not a 16s nested PCR method, and/or is not a method used to probe VDJ recombination in human B cells.

The skilled person will appreciate that it is possible to amplify a given target sequence and/or tuned competitor sequence using just one primer, for example asymmetric amplification or EXPAR, an exponential amplification reaction (see Reid et al Angewandte Chemie 2018 57: 11856-11866), or with two primers, for example as in the standard PCR. It is not considered necessary that two primers are used to amplify a given target sequence or a given competitor sequence, though typically two primers will be used, arranged so that the first and second primer hybridise on opposite strands of a double stranded target sequence or competitor sequence, so as to result in the production of a target product or competitor product. Two primers may be used to amplify the target sequence, and/or may be used to amplify a portion of or all of the tuned competitor polynucleotide. The skilled person will understand what is required for an appropriate primer, for example length, sequence identify to a portion of the target/competitor sequence.

Accordingly, in some embodiments the method comprises providing a second primer.

In some embodiments the second primer is capable of hybridising to the first target polynucleotide, wherein the first and second primer hybridise on opposite strands of the target so as to result in the production of the first target product, optionally a first target polymerase chain reaction (PCR) product.

The skilled person will also understand that for a first primer to be capable of hybridising to a first target polynucleotide and to a first tuned competitor polynucleotide, a portion of the first target polynucleotide and a portion of the first tuned competitor will have the same, or substantially the same sequence, so as to allow a single primer to hybridise to the two different polynucleotides. The remaining sequence of the target and competitor can be entirely different.

In some instances, where the method comprises the use of a second primer that is capable of hybridising to the first target polynucleotide, the same second primer is also capable of hybridising to the first tuned competitor polynucleotide, wherein the first and second primer hybridise on opposite strands of the first tuned competitor polynucleotide so as to result in the production of the first tuned competitor product, optionally first tuned competitor PCR product. In this case, the first target polynucleotide and the first tuned competitor polynucleotide will share two regions that are identical, or that are substantially identical, so as to allow the hybridisation of the first and second primer to each polynucleotide. The skilled person will understand how similar two sequences need to be so as to allow hybridisation of the same primer.

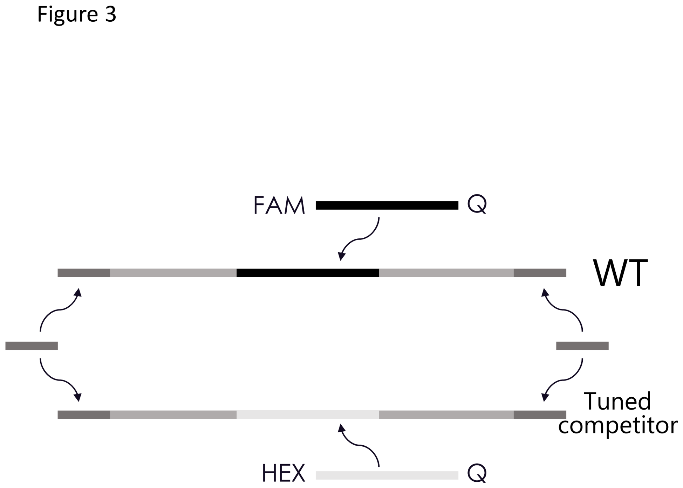

This arrangement, whereby the first target and the first competitor polynucleotides are amplified using the same first and second primers is depicted in Figure 3, and can be termed a "direct" method, or a direct CAN. The first also shows one particular embodiment which uses two labelled probes. However, as described herein, different probe systems, and different detection methods can be used. Typically, the method will require a labelled probe that can hybridise to the target polynucleotide product, and a probe labelled with a different label that can bind to the first competitor polynucleotide product.

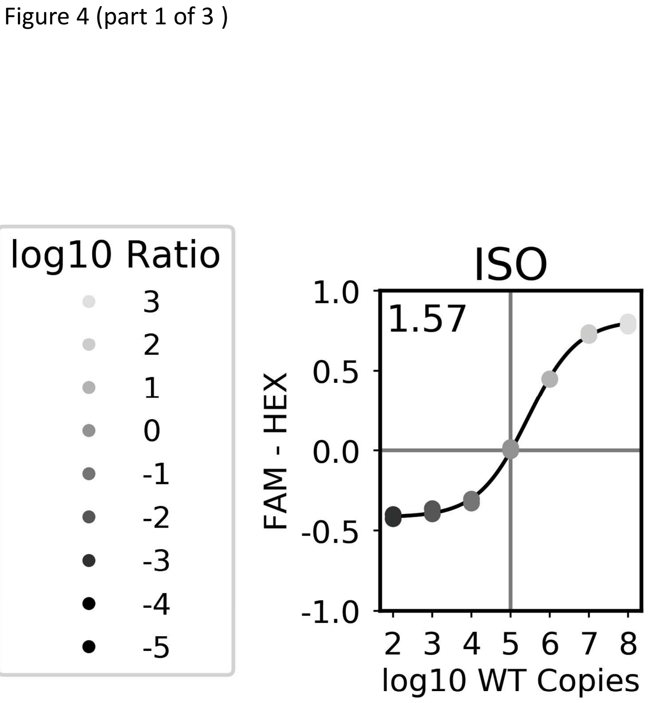

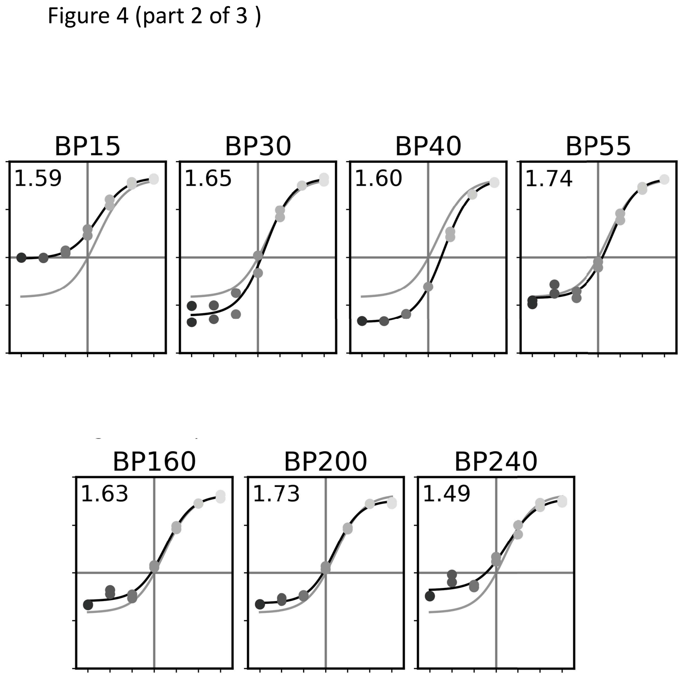

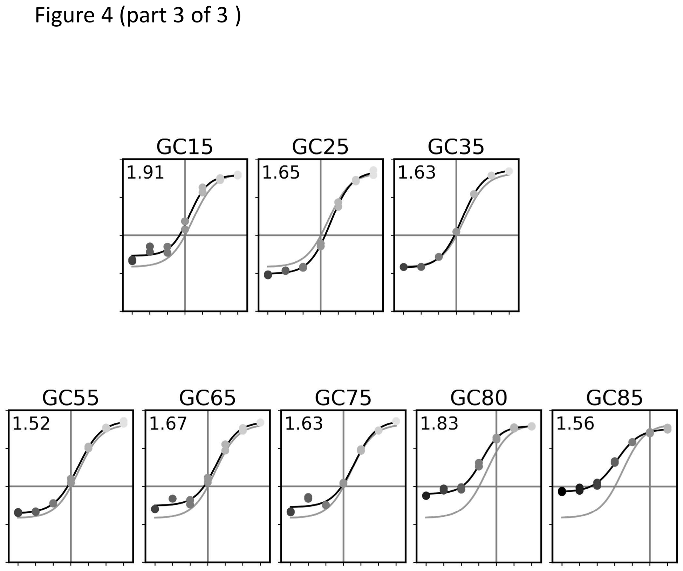

When the target and the competitor are amplified in the same amplification reaction, they compete for the primers. Since primers are consumed by each replication of a target strand, the amplification of both sequences stops as soon as the primer pool is exhausted. The quantity of each amplification product at the end of the reaction depends on the relative starting quantity of the two targets. This is reflected in the resulting fluorescent signal (see for example Figure 4). For two targets with the same amplification rate (such as the WT and the ISO from Figure 3) that begin at the same concentration, the fluorescent signal derived from each will be the same at the end of the reaction. If there is more "target" than competitor at the start of the reaction, the fluorescence associated with the target product will be more intense at the end, and vice versa. The sharpness or gradient of the transition from pure target signal to pure competitor signal can be tuned by adjusting the amplification rate of the competitor. Methods of designing the competitor polynucleotide sequence and length to adjust the amplification rate are described herein.

### *Testing and predicting competitor amplification behavior*

To estimate parameters governing amplification behaviour, each competitor can be amplified in a reaction containing the appropriate primers, the relevant fluorophore-labelled probe, and standard qPCR master mix (TaqMan Fast Advanced Master Mix from ThermoFisher Scientific). The resulting fluorescent data should be fitted with one of a number of algorithms which the skilled person will able to select, for example (herein referred to as the *mechanistic model* as used in the Examples) using standard non-linear least squares estimation,

$$F \;=\; f \cdot \left(1 + \frac{f}{K} \cdot m \cdot \left(t + \frac{\log F_0}{r}\right)\right) \tag{2}$$

where *f* is defined as

$$f \;=\; \frac{K}{1 + \dfrac{K - F_0}{F_0}\, 2^{-rt}} \tag{3}$$

where *r* is the amplification rate, $F_0$ is the initial fluorescence at the beginning of the reaction, *m* indicates the degree of drift of the steady-state fluorescence, and *K* gives the steady-state fluorescence in the absence of drift. The above equation is merely exemplary, other models which describe amplification behaviour may also be used. As described below, one way of estimating the parameters of this mechanistic model is via a Generalized Linear Model, specified as follows. To allow efficient estimation, the following variable substitution on $F_0$ and r is first applied:

$$\tau = -\frac{\log F_0}{r} \tag{4}$$

$$\rho = -\frac{\log r}{\log \tau} \tag{5}$$

The input parameters to the model are the length of region of the sequence between the primers, in base pairs (BP), the GC content of that region in percent (GC), and the concentration of the sequence in copies (Q). The input and output ($\rho, \tau, K,$ and *m*) parameters are first put into "standardized" form (indicated by a ^) as follows:

$$\log BP = \widehat{BP} \cdot \sigma_{BP} + \mu_{BP} \tag{6}$$

$$\operatorname{logit} GC = \widehat{GC} \cdot \sigma_{GC} + \mu_{GC} \tag{7}$$

$$\log_{10} Q = \widehat{Q} \cdot \sigma_{Q} + \mu_{Q} \tag{8}$$

$$\text{logit } \rho = \hat{\rho} \cdot \sigma_\rho + \mu_\rho \tag{9}$$

$$\tau = \hat{\tau} \cdot \sigma_\tau + \mu_\tau - \left(Q - \mu_Q\right) \cdot \log_2 10 \tag{10}$$

$$\text{logit } K = \widehat{K} \cdot \sigma_K + \mu_K \tag{11}$$

$$\log m = \widehat{m} \cdot \sigma_m + \mu_m \tag{12}$$

$$\text{logit } \rho = \hat{\rho} \cdot \sigma_\rho + \mu_\rho \tag{13}$$

The regression model is then given by:

$$\hat{\rho} = \alpha_\rho + \beta_{\rho,BP} \cdot \widehat{BP} + \beta_{\rho,GC} \cdot \widehat{GC} + \epsilon_\rho + \left(\gamma_\rho + \zeta_{\rho,BP} \cdot \widehat{BP} + \zeta_{\rho,GC} \cdot \widehat{GC} + \epsilon_{\rho,Q}\right) \cdot \widehat{Q} \tag{14}$$

$$\hat{\tau} = \alpha_\tau + \beta_{\tau,BP} \cdot \widehat{BP} + \beta_{\tau,GC} \cdot \widehat{GC} + \epsilon_\tau + \left(\gamma_\tau + \zeta_{\tau,BP} \cdot \widehat{BP} + \zeta_{\tau,GC} \cdot \widehat{GC} + \epsilon_{\tau,Q}\right) \cdot \widehat{Q} \tag{15}$$

$$\widehat{K} = \alpha_K + \beta_{K,BP} \cdot \widehat{BP} + \beta_{K,GC} \cdot \widehat{GC} + \epsilon_K + \left(\gamma_K + \zeta_{K,BP} \cdot \widehat{BP} + \zeta_{K,GC} \cdot \widehat{GC} + \epsilon_{K,Q}\right) \cdot \widehat{Q} \tag{16}$$

$$\widehat{m} = \alpha_m + \beta_{m,BP} \cdot \widehat{BP} + \beta_{m,GC} \cdot \widehat{GC} + \epsilon_m + \left(\gamma_m + \zeta_{m,BP} \cdot \widehat{BP} + \zeta_{m,GC} \cdot \widehat{GC} + \epsilon_{m,Q}\right) \cdot \widehat{Q} \tag{17}$$

where $\alpha$ denotes the "typical" value of the given parameter across all sequences and concentrations, $\beta$ indicates the dependence on the length or GC content of a given sequence, respectively, $\gamma$ represents the "typical" dependence on concentration across all sequences, and $\zeta$ defines how the dependence on concentration varies with length and GC content. In the *regression* model, which seeks to estimate parameter values from observed data, $\varepsilon$ represents the deviation of a given sequence's behavior from the global trend indicated by
the remaining parameters; the *prediction* model, which supplies parameter values for new, untested sequences, is the same as the regression model but without the $\varepsilon$ components.

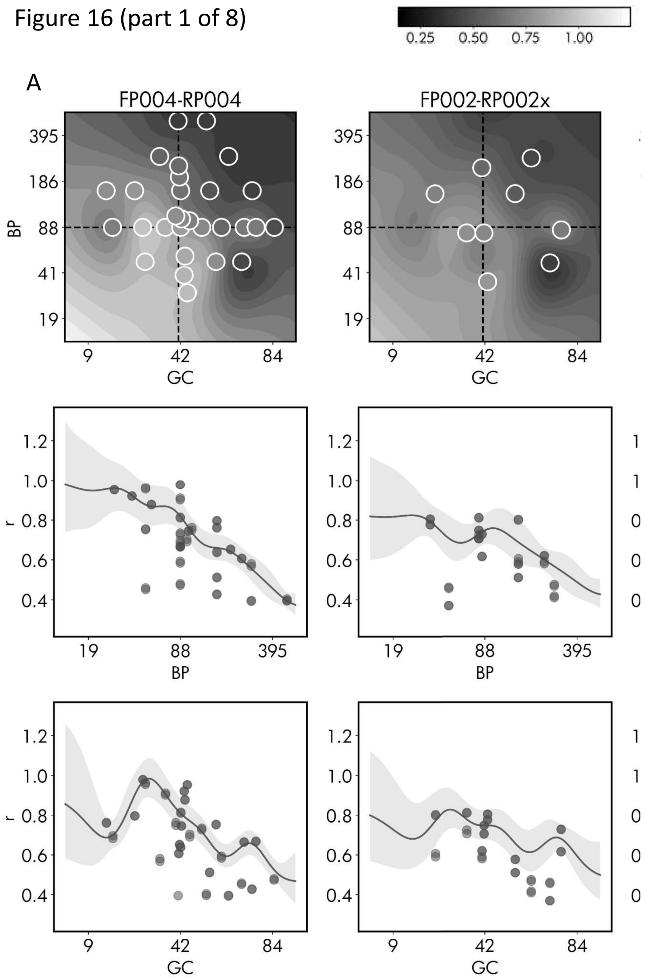

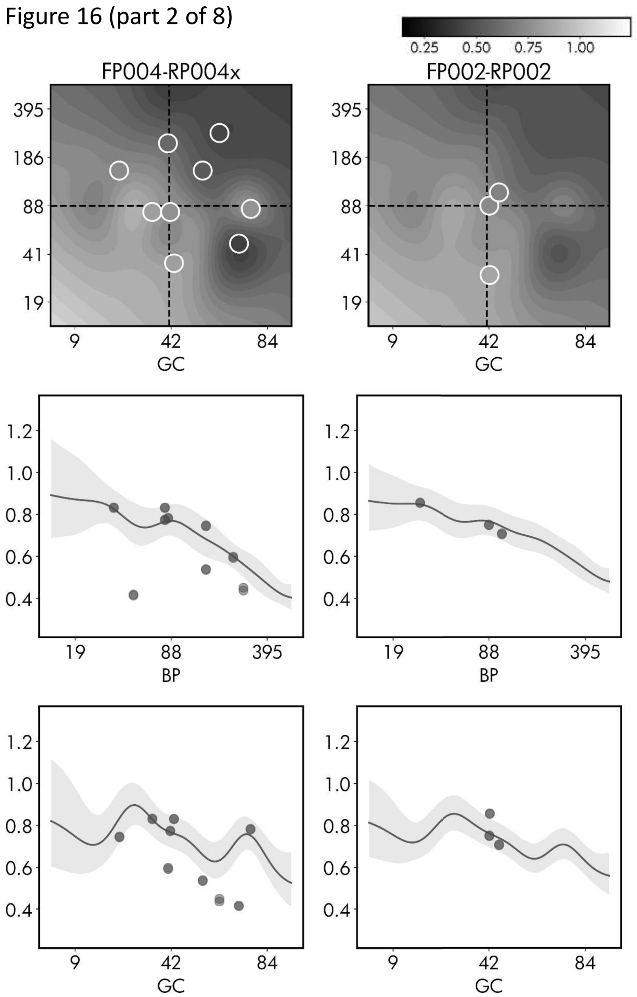

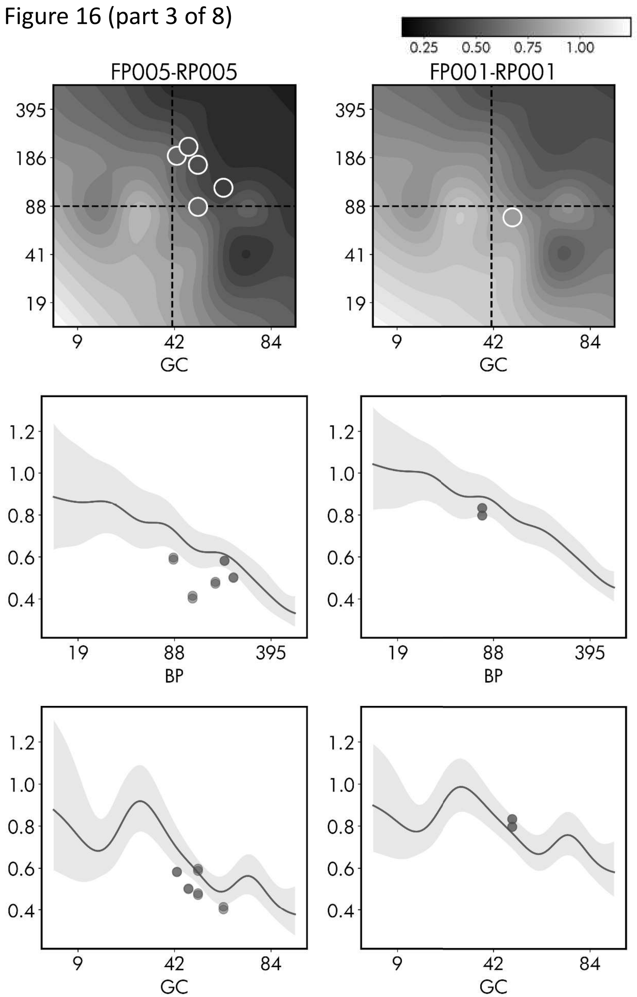

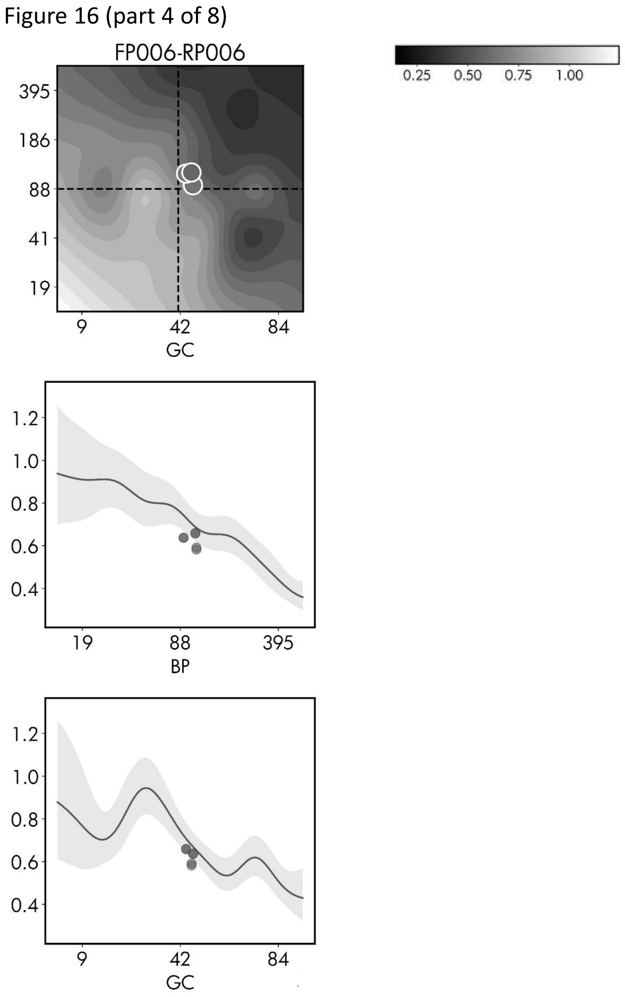

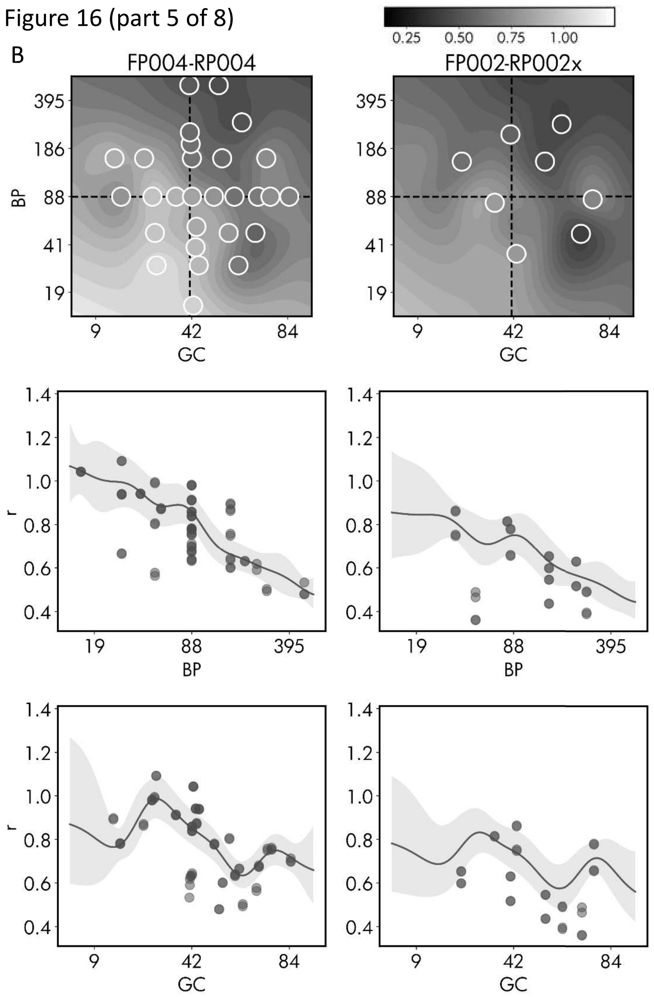

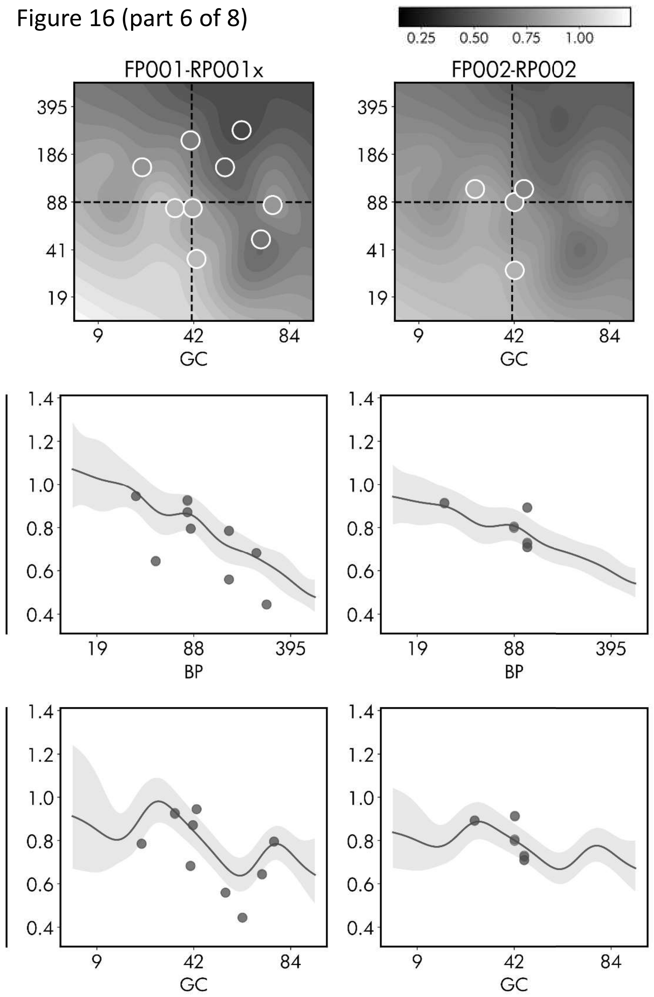

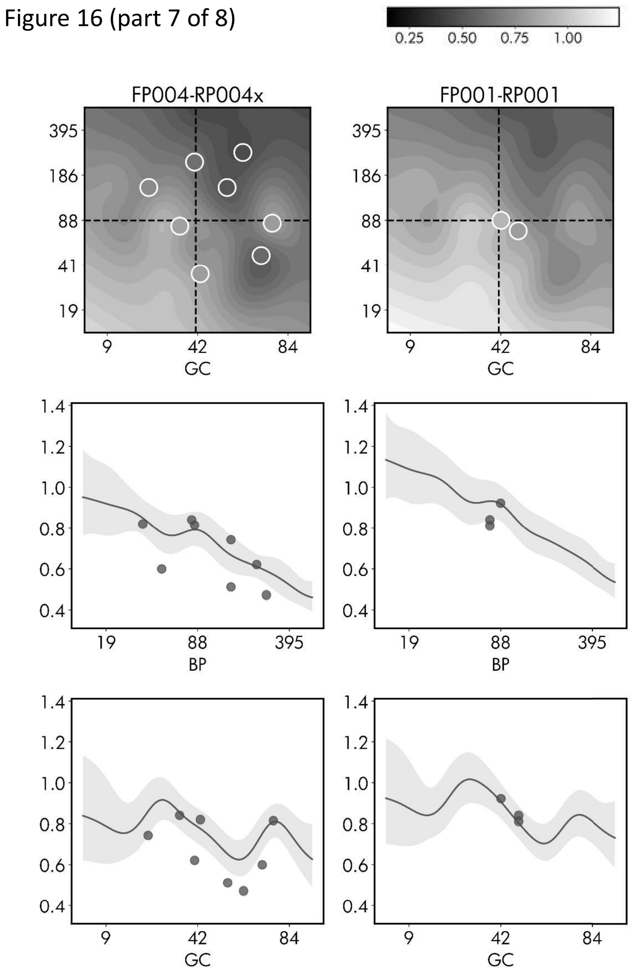

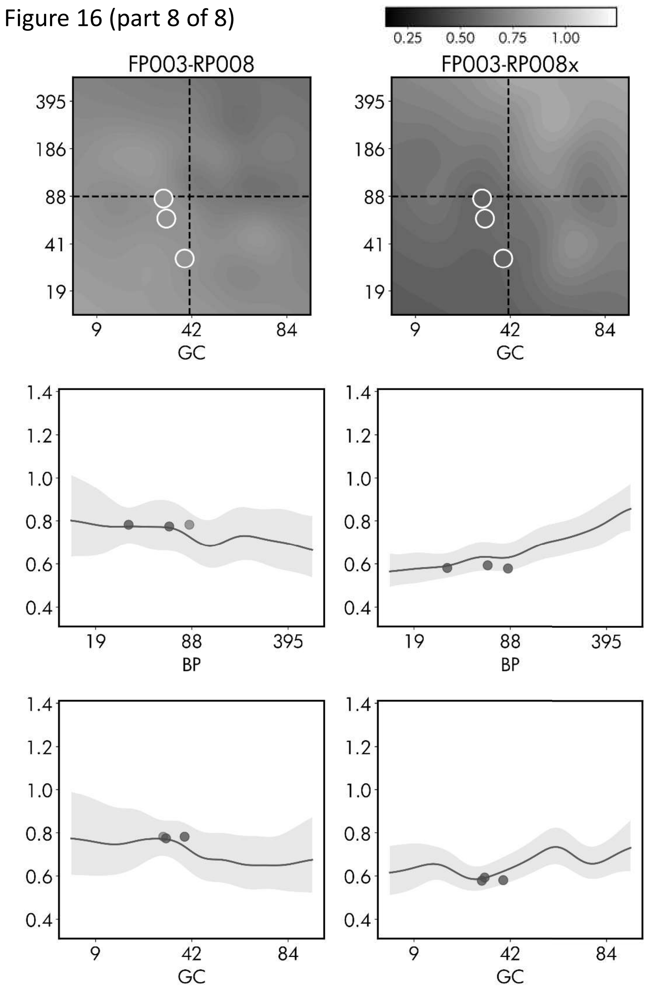

As shown in the Examples, in one embodiment 16 different competitors ranging in length from 30 to 240 base pairs and GC content from 15% to 85% are amplified. Each competitor at seven different concentrations (i.e., the reaction contained $10^2$, $10^3$, $10^4$, $10^5$, $10^6$, $10^7$, or $10^8$ copies of the competitor) in duplicate. The skilled person will be able to select an appropriate number of competitors, appropriate length, appropriate GC content and concentration, depending on the particular circumstances. The parameter values for the model above can be estimated using a Bayesian approach; however, other linear regression techniques could be used, including but not limited to maximum-likelihood estimation, least-squares estimation, ridge regression, and lasso regression.
The results of the regression of the 16 competitors described in the Examples are shown in Figure 16, and the estimated parameter values in the table below. In the figure, "Intercept" refers to the sum of the $\alpha$ and $\beta$ components while "Slope" refers to the sum of the $\gamma$ and $\zeta$ components. Dots represent the values estimated for specific sequences, while the line and shaded area give the overall trend and accompanying uncertainty, respectively.

|  | $\rho$ | T | K | m | BP | GC | Q |
|---|---|---|---|---|---|---|---|
| $\mu$ | -1.32 | 27.5 | 0.74 | -5.25 | 4.48 | -0.282 | 5 |
| $\sigma$ | 0.31 | 3.6 | 0.38 | 0.70 | 0.75 | 1.0 | 2 |
| $\alpha$ | -0.705 | -0.240 | -0.119 | 0.306 |  |  |  |
| $\beta_{BP}$ | 1.180 | 0.128 | -0.546 | -0.383 |  |  |  |
| $\beta_{GC}$ | 0.715 | 0.366 | -0.277 | 0.669 |  |  |  |
| $\gamma$ | 0.409 | -0.118 | 0.036 | -0.154 |  |  |  |
| $\zeta_{BP}$ | 0.105 | -0.018 | -0.006 | -0.061 |  |  |  |
| $\zeta_{GC}$ | 0.076 | -0.104 | 0.010 | 0.011 |  |  |  |

Besides a Generalized Linear Model, other regression techniques could be used, including but not limited to non-linear regression and non-parametric regression such as polynomial regression, Gaussian Processes, Artificial Neural Networks, Support Vector Machines, Nearest Neighbours, Decision Trees, Random Forests, and Naïve Bayes.

*Simulating competitive amplification*

The above equations describe the amplification of a given sequence in isolation. To simulate amplification behaviour when multiple oligos compete with one another, a more fine-grained model is used. Competitive amplification is modelled as an example of Monod growth (Monod, Jacques (1949). "The growth of bacterial cultures". Annual Review of Microbiology. 3: 371-394. doi:10.1146/annurev.mi.03.100149.002103).

Commonly used to model growth of microorganisms, this approach describes replication at some maximal rate that is dampened as the limiting substrate is consumed. Each of the two strands of a given oligonucleotide are considered as a separate "organism" that generates its complement at the maximum rate described above as the sequence's amplification rate. In doing so, it consumes the corresponding primer; the decreasing concentration of this primer depresses the generation rate of new strands. The magnitude of this dampening is given by the ratio of the given primer concentration to the sum of that same concentration and the concentration of all strands which bind to the primer. For simple, non-competitive PCR (one target, two primers), the model consists of the following system of ordinary differential equations:

$$\frac{dA_+}{dt} = rA_-\mu_{p1}, \quad \frac{dA_-}{dt} = rA_+\mu_{p2} \qquad (18.1, 18.2)$$

$$\frac{dp1}{dt} = -\frac{dA_+}{dt}, \quad \frac{dp2}{dt} = -\frac{dA_-}{dt} \qquad (19.1, 19.2)$$

$$\mu_{p1} = \frac{p1}{A_- + p1}, \quad \mu_{p2} = \frac{p2}{A_+ + p2} \qquad (20.1, 20.2)$$

where $A_+$ and $A_-$ are the concentrations of the positive and negative strands of a sequence A, $p1$ and $p2$ are the concentration of two primers, and $r$ is the amplification rate for the sequence (note that the $\mu$ here is unrelated to the $\mu$ in the previous equations.

The model for direct competitive PCR (two targets WT and REF, two primers) is as follows:

$$\frac{dWT_+}{dt} = rWT_-\mu_{p1}, \quad \frac{dWT_-}{dt} = rWT_+\mu_{p2} \qquad (21.1, 21.2)$$

$$\frac{dREF_+}{dt} = rREF_-\mu_{p1}, \quad \frac{dREF_-}{dt} = rREF_+\mu_{p2} \qquad (22.1, 22.2)$$

$$\frac{dp1}{dt} = -\frac{dREF_+}{dt} - \frac{dWT_+}{dt}, \quad \frac{dp2}{dt} = -\frac{dREF_-}{dt} - \frac{dWT_-}{dt} \qquad (23.1, 23.2)$$

$$\mu_{p1} = \frac{p1}{WT_- + REF_- + p1}, \quad \mu_{p2} = \frac{p2}{WT_+ + REF_+ + p2} \qquad (24.1, 24.2)$$

A skilled person could thus describe all the competitive amplification systems contained herein in a similar manner. These systems of differential equations can be solved using any of many analytical or numerical techniques known in the art to yield curves which describe the concentration of each species in the reaction over time. To obtain curves of the signal from a given probe or set of probes over time, the practitioner would combine the concentrations of the strands cognate to those probes. For example, in the above example of direct competitive PCR, consider a case where a FAM-labeled probe was designed to bind to the WT- strand (i.e., it shares sequence identity with the $WT_+$ strand), and a HEX-labeled probe was designed to bind to the REF- strand. The FAM signal is thus given by the concentration of the $WT_+$ strand, and the HEX signal is given by the concentration of the $REF_+$ strand. If an additional FAM-labeled probe was designed to bind to the $REF_+$ strand, the FAM signal would be given by the sum of the $WT_+$ and REF- strand concentrations.

The scenario described so far, i.e. one target polynucleotide and one corresponding competitor polynucleotide represents one of the simplest applications of the invention. However, assessing the expression level of one gene does not really represent a gene network. The expression level of multiple genes in a gene network can be assessed using a combination of amplifying more than one target polynucleotide and/or providing more than one competitor polynucleotide. The invention provides different combinations, some of which will be described in more detail, but the skilled person will understand that a large number of combinations of different target polypeptides, different competitors and different arrangements of primers, e.g. primers shared between target and competitor, shared between competitor and competitor, and/or shared between target and target.

Some of these methods are termed "indirect" methods, or indirect CAN.

The indirect CAN methods described herein are considered to be less expensive when larger gene signatures are to be analysed, since in the "direct" methods at least one if not two probes need to be designed for each transcript targeted. For gene signatures (e.g. gene expression levels, presence or absence of particular mutations, abundance of non-coding RNA) with 20-50 targets iterating on sequence designs becomes prohibitively expensive. To address this issue, indirect CANs provide similar functionality at a more or less fixed cost regardless of the number of genes under investigation. Indirect competition also opens the possibility of higher-order networks capable of complex, non-linear analysis of multiple targets simultaneously. Finally, redundant targeting allows additional flexibility for all CAN architectures.

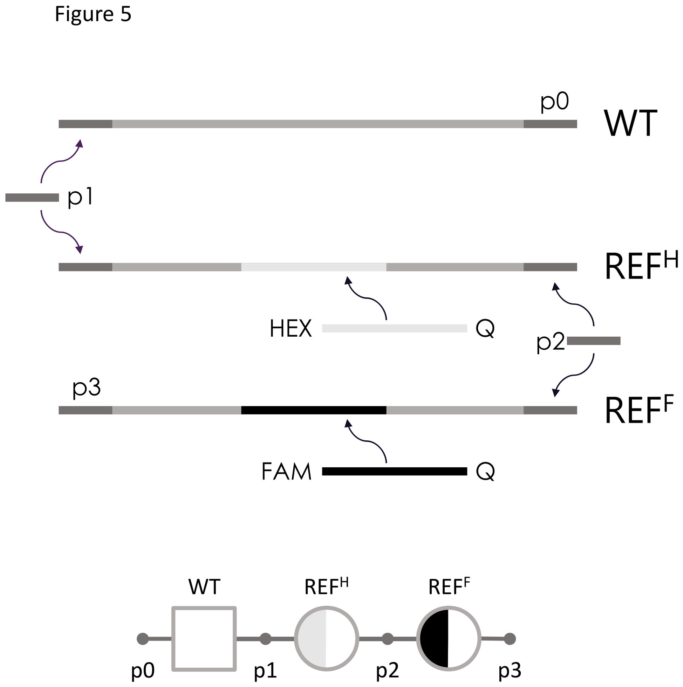

The direct competition methods described herein use competition between a probed target polynucleotide product and a probed competitor polynucleotide product. The indirect method uses an un-probed target polynucleotide to simply mediate the competition between competitor polynucleotide. Because both primers are necessary for exponential amplification of a given target, replication can be arrested by depletion of only one primer. In this embodiment of the invention a competitor polynucleotide, shown as REFH in Figure 5, is designed that shares one primer with a target polynucleotide, WT, and its second primer with a second competitor polynucleotide, REFF (Figure 5). If all components have equal amplification rate and the two competitors (REFs) start at equal concentration, without any WT present the HEX and FAM signals (labels on the nucleic acid probes) will amplify equally. However, increasing WT begins to outcompete REFH, dampening the HEX signal. This, in turn, creates more room for REFF to grow, leading to a greater FAM signal at the end of the reaction. The result is an S-shaped response curve to various WT concentrations, similar to that observed from direct competition (Figure 5A). This response curve can be tuned by adjusting the amplification rate of any of the targets, the starting concentration of the competitor polynucleotides, the concentration of any of the primers, or the topology of the network itself (Figure 5B,C). The key advantage of this system is that, because the sequence of the competitor polynucleotide is not restricted (only the regions that hybridise to the primers have any sequence constraints), the same two probe sequences can be reused to probe multiple competitor polynucleotide products, minimizing development costs regardless of how many natural targets are utilized or how complex the network is.

Accordingly, in some embodiments the method comprises providing a second tuned competitor polynucleotide.

In some embodiments the second primer is:

a) capable of hybridising to the first tuned competitor polynucleotide, wherein the first and second primer hybridise on opposite strands of the first tuned competitor polynucleotide so as to result in the production of the first tuned competitor product, optionally first tuned competitor PCR product; and

b) is capable of hybridising to the second tuned competitor polynucleotide and initiating a primer extension reaction such that the second tuned competitor polynucleotide is amplified so as to result in the production of the second tuned competitor product, optionally in combination with a further primer wherein the second and further primer hybridise on opposite strands of the second tuned competitor polynucleotide so as to result in the production of the second tuned competitor product, optionally a first target polymerase chain reaction (PCR) product,

optionally wherein the second primer is not capable of hybridising to the first target polynucleotide.

In other less preferred embodiments of the indirect method, the second primer is:

a) capable of hybridising to the first target polynucleotide, wherein the first and second primer hybridise on opposite strands of the target so as to result in the production of the first target product, optionally a first target polymerase chain reaction (PCR) product; and

b) capable of hybridising to the second tuned competitor polynucleotide,

and is optionally not capable of hybridising to the first competitor polynucleotide.

In some embodiments, the second primer is capable of hybridising to a second target polynucleotide, and is optionally not capable of hybridising to the first target polynucleotide.

It will be appreciated that, as described above, the method can be used in the context of more than one target polynucleotide. In some instances, the method is used to determine the expression of more than one gene, the presence or absence of more than one particular mutation, and/or the abundance of more than one non-coding RNA. In other embodiments, the skilled person will understand that the relevant primers may be designed so that the more than one target polynucleotide are part of the same actual RNA molecule. For example several primer pairs can be designed to amplify several different regions from a single mRNA. In conjunction with the appropriate competitor polynucleotides this embodiment of the methods of the invention is termed a "redundant" method.

Accordingly, in one embodiment the second target polynucleotide is part of the same polynucleotide molecule as the first target polynucleotide.

In other embodiments the second target polynucleotide is on a different polynucleotide molecule to the first target polynucleotide.

It will be appreciated that typically two primers are used to amplify each target. Accordingly, the methods of the invention may comprise more than two primers, for example at least 3, 4, 5, 6 or more primers.

For example, in one embodiment, the second primer is:

a) capable of hybridising to the first target polynucleotide, wherein the first and second primer hybridise on opposite strands of the target so as to result in the production of the first target product, optionally a first target polymerase chain reaction (PCR) product; and
b) is not capable of hybridising to the first or second tuned competitor polynucleotide

and wherein the method comprises a third primer capable of hybridising to the first and to the second tuned competitor polynucleotide.

In other embodiments the method comprises providing a fourth primer, wherein the fourth primer is capable of hybridising to the first target polynucleotide, wherein the first and fourth primer hybridise on opposite strands of the target so as to permit formation of the first target product, optionally a first target PCR product.

As can be seen, any suitable arrangement of primers is provided by the methods of the invention, so that each relevant target or competitor is amplified, and so that each target and competitor compete appropriately for the relevant primers.

To further exemplify the different combinations of target, competitor and primer arrangement provided by the invention, in some embodiments the method comprises providing:

a) a second primer capable of

i) hybridising to the first target polynucleotide, wherein the first and second primer hybridise on opposite strands of the target so as to result in the production of the first target product, optionally a first target polymerase chain reaction (PCR) product; and
ii) capable of hybridising to the first tuned competitor polynucleotide; and

b) a third primer capable of

i) hybridising to the first tuned competitor polynucleotide wherein the third and second primer hybridise on opposite strands of the first tuned competitor polynucleotide so as to result in the production of the first tuned competitor polynucleotide product, optionally a first tuned competitor polynucleotide polymerase chain reaction (PCR) product; and
ii) capable of hybridising to the second tuned competitor polynucleotide;
and

c) a fourth primer capable of hybridising to the second tuned competitor polynucleotide wherein the third and fourth primer hybridise on opposite strands of the second tuned competitor polynucleotide so as to result in the production of the second tuned competitor polynucleotide product, optionally a second tuned competitor polynucleotide polymerase chain reaction (PCR) product.

It will be clear that the fourth and fifth primers may bind to other target polynucleotides and/or to other competitor polynucleotides, expanding the complexity of the network that is assessed.

As described above, a key feature of the present invention is the use of one or more tuned competitor polynucleotides, that has an amplification rate that has been specifically tuned relative to the corresponding target polynucleotide or relative to the amplification rate of other target or competitor polynucleotides within the network. This tuning provides the discrimination in amplification that translates the predictive relationship, decision surface, or differential target oligonucleotide pattern (such as a differential gene regulation signature or presence or absence of particular mutations) into a relative abundance of each amplification product that can be simply interrogated, for example by using labelled nucleic acid probes.

Accordingly, in one embodiment the amplification rate of the first target polynucleotide is different to the amplification rate of the first tuned competitor polynucleotide. In other embodiments the amplification rate of a target polynucleotide is different to the amplification rate of its corresponding tuned competitor polynucleotide.

Typically, in prior art amplification methods, when trying to amplify a product the amplification rates are optimised, so that amplification is as efficient as possible. The skilled person is aware of techniques to increase the efficiency of amplification, for example altering the length of the product, altering the G/C content and changing the concentration of the primers. Since the skilled person knows how to improve amplification, so the skilled person knows how to make amplification less efficient, i.e. decrease the rate of amplification.

The skilled person will understand that it is the relative amplification rate between the target and the competitor (or in some cases between the target and competitors, or between the targets and competitor, or between the targets and competitors) that is important, not necessarily the absolute amplification rate. Accordingly, it is important that the most appropriate region of the target is chosen for amplification, for example the most appropriate 200bp region of a particular target mRNA, so that the relative amplification rate between target and competitor is appropriate.

Accordingly, in one embodiment the amplification rate of any of the target polynucleotides or competitor polynucleotides can be altered by one or more of:

a) Selecting the target nucleic acid sequence based on length and/or percentage GC content;
b) Designing the competitor nucleic acid sequence to alter length and/or percentage GC content;
c) Increasing or decreasing the starting concentration of the competitor nucleic acid sequence; and/or
d) Increasing or decreasing the starting concentration of any of the nucleic acid primers.

Accordingly, in one embodiment the amplification rate of the competitor polynucleotide can be altered by increasing or decreasing the number of base pairs of the competitor polynucleotide product.

In some embodiments the amplification rate of the competitor polynucleotide is:

increased by decreasing the number of base pairs;

reduced by increasing the number of base pairs;

altered by increasing or decreasing the percentage GC content of the competitor polynucleotide;

is decreased by increasing percentage GC content; and/or

is reduced by increasing percentage GC content.

As examples, the sequences of pairs of target product and corresponding competitor product, tuned to provide various relative rates of amplification and exemplified in the Examples, are provided below.

The amplification rate can be defined as the "r" estimated from fitting the following equation to a fluorescent trace of standard quantitative PCR run on the polynucleotide with only the primers capable of hybridizing to it, in the absence of any other polynucleotides:

$$F(t) = \frac{K}{1 + \dfrac{K - F_0}{F_0} 2^{-rt}} \qquad (25)$$

This and other suitable equations are known in the art, see for example Spiess et al BMC Bioinformatics 9 article number 221 (2008); Rutledge NAR 2004 32: e178; and Liu et al Cell Culture and Tissue Engineering 2001 27: 1407-1414.

Where t is the cycle at which each fluorescence value was measured. A typical reaction would include commercially available qPCR master mix, 125 nM of each of the two primers, 250 nM of the respective probe, run for 60 cycles at 60°C. The curve fitting would typically be performed through a non-linear least-squares (NLLS) algorithm. Variations in this procedure, including substituting the probe with a fluorescent dye (e.g., Sybr Green, EvaGreen), altering the duration, temperature, or concentrations involved, or alternative statistical approaches such as Bayesian estimation are permissible as long as the same approach is used for all polynucleotides being evaluated. In a similar vein, different equations can be used to estimate "r", including but not limited to:

$$F(t) = \frac{K}{1 + \frac{K - F_0}{F_0} e^{-rt}} \tag{26}$$

$$F(t) = \frac{K}{\left(1 + \frac{K - F_0}{F_0} 2^{-rt}\right)^g} \tag{27}$$

$$\frac{dF}{dt} = rF\left(1 - \frac{F}{K}\right) \tag{28}$$

$$F(t) = f \cdot \left(1 + \frac{f}{K} m \cdot (t - \tau)\right) \tag{29}$$

where *f* is defined as *F* from any of the above equations

Since the competitor polynucleotide is tuned to have a different amplification rate to the target polynucleotide, in a situation wherein the amplification reaction comprises the same or substantially the same number of initial target and competitor template molecules, the number of target product polynucleotides generated is different to the number of tuned competitor product polynucleotides generated. Accordingly, in one embodiment of the method, the number of target product polynucleotides generated is different to the number of tuned competitor product polynucleotides generated, when the initial number of target polynucleotides and the number of tuned competitor polynucleotides prior to primer extension is the same or is substantially the same.

The premise of tuning the competitor polynucleotide so that the target and competitor have particular relative amplification rates is ultimately to mimic the predictive relationship, decision surface or differential gene regulation signature or presence/absence of particular mutations that underlies the purpose of the method, for example in diagnostics, prognostics, or simply taking a snapshot of the current state of a system or gene network. Accordingly, in one embodiment the sequence of the first target polynucleotide to be amplified, and the sequence of the at least first tuned competitor polynucleotide, is selected so as to result in a final detectable signal that varies with the initial concentration of the first target polynucleotide in such a way that approximates, reproduces or matches the predictive relationship or differential gene regulation signature of the target to one or more states.

In this way, if a particular sample has a low level of expression of a gene, but that low expression is, for instance, highly predictive of a disease state (or has a particular mutation but that mutation is more highly predictive of a disease state than a second mutation), the final detectable level of the target product may be high (the corresponding competitor polynucleotide is designed to have a sequence that is a poor competitor); whereas a gene that has a high level of expression but is poorly predictive of a disease may have a lower final detectable level of target product (i.e. the corresponding competitor polynucleotide is designed to have a sequence that is highly competitive, converting the high gene expression to a lower amount of target product), since the competitor sequences are chosen to apply the correct weighting to the amplification of each target.

This same premise applies to direct methods, whereby each target polynucleotide is amplified by two primers, which also amplify a corresponding tuned competitor polynucleotide (keeping in mind that in each reaction is it possible to have a number of different targets and different corresponding competitor polynucleotides being amplified, as described below); and also applies to indirect methods whereby for example the target is amplified by two primers, one of which is also used to amplify a first competitor along with a second competitor primer, which itself is used to amplify a second competitor polynucleotide, e.g.- target - competitor1 - competitor2 -, wherein each "-" is a primer. The skilled person is able to generate such amplification networks that effectively encode the predictive relationship or differential gene regulation signature, such that the output, i.e. the amount of product of target and competitor, is diagnostic, prognostic, or otherwise predicts the probability of state A versus state B.

Accordingly, in one embodiment, the rate of amplification of a first target polynucleotide and the rate of amplification of a second target polynucleotide approximates, reproduces or matches a pre-defined weighting. The skilled person will understand that the weighting is derived from whatever is necessary for the assay signal to approximate, reproduce or match the predictive signal, which will typically be identified via simulation.

Prior art methods that involve competitive amplification require that the competitor be as close as possible in sequence to the target sequence - since the methods are used to quantify the amount of starting target template, any difference in amplification rate would skew the results. It is clear from the disclosure herein that the competitor polynucleotides of the present invention are intentionally designed to have a different amplification rate to the target.

This can be achieved by having a different sequence to the target. In one embodiment, the sequence of the first tuned competitor polynucleotide to be amplified shares less than 95%, 90%, 88%, 85%, 80%, 75%, 70%, 65%, 60%, 55%, 50%, 45%, 40%, 35%, 30% sequence identity with the sequence of the first target polynucleotide to be amplified. It will be clear to the skilled person that the target sequence to be amplified is typically a subsequence within a larger polynucleotide, for example a 200 nucleotide region of a 500 nucleotide polynucleotide. The skilled person will understand that the requirement for a particular sequence identity, or amplification rate, applies only to this portion of the polynucleotide that is to be amplified, and the sequence of the flanking regions is largely irrelevant.

As described above, a different amplification rate can be achieved by altering the GC content of the sequence to be amplified. Accordingly, in one embodiment, the sequence of the first tuned competitor polynucleotide to be amplified (i.e. the sequence of the first tuned competitor product) comprises least 15% GC, or at least 25%, is at least 35%, is at least 55%, is at least 65%, is at least 75%, is at least 85%, or at least 85% GC.

In the same or different embodiments the difference in GC content of the first target polynucleotide portion to be amplified and the first competitor polynucleotide to be amplified is at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85% or at least 90% or 95%. For example, the first target polynucleotide portion to be amplified may comprise a sequence that is 20% GC, and the first competitor polynucleotide to be amplified may comprise a sequence that is 25% GC, resulting a difference in GC content of 5%.

Altering the length of the product to be generated, i.e. the distance between the sites of hybridisation of the two primers used in any given amplification, can also be used (alone or in combination with other methods described here such as altering the GC content) to tune the amplification rate. Accordingly, in the same or different embodiment, the first tuned competitor product is at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product.

In some embodiments the first tuned competitor product is at least 5 nucleotides shorter than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the first target product.

In some embodiments the first tuned competitor product is at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product.

The skilled person will appreciate that any combination of one or all of the above parameters, i.e. GC content, sequence identity and length of amplicon can be used to produce an appropriately tuned competitor polynucleotide.

Following amplification, it will be apparent to the skilled person that the amplification products are detected. In some instances it is sufficient to detect the presence or absence of a particular product. In other instances determination of the actual or relative abundance of a product is required. Various means are available to the skilled person to determine the presence or amount of an amplification product, including gel based electrophoresis assays, affinity-based capture of the amplification products for example on lateral flow strips, and fluorescence labelled probe based assays.

The present invention is particularly powerful when used to determine the relative abundance of at least two target polynucleotides. Accordingly in some embodiments the one or more target products, optionally one or more target PCR products; and the one or more tuned competitor products, optionally one or more competitor polynucleotide PCR products are detected.

In preferred embodiments, each target product and each corresponding competitor product is detected. In particularly preferred embodiments, the detection involves the use of fluorescently labelled probes wherein no matter how many targets and competitors are detected, the detection only uses two different fluorophores. Summing the fluorescence from each probe (i.e. just a single reading of fluorescence from both fluorophores) produces a single overall value, i.e. which of the fluorescence labels is higher. In turn, this corresponds to a diagnosis or prognosis.

Accordingly, in some embodiments the method comprises providing one or more probe groups, wherein each probe group comprises at least one probe polynucleotide labelled with a first label and at least one probe polynucleotide labelled with a second label, and wherein the first and the second label are different.

In some instances the at least one probe labelled with the first label is capable of hybridising to the first target product; and the at least one probe labelled with a second label is capable of hybridising to the first tuned competitor product. In some embodiments neither probe is capable of hybridising to the first target product.

In other instance the at least one probe labelled with the first label is capable of hybridising to the first tuned competitor product; and the at least one probe labelled with the second label is capable of hybridising to the second tuned competitor product. In some embodiments neither probe is capable of hybridising to the first target product.

The above reflects the fact that some genes may be predictive or diagnostic when the expression level is increased as compared to a control (e.g. non-diseased) sample; and that some genes may be predictive or diagnostic when the expression level is decreased as compared to a control sample. The skilled person will be able to ensure that the correct label is assigned to the correct probe so that combining the total fluorescence takes into account the direction of gene expression. A key feature of the present invention is that it is the *difference* between labels that provides the information; which label provides the "positive" signal and which provides a "negative" signal is decided by the skilled person.

A particular probe group represents a set of probes that are each labelled with one of only two different labels. It will be clear that as described above, the methods may be used to detect a number of different target products and competitor products. Accordingly, in some embodiments, within a single probe group there are at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the first label. In the same or other embodiments within a single probe group there are at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the second label. The direct method described above will typically require one probe with one label that can hybridise to the target product, and a corresponding probe labelled with the second label that can hybridise to the corresponding competitor product, i.e. a 1:1 ratio of probes (though the labels may be swapped as described above depending on the predictive relationship or differential gene regulation signature). The indirect method does not necessarily require this 1:1 ratio, since for example a single target product may be associated with two or more competitor products.

Accordingly, in some embodiments, within a single probe group there are:

at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the first label; and
at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the second label.

In some embodiments, appropriate probes are as follows:

| | |
|---|---|
| /56-FAM/AGCTGTGAG/Zen/ACGAAGGCTTCATGC/3IABkFQ/ | SEQ ID NO: 77 |
| /5HEX/TAGAGAGGT/ZEN/TACCAGAGCGTTGCC/3IABkFQ/ /56- | SEQ ID NO: 78 |
| FAM/AGTTTCTCA/Zen/AGCAGACCAGCCTTTCTC/3IABkFQ/ | SEQ ID NO: 79 |
| /56-HEX/CCAGAGTTC/Zen/CCAGACGATTCCCA/3IABkFQ/ | SEO ID NO: 80 |

As described above, the power in the methods comes at least from combining the detection of a number of different targets and competitors into two single readings (i.e. a reading of the first label and a reading of the second label, both of which can be done in one single reading), which themselves are combined into a single reading - how much first label versus how much second label.

However, if analysis of the expression of a larger number of genes is required, or the analysis of more complex networks, it is possible to use further probe groups, labelled with a third and fourth primer for instance (or, a 3rd probe group labelled with a fifth and sixth label etc). In this way, one set of genes may be analysed using a first probe group (reading the first and second label, followed by how much first label versus how much second label) and a second probe group (reading the third and fourth label, followed by how much third label versus how much fourth label). If necessary the overall reading of first:second:third:fourth label can be taken. This will all depend on the predictive relationship or differential gene regulation signature that is being employed.

Accordingly, in some embodiments the method comprises providing at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probe groups, wherein no particular label is used in more than one probe group.

In some embodiments the method comprises providing a number of labelled probe polynucleotides such that each target product has a corresponding labelled target probe polynucleotide and each tuned competitor product has a corresponding labelled competitor probe,
and wherein the labelled probes corresponding to the target product and the tuned competitor product are labelled with different labels.

In some embodiments the only labels present on the probes are the first label and the second label.

In some embodiments, each probe is labelled with a single type of label. For example, each probe is labelled only with HEX, or is only labelled with FAM, and is not labelled with both HEX and FAM. It will be clear to the skilled person however that each probe may be labelled with more than one molecule of the same label, for example may be labelled with 1, 2, 3, 4, 5 or more HEX molecules.

The probes may be labelled with any type of detectable label for example an enzyme based label that results in a colour change. Preferably, the label is a fluorophore. Accordingly, in some embodiments the first and second label are fluorophores. Examples of fluorophore labelled probes are "TaqMan" probes (that require degradation to release the fluorophore from proximity to a quencher), Hybeacons (which light up only when bound to the target), and Molecular Beacons (which physically distance two fluorophores when bound to an amplicon though the fluorophores remain tethered through the probe), and Scorpion probes.

It will be clear then from the above that reference to a fluorophore does not mean that a quencher may not also be present. For example in some embodiments the probes are labelled with a first and a second fluorophore. However, each probe may also be labelled with an appropriate quencher, as will be understood by the skilled person.

Alternatively, probes may be labelled in a manner intended for affinity-based separation (see for example Abingdon probes for Nucleic acid lateral flow immunoassays https://www.abingdonhealth.com/other-products/nucleic-acid-detection-pcrd/and the probes provided by Twistdx https://www.twistdx.co.uk/docs/default-source/Application-notes/app-note-001-pcrd-rpa-use-v1-7.pdf?sfvrsn=615403fc_46). As an example of one such embodiment, one probe is labelled with FAM and the other with the hapten digoxigenin (DIG). A primer for each the target and the competitor is labelled with biotin; thus amplification produces some amplicons labelled at one end with biotin and at the other with FAM, as well as other amplicons labelled at one end with biotin and at the other with DIG. The amplicons are mixed with a solution of streptavidin-coated gold nanoparticles, which binds to the biotin to form nanoparticle-amplicon complexes, then allowed to flow up a lateral flow strip. Anti-FAM and anti-DIG antibodies printed in separate lines on this strip act act as affinity purification agents, binding to the respective amplicons. This causes gold nanoparticles to be trapped at the printed lines, producing a dark red band visible to the naked eye. The relative intensity of these two bands provides the "signal" in the same manner as the relative intensity of two fluorophores described above.

The skilled person understands what is required of a probe that functions via hybridisation to a nucleic acid target. For example, the probe could have a sequence that is 100% identical to the relevant region of the target. However, the skilled person also understands that the sequences do not have to be 100% identical. Designing such hybridisation probes is entirely routine for the skilled person.

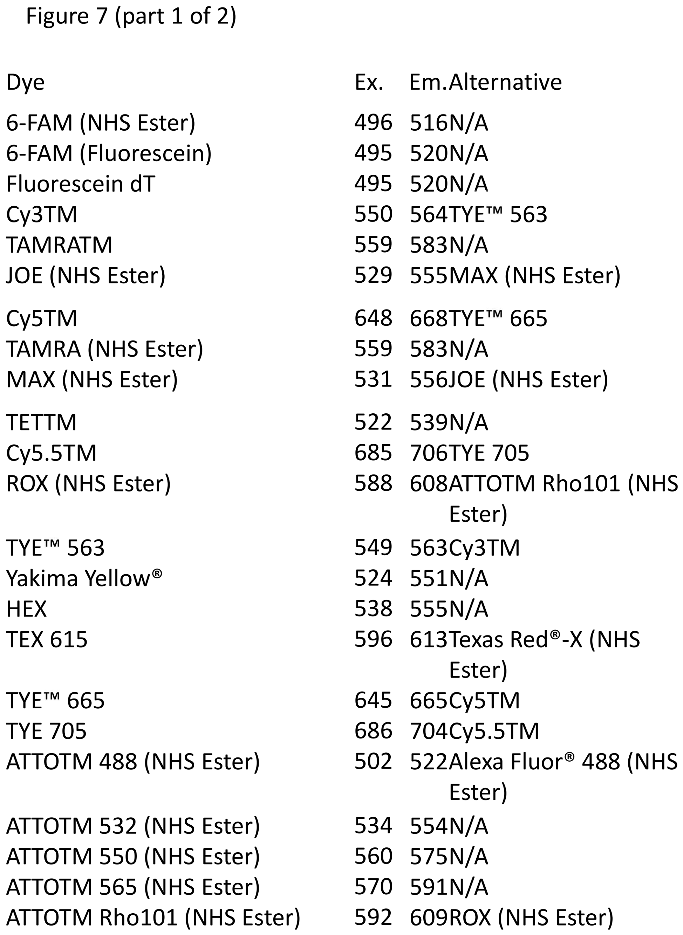

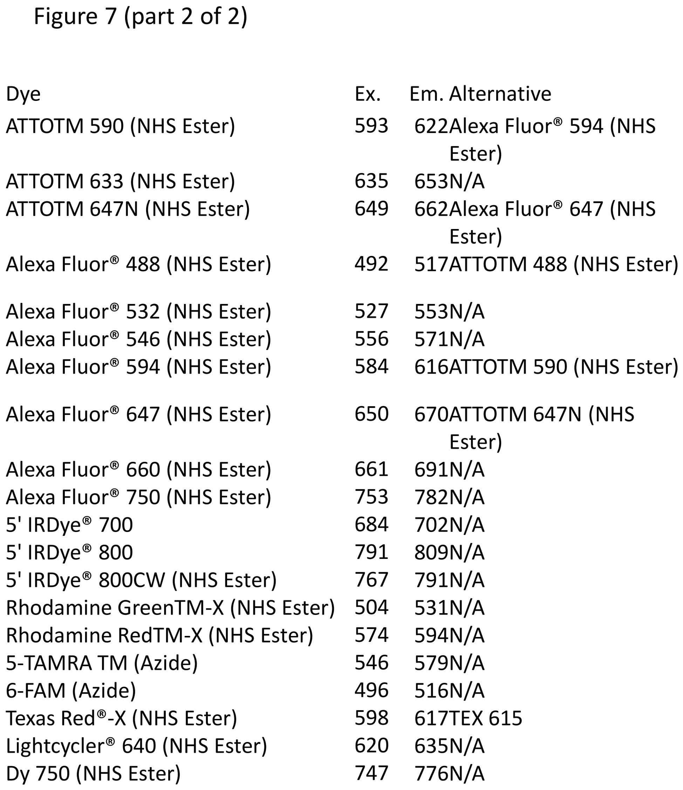

The skilled person will understand what is meant by a fluorophore and is capable of identifying appropriate fluorophores or fluorophore pairs. Preferably, the first and second fluorophore are chosen so that they have distinct emission spectra. Exemplary fluorophores are TAM, SUN, VIC, TET, JOE, the cyanine dyes (Cy3, Cy3.5, Cy5, Cy5.5), the Atto dyes, and the Alexa Fluors (see for example https://eu.idtdna.com/site/Catalog/modifications/dyes and https://www.trilinkbiotech.com/omi - Figure 7).

Particularly useful combinations are considered to be FAM and HEX; CY3 and CY5; and any combination of FAM, HEX, TET and Cy5.

A particularly useful pair of fluorophores are FAM and HEX.

Accordingly, in one embodiment, the first label is FAM and the second label is HEX. In another embodiment, the first label is HEX and the second label is FAM.

It is important that the probe that binds to the target product and the probe that binds to the corresponding competitor product are labelled with different labels, so the relative amounts of each product can be either determined, or incorporated into an overall determination of the amount of different target products and different competitor products.

Accordingly, in one embodiment, the at least one probe that is capable of hybridising to the first target product; and the at least one probe that is capable of hybridising to the first tuned competitor product are labelled with different labels.

In the same or different embodiment, the at least one probe that is capable of hybridising to the first tuned competitor product; and the at least one probe that is capable of hybridising to the second tuned competitor product are labelled with different labels.

In some embodiments where a group of genes are all predictive of the particular state (e.g. disease, prognosis) when the expression of the genes is increased relative to a control sample or control level, then it is appropriate that each probe that is capable of hybridising to the a target product is labelled with the same first label; and each probe that is capable of hybridising to a tuned competitor product are labelled with the same second label.

However, in some embodiments as described above, some genes are predictive of a particular state when the gene expression is repressed. Since many predictive relationships or differential gene regulation signatures and networks involve an increased expression of some genes and a concomitant repression of other genes, it is important that this can be reflected in the simple output from the method. Accordingly in some embodiments at least one of the probes that are capable of hybridising to a target product is labelled with a first label, and at least one of the probes that are capable of hybridising to a tuned competitor product are labelled with the same first label.

In some instances, within a given amplification reaction, there will be probes that are capable of hybridising to a target product that are labelled with a first label, probes that are capable of hybridising to a target product that are labelled with a second label, probes that are capable of hybridising to a competitor product that are labelled with a first label, and probes that are capable of hybridising to a competitor product that are labelled with a second label.

In some embodiments each probe that is capable of hybridising to a target polynucleotide product that is associated with a positive predictive relationship or differential gene regulation signature of a particular state is labelled with the first label, and the corresponding probe that is capable of hybridising to the tuned competitor polynucleotide product is labelled with the second label; and/or

wherein each probe that is capable of hybridising to a target polynucleotide product that is associated with a negative predictive relationship or differential gene regulation signature of the particular state is labelled with the second label, and the corresponding probe that is capable of hybridising to the tuned competitor polynucleotide product is labelled with the first label.

In some instances, wherein following amplification the actual amount of each product detected by the first probe and the amount of product detected by the second probe is determined.

In other embodiments, it is the relative amounts of each probe that are determined. For instance in some embodiments the relative amounts of each probe are compared to a standard curve to determine the relative probability of one or more states.

Generating an appropriate standard curve is routine for the skilled person and will require calibration, either by the individual user or the manufacturer, to relate a raw signal (or, in this case, the difference between signals) to a prediction/diagnosis.

An advantage of the present invention is that it allows the interrogation of a number of different expression patterns simultaneously, for example via multiplex PCR, and due to the use of only 2, or perhaps a small number for example 3, 4, 5, 6 different fluorophores, allows the abundance, or relative abundance, or each product to be condensed into a single reading, for example a single reading over multiple wavelengths (channels) to detect the amount of fluorescence from each probe label, or multiple readings performed in quick succession on the same sample.

It will be clear then that the methods of the invention translate the information provided by a given gene transcript or set of transcripts into the relative probability of a particular state.

The methods described herein capture the state of a portion of a gene expression network, optionally as a single value.

It will be clear to the skilled person that the target polynucleotide can be any nucleic acid from any source, provided that it is capable of being amplified. In one embodiment the target polynucleotide is RNA, optionally is an RNA transcript, optionally is an mRNA. In some embodiments the target polynucleotide is an miRNA, lncRNA or an siRNA.

The target polynucleotide may also be DNA. The DNA may be a modified form of DNA.

The sample may be any sample provided it comprises, or is expected to comprise, nucleic acid.

The methods of the present invention have both medical uses and biotechnological/bioproduct uses. The sample may be selected from the group comprising or consisting of: tissue, biopsy, blood, plasma, serum, pathogens, microbial cells, cell culture and cell lysate.

The sample may comprise any source of nucleic acid. In some examples the sample comprises any one or more of: cells, optionally white blood cells and/or red blood cells; exosomes; circulating tumour DNA (ctDNA); cell-free DNA (cfDNA); RNA; or pathogen nucleic acid.

The cells may be of any cell type. For example the cells may be mammalian cells, bacterial cells, yeast cells or plant cells. The mammalian cells may be human cells or are derived from human cells.

The cells may be cultured cells, optionally primary patient-derived cells or immortalized cell lines.

The cells may be mammalian stem cells.

In some embodiments, the cells are engineered cells, optionally engineered cells used in the bioproduction of metabolites and compounds.

The cells may be yeast cells, optionally wherein the yeast cells are used in brewing.

As is clear from the above, the method of the invention the is, in some preferred embodiments, for the amplification of at least a first and a second target polynucleotide.

In some embodiments, the method is for the amplification of at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 target polynucleotides.

As described above, the present methods also include what is termed a "redundant" model, whereby at least two or more portions of the same physical target polynucleotide molecule are amplified.

Accordingly, in some embodiments the first and the second target polynucleotides are target sequences within the same single polynucleotide.

In some particular embodiments, the method comprises amplification of a tuned competitor polynucleotide with at least one primer that is capable of hybridising to the first and to the second target polynucleotide and producing a first target product and a second target product.

In some embodiments the method comprises amplification of two tuned competitor polynucleotides, wherein the method comprises:

amplification of a first tuned competitor polynucleotide with at least one primer that is capable of hybridising to the first target polynucleotide; and

amplification of a second tuned competitor polynucleotide with at least one primer that is capable of hybridising to the second target polynucleotide.

It will be clear that following amplification, detection of the product, for example detection of the signal produced by the fluorophore labelled probes, is indicative of any one or more of:

i) the presence or absence;
ii) a particular pre-determined starting concentration;
iii) a starting concentration above or below a pre-determined level; and/or
iv) starting concentrations falling within a pre-determined range,

of one or more target polynucleotides.

In some embodiments, (i), (ii), (iii) and/or (iv) above is indicative of one or more of:

a) the relative expression of a specific gene;
b) the relative expression of two or more specific genes;
c) expression of one or more housekeeping genes
d) expression of a particular gene expression signature;
e) expression of a particular allelic variant of a gene or genes;
f) expression of a mutant version of a gene;
g) expression of cell-free tumour DNA,

wherein the target polynucleotide is selected from one or more portions of a known sequence of (a)-(g).

As mentioned herein, the methods of the present invention can be used to determine whether a particular sample more likely to be in a particular state A rather than a particular state B. The states are the states on which the predictive relationship or differential gene regulation signature is based. In some instances the states may be "particular disease" vs "no disease" or vs "other disease" or vs "not particular disease".

Any of the methods provided by the invention can be for the diagnosis and/or prognosis of a disease or condition in a subject.

Accordingly, the invention also provides a method for the diagnosis and/or prognosis of a disease or condition in a subject.

In some instances, to diagnose a disease or condition requires the assessment of the relative expression levels of at least two genes, optionally requires the assessment of the relative expression levels of at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 genes.

In some embodiments, the disease or condition is selected from: human tuberculosis, human tuberculosis with HIV co-infection, human tuberculosis without HIV co-infection, cancer, optionally prostate cancer, sepsis, bloodstream candidiasis, bovine tuberculosis, bovine mastitis. In particular embodiments the disease is tuberculosis.

In very particular embodiments, the disease is tuberculosis, and the differential gene regulation signature and/or predictive relationship or differential gene regulation signature is identified from the white blood cells of the subject.

In some embodiments, where the disease is tuberculosis, the degree of differential regulation of GBP6, ARG1 and TMCC1 contributes to an overall probability of having tuberculosis as compared to having some "other disease". The gene expression signature is upregulation of GBP6, and downregulation of ARG1 and TMCC1, compared to the levels of these genes in patients not having tuberculosis.

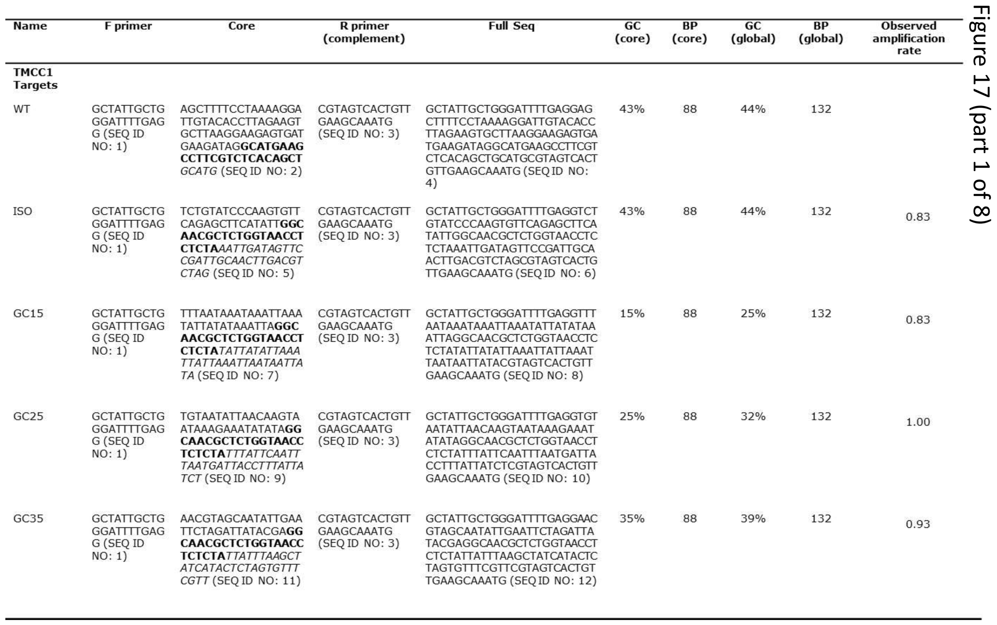

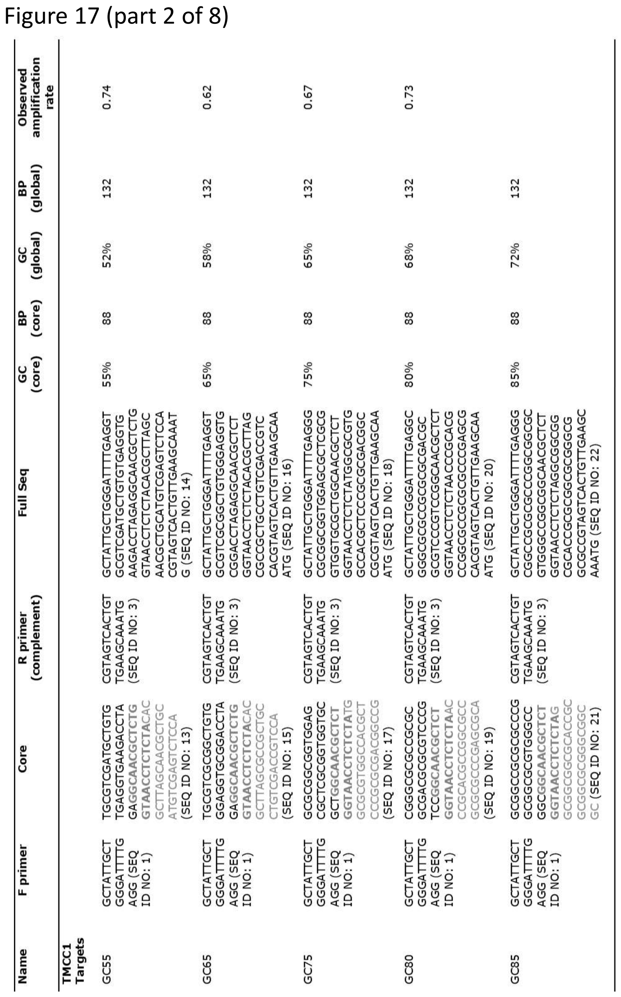

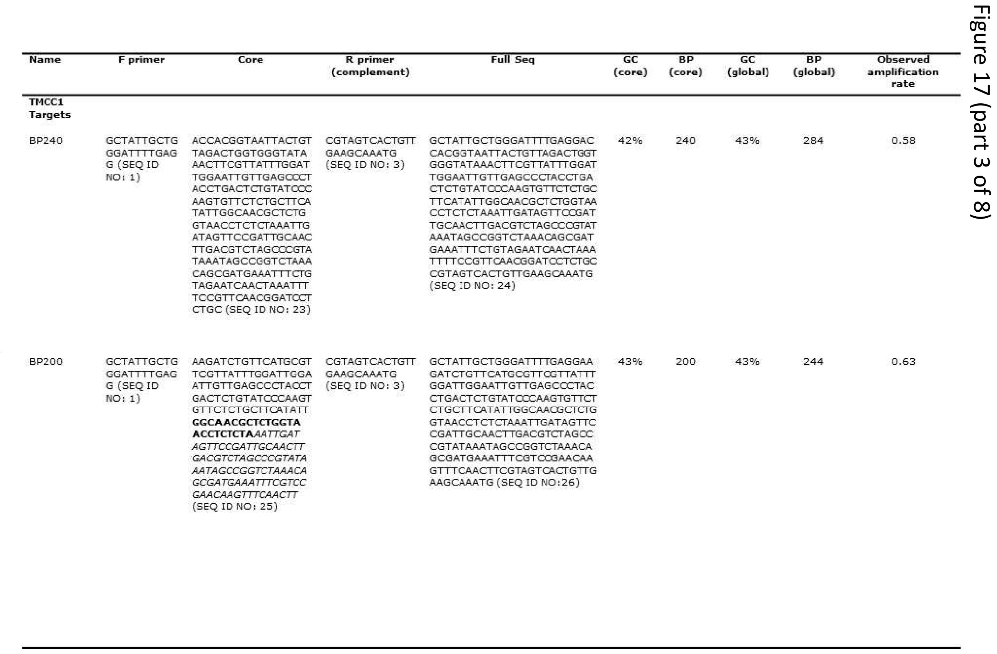

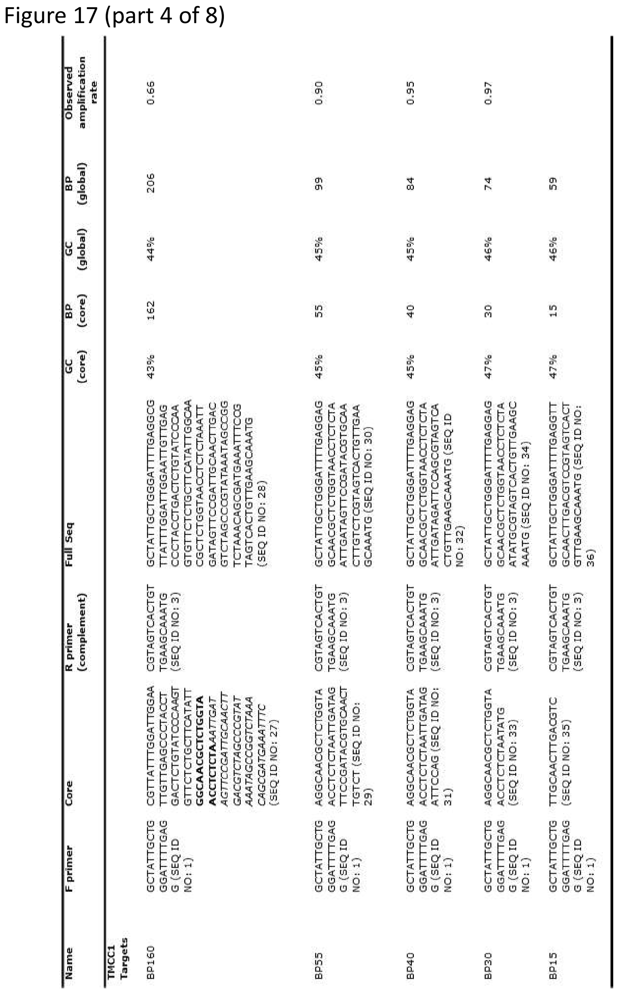

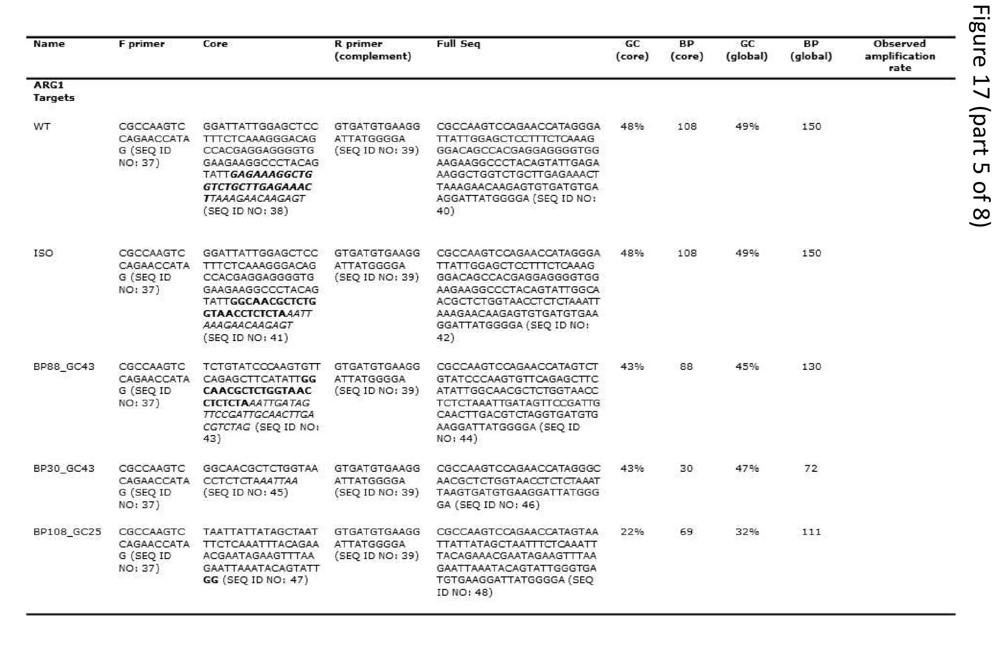

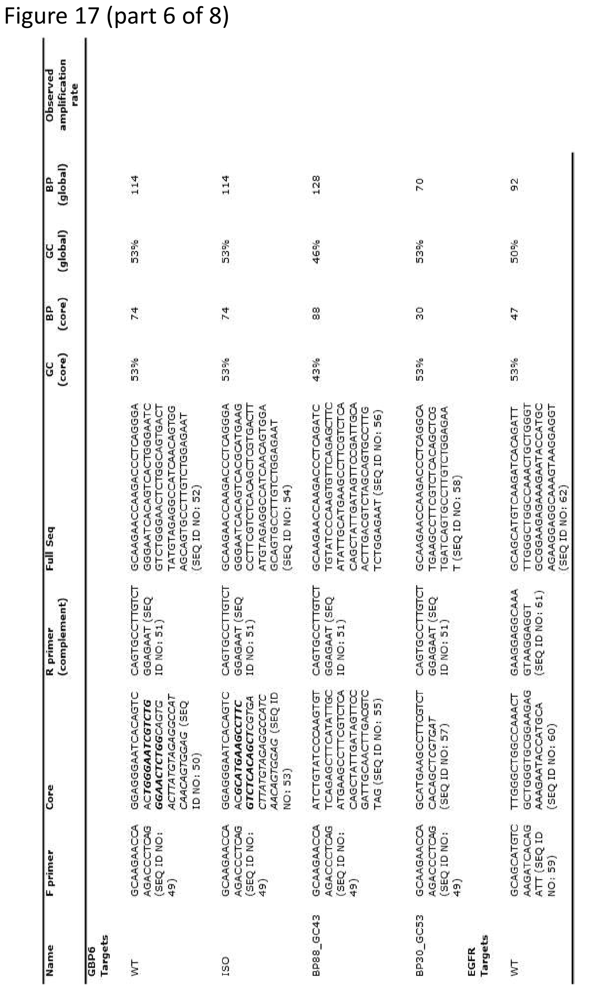

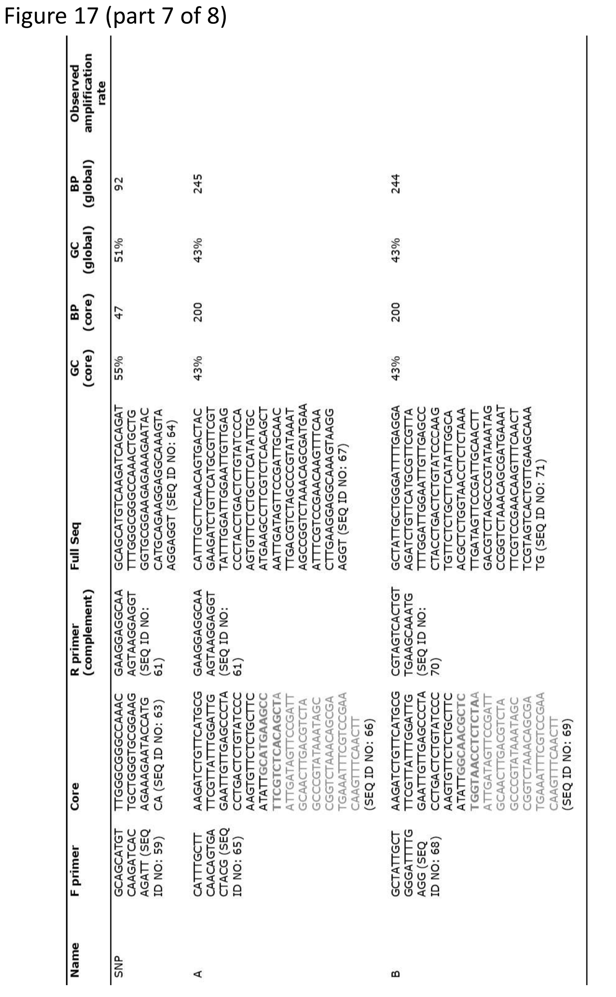

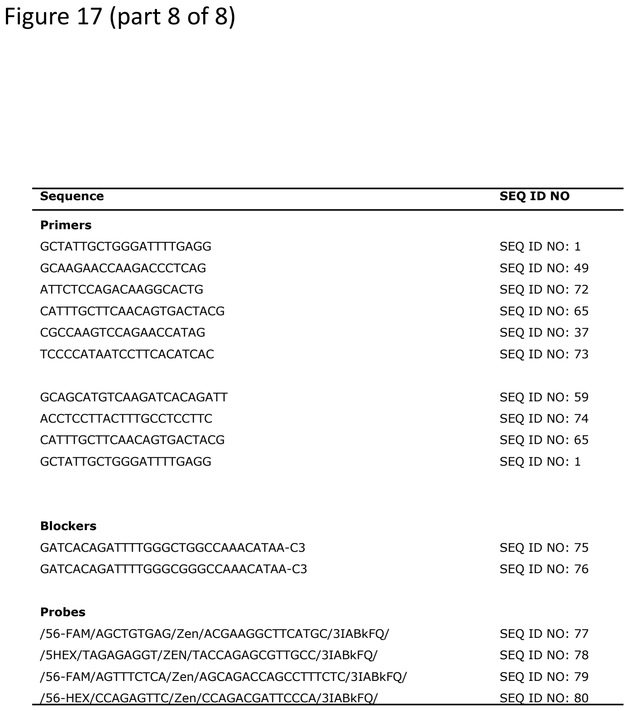

In the embodiments where the disease is tuberculosis and the degree of differential regulation of GBP6, ARG1 and TMCC1 contributes to an overall probability of having tuberculosis as compared to having some "other disease", examples of the primers and competitor sequences that can be used are shown in Figure 17.

In Figure 17, the WT sequence in each case is the target sequence. The F primer and R primer sequences are the sequences used to amplify the target and corresponding competitor sequences. The "Core" sequence is the sequence of the competitor between the two primer annealing sites, and the "Full seq" is the sequence of the full target or competitor oligonucleotide that is amplified by the two primers.

In one embodiment, where the target is TMCC1 and the target sequence is SEQ ID NO: 4, appropriate competitor sequences used to determine the most optimum competitor are considered to be SEQ ID NO: 6, 8, 10, 12, 14, 16, 18, 20, 22, 24, 26, 28, 30, 32, 34 and 36. Appropriate primers for amplification of the target and competitors are shown in SEQ ID NO: 1 and 3. Appropriate probes for detection of this target's contribution are shown in SEQ ID NO: 77 and 78.

In one embodiment, where the target is ARG1 and the target sequence is SEQ ID NO: 40, appropriate competitor sequences used to determine the most optimum competitor are considered to be SEQ ID NO: 42, 44, 46 and 48. Appropriate primers for amplification of the target and competitors are shown in SEQ ID NO: 37 and 39. Appropriate probes for detection of this target's contribution are shown in SEQ ID NO: 79 and 78.

In one embodiment, where the target is GBP6 and the target sequence is SEQ ID NO: 52, appropriate competitor sequences used to determine the most optimum competitor are considered to be SEQ ID NO: 54, 56, and 58. Appropriate primers for amplification of the target and competitors are shown in SEQ ID NO: 49 and 51. Appropriate probes for detection of this target's contribution are shown in SEQ ID NO: 80 and 77.

In other embodiments, the disease is cancer, for example is prostate cancer or breast cancer, optionally prostate cancer.

Where the disease is prostate cancer, the primers and probes that can be used are as follows:

In some embodiments, the disease is cancer, and the relative expression of a mutant version of a gene, particular allelic variant and/or cell-free tumour DNA is detected.

In any of the methods and embodiments described herein, the target polynucleotides may comprise SNPs, SNVs (single nucleotide variants) indels or copy-number variants (CNVs) associated with a disease state, optionally associated with the presence of a tumour and/or cancer, for example may comprise snps, snvs or indels in cell-free tumour DNA.

In some embodiments the target is EGFR, in particular a SNP in EGFR. In some embodiments the target sequence is SEQ ID NO: 62, and appropriate competitor sequences are SEQ ID NO: 64, 67 and 71. Appropriate primer sequences are SEQ ID NO: 68 and 70.

In some methods, a blocker oligonucleotide is used, wherein the blocker oligonucleotide cannot undergo extension of its 3' end, and wherein the blocker oligonucleotide is not complementary to the portion of the sequence in the at least one target polynucleotide containing the single-nucleotide polymorphism, optionally wherein the snp is a snv, but wherein the blocker oligonucleotide is complementary to the corresponding wild-type sequence and wherein the sequence in the target polynucleotide that comprises the sequence that is complementary to the blocker oligonucleotide overlaps with at least a portion of the sequence complementary to one of the primers.

In some instances, appropriate blocker sequences are SEQ ID NO: 75 and 76.

In some instance, the sample is obtained from a subject that is already suspected of having a particular disease or condition. In other instances, the method may be used as part of a routine screening programme, in which case the target polynucleotide may be derived from a sample obtained from a subject not suspected of having a particular disease or condition. The subject may be considered to be at risk of a particular disease or condition, for example due to age or lifestyle.

As mentioned here, in addition to medical uses, the present invention is useful in the field of bioengineering and industrial biotechnology. In some embodiments the detection of the relative expression of a specific gene or genes is indicative of the expression of specific natural and/or engineered genes in cells in culture and can for example allow the skilled person to determine whether a cell or system is behaving favourable or if culture parameters need to be optimised, for example.

As described above, any means of amplification is suitable for use with the present invention. However, preferred methods of amplification include the polymerase chain reaction (PCR) or the recombinase polymerase reaction (RPA).

As can be seen above, the invention provides numerous methods for the amplification of one or more target polynucleotides. As indicated at the outset, the invention provides:

a method of translating the relative abundance of at least two oligonucleotides, for example the relative expression of at least two genes, or presence or absence of at least two mutations, in a sample into the relative probability of a particular state;

a method of detecting the relative abundance of at least three oligonucleotides, for example the relative expression of at least three genes, or presence or absence of at least three mutations, in a sample using only two fluorophore labelled probes;

a method of combining the relative abundance of at least two oligonucleotides, for example the relative expression of at least two genes, or presence or absence of at least two mutations, in a sample into a single value;

a method of converting the predictive relationship, decision surface or differential target oligonucleotide pattern such as a differential gene regulation signature provided by the relative abundance of at least two oligonucleotides or the presence or absence of at least two mutations, in a sample into a single value;

a method of mimicking statistical information with a competitive amplification network; ; and

a method of reducing complex gene expression patterns to a single value;

wherein the method comprises the step of amplifying one or more target polynucleotides in a sample. The step of amplifying one or more target polynucleotides can be performed according to any of the methods of amplification described herein.

The invention further provides a method of diagnosis or prognosis of a disease or condition in a subject wherein the method comprises any of the methods of amplification of the invention. In some embodiments the subject is diagnosed as having a disease or condition or prognosis of a disease or condition when the relative amounts of the first label and the second label indicate prognosis of disease or condition.

As described above, the disease or condition may be selected from: human tuberculosis, human tuberculosis with HIV co-infection, human tuberculosis without HIV co-infection, cancer optionally prostate or breast cancer, sepsis, bloodstream candidiasis, bovine tuberculosis, bovine mastitis. Preferences for the disease or condition are as described elsewhere herein.

The invention also provides various compositions and kits that can be used to put the methods of the invention into practice. For example, the invention provides a composition comprising one or more of:

a) At least one target polynucleotide as described herein;
b) At least one tuned competitor polynucleotide as described herein;
c) At least one primer, preferably at least two primers, as defined herein;
d) At least one or more probe groups as defined herein, wherein each probe group comprises at least one probe polynucleotide labelled with a first label and at least one probe polynucleotide labelled with a second label.

The skilled person will appreciate that a composition for nucleic acid amplification may comprise one or more standard amplification components, such as a polymerase enzyme; appropriate amounts of each of four nucleotides A, C, T and G; a recombinase enzyme; a single stranded binding protein; and/or appropriate amounts of each of the nucleotides A, C, T, G and U.

The invention also provides a tuned competitor polynucleotide as defined herein. Preferences for features of the tuned competitor polynucleotide are described elsewhere herein.

The invention also provides a kit for carrying out any of the methods of the invention, for example wherein the kit comprises one or more of:

a) One or more tuned competitor polynucleotides as described herein;
b) One or more primers as described herein;
c) A first probe polynucleotide labelled with a first label as described herein and a second probe polynucleotide labelled with a second label as described herein;
d) Suitable buffers;
e) Instructions for use.

In particular embodiments the kit comprises;

a) One or more tuned competitor polynucleotides as described herein;
b) One or more primers as described herein;
c) A first probe polynucleotide labelled with a first label as described herein and a second probe polynucleotide labelled with a second label as described herein.

The invention also provides a composition comprising any one or more of:

a) One or more tuned competitor polynucleotides as described herein;
b) One or more primers as described herein;
c) A first probe polynucleotide labelled with a first label as described herein and a second probe polynucleotide labelled with a second label as described herein.

In one embodiment the composition comprises:

a) One or more tuned competitor polynucleotides as described herein; and
b) One or more primers as described herein.

In one embodiment the composition comprises:

a) One or more tuned competitor polynucleotides as described herein; and
c) A first probe polynucleotide labelled with a first label as described herein and a second probe polynucleotide labelled with a second label as described herein.

In one embodiment the composition comprises:

b) One or more primers as described herein; and
c) A first probe polynucleotide labelled with a first label as described herein and a second probe polynucleotide labelled with a second label as described herein.

In one embodiment the composition comprises:

a) One or more tuned competitor polynucleotides as described herein;
b) One or more primers as described herein; and
c) A first probe polynucleotide labelled with a first label as described herein and a second probe polynucleotide labelled with a second label as described herein.

In one embodiment, the kit or composition comprises any one more of the sequences shown in Figure 17.
In one embodiment, the kit or composition is for amplifying a portion of TMCC1 mRNA and comprises any one more of the competitor sequences of SEQ ID NO: 6, 8, 10, 12, 14, 16, 18, 20, 22, 24, 26, 28, 30, 32, 34 and 36. In some embodiments the kit or composition also comprises appropriate primers for amplification of the target and competitors, such as those of SEQ ID NO: 1 and 3.
In the same or different embodiment, the kit or composition is for, or is also for, amplifying a portion of ARG1 mRNA and comprises any one more of the competitor sequences of SEQ ID NO: 42, 44, 46 and 48. In some embodiments the kit or composition also comprises appropriate primers for amplification of the target and competitors, such as those of SEQ ID NO: 39 and 39.
In the same or different embodiment, the kit or composition is for, or is also for, amplifying a portion of GBP6 mRNA and comprises any one more of the competitor sequences of SEQ ID NO: 54, 56, and 58. In some embodiments the kit or composition also comprises appropriate primers for amplification of the target and competitors, such as those of SEQ ID NO: 49 and 51.
In other embodiments, the kit or composition is for amplifying a portion of EGFR genomic DNA, for example genomic DNA that is in a sample of ctDNA, for example in order to distinguish between the wild-type allele and a particular mutation, such as the L858R SNP, and comprises any one more of the competitor sequences of SEQ ID NO: 64, 67 and 71. In some embodiments the kit or composition also comprises appropriate primers for amplification of the target and competitors, such as those of SEQ ID NO: 68 and 70.
The invention also provides a collection or kit that comprises at least two tuned competitor polynucleotides as described herein, wherein the collection comprises at least 2, 3, 4, 5, 6, 7, 8, 9, 10, 11, 12, 13, 14, 15, 16, 17, 18, 19, 20, 22, 24, 25, 26, 28, 30, 32, 34, 35, 36, 38, 40, 45, 50, 55, 60, 65, 70, 75, 80, 85, 90, 95, 100, 110, 120, 130, 140, 150, 160, 170, 180, 190, or at least 200 tuned competitor polynucleotides.
The invention also provides a collection or kit that comprises at least two tuned competitor polynucleotides and at least two corresponding labelled probes.
The invention also provides a collection or kit that comprises:

at least two tuned competitor polynucleotides;
at last two corresponding labelled probes; and
at least two primers.

Further, the invention provides a collection or kit that comprises:

at least two tuned competitor polynucleotides as defined by any of the preceding claims;
at last two corresponding labelled probes as defined by any of the preceding claims; and
at least two primers as defined by any of the preceding claims.

The invention also provides a method of tuning a first competitor polynucleotide that competes for hybridisation with at least a first primer with a first target polynucleotide and which results in amplification of a first target product and a first tuned competitor product, and wherein:

a) a different proportion of target polynucleotides are amplified compared to the proportion of tuned competitor polynucleotides that are amplified;
b) amplification of the first target polynucleotide approximates, reproduces or matches the predictive relationship or differential gene regulation signature of the target polynucleotide to a particular state; and/or
c) the rate of amplification of the first target polynucleotide and optionally the rate of amplification of a second target polynucleotide approximates, reproduces or matches a pre-defined weighting,

the method comprising

optimising the sequence of the tuned competitor polynucleotide and/or length of tuned competitor amplification product with respect to the sequence of the first target product and/or length of the first target product. Detailed discussion as to how the skilled person tunes a competitor polynucleotide accordingly to the particular situation is given above, and also see for example the section "Mimicking logistic regression" below.

The method of tuning a competitor polynucleotide of the invention may also comprise:

a second primer is used in said amplification that is capable of hybridising to the first target polynucleotide so that the first target product is produced by primer extension from two primers, optionally produced by PCR;
a third primer is used in said amplification that is capable of hybridising to the first tuned competitor polynucleotide so that the first tuned competitor product is produced by primer extension from two primers, optionally produced by PCR;
optionally wherein the second and the third primer have the same sequence.

In some instances said optimising comprises producing two or more test tuned competitor polynucleotides that following amplification result in:

a) a different proportion of target polynucleotides are amplified compared to the proportion of tuned competitor polynucleotides that are amplified;
b) amplification of the first target polynucleotide approximates, reproduces or matches the predictive relationship or differential gene regulation signature of the target to a particular state; and/or
c) the rate of amplification of the first target polynucleotide and optionally the rate of amplification of a second target polynucleotide approximates, reproduces or matches a pre-defined weighting,

and selecting the tuned competitor that results in the most preferred amplification of the first target polynucleotide.
In some instances, said optimising comprises producing at least 3, 4, 5, 6, 7, 8, 9, 10, 11, 12, 13, 14, 15, 16, 17, 18, 19, 20 different test tuned competitor polynucleotides.
In some embodiments said optimising comprises performing at least 3, 4, 5, 6, 7, 8, 9, 10, 11, 12, 13, 14, 15, 16, 17, 18, 19, 20 test amplification reactions with each test tuned competitor polynucleotide,
optionally wherein at least 3, 4, 5, 6, 7, 8, 9, 10, 11, 12, 13, 14, 15, 16, 17, 18, 19, 20 amplification reactions are performed using at least 3, 4, 5, 6, 7, 8, 9, 10, 11, 12, 13, 14, 15, 16, 17, 18, 19, 20 different concentrations of target polynucleotide and/or number of target polynucleotide molecules.
In a preferred embodiment, at least two replicates of five amplification reactions are performed, wherein each of the five amplification reactions employs a different tuned competitor polynucleotide.
In some instances, each test amplification using a particular test tuned competitor polynucleotide is performed using a different concentration and/or number of target polynucleotide templates.
In some embodiments the test amplification reactions are performed with a range of concentrations and/or number of target polynucleotide templates that span 100 copies/ $\mu$L to $10^8$ copies/$\mu$L.
As described herein, in some instances the test tuned competitor polynucleotides are designed to have different GC contents.
Also provided by the present invention is a method of optimising a competitive amplification reaction according to any of the preceding claims, wherein said optimising comprises:

a) Increasing or decreasing the starting concentration of the synthetic nucleic acid sequence; and/or
b) Increasing or decreasing the starting concentration of any of the nucleic acid primers.

The invention also provides a method of multiplexed competitive amplification of at least two target polynucleotides wherein the method comprises at least one competitive polynucleotide and wherein the target amplification products are detected using probes labelled with the same label, optionally labelled with the same fluorophore, optionally wherein the competitive polynucleotide is a tuned competitive polynucleotide according to any of the preceding claims.
The invention also provides a method of determining the transcriptional state of a system wherein the method comprises competitive amplification according to any method of the invention.
The invention also provides a method of determining whether a system is in state A or in state B wherein the method comprises competitive amplification according to any method of the invention.

The method also provides a method of simultaneous competitive amplification of at least two target polynucleotides in a sample wherein the method comprises providing

a) a sample comprising polynucleotides;

b) a first and a second tuned competitor polynucleotide;

c) a first primer set, wherein the primer set comprises two primers capable of hybridising on opposite strands of a first target polynucleotide and the first competitive polynucleotide, so as to allow production of a first target amplification product and a first competitive amplification product;

d) a second primer set, wherein the primer set comprises two primers capable of hybridising on opposite strands of a second target polynucleotide and the second competitive polynucleotide, so as to allow production of a second target product and a second competitive product;

e) a first probe group, wherein the first probe group comprises a first labelled target probe capable of hybridising to the first target amplification product and a first labelled competitor probe capable of hybridising to the first competitive amplification product;

d) a second probe group, wherein the second probe group comprises a second labelled target probe capable of hybridising to the second target amplification product and a second labelled competitor probe capable of hybridising to the second competitive amplification product;

and wherein:

i) the first labelled target probe and the second target labelled probe are labelled with the same first label; and wherein the first labelled competitor probe and the second labelled competitor probe are labelled with the same second label; or

ii) the first labelled target probe and the second labelled competitor probe are labelled with the same first label; and wherein the first labelled competitor probe and the second labelled target probe are labelled with the same second label

and allowing the first and second primer sets to hybridise to the target and competitive polynucleotides.

In some embodiments of the method of simultaneous competitive amplification of at least two target polynucleotides the method comprises providing

e) a further 1, 2, 3, 4, 5, 6, 7, 8, 9, 10 primer sets and corresponding probe groups.

In some embodiments of the method of simultaneous competitive amplification of at least two target polynucleotides one of the labelled target probes is labelled with the second label and the corresponding labelled competitor probe is labelled with the first label.

In some embodiments of the method of simultaneous competitive amplification of at least two target polynucleotides the method further comprises simultaneously detecting the amount of the first label and the second label following multiplexed amplification.

The listing or discussion of an apparently prior-published document in this specification should not necessarily be taken as an acknowledgement that the document is part of the state of the art or is common general knowledge.

Preferences and options for a given aspect, feature or parameter of the invention should, unless the context indicates otherwise, be regarded as having been disclosed in combination with any and all preferences and options for all other aspects. For example, exemplary combinations of features provided by the invention include:

1) a method of combining the relative expression of at least two genes in a sample into a single value, wherein the method comprises the step of amplifying six target polynucleotides and six tuned competitor polynucleotides, wherein the six target amplification products are each probed with a different hybridisation probe labelled with HEX, and each of the six tuned competitor amplification products are probed each probed with a different hybridisation probe labelled with FAM;

2) a method of diagnosing cancer, wherein the method comprises a step of amplifying one or more target polynucleotides in a sample, wherein the method comprises:

providing:

a) a sample comprising polynucleotides

b) a first tuned competitor polynucleotide

c) at least a first primer wherein at least the first primer is capable of hybridising to:

a first target polynucleotide in the sample; and

the first tuned competitor polynucleotide; and

initiating a primer extension reaction such that the target polynucleotide (if present in the sample) and the first tuned competitor polynucleotide are amplified,

wherein amplification results in a first target product and a first tuned competitor product.

A summary of the overall approach that may be taken by the skilled person to put the invention into practice for specific applications is as follows:

a) The practitioner begins by performing regression, e.g. logistic regression, on patient data to determine both which gene transcripts to target as well as the appropriate relationship between expression level and diagnostic probability for each transcript. The skilled person may obtain pre-existing data on which the logistic regression may be performed.

b) Next, the practitioner selects a CAN architecture, i.e., the number of competitor sequences and the arrangement of shared primers, for each target transcript. Exemplary methods of selecting the CAN architecture are described elsewhere herein.

c) The practitioner then computationally determines the ideal components of each CAN module that will optimally recapitulate the patient data regression results, for example the concentration of each oligonucleotide and the desired amplification behavior. Using previously-acquired data, the practitioner proposes design parameters (length and GC content) for each competitor oligonucleotide, choosing those most likely to result in the desired amplification behavior. These parametric designs can then be used to produce sequence designs, which are obtained, experimentally tested via standard PCR amplification, and analyzed to describe their behavior. These new observations are combined with prior observations in a multitask regression framework, wherein a statistical model learns the empirical relationship between design parameters and each amplification parameter jointly.

d) If further optimization is necessary, this statistical model can be used to propose new sequence designs which, in light of the newly-acquired data, are now the most likely to produce the desired amplification behavior. This process continues until suitable competitor sequences are found that allow recapitulation of the logistic regression results via the CAN reaction.

A summary of an exemplary method of tuning a competitor polynucleotide is as follows:
Simulations were carried out to identify ideal parameters values describing optimal behaviour. Designing a competitor sequence which displays behaviour reflected by one or more of these parameter values is the goal of tuning. First, numerous amplicon sequences are designed and obtained with identical primer sequences and variable "core" sequences between the primers. These sequences are tested experimentally, and their behaviour analysed to derive values for the descriptive parameters. Assuming none of these sequences displayed ideal amplification behaviour, the data is used to rationally design a new sequence with the best chance of matching the target behaviour. To this end, performed regression is performed to determine how various sequence design parameters predicted the parameters of interest describing amplification behaviour. Specifically, a Gaussian Process regressor can be trained to relate the length and GC-content of the "core" sequence to the "amplification rate" parameter. This, or any other such regressor, could then be used to predict the behaviour of a given designed amplicon as well as provide the sequence descriptors (length and GC content) most likely to achieve the desired objective. This process of simulation, design, experimentation, analysis, and regression is iterated for every sequence in the Competitive Amplification Network until a suitable sequence is found. Modifications of this approach include incorporating information on the primer sequences themselves within the regression. This allows determination of both a global relationship between design parameters and amplification parameters as well as the idiosyncrasies of that relationship specific to a given pair of primers.
The invention is further described in the following numbered embodiment paragraphs:

1. A method of translating the relative abundance of at least two target oligonucleotides in a sample into the relative probability of a particular state.
2. A method of detecting the relative abundance of at least three target oligonucleotides in a sample using only two fluorophore labelled probes.
3. A method of combining the relative abundance of at least two target oligonucleotides in a sample into a single value.
4. A method of converting the predictive relationship or decision surface provided by the relative abundance of at least two oligonucleotides in a sample into a single value.
5. A method of mimicking statistical information with a competitive amplification network.

6. The method according to any of embodiments 1-5 wherein the method comprises the step of amplifying one of more target polynucleotides in a sample, optionally wherein the step of amplifying is the step of amplifying according to any one of embodiments 7-82.

7. A method of amplifying one or more target polynucleotides in a sample, wherein the method comprises: providing:

> a) a sample potentially comprising one or more target polynucleotides
> b) a first tuned competitor polynucleotide
> c) at least a first primer wherein at least the first primer is capable of hybridising to:

>> a first target polynucleotide in the sample; and
>> the first tuned competitor polynucleotide; and

> initiating a primer extension reaction such that the target polynucleotide (if present in the sample) and the first tuned competitor polynucleotide are amplified,
> wherein amplification results in a first target product and a first tuned competitor product.

8. The method according to embodiment 7 wherein the method comprises providing:

> a second primer;
> a second competitor polynucleotide; and/or
> a second target polynucleotide.

9. The method according to embodiment 8 wherein the second primer is capable of hybridising to the first target polynucleotide, wherein the first and second primer hybridise on opposite strands of the target so as to result in the production of the first target product, optionally a first target polymerase chain reaction (PCR) product.

10. The method according to any one of embodiments 7 or 8 wherein the second primer is also capable of hybridising to the first tuned competitor polynucleotide, wherein the first and second primer hybridise on opposite strands of the first tuned competitor polynucleotide so as to result in the production of the first tuned competitor product, optionally first tuned competitor PCR product.

11. The method according to any one of embodiments 7-10 wherein the method comprises providing a second tuned competitor polynucleotide.

12. The method according to any one of embodiments 8 or 11 wherein the second primer is:

> a) capable of hybridising to the first tuned competitor polynucleotide, wherein the first and second primer hybridise on opposite strands of the first tuned competitor polynucleotide so as to result in the production of the first tuned competitor product, optionally first tuned competitor PCR product; and
> b) is capable of hybridising to the second tuned competitor polynucleotide and initiating a primer extension reaction such that the second tuned competitor polynucleotide is amplified so as to result in the production of the second tuned competitor product, optionally in combination with a further primer wherein the second and further primer hybridise on opposite strands of the second tuned competitor polynucleotide so as to result in the production of the second tuned competitor product, optionally a first target polymerase chain reaction (PCR) product,

optionally wherein the second primer is not capable of hybridising to the first target polynucleotide.

13. The method according to any one of embodiments 7-12 wherein the second target polynucleotide is part of the same polynucleotide molecule as the first target polynucleotide.

14. The method according to 7-12 wherein the second target polynucleotide is on a different polynucleotide molecule to the first target polynucleotide.

16. The method according to any one of embodiments 7-14 wherein the second primer is:

> a) capable of hybridising to the first target polynucleotide, wherein the first and second primer hybridise on opposite strands of the target so as to result in the production of the first target product, optionally a first target polymerase chain reaction (PCR) product; and
> b) is not capable of hybridising to the first or second tuned competitor polynucleotide

and wherein the method comprises a third primer capable of hybridising to the first and to the second tuned competitor polynucleotide.

17. The method according to any one of embodiments 7-16 wherein the method comprises providing a fourth primer, wherein the fourth primer is capable of hybridising to the first target polynucleotide, wherein the first and fourth primer hybridise on opposite strands of the target so as to permit formation of the first target product, optionally a first target PCR product.

18. The method according to any one of embodiments 7-175 wherein the method comprises providing:

a) a second primer capable of

i) hybridising to the first target polynucleotide, wherein the first and second primer hybridise on opposite strands of the target so as to result in the production of the first target product, optionally a first target polymerase chain reaction (PCR) product; and
ii) capable of hybridising to the first tuned competitor polynucleotide; and

b) a third primer capable of

i) hybridising to the first tuned competitor polynucleotide wherein the third and second primer hybridise on opposite strands of the first tuned competitor polynucleotide so as to result in the production of the first tuned competitor polynucleotide product, optionally a first tuned competitor polynucleotide polymerase chain reaction (PCR) product; and
ii) capable of hybridising to the second tuned competitor polynucleotide;
and

c) a fourth primer capable of hybridising to the second tuned competitor polynucleotide wherein the third and fourth primer hybridise on opposite strands of the second tuned competitor polynucleotide so as to result in the production of the second tuned competitor polynucleotide product, optionally a second tuned competitor polynucleotide polymerase chain reaction (PCR) product.

19. The method of any one of embodiments 7-18, wherein the amplification rate of the first target polynucleotide is different to the amplification rate of the first tuned competitor polynucleotide.

20. The method according to any one of embodiments 7 or 19 wherein the number of target product polynucleotides generated is different to the number of tuned competitor product polynucleotides generated, when the initial number of target polynucleotides and the number of tuned competitor polynucleotides prior to primer extension is the same or is substantially the same.

21. The method according to any one of embodiments 7-20 wherein the sequence of the first target polynucleotide to be amplified, and the sequence of the at least first tuned competitor polynucleotide, is selected so as to result in a final detectable signal that varies with the initial concentration of the first target polynucleotide in such a way that approximates or reproduces or matches the predictive relationship of the target to one or more states.

22. The method according to any one of embodiments 7-21 wherein the rate of amplification of a first target polynucleotide and the rate of amplification of a second target polynucleotide matches a pre-defined weighting.

23. The method according to any of one of embodiments 7-22 wherein the sequence of the first tuned competitor polynucleotide to be amplified shares less than 95%, 90%, 88%, 85%, 80%, 75%, 70%, 65%, 60%, 55%, 50%, 45%, 40%, 35%, 30% sequence identity with the sequence of the first target polynucleotide to be amplified.

24. The method according to any one of embodiments 7-23 wherein the sequence of the first tuned competitor polynucleotide to be amplified comprises at least 15% GC, or at least 25%, is at least 35%, is at least 55%, is at least 65%, is at least 75%, is at least 85%, or at least 85% GC.

25. The method according to any one of embodiments 7-24 wherein the first tuned competitor product is at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product.

26. The method according to any one of embodiments 7-25 wherein the first tuned competitor product is:

at least 5 nucleotides shorter than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the first target product; or
at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product.

27. The method according to any one of embodiments 7-26 wherein the one or more target products, optionally one or more target PCR products; and the one or more tuned competitor products, optionally one or more competitor polynucleotide PCR products are detected.

28. The method according to any one of embodiments 7-27 wherein the method comprises providing one or more probe groups, wherein each probe group comprises at least one probe polynucleotide labelled with a first label and at least one probe polynucleotide labelled with a second label,

and wherein the first and the second label are different.

29. The method according to embodiment 28 wherein the at least one probe labelled with the first label is capable of hybridising to the first target product; and the at least one probe labelled with a second label is capable of hybridising to the first tuned competitor product.

30. The method according to any of embodiments 28 or 29 wherein the at least one probe labelled with the first label is capable of hybridising to the first tuned competitor product; and

the at least one probe labelled with the second label is capable of hybridising to the second tuned competitor product; and

optionally wherein neither probe is capable of hybridising to the first target product.

31. The method according to any of embodiments 28-30 wherein within a single probe group there are at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the first label.

32. The method according to any of embodiments 28-31 wherein within a single probe group there are at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the second label.

33. The method according to any of embodiments 28-32 wherein within a single probe group there are:

at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the first label; and

at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the second label.

34. The method according to any one of embodiments 28-33 wherein the method comprises providing at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probe groups,

optionally wherein no particular label, optionally a fluorophore, is used in more than one probe group.

35. The method according to any one of embodiments 28-34 wherein the only labels present on the probes are the first label and the second label.

36. The method according to any one of embodiments 28-35 wherein each probe is labelled with a single type of label.

37. The method according to any one of embodiments 28-36 wherein the first and second label are fluorophores, optionally wherein each probe comprises a quencher.

38. The method according to any one of embodiments 28-37 wherein the first label is FAM and the second label is HEX; or wherein the first label is HEX and the second label is FAM.

39. The method according to any one of embodiments 28-38 wherein

i) the at least one probe that is capable of hybridising to the first target product; and the at least one probe that is capable of hybridising to the first tuned competitor product are labelled with different labels; and/or

ii) the at least one probe that is capable of hybridising to the first tuned competitor product; and the at least one probe that is capable of hybridising to the second tuned competitor product are labelled with different labels.

40. The method according to any of embodiments 28-39 wherein each probe that is capable of hybridising to the target product is labelled with the same first label; and each probe that is capable of hybridising to a tuned competitor product are labelled with the same second label.

41. The method according to any of embodiments 28-40 wherein

at least one of the probes that are capable of hybridising to a target product is labelled with a first label, and at least one of the probes that are capable of hybridising to a tuned competitor product are labelled with the same first label; or

at least one probe that is capable of hybridising to a target product is labelled with a first label, at least one probe that is capable of hybridising to a target product that is labelled with a second label, at least one probe that is capable of hybridising to a competitor product is labelled with a first label, and at least on probe that is capable of hybridising to a competitor product is labelled with a second label.

42. The method according to any of embodiments 28-41 wherein each probe that is capable of hybridising to a target polynucleotide product that is associated with a positive predictive relationship of a particular state is labelled with the first label, and the corresponding probe that is capable of hybridising to the tuned competitor polynucleotide product is labelled with the second label;

and/or

wherein each probe that is capable of hybridising to a target polynucleotide product that is associated with a negative predictive relationship of the particular state is labelled with the second label, and the corresponding probe that is capable of hybridising to the tuned competitor polynucleotide product is labelled with the first label.

43. The method according to any of embodiments 28-42wherein following amplification the amount of the product detected by the first probe and the amount of product detected by the second probe is determined.

44. The method according to embodiment 43 wherein the relative amounts of each probe are compared to a standard curve to determine the relative probability of one or more states.

45. The method according to any of embodiments 7-44 wherein the method comprises a single reading of all fluorophores used.

46. The method according to any of embodiments 7-45 wherein the method captures the state of a portion of a gene expression network, optionally as a single value.

47. The method according to any of embodiments 7-46 wherein the target polynucleotide is RNA, optionally is an RNA transcript, optionally is an mRNA.

48. The method according to any of embodiments 7-47 wherein the target polynucleotide is a non-coding RNA, optionally is a miRNA, lncRNA or an siRNA.

49. The method according to any of embodiments 7-46 wherein the target polynucleotide is a DNA.

50. The method according to any of embodiments 7-49 wherein the sample is selected from the group comprising or consisting of: tissue, biopsy, blood, plasma, serum, pathogens, microbial cells, cell culture and cell lysate.

51. The method according to any of embodiments 7-50 wherein the sample comprises any one or more of: cells, optionally white blood cells and/or red blood cells; exosomes; circulating tumour DNA (ctDNA); cell-free DNA (cfDNA); RNA; or pathogen nucleic acid.

52. The method according to any of 50 or 51 wherein the cells are:

mammalian cells, bacterial cells, yeast cells or plant cells;

cultured cells, optionally primary patient-derived cells or immortalized cell lines;

mammalian stem cells;

engineered cells, optionally engineered cells used in the bioproduction of metabolites and compounds; and/or

yeast cells, optionally wherein the yeast cells are used in brewing.

53. The method according to any one of embodiments 7-52 wherein the method is for the amplification of at least a first and a second target polynucleotide, optionally wherein the method is for the amplification of at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 target polynucleotides.

54. The method according to embodiment 53 wherein the at least first and the second target polynucleotides are target sequences within the same single polynucleotide.

55. The method according to embodiment 7-54 wherein the method comprises amplification of a tuned competitor polynucleotide with at least one primer that is capable of hybridising to the first and to the second target polynucleotide and producing a first target product and a second target product.

56. The method according to embodiment 7-55 wherein the method comprises amplification of two tuned competitor polynucleotides, wherein the method comprises:

amplification of a first tuned competitor polynucleotide with at least one primer that is capable of hybridising to the first target polynucleotide; and

amplification of a second tuned competitor polynucleotide with at least one primer that is capable of hybridising to the second target polynucleotide.

57. The method of any of embodiments 7-56, wherein detection of the amplification products is indicative of:

i) the presence or absence;

ii) a particular pre-determined starting concentration;

iii) a starting concentration above or below a pre-determined level; and/or

iv) starting concentrations falling within a pre-determined range,

of one or more target polynucleotides.

58. The method of embodiment 57, wherein (i), (ii), (iii) and/or (iv) is indicative of one or more of:

a) the relative expression of a specific gene;

b) the relative expression of two or more specific genes;

c) the relative expression of one or more housekeeping genes

d) the relative expression of a particular gene expression signature;

e) the relative expression of a particular allelic variant of a gene or genes;

f) the relative expression of a mutant version of a gene;

g) the relative expression of cell-free tumour DNA,

wherein the target polynucleotide is selected from one or more portions of a known sequence of (a)-(g).

59. The method of any of embodiments 7-58, wherein the degree of differential gene regulation contributes to an overall probability of the sample being in a particular state 1 as compared to being in some other particular state 2.

60. The method of any of embodiments 1- wherein the particular state 1 is "particular disease" and particular state 2 is "no disease" or "other disease" or "not particular disease".

61. The method of any of embodiments 1-60 embodiment wherein the method is for the diagnosis and/or prognosis of a disease or condition in a subject.

62. The method according to embodiment 61 wherein diagnosis of the disease or condition requires the assessment of the relative expression levels of at least two genes, optionally requires the assessment of the relative expression levels of at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 genes.

63. The method of embodiment 61 or 62, wherein the disease or condition is selected from: human tuberculosis, human tuberculosis with HIV co-infection, human tuberculosis without HIV co-infection, cancer, optionally prostate cancer, sepsis, bloodstream candidiasis, bovine tuberculosis, bovine mastitis.

64. The method of embodiment 61 to 63, wherein the disease is tuberculosis.

65. The method of embodiment 64, wherein the disease is tuberculosis, and the differential gene regulation signature or predictive relationship is identified from the white blood cells of the subject.

66. The method of any of embodiments 63-65, wherein the degree of differential regulation of GBP6, ARG1 and TMCC1 contributes to an overall probability of having tuberculosis as compared to having some "other disease" and the gene expression signature is upregulation of GBP6, and downregulation of ARG1 and TMCC1, compared to the levels of these genes in patients not having tuberculosis.

67. The method of embodiment 61-63, wherein the disease is cancer, optionally prostate cancer or breast cancer, optionally prostate cancer.

68. The method of any of embodiments 61-67, wherein the relative expression of a mutant version of a gene, particular allelic variant and/or cell-free tumour DNA is detected.

69. The method of any of embodiments 61-68 wherein the disease is cancer.

70. The method of any of embodiments 7-69 wherein the target polynucleotide(s) comprise snps, snvs (single nucleotide variants) indels or copy-number variatns (CNVs) associated with a disease state, optionally associated with the presence of a tumour and/or cancer.

71. The method of embodiment 70 wherein the target polynucleotide(s) comprise snps, snvs or indels in cell-free tumour DNA.

72. The method of any of embodiments 7-71, wherein the method further comprises adding a blocker oligonucleotide, wherein the blocker oligonucleotide cannot undergo extension of its 3' end, and wherein the blocker oligonucleotide is not complementary to the portion of the sequence in the at least one target polynucleotide containing the single-nucleotide polymorphism, optionally wherein the snp is a snv, but wherein the blocker oligonucleotide is complementary to the corresponding wild-type sequence and wherein the sequence in the target polynucleotide that comprises the sequence that is complementary to the blocker oligonucleotide overlaps with at least a portion of the sequence complementary to one of the primers.

73. The method of any one of embodiments 7-72, wherein the target polynucleotide(s) is derived from a sample obtained from a subject suspected of having a particular disease or condition.

74. The method of any one of embodiments 7-72, wherein the target polynucleotide is derived from a sample obtained from a subject not suspected of having a particular disease or condition.

75. The method of any of embodiments 7-74, wherein the detection of expression of a specific gene or genes is indicative of the expression of specific natural and/or engineered genes in cells in culture.

76. The method of any of embodiments 7-75, wherein the cells are genetically engineered bacterial, plant or yeast cells.

77. The method of any of embodiments 7-76wherein the nucleic acids are amplified using the polymerase chain reaction (PCR) or the recombinase polymerase reaction (RPA).

78. A method of diagnosis or prognosis of a disease or condition in a subject wherein the method comprises the method of any one of embodiments 1-77.

79. The method according to embodiment 78 wherein the subject is diagnosed as having a disease or condition or prognosis of a disease or condition when the relative amounts of the first label and the second label indicate prognosis of disease or condition.

80. The method of any of embodiments 78 or 79, wherein the disease or condition is selected from: human tuberculosis, human tuberculosis with HIV co-infection, human tuberculosis without HIV co-infection, cancer optionally prostate or breast cancer, sepsis, bloodstream candidiasis, bovine tuberculosis, bovine mastitis.

81. The method of embodiment 78-80x, wherein the disease is tuberculosis, optionally wherein:

the differential gene regulation signature and/or predictive relationship is identified from the white blood cells of the subject; and/or
the degree of differential regulation of GBP6, ARG1 and TMCC1 contributes to an overall probability of having tuberculosis as compared to having some "other disease".

82. The method of any of embodiments 78-80, wherein the disease is cancer, optionally prostate or breast cancer, optionally prostate cancer.

83. A composition comprising one or more of:

a) At least one target nucleic acid sequence as defined in any one of embodiments 7-82;
b) At least one tuned competitor polynucleotide as defined in any one of embodiments 7-82;
c) At least one primer as defined in any one of embodiments 7-82, optionally at least two primers as defined in anyone of embodiments 7-82;
d) at least one or more probe groups, wherein each probe group comprises at least one probe polynucleotide labelled with a first label and at least one probe polynucleotide labelled with a second label, optionally as defined in any of embodiments 28-82.

84. The composition of embodiment 83, further comprising:

d) A polymerase enzyme;
e) Appropriate amounts of each of four nucleotides A, C, T and G.

85. The composition of embodiment 83 or 84, further comprising:

f) A recombinase enzyme;
g) A single stranded binding protein;
h) A polymerase enzyme;
i) Appropriate amounts of each of the nucleotides A, C, T, G and U.

86. A tuned competitor polynucleotide as defined in any one of embodiments 7-85.

87. A kit for carrying out the method of any one of embodiments 1-82, wherein the kit comprises one or more of:

a) One or more tuned competitor polynucleotides as defined by embodiments 7-82;
b) One or more primers;
c) A first probe group as defined in any one of embodiments 28-82;
d) Suitable buffers;
e) Instructions for use,

optionally wherein the kit comprises at least 2, 3, 4, 5, 6, 7, 8, 9 or at least 10 different tuned competitor polynucleotides and/or at least 2, 3, 4, 5, 6, 7, 8, 9 or at least 10 different probe groups.

88. A method, nucleic acid sequence or kit substantially as described herein.

89. A collection or kit of at least two tuned competitor polynucleotides, wherein the collection comprises at least 2, 3, 4, 5, 6, 7, 8, 9, 10, 11, 12, 13, 14, 15, 16, 17, 18, 19, 20, 22, 24, 25, 26, 28, 30, 32, 34, 35, 36, 38, 40, 45, 50, 55, 60, 65, 70, 75, 80, 85, 90, 95, 100, 110, 120, 130, 140, 150, 160, 170, 180, 190, or at least 200 tuned competitor polynucleotides, optionally wherein the tuned competitors polynucleotides are defined by any of embodiments 7-88.

90. A collection/kit comprising at least two tuned competitor polynucleotides and at least two corresponding labelled probes.

91. A collection/kit comprising:

at least two tuned competitor polynucleotides;
at last two corresponding labelled probes; and
at least two primers.

92. A collection/kit comprising:

at least two tuned competitor polynucleotides as defined by any of the preceding embodiments;
at last two corresponding labelled probes as defined by any of the preceding embodiments; and
at least two primers as defined by any of the preceding embodiments.

93. A method of tuning a first competitor polynucleotide that competes for hybridisation of at least a first primer with a first target polynucleotide and which results in amplification of a first target product and a first tuned competitor product, and wherein:

a) a different proportion of target polynucleotides are amplified compared to the proportion of tuned competitor polynucleotides that are amplified;
b) amplification of the first target polynucleotide matches the predictive relationship of the target polynucleotide to a particular state; and/or
c) the rate of amplification of the first target polynucleotide and optionally the rate of amplification of a second target polynucleotide matches a pre-defined weighting, the method comprising

optimising the sequence of the tuned competitor polynucleotide and/or length of tuned competitor amplification product with respect to the sequence of the first target product and/or length of the first target product.

94. The method according to embodiment 93 wherein:

a second primer is used in said amplification that is capable of hybridising to the first target polynucleotide so that the first target product is produced by primer extension from two primers, optionally produced by PCR;
a third primer is used in said amplification that is capable of hybridising to the first tuned competitor polynucleotide so that the first tuned competitor product is produced by primer extension from two primers, optionally produced by PCR;
optionally wherein the second and the third primer have the same sequence.

95. The method according to embodiment 93 or 94 wherein said optimising comprises producing two or more test tuned competitor polynucleotides that following amplification result in:

a) a different proportion of target polynucleotides are amplified compared to the proportion of tuned competitor polynucleotides that are amplified;
b) amplification of the first target polynucleotide matches the predictive relationship of the target to a particular state; and/or
c) the rate of amplification of the first target polynucleotide and optionally the rate of amplification of a second target polynucleotide matches a pre-defined weighting,

and selecting the tuned competitor that results in the most preferred amplification of the first target polynucleotide.

96. The method according to any of embodiments 93-95 wherein said optimising comprises producing at least 3, 4, 5, 6, 7, 8, 9, 10, 11, 12, 13, 14, 15, 16, 17, 18, 19, 20 different test tuned competitor polynucleotides.

97. The method according to any of embodiments 93-96 wherein said optimising comprises performing at least 3, 4, 5, 6, 7, 8, 9, 10, 11, 12, 13, 14, 15, 16, 17, 18, 19, 20 test amplification reactions with each test tuned competitor polynucleotide, optionally wherein at least 3, 4, 5, 6, 7, 8, 9, 10, 11, 12, 13, 14, 15, 16, 17, 18, 19, 20 amplification reactions are performed using at least 3, 4, 5, 6, 7, 8, 9, 10, 11, 12, 13, 14, 15, 16, 17, 18, 19, 20 different concentrations of target polynucleotide and/or number of target polynucleotide molecules.

98. The method according to embodiment 97 wherein each test amplification using a particular test tuned competitor polynucleotide is performed using a different concentration and/or number of target polynucleotide templates.

99. The method according to any of embodiments 97 or 98 wherein the test amplification reactions are performed with a range of concentrations and/or number of target polynucleotide templates that span 100 copies/ μL to $10^8$ copies/μL.

100. The method according to any of embodiments 93-99 wherein the test tuned competitor polynucleotides are designed to have different GC contents.

100. A method of optimising a competitive amplification reaction according to any of the preceding embodiments, wherein said optimising comprises:

 a) Increasing or decreasing the starting concentration of the synthetic nucleic acid sequence; and/or
 b) Increasing or decreasing the starting concentration of any of the nucleic acid primers.

101. A method of multiplexed competitive amplification of at least two target polynucleotides wherein the method comprises at least one competitive polynucleotide and wherein the target amplification products are detected using probes labelled with the same label, optionally labelled with the same fluorophore, optionally wherein the competitive polynucleotide is a tuned competitive polynucleotide according to any of the preceding embodiments.

102. A method of determining the transcriptional state of a system wherein the method comprises competitive amplification according to any of the preceding embodiments.

103. A method of determining whether a system is in state A or in state B wherein the method comprises competitive amplification according to any of the preceding embodiments.

104. A method of simultaneous competitive amplification of at least two target polynucleotides in a sample wherein the method comprises providing

 a) a sample comprising polynucleotides;
 b) a first and a second tuned competitor polynucleotide;
 c) a first primer set, wherein the primer set comprises two primers capable of hybridising on opposite strands of a first target polynucleotide and the first competitive polynucleotide, so as to allow production of a first target amplification product and a first competitive amplification product;
 d) a second primer set, wherein the primer set comprises two primers capable of hybridising on opposite strands of a second target polynucleotide and the second competitive polynucleotide, so as to allow production of a second target product and a second competitive product;
 e) a first probe group, wherein the first probe group comprises a first labelled target probe capable of hybridising to the first target amplification product and a first labelled competitor probe capable of hybridising to the first competitive amplification product;
 d) a second probe group, wherein the second probe group comprises a second labelled target probe capable of hybridising to the second target amplification product and a second labelled competitor probe capable of hybridising to the second competitive amplification product;
 and wherein:

 i) the first labelled target probe and the second target labelled probe are labelled with the same first label; and wherein the first labelled competitor probe and the second labelled competitor probe are labelled with the same second label; or
 ii) the first labelled target probe and the second labelled competitor probe are labelled with the same first label; and wherein the first labelled competitor probe and the second labelled target probe are labelled with the same second label

and allowing the first and second primer sets to hybridise to the target and competitive polynucleotides.

105. The method according to 104 wherein the method comprises providing e) a further 1, 2, 3, 4, 5, 6, 7, 8, 9, 10 primer sets and corresponding probe groups.

106. The method according to embodiment 105 wherein the method further comprises simultaneously detecting the amount of the first label and the second label following multiplexed amplification.

107. Any of the preceding embodiments wherein:

 where the target is TMCC1, the target sequence is SEQ ID NO: 4, competitor sequences used to determine the most optimum competitor are SEQ ID NO: 6, 8, 10, 12, 14, 16, 18, 20, 22, 24, 26, 28, 30, 32, 34 and 36, optionally wherein primers for amplification of the target and competitors are shown in SEQ ID NO: 1 and 3; and/or
 where the target is ARG1, the target sequence is SEQ ID NO: 40, competitor sequences used to determine the most optimum competitor are SEQ ID NO: 42, 44, 46 and 48, optionally wherein primers for amplification of the target and competitors are shown in SEQ ID NO: 37 and 39; and/or

where the target is GBP6, the target sequence is SEQ ID NO: 52, competitor sequences used to determine the most optimum competitor are SEQ ID NO: 54, 56, and 58, optionally wherein primers for amplification of the target and competitors are shown in SEQ ID NO: 49 and 51; and/or

where the target is EGFR, the target sequence is SEQ ID NO: 62, competitor sequences are SEQ ID NO: 64, 67 and 71, optionally wherein primer sequences are SEQ ID NO: 68 and 70.

**The invention is also further defined by the following numbered embodiments:**

1. A method of amplifying one or more target polynucleotides in a sample, wherein the method comprises: providing:

a) a sample potentially comprising one or more target polynucleotides
b) a first tuned competitor polynucleotide
c) at least a first primer wherein at least the first primer is capable of hybridising to:

a first target polynucleotide in the sample; and
the first tuned competitor polynucleotide; and

initiating a primer extension reaction such that the target polynucleotide (if present in the sample) and the first tuned competitor polynucleotide are amplified,

wherein amplification results in a first target product and a first tuned competitor product.

2. The method according to embodiment 1 wherein the method comprises providing:

a second primer;
a second competitor polynucleotide; and/or
a second target polynucleotide.

3. The method according to embodiment 2 wherein the second primer is capable of hybridising to the first target polynucleotide, wherein the first and second primer hybridise on opposite strands of the target so as to result in the production of the first target product, optionally a first target polymerase chain reaction (PCR) product.

4. The method according to any one of embodiments 2 or 3 wherein the second primer is also capable of hybridising to the first tuned competitor polynucleotide, wherein the first and second primer hybridise on opposite strands of the first tuned competitor polynucleotide so as to result in the production of the first tuned competitor product, optionally first tuned competitor PCR product.

5. The method according to any one of embodiments 1-4 wherein the method comprises providing a second tuned competitor polynucleotide. 6. The method according to any one of embodiments 2 or 5 wherein the second primer is:

a) capable of hybridising to the first tuned competitor polynucleotide, wherein the first and second primer hybridise on opposite strands of the first tuned competitor polynucleotide so as to result in the production of the first tuned competitor product, optionally first tuned competitor PCR product; and

b) is capable of hybridising to the second tuned competitor polynucleotide and initiating a primer extension reaction such that the second tuned competitor polynucleotide is amplified so as to result in the production of the second tuned competitor product, optionally in combination with a further primer wherein the second and further primer hybridise on opposite strands of the second tuned competitor polynucleotide so as to result in the production of the second tuned competitor product, optionally a first target polymerase chain reaction (PCR) product,

optionally wherein the second primer is not capable of hybridising to the first target polynucleotide.

7. The method according to any one of embodiments 1-6 wherein the second target polynucleotide is part of the same polynucleotide molecule as the first target polynucleotide.

8. The method according to 1-7 wherein the second target polynucleotide is on a different polynucleotide molecule to the first target polynucleotide.

9. The method according to any one of embodiments 1-8 wherein the second primer is:

a) capable of hybridising to the first target polynucleotide, wherein the first and second primer hybridise on opposite strands of the target so as to result in the production of the first target product, optionally a first target polymerase chain reaction (PCR) product; and

b) is not capable of hybridising to the first or second tuned competitor polynucleotide

and wherein the method comprises a third primer capable of hybridising to the first and to the second tuned competitor polynucleotide.

10. The method of any one of embodiments 1-9, wherein the amplification rate of the first target polynucleotide is different to the amplification rate of the first tuned competitor polynucleotide.

11. The method according to any one of embodiments 1 or 10 wherein the number of target product polynucleotides generated is different to the number of tuned competitor product polynucleotides generated, when the initial number of target polynucleotides and the number of tuned competitor polynucleotides prior to primer extension is the same or is substantially the same.

12. The method according to any one of embodiments 1-11 wherein the sequence of the first target polynucleotide to be amplified, and the sequence of the at least first tuned competitor polynucleotide, is selected so as to result in a final detectable signal that varies with the initial concentration of the first target polynucleotide in such a way that approximates or reproduces or matches the predictive relationship of the target to one or more states.

12. The method according to any one of embodiments 1-11 wherein the rate of amplification of a first target polynucleotide and the rate of amplification of a second target polynucleotide matches a pre-defined weighting.

13. The method according to any of one of embodiments 1-12 wherein the sequence of the first tuned competitor polynucleotide to be amplified shares less than 95%, 90%, 88%, 85%, 80%, 75%, 70%, 65%, 60%, 55%, 50%, 45%, 40%, 35%, 30% sequence identity with the sequence of the first target polynucleotide to be amplified.

14. The method according to any one of embodiments 1-13 wherein the first tuned competitor product is:

at least 5 nucleotides shorter than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the first target product; or
at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product.

15. The method according to any one of embodiments 1-14 wherein the one or more target products, optionally one or more target PCR products; and the one or more tuned competitor products, optionally one or more competitor polynucleotide PCR products are detected.

16. The method according to any one of embodiments 1-15 wherein the method comprises providing one or more probe groups, wherein each probe group comprises at least one probe polynucleotide labelled with a first label and at least one probe polynucleotide labelled with a second label,
and wherein the first and the second label are different.

17. The method according to embodiment 16 wherein the at least one probe labelled with the first label is capable of hybridising to the first target product; and the at least one probe labelled with a second label is capable of hybridising to the first tuned competitor product.

18. The method according to any of embodiments 16 or 17 wherein the at least one probe labelled with the first label is capable of hybridising to the first tuned competitor product; and

the at least one probe labelled with the second label is capable of hybridising to the second tuned competitor product; and
optionally wherein neither probe is capable of hybridising to the first target product.

19. The method according to any of embodiments 16-18 wherein within a single probe group there are:

at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the first label; and/or
at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the second label.

20. The method according to any one of embodiments 16-19 wherein the method comprises providing at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probe groups,

optionally wherein no particular label, optionally a fluorophore, is used in more than one probe group.

20. The method according to any one of embodiments 16-19 wherein the only labels present on the probes are the first label and the second label.

21. The method according to any one of embodiments 16-20 wherein the first and second label are fluorophores, optionally

wherein each probe comprises a quencher; and/or
wherein the first label is FAM and the second label is HEX; or wherein the first label is HEX and the second label is FAM.

22. The method according to any one of embodiments 16-21 wherein

i) the at least one probe that is capable of hybridising to the first target product; and the at least one probe that is capable of hybridising to the first tuned competitor product are labelled with different labels; and/or
ii) the at least one probe that is capable of hybridising to the first tuned competitor product; and the at least one probe that is capable of hybridising to the second tuned competitor product are labelled with different labels.

23. The method according to any of embodiments 16-22 wherein each probe that is capable of hybridising to a target polynucleotide product that is associated with a positive predictive relationship of a particular state is labelled with the first label, and the corresponding probe that is capable of hybridising to the tuned competitor polynucleotide product is labelled with the second label;
and/or
wherein each probe that is capable of hybridising to a target polynucleotide product that is associated with a negative predictive relationship of the particular state is labelled with the second label, and the corresponding probe that is capable of hybridising to the tuned competitor polynucleotide product is labelled with the first label.

24. The method according to any of embodiments 16-23 wherein following amplification the amount of the product detected by the first probe and the amount of product detected by the second probe is determined.

25. The method according to embodiment 24 wherein the relative amounts of each probe are compared to a standard curve to determine the relative probability of one or more states.

26. The method according to any of embodiments 1-25 wherein the method comprises a single reading of all fluorophores used.

27. The method according to any one of embodiments 1-26 wherein the method is for the amplification of at least a first and a second target polynucleotide, optionally wherein the method is for the amplification of at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 target polynucleotides.

28. The method according to embodiment 1-27 wherein the method comprises amplification of two tuned competitor polynucleotides, wherein the method comprises:

amplification of a first tuned competitor polynucleotide with at least one primer that is capable of hybridising to the first target polynucleotide; and
amplification of a second tuned competitor polynucleotide with at least one primer that is capable of hybridising to the second target polynucleotide.

29. The method of any of embodiments 1-28 embodiment wherein the method is for the diagnosis and/or prognosis of a disease or condition in a subject.

30. The method of any of embodiments 1-29 wherein the nucleic acids are amplified using the polymerase chain reaction (PCR) or the recombinase polymerase reaction (RPA).

31. A method of diagnosis or prognosis of a disease or condition in a subject wherein the method comprises the method of any one of embodiments 1-30.

32. The method according to embodiment 31 wherein the subject is diagnosed as having a disease or condition or prognosis of a disease or condition when the relative amounts of the first label and the second label indicate prognosis of disease or condition.

33. The method of any of embodiments 31 or 32, wherein the disease or condition is selected from: human tuberculosis, human tuberculosis with HIV co-infection, human tuberculosis without HIV co-infection, cancer optionally prostate or breast cancer, sepsis, bloodstream candidiasis, bovine tuberculosis, bovine mastitis.

34. The method of any of embodiments 31-33, wherein the disease is tuberculosis, optionally wherein:

the differential gene regulation signature and/or predictive relationship is identified from the white blood cells of the subject; and/or

the degree of differential regulation of GBP6, ARG1 and TMCC1 contributes to an overall probability of having tuberculosis as compared to having some "other disease", optionally wherein the gene expression signature is upregulation of GBP6, and downregulation of ARG1 and TMCC1, compared to the levels of these genes in patients not having tuberculosis.

35. The method of any of embodiments 31-34, wherein the disease is cancer, optionally prostate or breast cancer, optionally prostate cancer.

36. The method according to any of embodiments 31-35 wherein diagnosis of the disease or condition requires the assessment of the relative expression levels of at least two genes, optionally requires the assessment of the relative expression levels of at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 genes.

37. A composition comprising one or more of:

a) At least one target nucleic acid sequence as defined in any one of embodiments 1-30;
b) At least one tuned competitor polynucleotide as defined in any one of embodiments 1-30;
c) At least one primer as defined in any one of embodiments 1-30, optionally at least two primers as defined in anyone of embodiments 1-30;
d) at least one or more probe groups, wherein each probe group comprises at least one probe polynucleotide labelled with a first label and at least one probe polynucleotide labelled with a second label, optionally as defined in any of embodiments 16-30.

38. A tuned competitor polynucleotide as defined in any one of embodiments 1-30.

39. A kit for carrying out the method of any one of embodiments 1-36, wherein the kit comprises one or more of:

a) One or more tuned competitor polynucleotides as defined by embodiments 1-30;
b) One or more primers, optionally as defined in any one of embodiments 1-30;
c) A first probe group as defined in any one of embodiments 16-30;
d) Suitable buffers;
e) Instructions for use,

optionally wherein the kit comprises at least 2, 3, 4, 5, 6, 7, 8, 9 or at least 10 different tuned competitor polynucleotides and/or at least 2, 3, 4, 5, 6, 7, 8, 9 or at least 10 different probe groups.

40. A method of tuning a first competitor polynucleotide that competes for hybridisation of at least a first primer with a first target polynucleotide and which results in amplification of a first target product and a first tuned competitor product, and wherein:

a) a different proportion of target polynucleotides are amplified compared to the proportion of tuned competitor polynucleotides that are amplified;
b) amplification of the first target polynucleotide matches the predictive relationship of the target polynucleotide to a particular state; and/or
c) the rate of amplification of the first target polynucleotide and optionally the rate of amplification of a second target polynucleotide matches a pre-defined weighting, the method comprising

optimising the sequence of the tuned competitor polynucleotide and/or length of tuned competitor amplification product with respect to the sequence of the first target product and/or length of the first target product.

41. The method according to embodiment 40 wherein:

a second primer is used in said amplification that is capable of hybridising to the first target polynucleotide so that the first target product is produced by primer extension from two primers, optionally produced by PCR;
a third primer is used in said amplification that is capable of hybridising to the first tuned competitor polynucleotide so that the first tuned competitor product is produced by primer extension from two primers, optionally produced by PCR;
optionally wherein the second and the third primer have the same sequence.

42. The method according to embodiment 40 or 41 wherein said optimising comprises producing two or more test tuned competitor polynucleotides that following amplification result in:

a) a different proportion of target polynucleotides are amplified compared to the proportion of tuned competitor polynucleotides that are amplified;

b) amplification of the first target polynucleotide matches the predictive relationship of the target to a particular state; and/or

c) the rate of amplification of the first target polynucleotide and optionally the rate of amplification of a second target polynucleotide matches a pre-defined weighting,

and selecting the tuned competitor that results in the most preferred amplification of the first target polynucleotide.

43. A method of determining the transcriptional state of a system wherein the method comprises competitive amplification according to any of the preceding embodiments.

44. A method of determining whether a system is in state A or in state B wherein the method comprises competitive amplification according to any of the preceding embodiments.

45. A method of simultaneous competitive amplification of at least two target polynucleotides in a sample wherein the method comprises

providing

a) a sample comprising polynucleotides;

b) a first and a second tuned competitor polynucleotide;

c) a first primer set, wherein the primer set comprises two primers capable of hybridising on opposite strands of a first target polynucleotide and the first competitive polynucleotide, so as to allow production of a first target amplification product and a first competitive amplification product;

d) a second primer set, wherein the primer set comprises two primers capable of hybridising on opposite strands of a second target polynucleotide and the second competitive polynucleotide, so as to allow production of a second target product and a second competitive product;

e) a first probe group, wherein the first probe group comprises a first labelled target probe capable of hybridising to the first target amplification product and a first labelled competitor probe capable of hybridising to the first competitive amplification product;

d) a second probe group, wherein the second probe group comprises a second labelled target probe capable of hybridising to the second target amplification product and a second labelled competitor probe capable of hybridising to the second competitive amplification product;

and wherein:

i) the first labelled target probe and the second target labelled probe are labelled with the same first label; and wherein the first labelled competitor probe and the second labelled competitor probe are labelled with the same second label; or

ii) the first labelled target probe and the second labelled competitor probe are labelled with the same first label; and wherein the first labelled competitor probe and the second labelled target probe are labelled with the same second label

and allowing the first and second primer sets to hybridise to the target and competitive polynucleotides.

46. The method according to 45 wherein the method comprises providing e) a further 1, 2, 3, 4, 5, 6, 7, 8, 9, 10 primer sets and corresponding probe groups.

47. The method according to embodiment 46 wherein the method further comprises simultaneously detecting the amount of the first label and the second label following multiplexed amplification.

The invention also provides the following numbered embodiment paragraphs:

1. A method of amplifying at least a first and at least a second target polynucleotide in a sample, wherein the method comprises:

providing:

a) a sample potentially comprising the at least a first and at least a second target polynucleotides

b) a first tuned competitor polynucleotide and a second tuned competitor polynucleotide;

c) at least a first primer wherein at least the first primer is capable of hybridising to:

a first target polynucleotide in the sample; and
the first tuned competitor polynucleotide; and

initiating a primer extension reaction such that the first and second target polynucleotides (if present in the sample) and the first tuned competitor polynucleotide and the second tuned competitor polynucleotide are amplified,

wherein amplification results in a first target product, a second target product, a first tuned competitor product and a second tuned competitor product.

2. The method according to paragraph 1 wherein the method comprises providing a second primer, optionally wherein;

a) the second primer is capable of hybridising to the first target polynucleotide, wherein the first and second primer hybridise on opposite strands of the target so as to result in the production of the first target product, optionally a first target polymerase chain reaction (PCR) product;

b) the second primer is capable of hybridising to the first tuned competitor polynucleotide, wherein the first and second primer hybridise on opposite strands of the first tuned competitor polynucleotide so as to result in the production of the first tuned competitor product, optionally first tuned competitor PCR product;

c) the second primer is:

i) capable of hybridising to the first tuned competitor polynucleotide, wherein the first and second primer hybridise on opposite strands of the first tuned competitor polynucleotide so as to result in the production of the first tuned competitor product, optionally first tuned competitor PCR product; and

ii) is capable of hybridising to the second tuned competitor polynucleotide and initiating a primer extension reaction such that the second tuned competitor polynucleotide is amplified so as to result in the production of the second tuned competitor product, optionally in combination with a further primer wherein the second and further primer hybridise on opposite strands of the second tuned competitor polynucleotide so as to result in the production of the second tuned competitor product, optionally a first target polymerase chain reaction (PCR) product,

optionally wherein the second primer is not capable of hybridising to the first target polynucleotide; and/or

d) the second primer is:

i) capable of hybridising to the first target polynucleotide, wherein the first and second primer hybridise on opposite strands of the target so as to result in the production of the first target product, optionally a first target polymerase chain reaction (PCR) product; and
ii) is not capable of hybridising to the first or second tuned competitor polynucleotide

and wherein the method comprises a third primer capable of hybridising to the first and to the second tuned competitor polynucleotide.

3. The method of any one of paragraphs 1 or 2 wherein the amplification kinetics of the first target polynucleotide are not the same as the amplification kinetics of the first tuned competitor polynucleotide, or are not substantially similar to the amplification kinetics of the first tuned competitor polynucleotide.

4. The method according to any one of paragraphs 1-3 wherein the number of target product polynucleotides generated is different to the number of tuned competitor product polynucleotides generated, when the initial number of target polynucleotides and the number of tuned competitor polynucleotides prior to primer extension is the same or is substantially the same.

5. The method according to any one of paragraphs 1-4 wherein:

the sequence of the first target polynucleotide to be amplified and the sequence of the at least first tuned competitor polynucleotide, and

the sequence of the second target polynucleotide to be amplified and the sequence of the at least second tuned competitor polynucleotide,

is selected so as to result in a final amount of first target amplification product and second target amplification product that varies with the initial concentration of the first target polynucleotide and the second target polynucleotide in such a way that approximates or reproduces or matches the predictive relationship of the target to one or more states.

6. The method according to any one of paragraphs 1-5 wherein the rate of amplification of the first target polynucleotide and the rate of amplification of the second target polynucleotide matches a pre-defined weighting.

7. The method according to any of one of paragraphs 1-6 wherein the sequence of the first tuned competitor polynucleotide to be amplified shares less than 95%, 90%, 88%, 85%, 80%, 75%, 70%, 65%, 60%, 55%, 50%, 45%, 40%, 35%, 30% sequence identity with the sequence of the first target polynucleotide to be amplified; and optionally wherein
the sequence of the second tuned competitor polynucleotide to be amplified shares less than 95%, 90%, 88%, 85%, 80%, 75%, 70%, 65%, 60%, 55%, 50%, 45%, 40%, 35%, 30% sequence identity with the sequence of the second target polynucleotide to be amplified;

8. The method according to any one of paragraphs 1-7 wherein:

the first tuned competitor product is:

i) at least 5 nucleotides shorter than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the first target product; or
ii) at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product; and/or

the second tuned competitor product is:

i) at least 5 nucleotides shorter than the second target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the second target product; or
ii) at least 5 nucleotides longer than the second target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the second target product;

9. The method according to any one of paragraphs 1-8 wherein the one or more target products, optionally one or more target PCR products; and the one or more tuned competitor products, optionally one or more competitor polynucleotide PCR products are detected, optionally wherein
the first target amplification product, the second target amplification product, the first tuned competitor amplification product and the second tuned competitor amplification product are detected.

10. The method according to any one of paragraphs 1-9 wherein the method comprises providing one or more probe groups, wherein each probe group comprises at least one probe polynucleotide labelled with a first label and at least one probe polynucleotide labelled with a second label,
and wherein the first and the second label are different.

11. The method according to paragraph 10 wherein the at least one probe labelled with the first label is capable of hybridising to the first target product; and the at least one probe labelled with a second label is capable of hybridising to the first tuned competitor product.

12. The method according to any of paragraphs 10 or 11 wherein the at least one probe labelled with the first label is capable of hybridising to the first tuned competitor product; and

the at least one probe labelled with the second label is capable of hybridising to the second tuned competitor product; and

optionally wherein neither probe is capable of hybridising to the first target product.

13. The method according to any of paragraphs 10-12 wherein within a single probe group there are:

at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the first label; and/or
at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probes each labelled with the second label.

14. The method according to any one of paragraphs 10-13 wherein the method comprises providing at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 different probe groups,
optionally wherein no particular label, optionally a fluorophore, is used in more than one probe group.

15. The method according to any one of paragraphs 10-14 wherein the only labels present on the probes are the first label and the second label.

16. The method according to any one of paragraphs 10-15 wherein the first and second label are fluorophores, optionally

wherein each probe comprises a quencher; and/or
wherein the first label is FAM and the second label is HEX; or wherein the first

label is HEX and the second label is FAM.

17. The method according to any one of paragraphs 10-16 wherein

i) the at least one probe that is capable of hybridising to the first target product; and the at least one probe that is capable of hybridising to the first tuned competitor product are labelled with different labels; and/or

ii) the at least one probe that is capable of hybridising to the first tuned competitor product; and the at least one probe that is capable of hybridising to the second tuned competitor product are labelled with different labels.

18. The method according to any of paragraphs 10-17 wherein

each probe that is capable of hybridising to a target polynucleotide product that is associated with a positive predictive relationship of a particular state is labelled with the first label, and the corresponding probe that is capable of hybridising to the tuned competitor polynucleotide product is labelled with the second label; and/or

each probe that is capable of hybridising to a target polynucleotide product that is associated with a negative predictive relationship of the particular state is labelled with the second label, and the corresponding probe that is capable of hybridising to the tuned competitor polynucleotide product is labelled with the first label.

19. The method according to any of paragraphs 10-18 wherein following amplification the amount of the product detected by the first probe and the amount of product detected by the second probe is determined.

20. The method according to paragraph 19 wherein the relative amounts of each probe are compared to a standard curve to determine the relative probability of one or more states.

21. The method according to any of paragraphs 1-20 wherein the method comprises detecting the relative abundance of all amplification products by taking a single reading of all fluorophores used.

22. The method according to any one of paragraphs 1-21 wherein the method is for the amplification of at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 target polynucleotides.

23. The method according to paragraph 1-22 wherein the method comprises amplification of two tuned competitor polynucleotides, wherein the method comprises:

amplification of a first tuned competitor polynucleotide with at least one primer that is capable of hybridising to the first target polynucleotide; and

amplification of a second tuned competitor polynucleotide with at least one primer that is capable of hybridising to the second target polynucleotide.

24. The method of any of paragraphs 1-23 wherein the polynucleotides are amplified using the polymerase chain reaction (PCR) or the recombinase polymerase reaction (RPA).

25. A method of:

converting the predictive relationship, decision surface or differential target oligonucleotide pattern, optionally a differential gene regulation signature provided by the relative abundance of at least two oligonucleotides or the presence or absence of at least two mutations, in a sample into a single value;

translating the relative abundance of at least two oligonucleotides, for example the relative expression of at least two genes, or presence or absence of at least two mutations, in a sample into the relative probability of a particular state;

detecting the relative abundance of at least three oligonucleotides, for example the relative expression of at least three genes, or presence or absence of at least three mutations, in a sample using only two fluorophore labelled probes;

combining the relative abundance of at least two oligonucleotides, for example the relative expression of at least two genes, or presence or absence of at least two mutations, in a sample into a single value

wherein the method comprises the method of amplifying at least a first and at least a second target polynucleotide in a sample according to any of paragraphs 1-24.

26. The method of any of paragraphs 1-25 wherein the method is for the diagnosis and/or prognosis of a disease or condition in a subject.

27. A method of diagnosis or prognosis of a disease or condition in a subject wherein the method comprises the method of any one of paragraphs 1-25.

28. The method according to paragraph 27 wherein the subject is diagnosed as having a disease or condition or prognosis of developing a disease or condition when the relative amounts of the first label and the second label indicate diagnosis or prognosis of disease or condition.

29. The method of any of paragraphs 26-28, wherein:

a) the disease or condition is selected from: human tuberculosis, human tuberculosis with HIV co-infection, human tuberculosis without HIV co-infection, cancer optionally prostate or breast cancer, sepsis, bloodstream candidiasis, bovine tuberculosis, bovine mastitis, optionally
wherein the disease is tuberculosis, optionally wherein:

the predictive relationship, decision surface or differential target oligonucleotide pattern, optionally a differential gene regulation signature is identified from the white blood cells of the subject; and/or

the degree of differential regulation of GBP6, ARG1 and TMCC1 contributes to an overall probability of having tuberculosis as compared to having some "other disease", optionally wherein the gene expression signature is upregulation of GBP6, and downregulation of ARG1 and TMCC1, compared to the levels of these genes in patients not having tuberculosis.

30. The method of any of paragraphs 26-29, wherein the disease is cancer, optionally prostate or breast cancer, optionally prostate cancer.

31. The method according to any of paragraphs 26-30 wherein diagnosis of the disease or condition requires the assessment of the relative expression levels of at least two genes, optionally requires the assessment of the relative expression levels of at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or at least 100 genes.

32. A composition comprising one of, at least two or, or all of:

a) At least one tuned competitor polynucleotide as defined in any one of paragraphs 1-26;
b) At least one primer as defined in any one of paragraphs 1-26, optionally at least two primers as defined in anyone of paragraphs 1-26;
c) at least one or more probe groups, wherein each probe group comprises at least one probe polynucleotide labelled with a first label and at least one probe polynucleotide labelled with a second label, optionally as defined in any of paragraphs 10-26.

33. A tuned competitor polynucleotide as defined in any one of paragraphs 1-26.

34. A kit for carrying out the method of any one of paragraphs 1-31, wherein the kit comprises one or more of:

a) One or more tuned competitor polynucleotides as defined by paragraphs 1-26;
b) One or more primers, optionally as defined in any one of paragraphs 1-26;
c) A first probe group as defined in any one of paragraphs 10-26;
d) Suitable buffers;
e) Instructions for use,

optionally wherein the kit comprises at least 2, 3, 4, 5, 6, 7, 8, 9 or at least 10 different tuned competitor polynucleotides and/or at least 2, 3, 4, 5, 6, 7, 8, 9 or at least 10 different probe groups.

35. The kit according to paragraph 34 wherein the kit comprises:

a)

i) One or more tuned competitor polynucleotides as defined by paragraphs 1-26, optionally at least two tuned competitor polynucleotides as defined by paragraphs 1-26; and
ii) One or more primers, optionally as defined in any one of paragraphs 1-26; or

b)

i) One or more tuned competitor polynucleotides as defined by paragraphs 1-26, optionally at least two tuned competitor polynucleotides as defined by paragraphs 1-26; and
ii) A first probe group as defined in any one of paragraphs 10-26; or

c)

i) One or more primers, optionally as defined in any one of paragraphs 1-26; and
ii) A first probe group as defined in any one of paragraphs 10-26; or

d)

i) One or more tuned competitor polynucleotides as defined by paragraphs 1-26, optionally at least two tuned competitor polynucleotides as defined by paragraphs 1-26;
ii) One or more primers, optionally as defined in any one of paragraphs 1-26; and
iii) A first probe group as defined in any one of paragraphs 10-26.

36. A method of tuning a first competitor polynucleotide that competes for hybridisation of at least a first primer with a first target polynucleotide and which results in a discrimination in amplification of a first target product and a first tuned competitor product that translates a predictive relationship, decision surface, or differential target oligonucleotide pattern into a relative abundance of the first target polynucleotide amplification product and wherein:

a) the first competitor polynucleotide is designed to have different amplification kinetics to the target polynucleotide;

b) a different proportion of target polynucleotides are amplified compared to the proportion of tuned competitor polynucleotides that are amplified;
c) amplification of the first target polynucleotide matches the predictive relationship of the target polynucleotide to a particular state; and/or
d) the rate of amplification of the first target polynucleotide and optionally the rate of amplification of a second target polynucleotide matches a pre-defined weighting,

the method comprising

optimising the sequence of the tuned competitor polynucleotide and/or length of tuned competitor amplification product with respect to the sequence of the first target product and/or length of the first target product.

37. The method according to paragraph 36 wherein:

a second primer is used in said amplification that is capable of hybridising to the first target polynucleotide so that the first target product is produced by primer extension from two primers, optionally produced by PCR;
a third primer is used in said amplification that is capable of hybridising to the first tuned competitor polynucleotide so that the first tuned competitor product is produced by primer extension from two primers, optionally produced by PCR;
optionally wherein the second and the third primer have the same sequence.

38. The method according to paragraph 36 or 37 wherein said method is a method for tuning at least two or more test tuned competitor polynucleotides that results in a discrimination in amplification of a first target product and a first tuned competitor product, and in a discrimination in amplification of a second target product and a second tuned competitor product that translates a predictive relationship, decision surface, or differential target oligonucleotide pattern into a relative abundance of the first target polynucleotide amplification product and second target polynucleotide amplification product, and optionally wherein:

a) the first competitor polynucleotide is designed to have different amplification kinetics to the target polynucleotide;
b) a different proportion of target polynucleotides are amplified compared to the proportion of tuned competitor polynucleotides that are amplified;
c) amplification of the first target polynucleotide matches the predictive relationship of the target to a particular state; and/or
d) the rate of amplification of the first target polynucleotide and optionally the rate of amplification of a second target polynucleotide matches a pre-defined weighting,

and selecting the tuned competitor that results in the most preferred amplification of the first target polynucleotide.

39. A method of determining the transcriptional state of a system wherein the method comprises a method of amplification according to any of the preceding paragraphs.

40. A method of determining whether a system is in state A or in state B wherein the method comprises a method of amplification according to any of the preceding paragraphs.

41. A method of simultaneous competitive amplification of at least two target polynucleotides in a sample wherein the method comprises
providing

a) a sample comprising polynucleotides;
b) a first and a second tuned competitor polynucleotide;
c) a first primer set, wherein the primer set comprises two primers capable of hybridising on opposite strands of a first target polynucleotide and the first competitive polynucleotide, so as to allow production of a first target amplification product and a first competitive amplification product;
d) a second primer set, wherein the primer set comprises two primers capable of hybridising on opposite strands of a second target polynucleotide and the second competitive polynucleotide, so as to allow production of a second target product and a second competitive product;
e) a first probe group, wherein the first probe group comprises a first labelled target probe capable of hybridising to the first target amplification product and a first labelled competitor probe capable of hybridising to the first competitive amplification product;

d) a second probe group, wherein the second probe group comprises a second labelled target probe capable of hybridising to the second target amplification product and a second labelled competitor probe capable of hybridising to the second competitive amplification product;

and wherein:

i) the first labelled target probe and the second target labelled probe are labelled with the same first label; and wherein the first labelled competitor probe and the second labelled competitor probe are labelled with the same second label; or

ii) the first labelled target probe and the second labelled competitor probe are labelled with the same first label; and wherein the first labelled competitor probe and the second labelled target probe are labelled with the same second label

and allowing the first and second primer sets to hybridise to the target and competitive polynucleotides.

42. The method according to 41 wherein the method comprises providing

e) a further 1, 2, 3, 4, 5, 6, 7, 8, 9, 10 primer sets and corresponding probe groups.

43. The method according to paragraph 42 wherein the method further comprises simultaneously detecting the amount of the first label and the second label following multiplexed amplification.

**Figure legends**

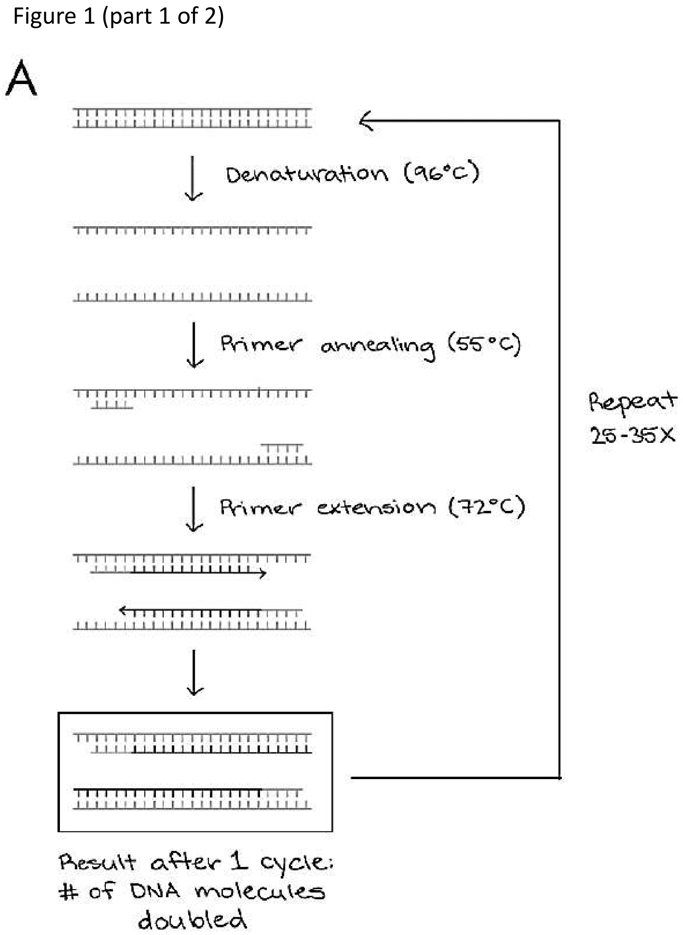

Figure 1 - **Mechanism of traditional PCR. A)** In PCR, cycling through different temperature stages duplicates or "amplifies" the target sequence many times over. This doubling is facilitated by short synthetic "primer" oligonucleotides specific to the target of interest. Once the primers are used up, the reaction stops. **B)** In quantitative PCR, a synthetic "probe" sequence is included as well to generate a fluorescent signal with each duplication of the target. The probe is designed with a fluorophore at one end and a quencher at the other. While the probe remains intact, the quencher absorbs the light emitted by the fluorophore, preventing it from being detected. As the reaction proceeds, however, the polymerase degrades the probe, chewing it up into tiny pieces. This separates the fluorophore from the quencher, leading to a detectable fluorescent signal.

Figure 2 **Changing the composition of the target sequence changes amplification behaviour.** Variations on a natural PCR target sequence (WT) were designed to utilize the same primer sequence but differ in number of base pairs (BP) and percentage of nucleotides that are guanine or cytosine (GC) between primer regions. The ISO target has the same length (88 bp) and GC content (43%) as the WT, but a different sequence. **A)** PCR reactions of these targets were fit with equation (1), grey lines show the ISO fits for reference. **B)** These targets displayed a wide range of exponential growth rates (* indicates the ISO target). This diversity of amplification behaviour is used to tune the characteristics of a CAN to specific applications.

Figure 3 **Target design for direct competitive PCR.** The synthetic REF sequence competes with the WT sequence for the same primers, but the two are targeted by distinct probes with different labels.

Figure 4 **Direct Competitive Amplification endpoints.** The WT sequence from Figure 3 was amplified in the same reaction as the indicated REF sequence. The difference between WT (FAM) and REF (HEX) fluorescence after 45 PCR cycles is shown as a function of WT starting quantity. The initial concentration of the respective REF sequence is indicated in each plot by the vertical grey line. The dose-response relationships are fit with sigmoid curves (black curves, grey curve reflects ISO fit). The inset numbers indicate the sigmoid exponent; a higher number indicates a steeper curve. Reactions with a fast competitor sequence (shorter sequences and those with low GC content) displayed sharp transitions, while slow competitor sequences led to gradual curves.

Figure 5 **Indirect CAN principle. A)** In indirect competition, the natural target does not directly interact with a fluorescent probe. Instead, multiple synthetic targets are used, each of which might share only one primer with another sequence. **B)** Abstract network diagram. Natural targets are shown as squares, synthetic targets as circles, and primers as dots. "Uncontested" primers not shared by multiple targets (here, p0 and p3) are generally omitted from the diagram.

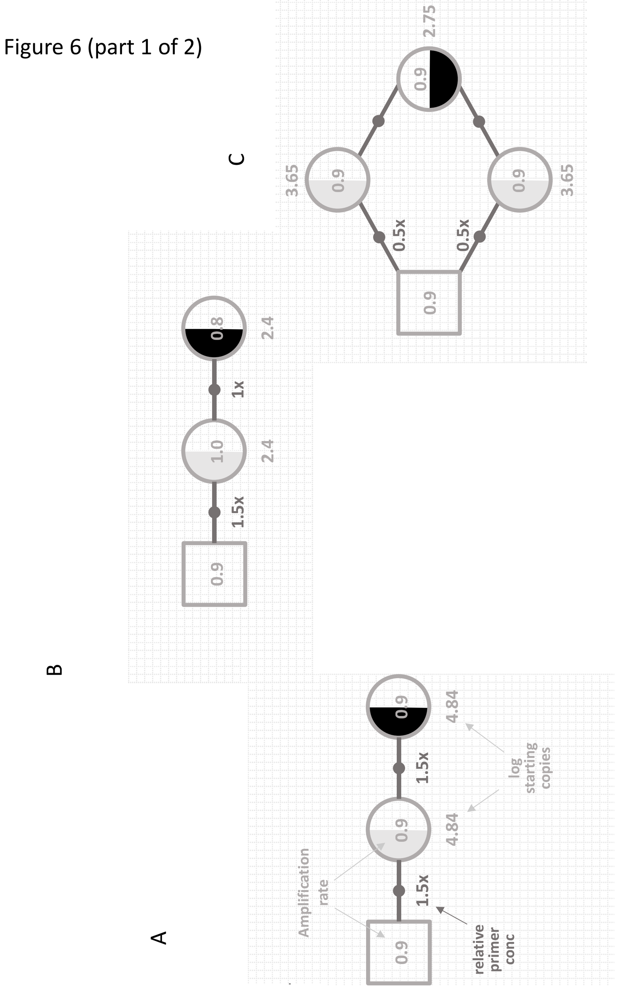

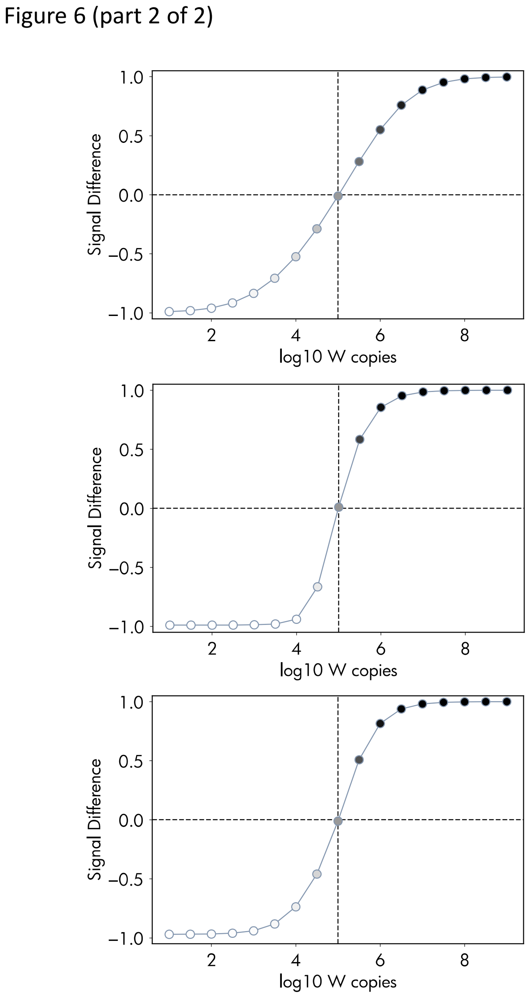

Figure 6 **Simulated outputs for various indirect CAN architectures.** Indirect CANs can be tuned by adjusting parameters of individual components (amplification rate, concentration) or by modifying the connectivity between components. As shown here, indirect CANs can achieve a wide range of dynamic ranges (DR), defined as the WT concentration range between 10% and 90% maximum signal difference.

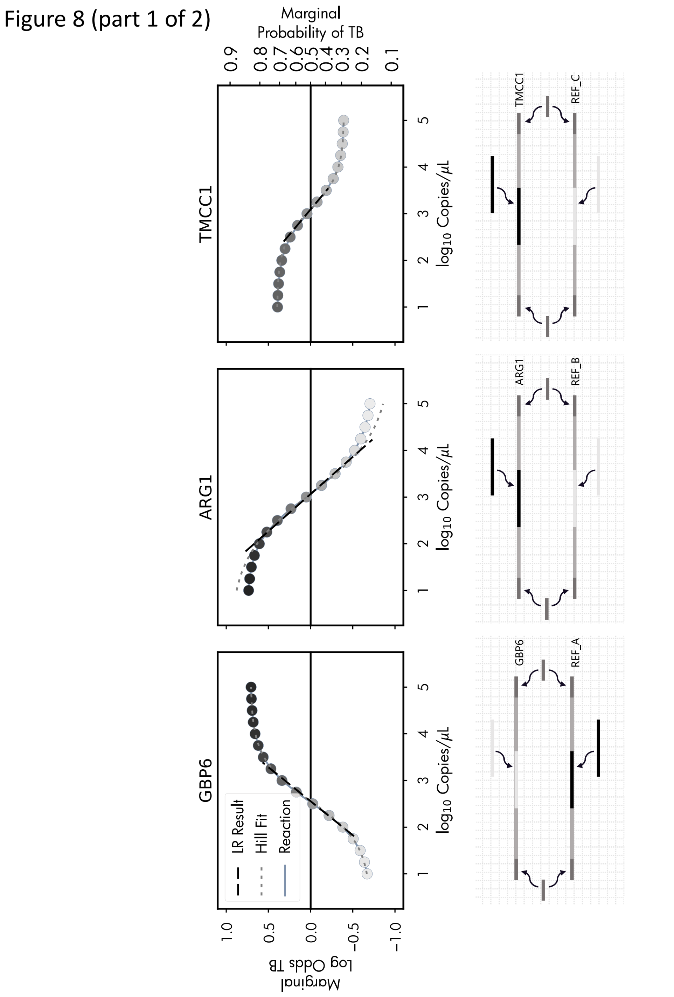

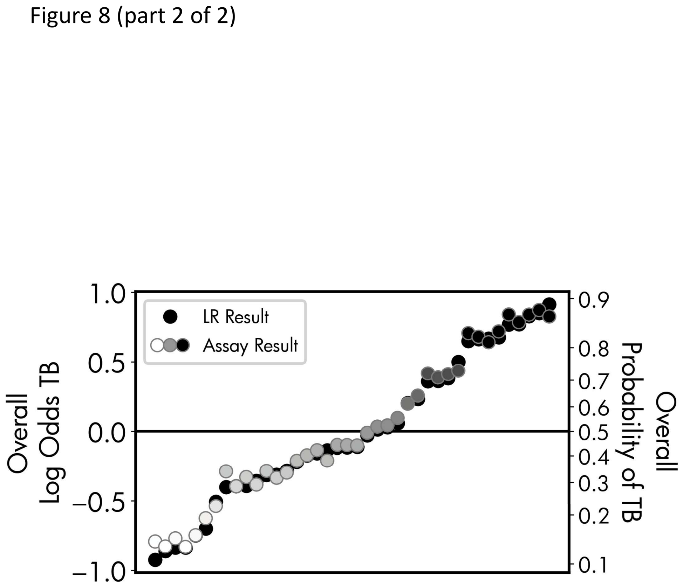

Figure 8 **Three-pair direct CAN for diagnosing tuberculosis.** A) The CAN consists of three direct competitive pairs, one for each transcript in the gene expression signature. Each pair is designed to exhibit a signal response to various concentrations of the natural target that mimics the respective marginal log-odds from logistic regression (Figure 6). Simulated reaction results are shown here. B) When all three pairs are amplified in the same reaction, the resulting fluorescence aggregates their individual contributions. The overall fluorescence difference between teal and orange signals provides a final diagnosis which differs insubstantially from the log-odds provided by logistic regression.

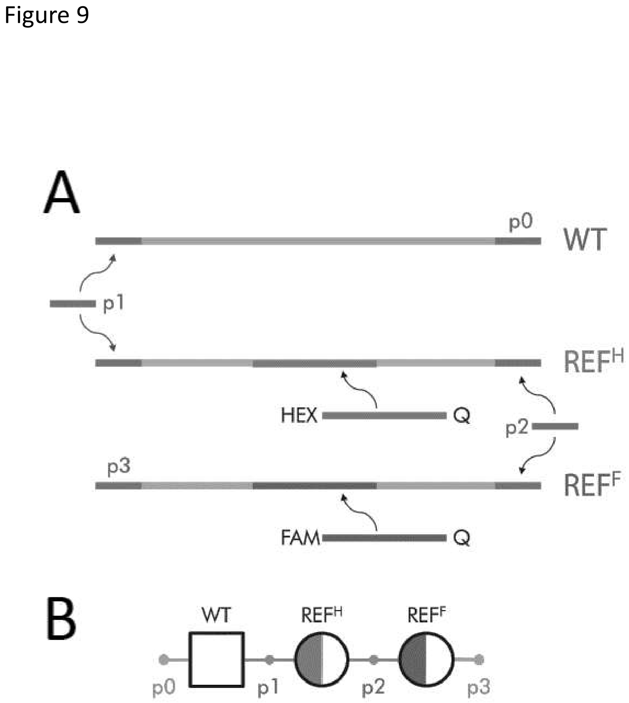

Figure 9: **Indirect CAN principle.** A) In indirect competition, the natural target does not directly interact with a fluorescent probe. Instead, multiple synthetic targets are used, each of which might share only one primer with another sequence. B) Abstract network diagram. Natural targets are shown as squares, synthetic targets as circles, and primers as dots. "Uncontested" primers not shared by multiple targets (here, p0 and p3) are generally omitted from the diagram.

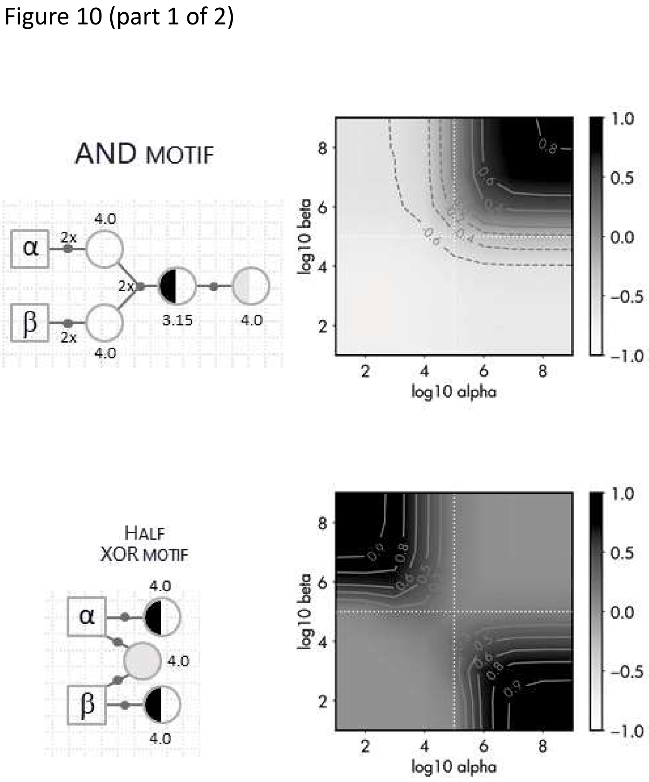

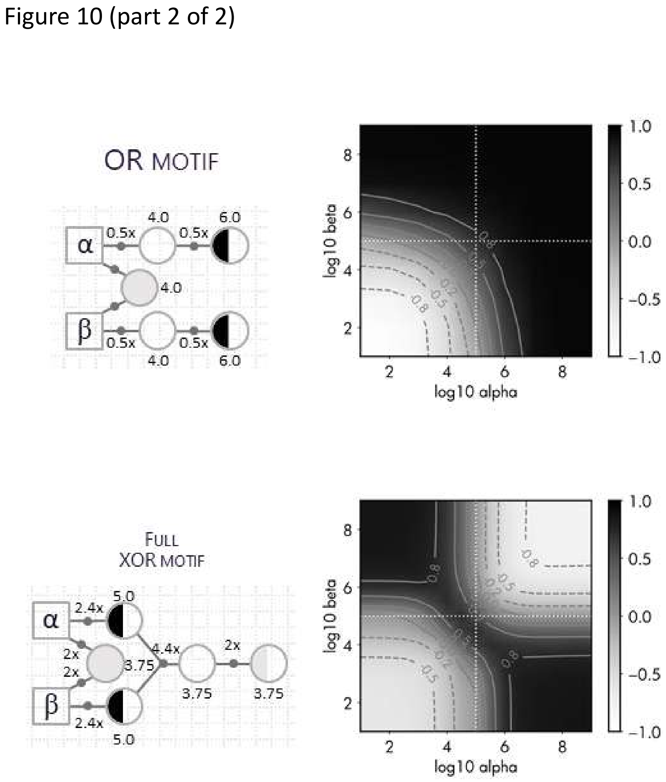

Figure 10: **Higher-order CANs can be designed to approximate Boolean logic.** Indirect competition can recognize combinatorial comprised of patterns of multiple targets. Here, CAN motifs act as Boolean gates, signalling teal/high when the specified condition is true and orange/low when it is false. The "half" XOR is an exception, producing signal parity when false. The full XOR shown here is imperfect, needing further tuning, but demonstrates the rich behaviour possible from higher-order CANs. Tuning network parameters can determine the abruptness and location of the transition regime. Note that the inverse gates, NAND, NOR, and XNOR, can all be obtained by simply swapping the probe labels. Simulated results, all targets are assumed to have a 0.9 amplification rate.

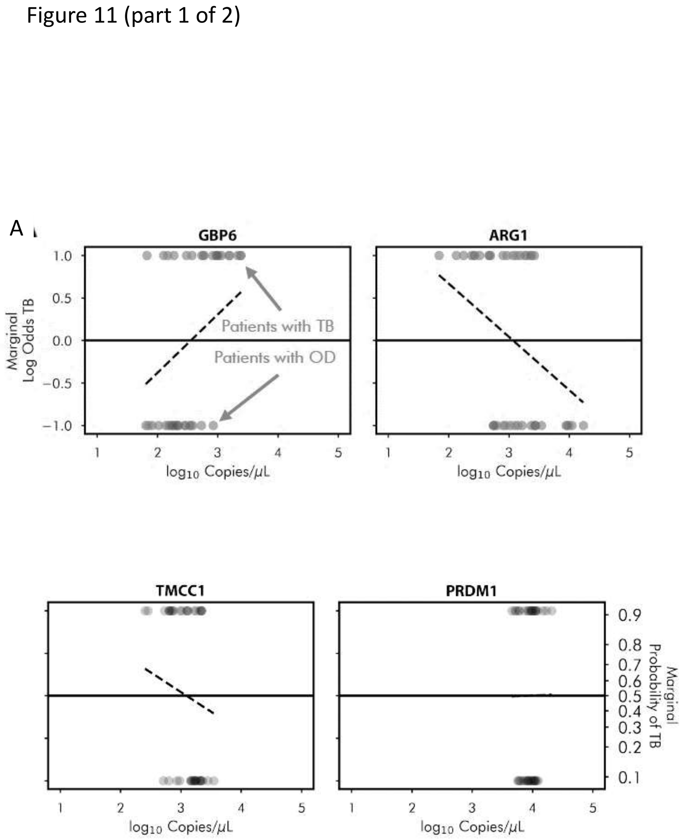

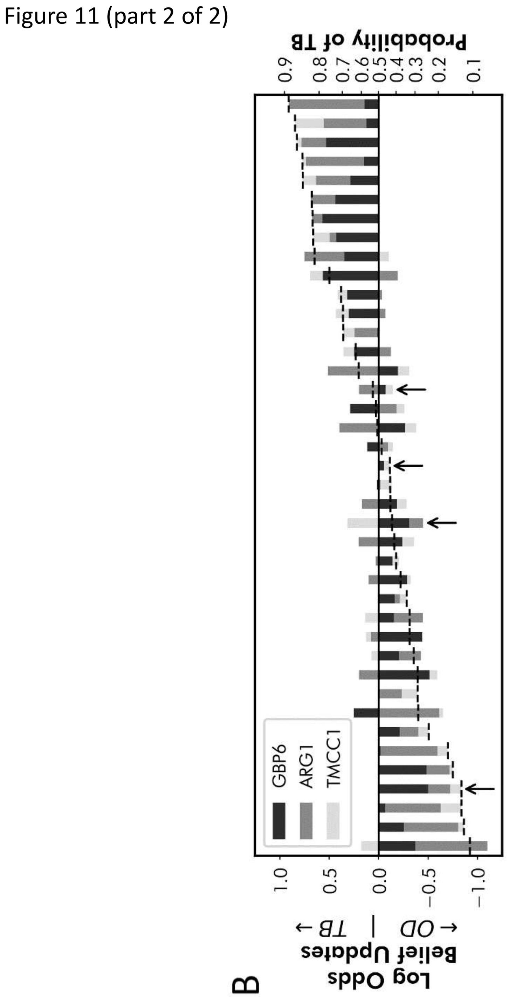

Figure 11: **Logistic regression on digital PCR data.** A) Grey dots indicate gene concentrations found in individual patients with either tuberculosis (TB) or some other disease (OD), while the dashed line is the result of the logistic regression. Log-odds (left) and probability (right) are interchangeable through a simple non-linear transform. From these results, we can see that the fourth gene, PRDM1, does not contribute meaningfully to the diagnosis. B) The individual contribution of each gene to the overall diagnosis is shown by the corresponding colour and the overall log-odds is indicated by the dashed lines. Arrows point to patients for whom the statistical diagnosis is discordant with the gold-standard diagnosis, microbial culture.

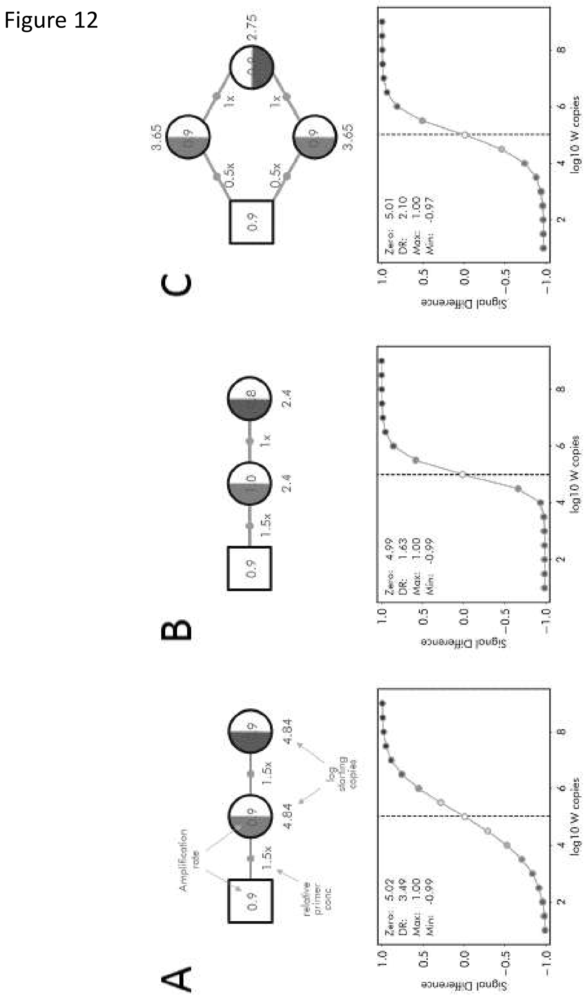

Figure 12: **Simulated outputs for various indirect CAN architectures.** Indirect CANs can be tuned by adjusting parameters of individual components (amplification rate, concentration) or by modifying the connectivity between components. As shown here, indirect CANs can achieve a wide range of dynamic ranges (DR), defined as the WT concentration range between 10% and 90% maximum signal difference.

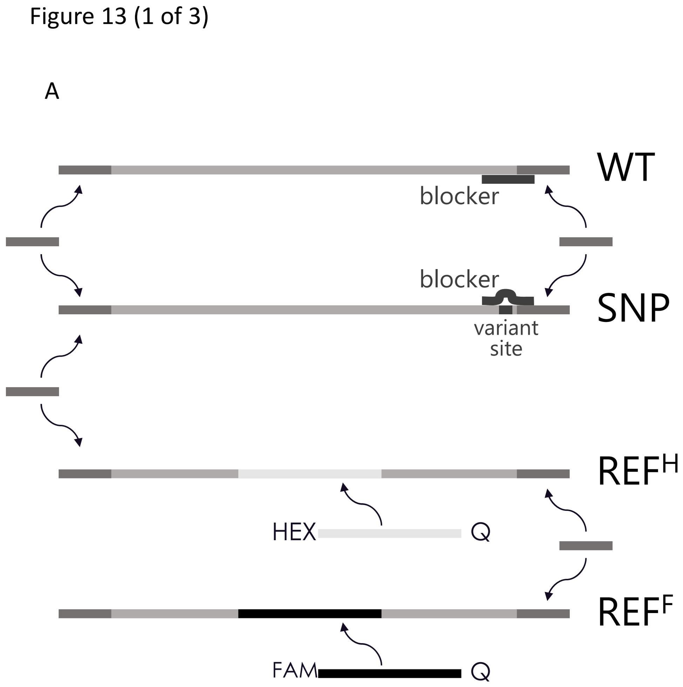

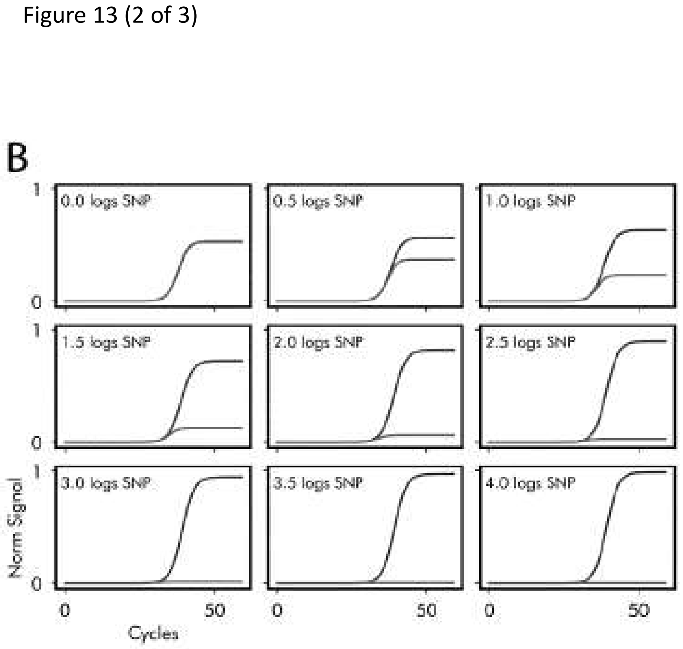

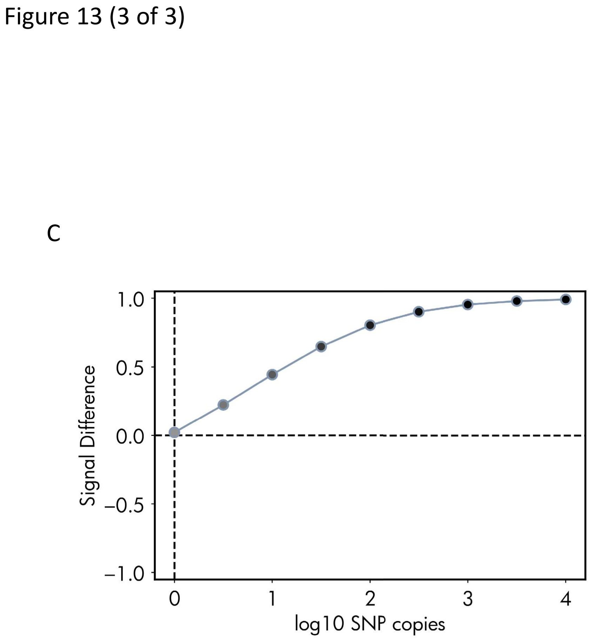

Figure 13: **CAN system for detection of trace cancerous SNPs in ctDNA.** A) An additional competitive mechanism suppresses WT amplification to produce a signal reflective of only the SNP concentration. A blocker oligo (dark purple), which cannot be extended by the polymerase, inhibits replication of the corresponding WT strand owing to its greater affinity for the WT allele than the SNP variant. The ratio of the final colour intensities corresponds to the amount of SNP, even at high WT concentration. B) Individual siumulated HEX and FAM fluorescent traces. C) The difference between fluorescent intensities (FAM-HEX) at the endpoint of the reaction for various concentrations of the SNP in the presence of 105 copies of WT. Multiple distinct mutations can be targeted simultaneously with such a system, so that the total SNP burden in the cfDNA can be estimated from endpoint signal difference.

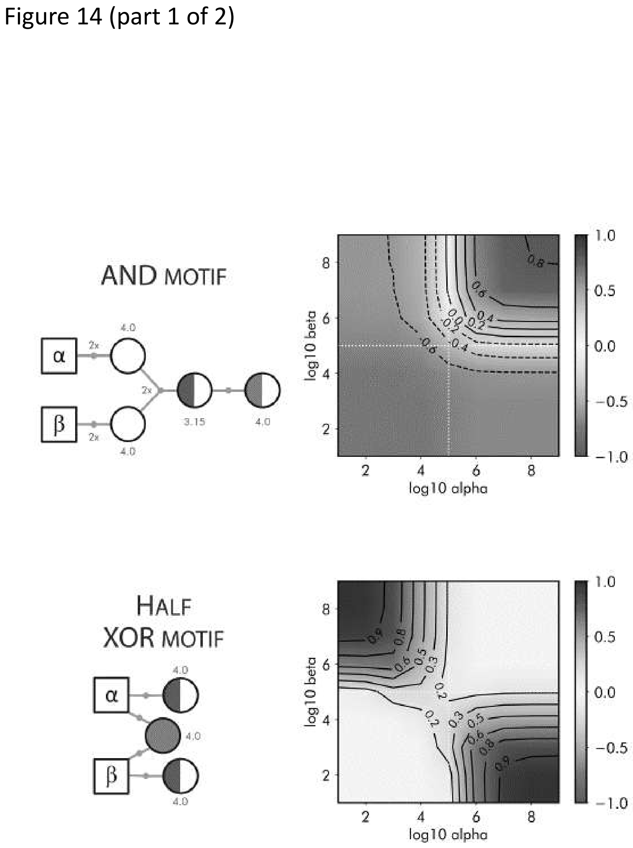

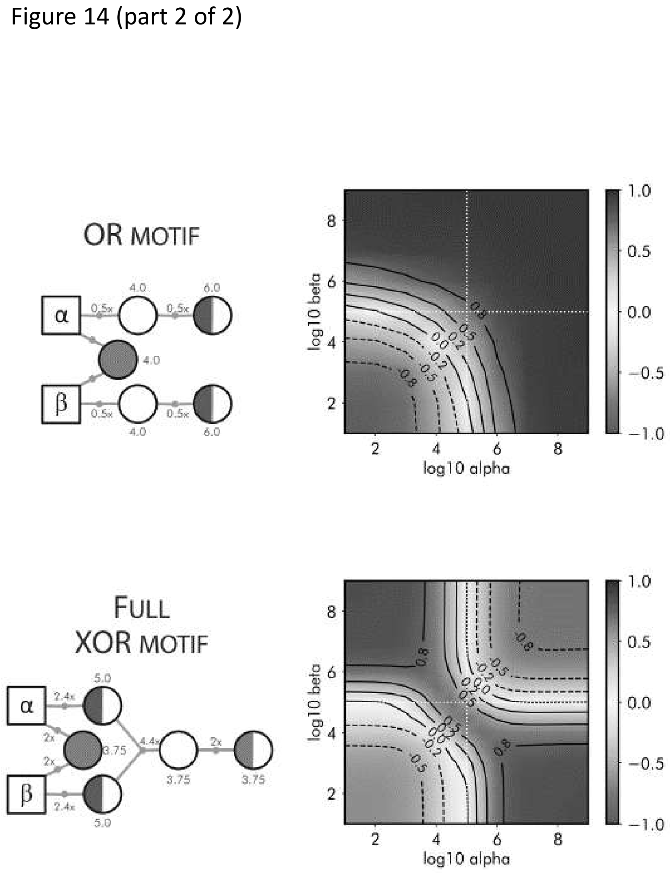

Figure 14: **Higher-order CANs can be designed to approximate Boolean logic.** Indirect competition can recognize combinatorial comprised of patterns of multiple targets. Here, CAN motifs act as Boolean gates, signalling teal/high when the specified condition is true and orange/low when it is false. The "half" XOR is an exception, producing signal parity when false. The full XOR shown here is imperfect, needing further tuning, but demonstrates the rich behaviour possible from higher-order CANs. Tuning network parameters can determine the abruptness and location of the transition regime. Note that the inverse gates, NAND, NOR, and XNOR, can all be obtained by simply swapping the probe labels. Simulated results, all targets are assumed to have a 0.9 amplification rate.

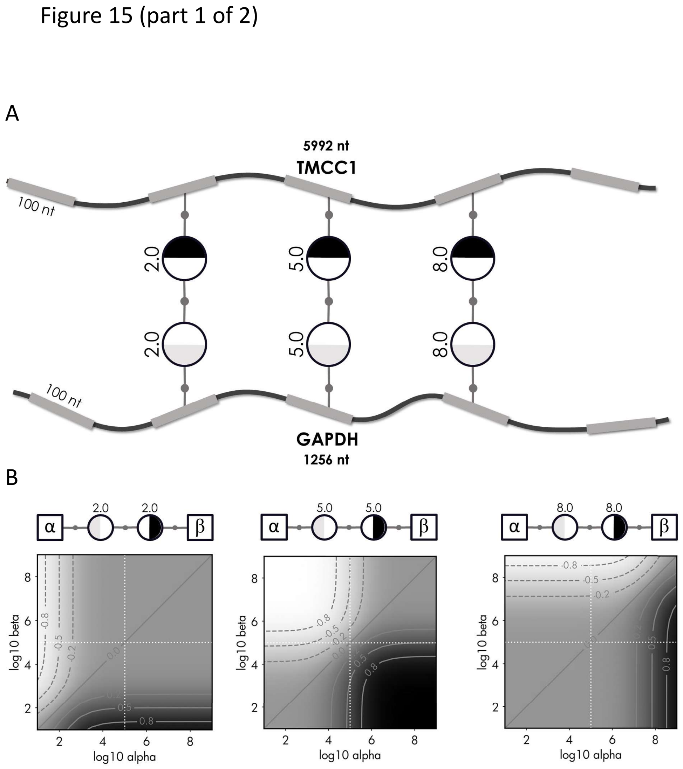

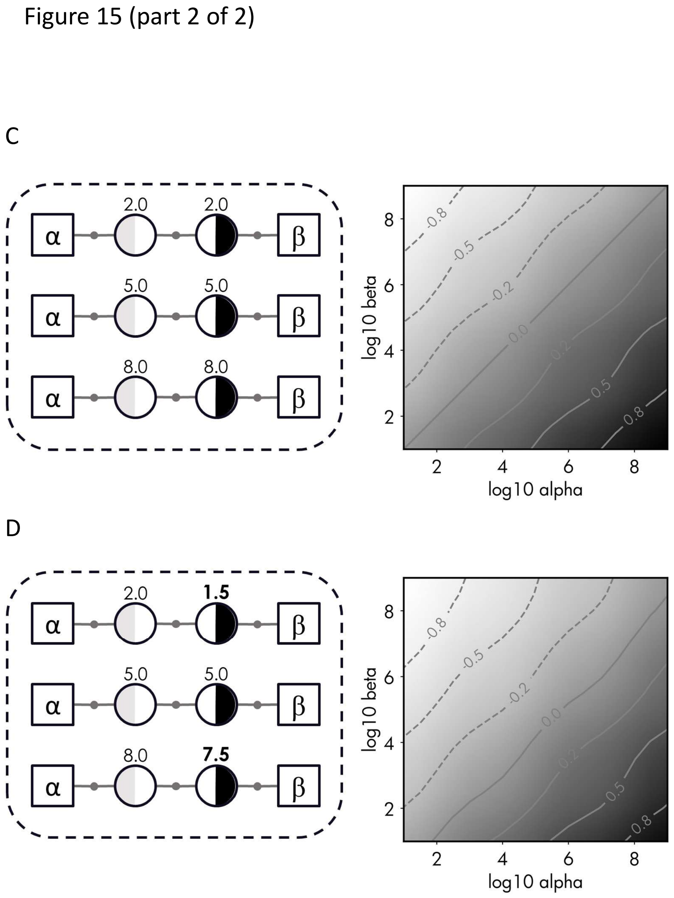

Figure 15: **Redundant targeting allows design of a CAN that reports the relative concentration of two targets, agnostic to their absolute concentrations.** A) Gene transcripts are typically thousands of nucleotides long, which PCR targets are on the order of one hundred nucleotides, implying that multiple PCR targets can be derived from a single transcript. This allows design of independent CAN motifs that each target different regions of the same sequence, such as to compare the concentration of a gene of interest (TMCC1) to a classical "housekeeping" gene (GAPDH). B) The CAN motifs shown here function roughly as comparators, reporting on whether one target is greater than the other, but only within narrow concentration regimes. C) Combining the motifs from B in a single reaction causes their fluorescent outputs to stack, producing a signal proportional to the (log) relative concentration of the two transcripts regardless of how diluted the sample is. D) The signal parity regime can be shifted by tuning the competitor concentrations, so the reaction now determines whether the concentration of alpha is greater or less than 100-fold greater than that of beta.

Figure 16: A) Measured amplification rate and estimated trend across length and GC content for probe-targeted reactions by primer pair. Titles on top row indicate forward and reverse primers used, circles indicate measured values for specific targets at 10^8 copies/reaction. B) Measured amplification rate and estimated trend across length and GC content for dye-targeted reactions by primer pair. Titles on top row indicate forward and reverse primers used, circles indicate measured values for specific targets at 10^8 copies/reaction.

Figure 17: Sequence information.

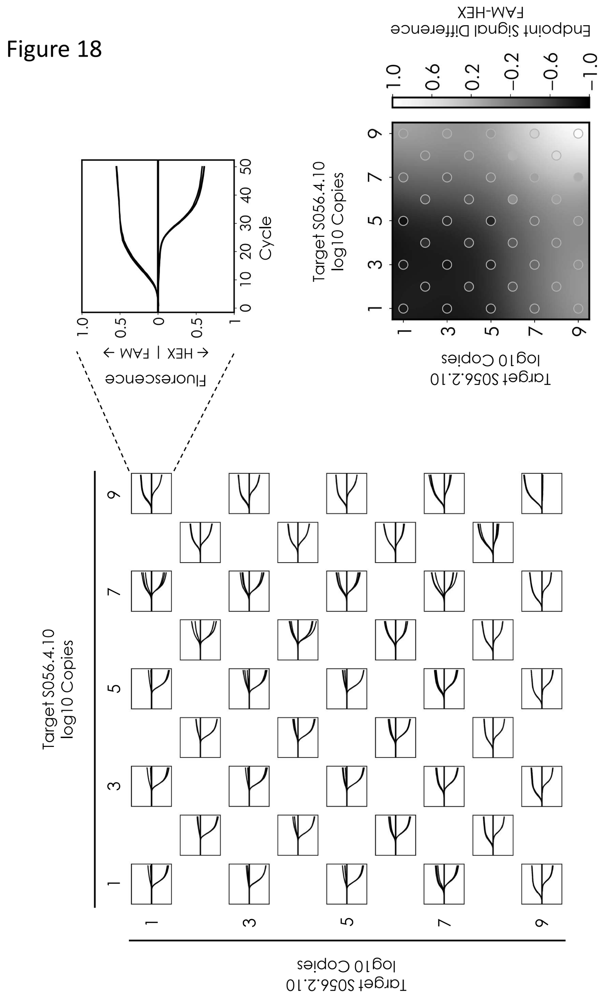

Figure 18: Combining CANs leads to additive behavior. Here, 10^3 copies of S056.2.2 and 10^3 copies of synthetic competitor S056.4.2 were included in every reaction, and two targets S056.2.10 and S056.4.10 were included at the indicated concentration. S056.2.10 shares primers with S056.2.2 and S056.4.10 with S056.4.2; S056.2.10 and S056.4.10 are targeted by FAM probes while S056.2.2 and S056.4.2 are targeted by HEX probes. Thus, this system consists of two CANs with independent endpoint responses to varying target concentration. Given that both CANs share probe fluorophores, their respective fluorescence behaviors combine additively: a greater concentration of either target leads to a stronger FAM and weaker HEX signal. The difference between FAM and HEX intensities at the end of all reactions is summarized in the plot in the lower right: this signal difference reaches a maximum when both targets are at their highest concentration.

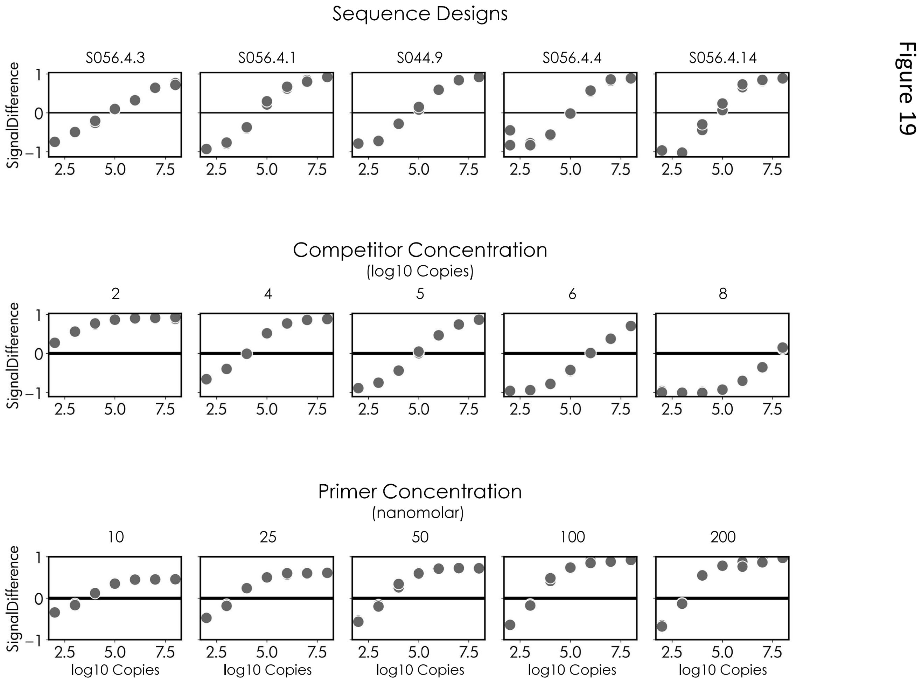

Figure 19: The endpoint response profile of a CAN is tunable by adjusting various components. Shown here are the response profiles of single-competitor CANs. The sharpness of the response can be varied through choice of competitor and wild type sequences. Adjusting the concentration of the competitor shifts the center point of the response profile. Finally, the minimum and maximum extent of the signal response can be constrained through reducing the concentration of the primers.

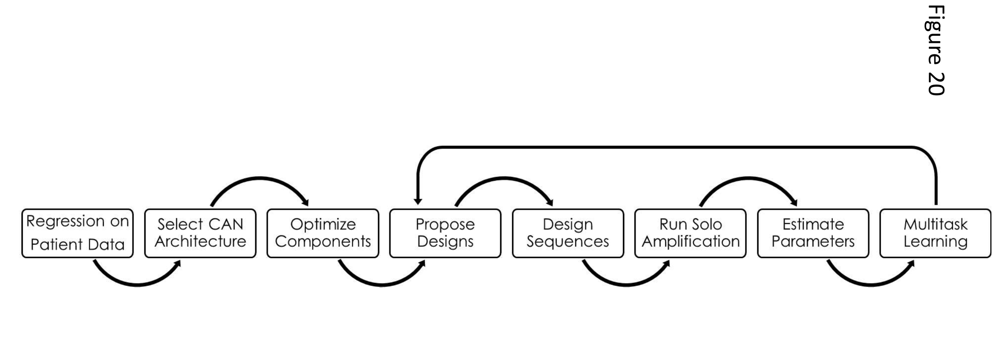

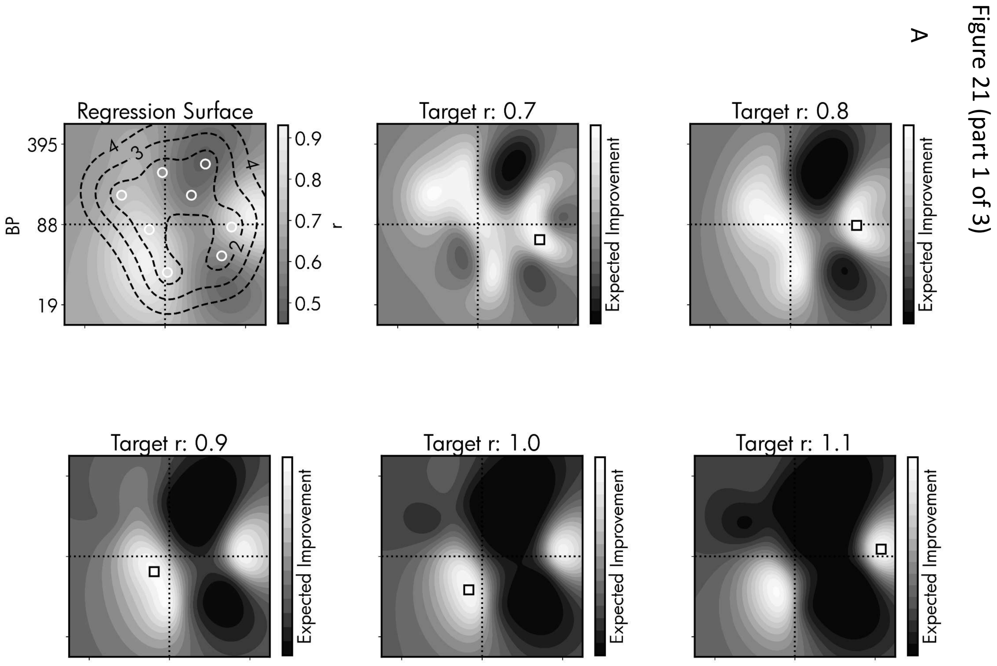

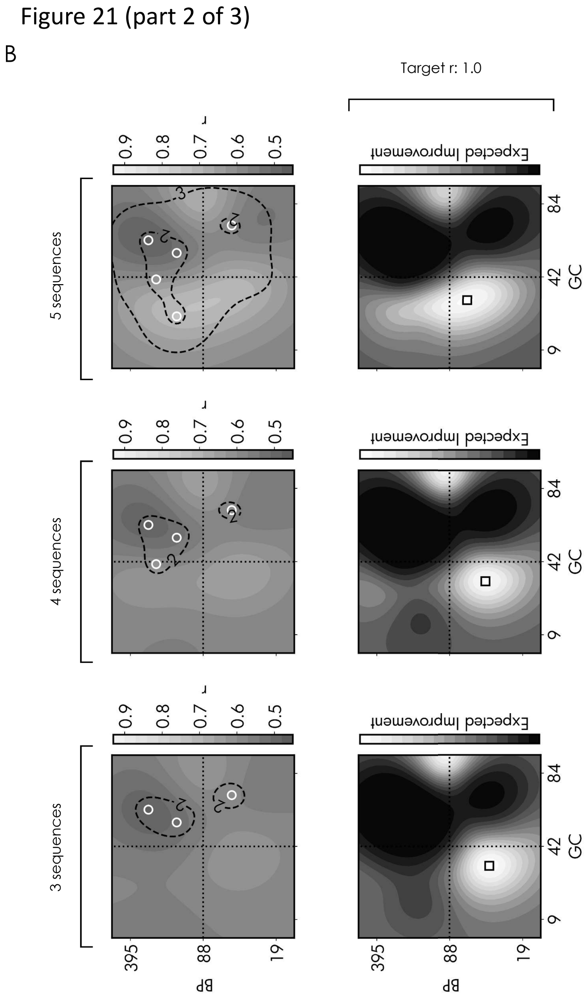

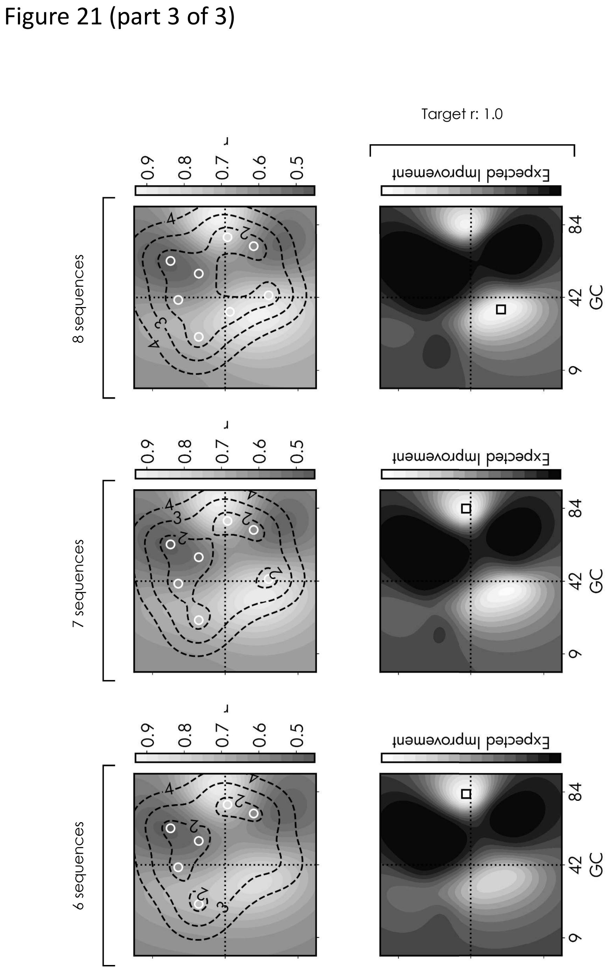

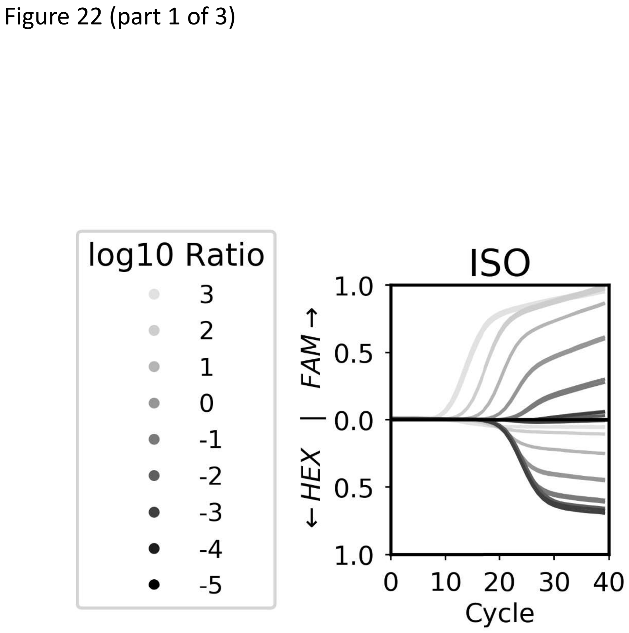

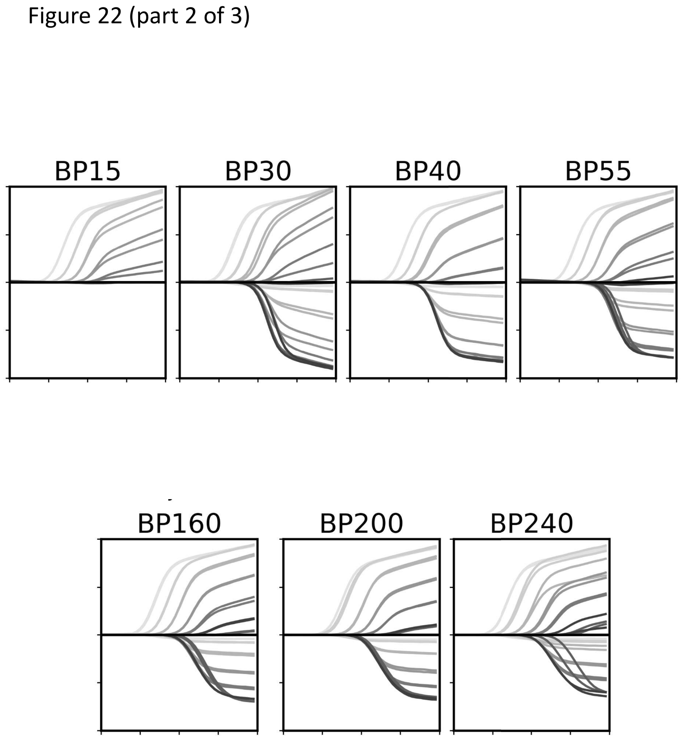

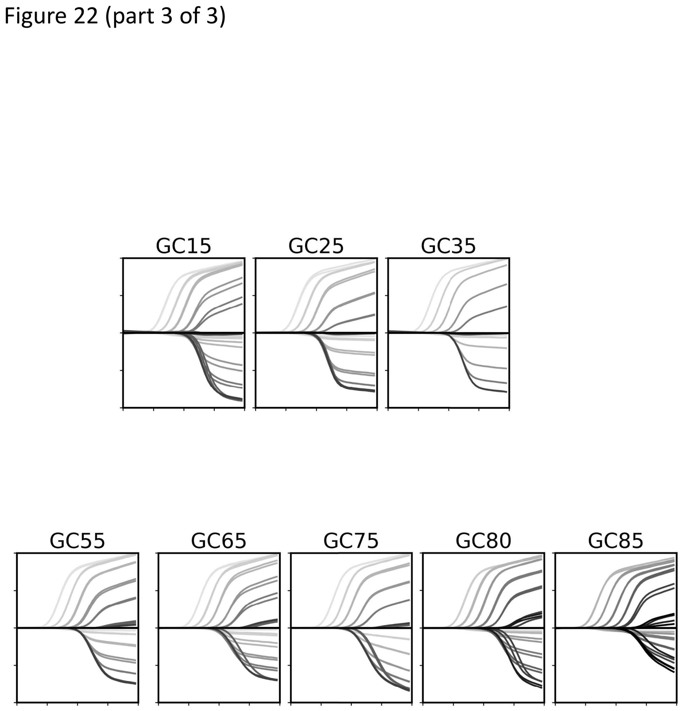

Figure 20: The process of designing a CAN for a specific application. The practitioner begins by performing regression, e.g. logistic regression, on patient data to determine both which gene transcripts to target as well as the appropriate relationship between expression level and diagnostic probability for each transcript. Next, the practitioner selects a CAN architecture, i.e., the number of competitor sequences and the arrangement of shared primers, for each target transcript. The practitioner then computationally determines the ideal components of each CAN module that will optimally recapitulate the patient data regression results, specifically the concentration of each oligonucleotide and the desired amplification behavior. Using previously-acquired data, the practitioner proposes design parameters (length and GC content) for each competitor oligonucleotide, choosing those most likely to result in the desired amplification behavior. These parametric designs can then be used to produce sequence designs, which are obtained, experimentally tested via standard PCR amplification, and analyzed to describe their behavior. These new observations are combined with prior observations in a multitask regression framework, wherein a statistical model learns the empirical relationship between design parameters and each amplification parameter jointly. If further optimization is necessary, this statistical model can be used to propose new sequence designs which, in light of the newly-acquired data, are now the most likely to produce the desired amplification behavior. This process continues until suitable competitor sequences are found that allow recapitulation of the logistic regression results via the CAN reaction.

Figure 21:
Illustration of how regression enables tuning of the competitors to achieve a given target amplification rate r. **A)** A regression surface (far left) is generated, for example through Gaussian Process regression, that relates the two competitor design parameters of length (BP, in nucleotides) and GC content (in percent) to the observed amplification rate, along with the uncertainty in that relationship. Here, observed points (i.e., competitor sequences which have been designed and experimentally tested) are denoted by circles shaded by amplification rate. Filled contours represent the expected amplification rate at each point determined by the regression algorithm, and dashed lines represent iso-uncertainty contours (the square root of the variance returned by the regressor), indicated as a multiple of the standard deviation of all observed r values thus far. From this regression surface, a metric such as Expected Improvement can be calculated that indicates a *new* design likely to display the desired target amplification rate. Shown here are the Expected Improvement surfaces for different targets, lighter shades indicating a higher likelihood of achieving the goal. **B)** The regression surface and expected improvement surfaces, shown here for a target amplification rate of 1.0, change as new sequences are tested and added to the model. In this way, the practitioner can iteratively tune the competitor sequences to achieve the desired amplification rate: i) regression is performed on data obtained thus far, ii) a new design is proposed which has high likelihood of achieving the desired rate, iii) a new sequence based on this design is obtained and experimentally tested, iv) if observed behavior is suboptimal, the regression surface can be updated to incorporate this data, and v) yet another design can be proposed.

Figure 22:

Shown here are the real-time fluorescence traces for competitive amplification reactions between each synthetic amplicon shown in Figure 2 and the "WT" shown in that figure. For each reaction, the competitor is kept at a fixed concentration and the WT is tested at a range of concentrations between 10^2 and 10^8 copies per reaction. The WT is targeted by a probe with the FAM fluorophore; the intensity of this signal is shown on the top half of each panel. The competitor amplicons are targeted by a probe with the HEX fluorophore; the intensity of this signal is shown inverted on the bottom half of each panel. The reactions are color-coded by the log of the relative concentration of the competitor and the WT. A "log10 Ratio" of 3 indicates that there is 1000-fold more WT in the reaction than the respective competitor, and a "log10 Ratio" of -5 implies there is 100000-fold more competitor in the reaction than WT. Note that the BP15 competitor was too short to permit a probe region, so no HEX signal is observed, but the dose-dependent change in endpoint fluorescence signal is still observed. The difference in FAM and HEX signal intensities for each reaction shown here are summarized in Figure 4.

Figure 23:

List of examined sequences, design characteristics, and observed amplification parameters used in this work, any of which may be used as components of any CAN. Each sequence listed here was amplified in using traditional PCR techniques and the resulting fluorescence curves were analyzed as described in this work. The measured parameters F0_Ig, K, r, and m are those that appear in equations 2 and 3, and tau and rho appear in equations 4 and 5. Figure 16 summarizes the findings in this table for the r parameter as determined by Gaussian Process regression, relating the length (BP) and GC content (GC) of each sequence to the observed rate r for each primer pair (denoted "FPname-RPname" on that figure) and reporter (dye or probe). (FP = Forward Primer; RP = Reverse Primer; CO = Competitor oligonucleotide).

Sequence information:

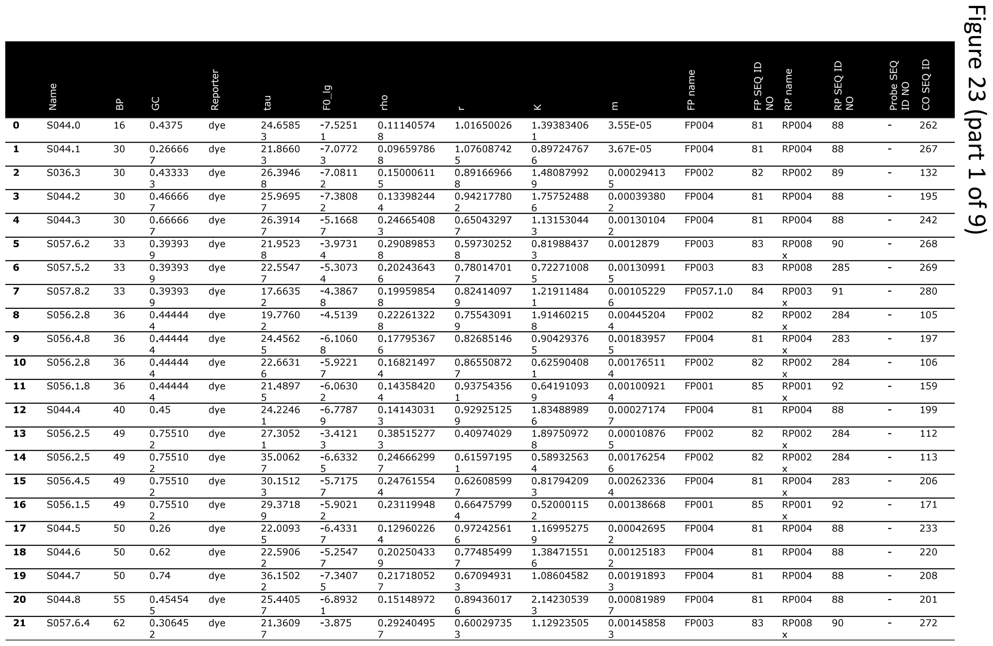

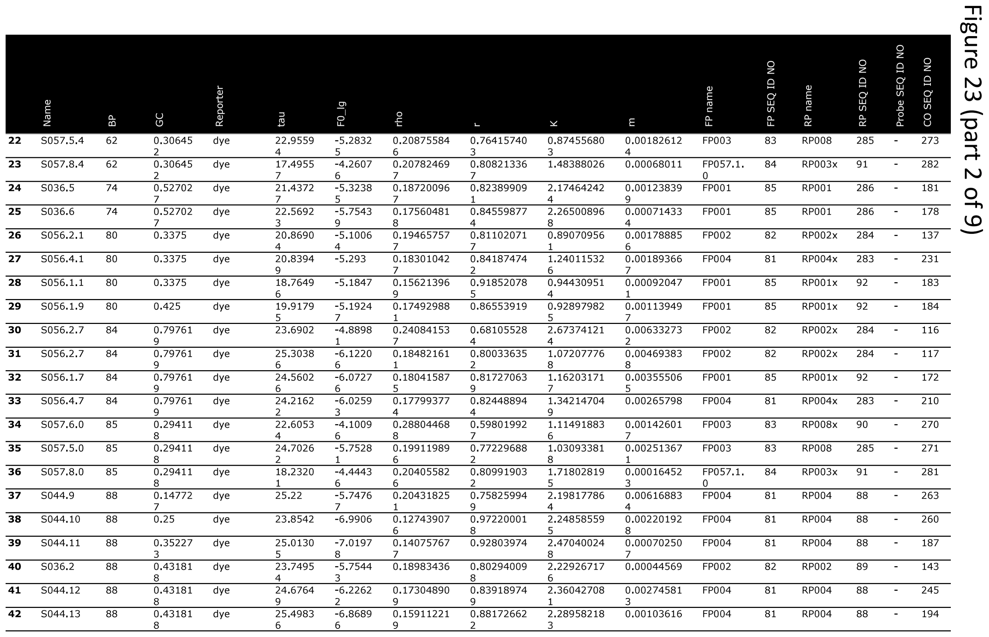

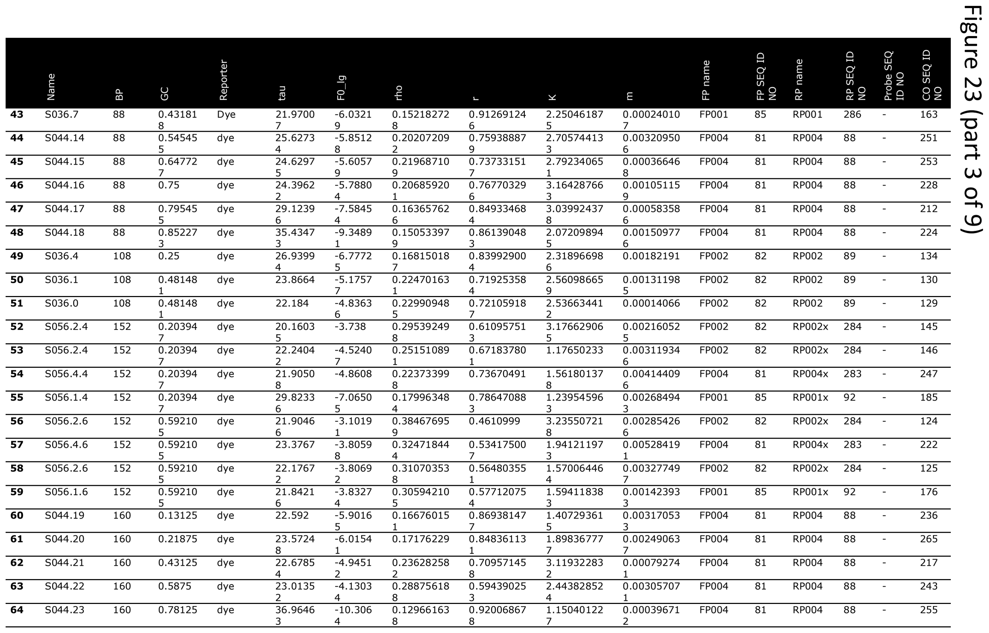

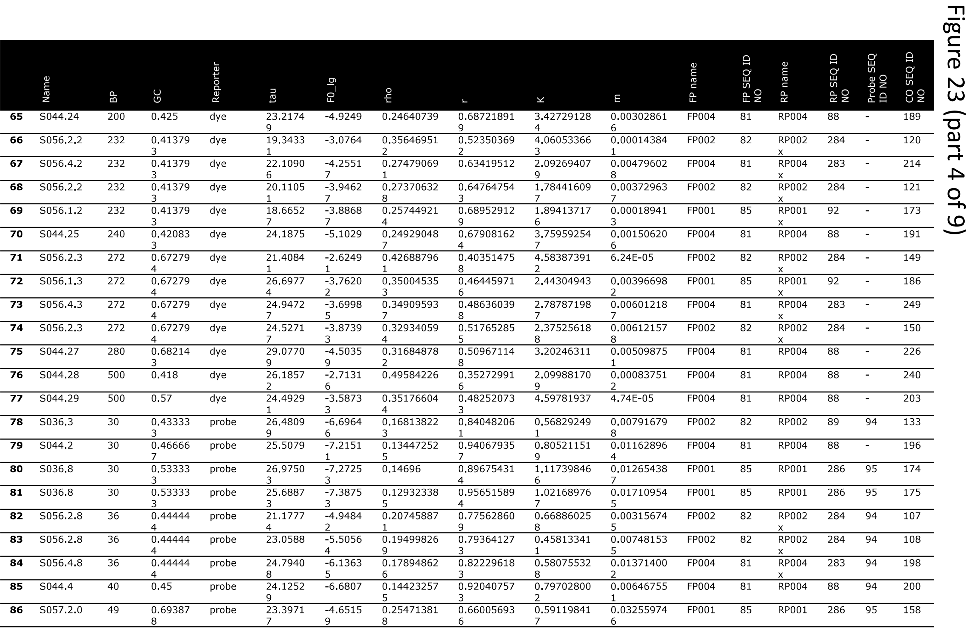

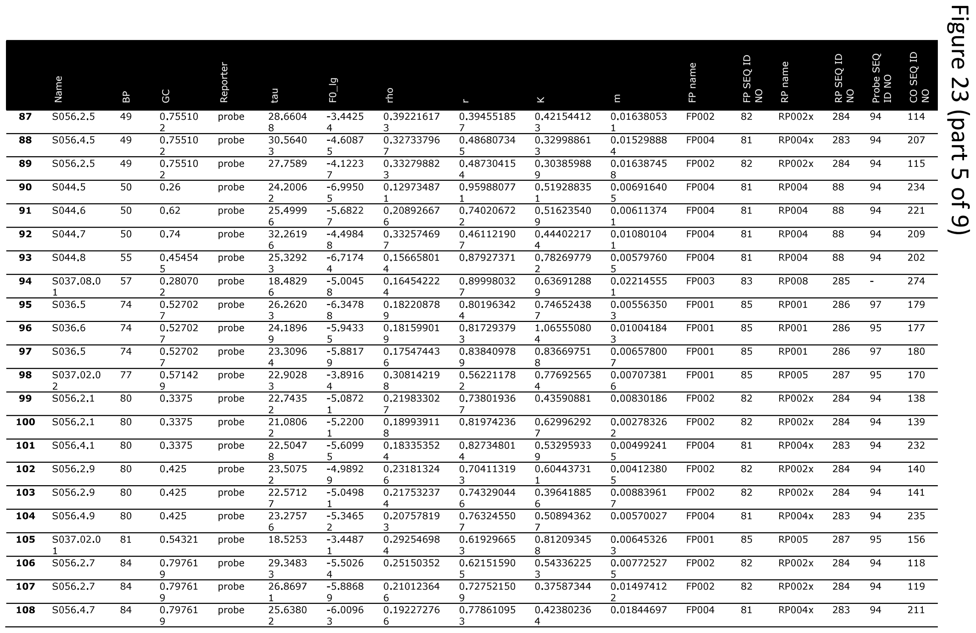

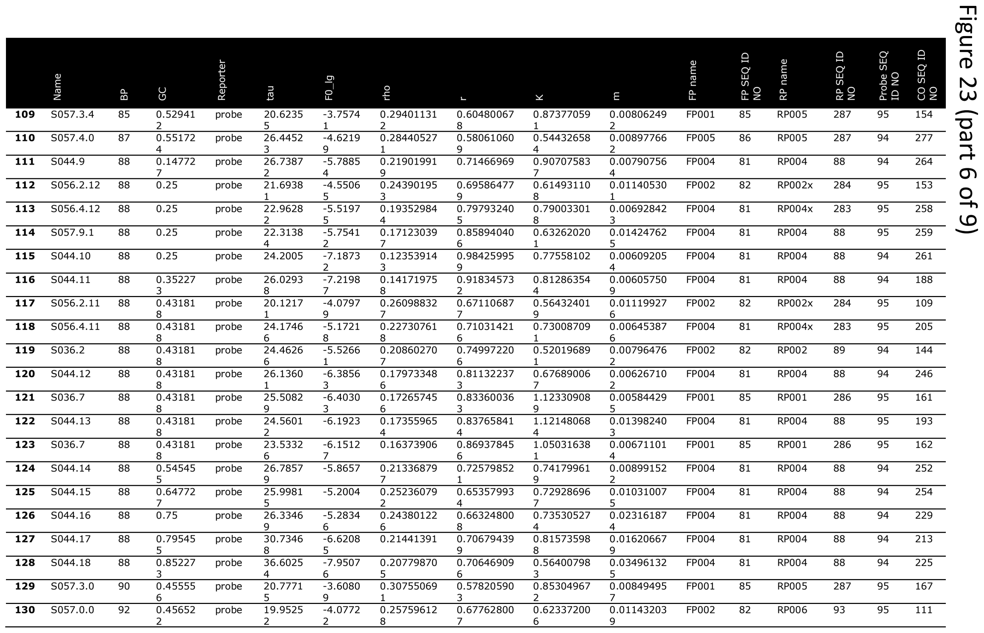

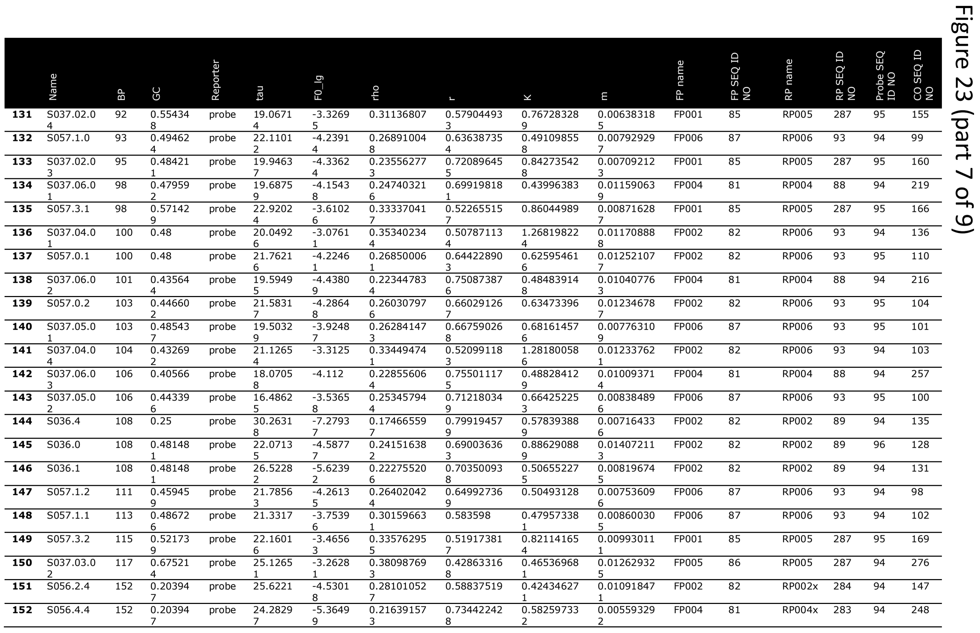

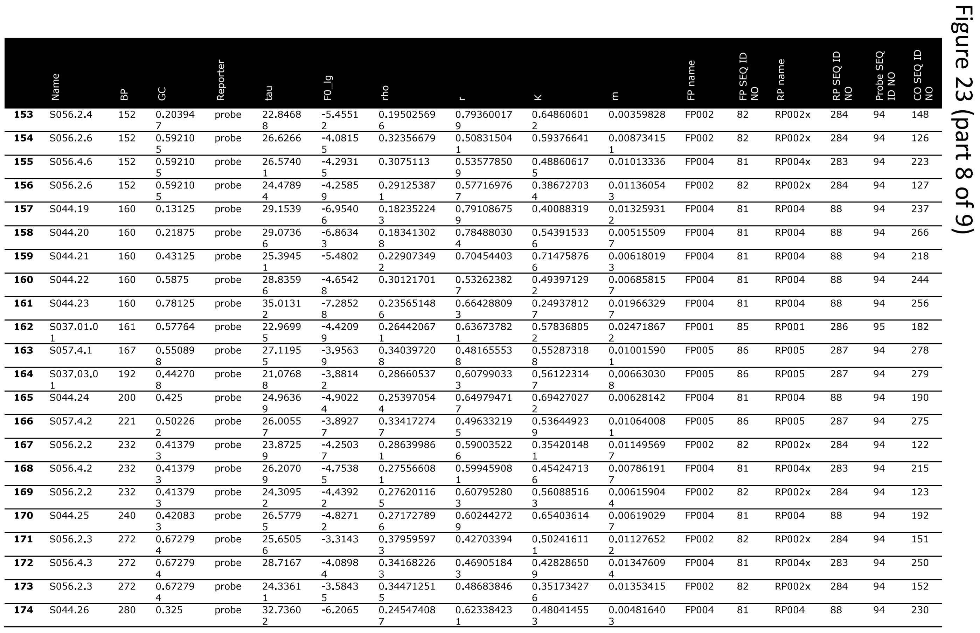

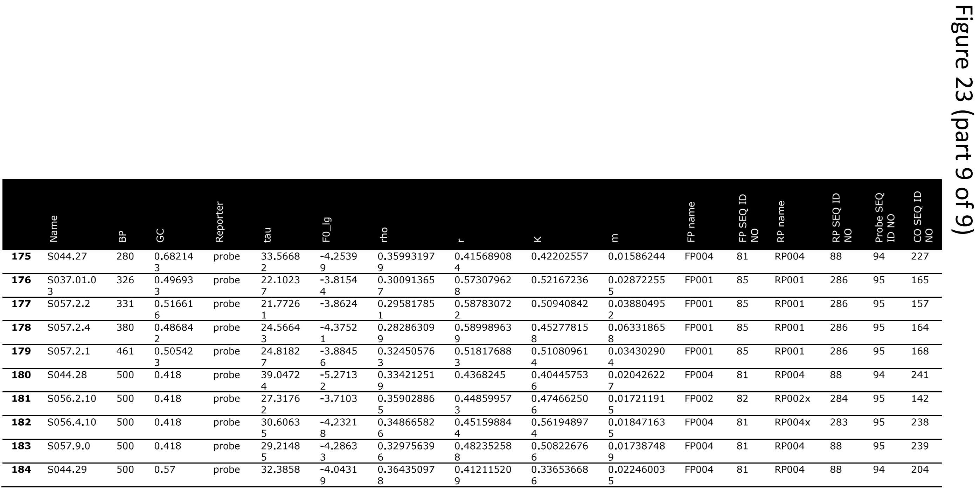

SEQ ID NOs: 1-80 are as set out in Figure 17. SEQ ID NOs: 81-287 are set out in Table 1 below and relate to the oligonucleotides described in Figure 23.

**Table 1**

| SEQ ID NO | Sequence |
|---|---|
| 81 | GCTATTGCTGGGATTTTGAGG |
| 82 | CGCCAAGTCCAGAACCATAG |
| 83 | GGAGAAAAGCCACATGAATGC |
| 84 | TGCAGAAACACTACCTGGTAC |
| 85 | GCAAGAACCAAGACCCTCAG |
| 86 | TCTCTGATCGGTCCCTTTACTC |
| 87 | AGTCAGTGTCAATATCCAAGCG |
| 88 | CATTTGCTTCAACAGTGACTACG |
| 89 | TCCCCATAATCCTTCACATCAC |
| 90 | CTGGAGAGAAACCATACCAATG |
| 91 | CCAAGTTCACCCAGTTTGTG |

(continued)

| SEQ ID NO | Sequence |
|---|---|
| 92 | CAGTGCCTTGTCTGGAGAAT |
| 93 | TAATGTATGTCGGCGGTGTATC |
| 94 | TAGAGAGGTTACCAGAGCGTTGCC |
| 95 | AGCTGTGAGACGAAGGCTTCATGC |
| 96 | AGTTTCTCAAGCAGACCAGCCTTTCTC |
| 97 | CCAGAGTTCCCAGACGATTCCCA |
| 98 | AGTCAGTGTCAATATCCAAGCGCAAATAAAACACAAAACCCCAACTCAAACAAACCACACACCACCAAC CCACCCTCCCTCTACTCCTCTTTCTCTTCTTTTCTGGCAACGCTCTGGTAACCTCTCTAACTCTGATACAC CGCCGACATACATTA |
| 99 | AGTCAGTGTCAATATCCAAGCGCAAATAACCAACAAACAACCCAACCACCCCACCTCCCACTCTCCCTCC TTCTACTTCTCTTCTTGGCAACGCTCTGGTAACCTCTCTAATCACGATACACCGCCGACATACATTA |
| 100 | AGTCAGTGTCAATATCCAAGCGCGAAAAGAGTGAAGATAGTACGTGATTATGGGTCGGGTCCTGGGCT TTCTTACTTCTGCTATGATTTGTACTTTTACGCATGAAGCCTTCGTCTCACAGCTAGTTCGATACACCGCC GACATACATTA |
| 101 | AGTCAGTGTCAATATCCAAGCGTAAGGCCCACCAACATAACCACCCAAAAGATCAAGATTAGTGTGACG TACCTACCCTGAAATGACAGCCGCCTAGCATGAAGCCTTCGTCTCACAGCTGATGAGATACACCGCCGA CATACATTA |
| 102 | AGTCAGTGTCAATATCCAAGCGTAATCAATCTCTCCTACCATCTCCCCTCCTCCCACCTCACCCTCAACC CACAACACACAAACCCCAACCTAACATAAACTCACTGGCAACGCTCTGGTAACCTCTCTAAAACTGATAC ACCGCCGACATACATTA |
| 103 | AGTCAGTGTCAATATCCAAGCGTAATCAATCTCTCCTACCATCTCCCCTCCTCCCACCTCACCCTCAACC CACAACACACAAACCCCAACCTAACATAAACTCACTGGCAACGCTCTGGTAACCTCTCTAAAACTGATAC ACCGCCGACATACATTA |
| 104 | CGCCAAGTCCAGAACCATAGAACAACACCAACAAACCACACACCCCACCACTCATCTCCCTTCTTCCTCT TTCTCTCCTATTTCCTTTACTTTTGCATGAAGCCTTCGTCTCACAGCTCTAAAGATACACCGCCGACATAC ATTA |
| 105 | CGCCAAGTCCAGAACCATAGAGGCAACGCTCTGGTAACCTCTCTAATTGATAGAAGTCCCCATAATCCT TCACATCAC |
| 106 | CGCCAAGTCCAGAACCATAGAGGCAACGCTCTGGTAACCTCTCTAATTGATAGAAGTCCCCATAATCCT TCACATCAC |
| 107 | CGCCAAGTCCAGAACCATAGAGGCAACGCTCTGGTAACCTCTCTAATTGATAGAAGTCCCCATAATCCT TCACATCAC |
| 108 | CGCCAAGTCCAGAACCATAGAGGCAACGCTCTGGTAACCTCTCTAATTGATAGAAGTCCCCATAATCCT TCACATCAC |
| 109 | CGCCAAGTCCAGAACCATAGATCTGTATCCCAAGTGTTCAGACCTTCATATTGCATGAAGCCTTCGTCTC ACAGCTATTGATAGTTCCGATTGCAACTTGACGTCTAGTCCCCATAATCCTTCACATCAC |
| 110 | CGCCAAGTCCAGAACCATAGCAACAGAAAAGAACACGAACAACCAAAACCCACAATAAACACACCTACA ACACCCAACCCCACCTCACCCCGCATGAAGCCTTCGTCTCACAGCTAACAAGATACACCGCCGACATAC ATTA |
| 111 | CGCCAAGTCCAGAACCATAGCCAAAACCAAACACCAACCACAACCTACCCCATCTCTCCCTCTCTTTTCT CCTTTTATTTCCTGCATGAAGCCTTCGTCTCACAGCTAAACAGATACACCGCCGACATACATTA |
| 112 | CGCCAAGTCCAGAACCATAGCCCGCCCCGCCCTGGCAACGCTCTGGTAACCTCTCTAGCCCGCCCCGC CTCCCCATAATCCTTCACATCAC |
| 113 | CGCCAAGTCCAGAACCATAGCCCGCCCCGCCCTGGCAACGCTCTGGTAACCTCTCTAGCCCGCCCCGC CTCCCCATAATCCTTCACATCAC |
| 114 | CGCCAAGTCCAGAACCATAGCCCGCCCCGCCCTGGCAACGCTCTGGTAACCTCTCTAGCCCGCCCCGC CTCCCCATAATCCTTCACATCAC |

(continued)

| SEQ ID NO | Sequence |
|---|---|
| 115 | CGCCAAGTCCAGAACCATAGCCCGCCCCGCCCTGGCAACGCTCTGGTAACCTCTCTAGCCCGCCCCGC CTCCCCATAATCCTTCACATCAC |
| 116 | CGCCAAGTCCAGAACCATAGCGGGCGCGCCGCGCGCGACGCGCGTCCCGTCCGGCAACGCTCTGGTA ACCTCTCTAACCCGCACGCCGGCGACCGCGCGCCCGGTCCCCATAATCCTTCACATCAC |
| 117 | CGCCAAGTCCAGAACCATAGCGGGCGCGCCGCGCGCGACGCGCGTCCCGTCCGGCAACGCTCTGGTA ACCTCTCTAACCCGCACGCCGGCGACCGCGCGCCCGGTCCCCATAATCCTTCACATCAC |
| 118 | CGCCAAGTCCAGAACCATAGCGGGCGCGCCGCGCGCGACGCGCGTCCCGTCCGGCAACGCTCTGGTA ACCTCTCTAACCCGCACGCCGGCGACCGCGCGCCCGGTCCCCATAATCCTTCACATCAC |
| 119 | CGCCAAGTCCAGAACCATAGCGGGCGCGCCGCGCGCGACGCGCGTCCCGTCCGGCAACGCTCTGGTA ACCTCTCTAACCCGCACGCCGGCGACCGCGCGCCCGGTCCCCATAATCCTTCACATCAC |
| 120 | CGCCAAGTCCAGAACCATAGCGGTAATTACTGTTAGACTGGTGGGTATAAACTTCGTTATTTGGATTGG AATTGTTGAGCCCTACCTGACTCTGTATCCCAAGTGTTCTCTGCTTCATATTGGCAACGCTCTGGTAACC TCTCTAAATTGATAGTTCCGATTGCAACTTGACGTCTAGCCCGTATAAATAGCCGGTCTAAACAGCGATG AAATTTCTGTAGAATCAACTAAATTTTCCGTTCAACGGATCCTTCCCCATAATCCTTCACATCAC |
| 121 | CGCCAAGTCCAGAACCATAGCGGTAATTACTGTTAGACTGGTGGGTATAAACTTCGTTATTTGGATTGG AATTGTTGAGCCCTACCTGACTCTGTATCCCAAGTGTTCTCTGCTTCATATTGGCAACGCTCTGGTAACC TCTCTAAATTGATAGTTCCGATTGCAACTTGACGTCTAGCCCGTATAAATAGCCGGTCTAAACAGCGATG AAATTTCTGTAGAATCAACTAAATTTTCCGTTCAACGGATCCTTCCCCATAATCCTTCACATCAC |
| 122 | CGCCAAGTCCAGAACCATAGCGGTAATTACTGTTAGACTGGTGGGTATAAACTTCGTTATTTGGATTGG AATTGTTGAGCCCTACCTGACTCTGTATCCCAAGTGTTCTCTGCTTCATATTGGCAACGCTCTGGTAACC TCTCTAAATTGATAGTTCCGATTGCAACTTGACGTCTAGCCCGTATAAATAGCCGGTCTAAACAGCGATG AAATTTCTGTAGAATCAACTAAATTTTCCGTTCAACGGATCCTTCCCCATAATCCTTCACATCAC |
| 123 | CGCCAAGTCCAGAACCATAGCGGTAATTACTGTTAGACTGGTGGGTATAAACTTCGTTATTTGGATTGG AATTGTTGAGCCCTACCTGACTCTGTATCCCAAGTGTTCTCTGCTTCATATTGGCAACGCTCTGGTAACC TCTCTAAATTGATAGTTCCGATTGCAACTTGACGTCTAGCCCGTATAAATAGCCGGTCTAAACAGCGATG AAATTTCTGTAGAATCAACTAAATTTTCCGTTCAACGGATCCTTCCCCATAATCCTTCACATCAC |
| 124 | CGCCAAGTCCAGAACCATAGGCATGTCGGCTCGGTCTGTCTCTTTCCCCTCATCTCTCGGTACACTCCTT ACCTCGCCCACCCCGGCAACGCTCTGGTAACCTCTCTATCTGGCCTGTCACGAATCACTGTCCATCTAA CCTCGGAGCCCTGTCACGCGGCGGACTTGGAGATCCCCATAATCCTTCACATCAC |
| 125 | CGCCAAGTCCAGAACCATAGGCATGTCGGCTCGGTCTGTCTCTTTCCCCTCATCTCTCGGTACACTCCTT ACCTCGCCCACCCCGGCAACGCTCTGGTAACCTCTCTATCTGGCCTGTCACGAATCACTGTCCATCTAA CCTCGGAGCCCTGTCACGCGGCGGACTTGGAGATCCCCATAATCCTTCACATCAC |
| 126 | CGCCAAGTCCAGAACCATAGGCATGTCGGCTCGGTCTGTCTCTTTCCCCTCATCTCTCGGTACACTCCTT ACCTCGCCCACCCCGGCAACGCTCTGGTAACCTCTCTATCTGGCCTGTCACGAATCACTGTCCATCTAA CCTCGGAGCCCTGTCACGCGGCGGACTTGGAGATCCCCATAATCCTTCACATCAC |
| 127 | CGCCAAGTCCAGAACCATAGGCATGTCGGCTCGGTCTGTCTCTTTCCCCTCATCTCTCGGTACACTCCTT ACCTCGCCCACCCCGGCAACGCTCTGGTAACCTCTCTATCTGGCCTGTCACGAATCACTGTCCATCTAA CCTCGGAGCCCTGTCACGCGGCGGACTTGGAGATCCCCATAATCCTTCACATCAC |
| 128 | CGCCAAGTCCAGAACCATAGGGATTATTGGAGCTCCTTTCTCAAAGGGACAGCCACGAGGAGGGGTGG AAGAAGGCCCTACAGTATTGAGAAAGGCTGGTCTGCTTGAGAAACTTAAAGAACAAGAGTGTGATGTGA AGGATTATGGGGA |
| 129 | CGCCAAGTCCAGAACCATAGGGATTATTGGAGCTCCTTTCTCAAAGGGACAGCCACGAGGAGGGGTGG AAGAAGGCCCTACAGTATTGAGAAAGGCTGGTCTGCTTGAGAAACTTAAAGAACAAGAGTGTGATGTGA AGGATTATGGGGA |
| 130 | CGCCAAGTCCAGAACCATAGGGATTATTGGAGCTCCTTTCTCAAAGGGACAGCCACGAGGAGGGGTGG AAGAAGGCCCTACAGTATTGGCAACGCTCTGGTAACCTCTCTAAATTAAAGAACAAGAGTGTGATGTGA AGGATTATGGGGA |
| 131 | CGCCAAGTCCAGAACCATAGGGATTATTGGAGCTCCTTTCTCAAAGGGACAGCCACGAGGAGGGGTGG AAGAAGGCCCTACAGTATTGGCAACGCTCTGGTAACCTCTCTAAATTAAAGAACAAGAGTGTGATGTGA AGGATTATGGGGA |

(continued)

| SEQ ID NO | Sequence |
|---|---|
| 132 | CGCCAAGTCCAGAACCATAGGGCAACGCTCTGGTAACCTCTCTAAATTAAGTGATGTGAAGGATTATGGGGA |
| 133 | CGCCAAGTCCAGAACCATAGGGCAACGCTCTGGTAACCTCTCTAAATTAAGTGATGTGAAGGATTATGGGGA |
| 134 | CGCCAAGTCCAGAACCATAGTAATTATTATAGCTAATTTCTCAAATTTACAGAAACGAATAGAAGTTTAAGAATTAAATACAGTATTGGCAACGCTCTGGTAACCTCTCTAAATTAAATAACAATAATGTGATGTGAAGGATTATGGGGA |
| 135 | CGCCAAGTCCAGAACCATAGTAATTATTATAGCTAATTTCTCAAATTTACAGAAACGAATAGAAGTTTAAGAATTAAATACAGTATTGGCAACGCTCTGGTAACCTCTCTAAATTAAATAACAATAATGTGATGTGAAGGATTATGGGGA |
| 136 | CGCCAAGTCCAGAACCATAGTACAAAGCACGATCGAGAACAGGGCAGGTAGATTGAACGAGATGGGGAATGATGGACGGATAAATGGGACTGGCAACGCTCTGGTAACCTCTCTAACATTGATACACCGCCGACATACATTA |
| 137 | CGCCAAGTCCAGAACCATAGTAGCAATATTGAATTCTAGATTATACGAGGCAACGCTCTGGTAACCTCTCTATTATTTAAGCTATCATACTCTAGTGTTTTCCCCATAATCCTTCACATCAC |
| 138 | CGCCAAGTCCAGAACCATAGTAGCAATATTGAATTCTAGATTATACGAGGCAACGCTCTGGTAACCTCTCTATTATTTAAGCTATCATACTCTAGTGTTTTCCCCATAATCCTTCACATCAC |
| 139 | CGCCAAGTCCAGAACCATAGTAGCAATATTGAATTCTAGATTATACGAGGCAACGCTCTGGTAACCTCTCTATTATTTAAGCTATCATACTCTAGTGTTTTCCCCATAATCCTTCACATCAC |
| 140 | CGCCAAGTCCAGAACCATAGTATCCCAAGTGTTCTCTGCTTCATATTGGCAACGCTCTGGTAACCTCTCTAAATTGATAGTTCCGATTGCAACTTGACGTTCCCCATAATCCTTCACATCAC |
| 141 | CGCCAAGTCCAGAACCATAGTATCCCAAGTGTTCTCTGCTTCATATTGGCAACGCTCTGGTAACCTCTCTAAATTGATAGTTCCGATTGCAACTTGACGTTCCCCATAATCCTTCACATCAC |
| 142 | CGCCAAGTCCAGAACCATAGTCATAGTTCTATATACATTGTCATGACACGAATTGGCTGAAAACGGTTGATAGAAGATATTGACTATATTCTCTTCCGCTGTATCCCGTTTCTTTTGGAAATTGACCGTATTATGGTCACCATCAGCCTAAGTGATCTCTGGACCGTCGAGAGACCCCATTGACTTGGTTCTTCGGTTTGATGCACTCATGTAAAATGTAGTCTCAATCAATACCATCCATTTCTAGCATACGGGTGAGCATGAAGCCTTCGTCTCACAGCTCCGGTACAGGTAATCGAGAGAACACTAAAACAGTCCGACATGAGATTCATTAAAAACCTATTTTCACCAATCGGTAGAACGGTTATGCGCAAAATATTTTCGGGGTCCACAGTGCACCTATGTAATCTGTAACATGAAGTTGTACGAAAATAGAGAACCCACCCAGCTTATCTAGGAAATTGATCTCTTCGATTTAAGGATGTGTCGACACGTATCATGCCAAGTGATCAGAGGCGTAATCCCCATAATCCTTCACATCAC |
| 143 | CGCCAAGTCCAGAACCATAGTCTGTATCCCAAGTGTTCAGAGCTTCATATTGGCAACGCTCTGGTAACCTCTCTAAATTGATAGTTCCGATTGCAACTTGACGTCTAGGTGATGTGAAGGATTATGGGGA |
| 144 | CGCCAAGTCCAGAACCATAGTCTGTATCCCAAGTGTTCAGAGCTTCATATTGGCAACGCTCTGGTAACCTCTCTAAATTGATAGTTCCGATTGCAACTTGACGTCTAGGTGATGTGAAGGATTATGGGGA |
| 145 | CGCCAAGTCCAGAACCATAGTGAATATTTTATTCCCTAATTTTATTATTATGTTCTAAAAGGTATTTAAATACTTTTCATTAATGGCAACGCTCTGGTAACCTCTCTATCATAAATATTTTAAATACTAGAATCTTATTTTATTCTTTAGAATAGTAGAAATTTAATTAAATTCCCCATAATCCTTCACATCAC |
| 146 | CGCCAAGTCCAGAACCATAGTGAATATTTTATTCCCTAATTTTATTATTATGTTCTAAAAGGTATTTAAATACTTTTCATTAATGGCAACGCTCTGGTAACCTCTCTATCATAAATATTTTAAATACTAGAATCTTATTTTATTCTTTAGAATAGTAGAAATTTAATTAAATTCCCCATAATCCTTCACATCAC |
| 147 | CGCCAAGTCCAGAACCATAGTGAATATTTTATTCCCTAATTTTATTATTATGTTCTAAAAGGTATTTAAATACTTTTCATTAATGGCAACGCTCTGGTAACCTCTCTATCATAAATATTTTAAATACTAGAATCTTATTTTATTCTTTAGAATAGTAGAAATTTAATTAAATTCCCCATAATCCTTCACATCAC |
| 148 | CGCCAAGTCCAGAACCATAGTGAATATTTTATTCCCTAATTTTATTATTATGTTCTAAAAGGTATTTAAATACTTTTCATTAATGGCAACGCTCTGGTAACCTCTCTATCATAAATATTTTAAATACTAGAATCTTATTTTATTCTTTAGAATAGTAGAAATTTAATTAAATTCCCCATAATCCTTCACATCAC |

(continued)

| SEQ ID NO | Sequence |
|---|---|
| 149 | CGCCAAGTCCAGAACCATAGTGCCGAGGGTCCAGGTCGAGACTCCATCCCGAGGCGTGTGTCCCCATG GCCGTCCTCCAGGCTAGTACTGTGCCCCGTCGCCGTCGCACAAGGCCGGTCGATCGTGGTGGCTGTCA GGCGGGGGTGGCAACGCTCTGGTAACCTCTCTACGGCGTAGTAGTTCGTGCCCCTCCCCTTGCGACCTC CCGCTACCACCCGTCACTCCCCGGTAAGAGGCTCTCACGGACGGCAGAGTCGGTCGCGCGCTCCGGAT GTGGTCCCCTCCCAGTCCTCTCCCCATAATCCTTCACATCAC |
| 150 | CGCCAAGTCCAGAACCATAGTGCCGAGGGTCCAGGTCGAGACTCCATCCCGAGGCGTGTGTCCCCATC GCCGTCCTCCAGGCTAGTACTGTGCCCCGTCGCCGTCGCACAAGGCCGGTCGATCGTGGTGGCTGTCA GGCGGGGGTGGCAACGCTCTGGTAACCTCTCTACGGCGTAGTAGTTCGTGCCCCTCCCCTTGCGACCTC CCGCTACCACCCGTCACTCCCCGGTAAGAGGCTCTCACGGACGGCAGAGTCGGTCGCGCGCTCCGGA GTGGTCCCCTCCCAGTCCTCTCCCCATAATCCTTCACATCAC |
| 151 | CGCCAAGTCCAGAACCATAGTGCCGAGGGTCCAGGTCGAGACTCCATCCCGAGGCGTGTGTCCCCATG GCCGTCCTCCAGGCTAGTACTGTGCCCCGTCGCCGTCGCACAAGGCCGGTCGATCGTGGTGGCTGTCA GGCGGGGGTGGCAACGCTCTGGTAACCTCTCTACGGCGTAGTAGTTCGTGCCCCTCCCCTTGCGACCTC CCGCTACCACCCGTCACTCCCCGGTAAGAGGCTCTCACGGACGGCAGAGTCGGTCGCGCGCTCCGGAT GTGGTCCCCTCCCAGTCCTCTCCCCATAATCCTTCACATCAC |
| 152 | CGCCAAGTCCAGAACCATAGTGCCGAGGGTCCAGGTCGAGACTCCATCCCGAGGCGTGTGTCCCCATG GCCGTCCTCCAGGCTAGTACTGTGCCCCGTCGCCGTCGCACAAGGCCGGTCGATCGTGGTGGCTGTCA GGCGGGGGTGGCAACGCTCTGGTAACCTCTCTACGGCGTAGTAGTTCGTGCCCCTCCCCTTGCGACCTC CCGCTACCACCCGTCACTCCCCGGTAAGAGGCTCTCACGGACGGCAGAGTCGGTCGCGCGCTCCGGAT GTGGTCCCCTCCCAGTCCTCTCCCCATAATCCTTCACATCAC |
| 153 | CGCCAAGTCCAGAACCATAGTGTAATATTAACAAGTAATAAAGAAATATATAGCATGAAGCCTTCGTCTC ACAGCTTTTATTCAATTTAATGATTACCTTTATTATCTTCCCCATAATCCTTCACATCAC |
| 154 | GCAAGAACCAAGACCCTCAGAAACACCAACCCAACAACACAAACCCGCAACCTAAAACCACCACAACTC CCTCCTCGCATGAAGCCTTCGTCTCACAGCTCCCCACCCCTTAATTTCCGCACCTATT |
| 155 | GCAAGAACCAAGACCCTCAGACACGACTCCCCGCCACAACCACACAATCCACTACCTGCCCACATCCTA ACCCTACCCTTCCTGCATGAAGCCTTCGTCTCACAGCTCTAGTCCCCTTAATTTCCGCACCTATT |
| 156 | GCAAGAACCAAGACCCTCAGACCAAACGCAACAACACAGACACCACAACTACCACTCACCCCAACTCCA ACCGCATGAAGCCTTCGTCTCACAGCTCTCCGCCCCTTAATTTCCGCACCTATT |
| 157 | GCAAGAACCAAGACCCTCAGACCAACAACCGCCAACTACAACGACACCAGAGCACACCCATATACATCA CCCCTTCCCCTATTTCTCTTCCGCTCCTTTCTTTCCGTCTGTTTCCCGCTGCTTTTCTGTCTCGCCCTAAT CCACCAAACCCGCCCACTCCAATATCCTACCTTCTTCACCTTGCCTGTACCGATGACTTTGCCCGAATAA TCTACTCTCCTAACCTGCACCCGACTCAACTCCTCATCTATCCCAACGCCGTCACTTCCTCCATACCTCTA CCATCCAACCCCACGACCCACCTACACAGATACCCAAATCCGCATGAAGCCTTCGTCTCACAGCTTATGT CAGTGCCTTGTCTGGAGAAT |
| 158 | GCAAGAACCAAGACCCTCAGACCGCCGCCCACCCCTCCCCGCATGAAGCCTTCGTCTCACAGCTCGCG TCAGTGCCTTGTCTGGAGAAT |
| 159 | GCAAGAACCAAGACCCTCAGAGGCAACGCTCTGGTAACCTCTCTAATTGATAGAAGATTCTCCAGACAA GGCACTG |
| 160 | GCAAGAACCAAGACCCTCAGATCCATTACCCAGATTGAGCTATTTACGACGACAACACATCCACATTCTA CCTGACCCACTACCGCGCATGAAGCCTTCGTCTCACAGCTTCGATCCCCTTAATTTCCGCACCTATT |
| 161 | GCAAGAACCAAGACCCTCAGATCTGTATCCCAAGTGTTCAGACCTTCATATTGCATGAAGCCTTCGTCTC ACAGCTATTGATAGTTCCGATTGCAACTTGACGTCTAGCAGTGCCTTGTCTGGAGAAT |
| 162 | GCAAGAACCAAGACCCTCAGATCTGTATCCCAAGTGTTCAGACCTTCATATTGCATGAAGCCTTCGTCTC ACAGCTATTGATAGTTCCGATTGCAACTTGACGTCTAGCAGTGCCTTGTCTGGAGAAT |
| 163 | GCAAGAACCAAGACCCTCAGATCTGTATCCCAAGTGTTCAGACCTTCATATTGCATGAAGCCTTCGTCTC ACAGCTATTGATAGTTCCGATTGCAACTTGACGTCTAGCAGTGCCTTGTCTGGAGAAT |
| 164 | GCAAGAACCAAGACCCTCAGCACCACCATCCCCACCTCCCACTCTACTCCACGCCTCAATTCCGACTAC CACTACGCCATTTCCCCTCTTCCATTCACTGTCCTTTCTCTCCTTATCCTGCTCCTCTGTCTCTTTTATTCT TTCCTTCCCTTTATCTCCCGTTACTTGCACTTTACCTATCCGAACCCACACATACCCCTGCCAAAACCCCA ACCTAAAACGAACACCCAAACAAAGCCACAATACAACACACCAACATAACAACCCGCACTCCCTAATATC ACCTTGCCCTCCTACTAACCTCATCATCTACCCGTCCGCTCTAACACTAATCACACTTACATCTGCCCGC CCCTTACCCTAGAAAACTCGCATGAAGCCTTCGTCTCACAGCTATTTTCAGTGCCTTGTCTGGAGAAT |

(continued)

| SEQ ID NO | Sequence |
|---|---|
| 165 | GCAAGAACCAAGACCCTCAGCCACCCTAAATCTCCGCACAGGCATTCACGACGATATACGGAAACAGCA CAAGTGGCACGCGGGAAGGTCATCAGTTACAGTCATGGTCAGGGTTAGTAGGTTGGGTAGGAGGGAAA TTGGACAGATTAACGAGGGCAGATCAGAGAAACGTGCATACTCTACTCCACACAACTTCCGACGCTTAG ATAACCACGCAACCCCGAATTTACTACAATAACTCTCCTTTCACCTAGCCATTCCTCCCCTATTCAGTCCT AGTCGCTAAAGTTCCCATCCCCGCATAGTTGAGTGTTGTTGCATGAAGCCTTCGTCTCACAGCTCGCGT CAGTGCCTTGTCTGGAGAAT |
| 166 | GCAAGAACCAAGACCCTCAGCCCAAACACAACACCACCAACCACACCCGCCCTCCCACTTCCCTTCTCC TTTCCCCTATCTTACCCTACGCATGAAGCCTTCGTCTCACAGCTCCCCACCCCTTAATTTCCGCACCTATT |
| 167 | GCAAGAACCAAGACCCTCAGCCCATACCCCACCCCTCCACTCCTCCTTCCTTTATTTTCGTTTCTCTGTTT TGTATTTGTTGCATGAAGCCTTCGTCTCACAGCTCTTTTCCCCTTAATTTCCGCACCTATT |
| 168 | GCAAGAACCAAGACCCTCAGCCCATCCCAGAAACAAGTTACGCGACAGTGAGGAGAGAGCCAAGTATA AGTAAGCAGATCCGTCCATTCAAGCGTCAGAGTCCCGTGCCATTGTTCCCTTCCTATACCCTTGCCACTA CTTTCTCGCTCCCATATTTCTACAGGTGCATCGTACTTCTTTATGCCGCGTTACTGTTCACTCTTTTCCTT AGGCTAGATCGGAACTCGCAACAAAACTAATCACAAACGGGCAAAGGGGATACGGACACTGGAATAAG ACTACACGCCGACTTGATGAAAGCTACTCCACACGACACAACCTCCTAAACCGACCACCGCCACCAACA CACCATCACCCAACCACTCAAAATCCCTACCCGTACCTGAGAGTAAAACCAGCGCCAAATCGACCTCAA CCCACCTAACACCCCTATCCATACCGTAAAGCCCTCCGCATGAAGCCTTCGTCTCACAGCTCGGGTCAG TGCCTTGTCTGGAGAAT |
| 169 | GCAAGAACCAAGACCCTCAGCCCCGACACAAAATAAAACCACACCAAACACCCAACAACCCCACATCCC ACCACCTCCCTACCCACTACCACTCCTCTCTAAACCCGCATGAAGCCTTCGTCTCACAGCTCTTTTCCCC TTAATTTCCGCACCTATT |
| 170 | GCAAGAACCAAGACCCTCAGCCCCGCCCGTAACACTCAGACCTAACTAAACCGAGCACCACACAACCC GCATGAAGCCTTCGTCTCACAGCTCCCATCCCCTTAATTTCCGCACCTATT |
| 171 | GCAAGAACCAAGACCCTCAGCCCGCCCCGCCCTGGCAACGCTCTGGTAACCTCTCTAGCCCGCCCCGC CATTCTCCAGACAAGGCACTG |
| 172 | GCAAGAACCAAGACCCTCAGCGGGCGCGCCGCGCGCGACGCGCGTCCCGTCCGGCAACGCTCTGGTA ACCTCTCTAACCCGCACGCCGGCGACCGCGCGCCCGGATTCTCCAGACAAGGCACTG |
| 173 | GCAAGAACCAAGACCCTCAGCGGTAATTACTGTTAGACTGGTGGGTATAAACTTCGTTATTTGGATTGG AATTGTTGAGCCCTACCTGACTCTGTATCCCAAGTGTTCTCTGCTTCATATTGGCAACGCTCTGGTAACC TCTCTAAATTGATAGTTCCGATTGCAACTTGACGTCTAGCCCGTATAAATAGCCGGTCTAAACAGCGATG AAATTTCTGTAGAATCAACTAAATTTTCCGTTCAACGGATCCTATTCTCCAGACAAGGCACTG |
| 174 | GCAAGAACCAAGACCCTCAGGCATGAAGCCTTCGTCTCACAGCTCGTGATCAGTGCCTTGTCTGGAGAA T |
| 175 | GCAAGAACCAAGACCCTCAGGCATGAAGCCTTCGTCTCACAGCTCGTGATCAGTGCCTTGTCTGGAGAA T |
| 176 | GCAAGAACCAAGACCCTCAGGCATGTCGGCTCGGTCTGTCTCTTTCCCCTCATCTCTCGGTACACTCCTT ACCTCGCCCACCCCGGCAACGCTCTGGTAACCTCTCTATCTGGCCTGTCACGAATCACTGTCCATCTAA CCTCGGAGCCCTGTCACGCGGCGGACTTGGAGAATTCTCCAGACAAGGCACTG |
| 177 | GCAAGAACCAAGACCCTCAGGGAGGGAATCACAGTCACGCATGAAGCCTTCGTCTCACAGCTCGTGAC TTATGTAGAGGCCATCAACAGTGGAGCAGTGCCTTGTCTGGAGAAT |
| 178 | GCAAGAACCAAGACCCTCAGGGAGGGAATCACAGTCACGCATGAAGCCTTCGTCTCACAGCTCGTGAC TTATGTAGAGGCCATCAACAGTGGAGCAGTGCCTTGTCTGGAGAAT |
| 179 | GCAAGAACCAAGACCCTCAGGGAGGGAATCACAGTCACTGGGAATCGTCTGGGAACTCTGGCAGTGAC TTATGTAGAGGCCATCAACAGTGGAGCAGTGCCTTGTCTGGAGAAT |
| 180 | GCAAGAACCAAGACCCTCAGGGAGGGAATCACAGTCACTGGGAATCGTCTGGGAACTCTGGCAGTGAC TTATGTAGAGGCCATCAACAGTGGAGCAGTGCCTTGTCTGGAGAAT |
| 181 | GCAAGAACCAAGACCCTCAGGGAGGGAATCACAGTCACTGGGAATCGTCTGGGAACTCTGGCAGTGAC TTATGTAGAGGCCATCAACAGTGGAGCAGTGCCTTGTCTGGAGAAT |

(continued)

| SEQ ID NO | Sequence |
|---|---|
| 182 | GCAAGAACCAAGACCCTCAGTACCGTTCGCATCGCCACCTTCACCTCCACTCCCTCCTTCCACACCCGT CTGCACCCCTCGAAGTCTCTGCGCTACTCTATCCCGGTCTGTGCGTTTTACCTCGTCCTCCCCTATGTGT TCCTGATCCCCGCGCATGAAGCCTTCGTCTCACAGCTCATTACAGTGCCTTGTCTGGAGAAT |
| 183 | GCAAGAACCAAGACCCTCAGTAGCAATATTGAATTCTAGATTATACGAGGCAACGCTCTGGTAACCTCT CTATTATTTAAGCTATCATACTCTAGTGTTTATTCTCCAGACAAGGCACTG |
| 184 | GCAAGAACCAAGACCCTCAGTATCCCAAGTGTTCTCTGCTTCATATTGGCAACGCTCTGGTAACCTCTCT AAATTGATAGTTCCGATTGCAACTTGACGTATTCTCCAGACAAGGCACTG |
| 185 | GCAAGAACCAAGACCCTCAGTGAATATTTTATTCCCTAATTTTATTATTATGTTCTAAAAGGTATTTAAAT ACTTTTCATTAATGGCAACGCTCTGGTAACCTCTCTATCATAAATATTTTAAATACTAGAATCTTATTTTAT TCTTTAGAATAGTAGAAATTTAATTAAATATTCTCCAGACAAGGCACTG |
| 186 | GCAAGAACCAAGACCCTCAGTGCCGAGGGTCCAGGTCGAGACTCCATCCCGAGGCGTGTGTCCCCATG GCCGTCCTCCAGGCTAGTACTGTGCCCCGTCGCCGTCGCACAAGGCCGGTCGATCGTGGTGGCTGTCA GGCGGGGTGGCAACGCTCTGGTAACCTCTCTACGGCGTAGTAGTTCGTGCCCCTCCCCTTGCGACCTC CCGCTACCACCCGTCACTCCCCGGTAAGAGGCTCTCACGGACGGCAGAGTCGGTCGCGCGCTCCGGAT GTGGTCCCCTCCCAGTCCTCATTCTCCAGACAAGGCACTG |
| 187 | GCTATTGCTGGGATTTTGAGGAACGTAGCAATATTGAATTCTAGATTATACGAGGCAACGCTCTGGTAA CCTCTCTATTATTTAAGCTATCATACTCTAGTGTTTCGTTCGTAGTCACTGTTGAAGCAAATG |
| 188 | GCTATTGCTGGGATTTTGAGGAACGTAGCAATATTGAATTCTAGATTATACGAGGCAACGCTCTGGTAA CCTCTCTATTATTTAAGCTATCATACTCTAGTGTTTCGTTCGTAGTCACTGTTGAAGCAAATG |
| 189 | GCTATTGCTGGGATTTTGAGGAAGATCTGTTCATGCGTTCGTTATTTGGATTGGAATTGTTGAGCCCTAC CTGACTCTGTATCCCAAGTGTTCTCTGCTTCATATTGGCAACGCTCTGGTAACCTCTCTAAATTGATAGTT CCGATTGCAACTTGACGTCTAGCCCGTATAAATAGCCGGTCTAAACAGCGATGAAATTTCGTCCGAACA AGTTTCAACTTCGTAGTCACTGTTGAAGCAAATG |
| 190 | GCTATTGCTGGGATTTTGAGGAAGATCTGTTCATGCGTTCGTTATTTGGATTGGAATTGTTGAGCCCTAC CTGACTCTGTATCCCAAGTGTTCTCTGCTTCATATTGGCAACGCTCTGGTAACCTCTCTAAATTGATAGTT CCGATTGCAACTTGACGTCTAGCCCGTATAAATAGCCGGTCTAAACAGCGATGAAATTTCGTCCGAACA AGTTTCAACTTCGTAGTCACTGTTGAAGCAAATG |
| 191 | GCTATTGCTGGGATTTTGAGGACCACGGTAATTACTGTTAGACTGGTGGGTATAAACTTCGTTATTTGGA TTGGAATTGTTGAGCCCTACCTGACTCTGTATCCCAAGTGTTCTCTGCTTCATATTGGCAACGCTCTGGT AACCTCTCTAAATTGATAGTTCCGATTGCAACTTGACGTCTAGCCCGTATAAATAGCCGGTCTAAACAGC GATGAAATTTCTGTAGAATCAACTAAATTTTCCGTTCAACGGATCCTCTGCCGTAGTCACTGTTGAAGCA AATG |
| 192 | GCTATTGCTGGGATTTTGAGGACCACGGTAATTACTGTTAGACTGGTGGGTATAAACTTCGTTATTTGGA TTGGAATTGTTGAGCCCTACCTGACTCTGTATCCCAAGTGTTCTCTGCTTCATATTGGCAACGCTCTGGT AACCTCTCTAAATTGATAGTTCCGATTGCAACTTGACGTCTAGCCCGTATAAATAGCCGGTCTAAACAGC GATGAAATTTCTGTAGAATCAACTAAATTTTCCGTTCAACGGATCCTCTGCCGTAGTCACTGTTGAAGCA AATG |
| 193 | GCTATTGCTGGGATTTTGAGGAGCTTTTCCTAAAAGGATTGTACACCTTAGAAGTGCTTAAGGAAGAGT GATGAAGATAGGCATGAAGCCTTCGTCTCACAGCTGCATGCGTAGTCACTGTTGAAGCAAATG |
| 194 | GCTATTGCTGGGATTTTGAGGAGCTTTTCCTAAAAGGATTGTACACCTTAGAAGTGCTTAAGGAAGAGT GATGAAGATAGGCATGAAGCCTTCGTCTCACAGCTGCATGCGTAGTCACTGTTGAAGCAAATG |
| 195 | GCTATTGCTGGGATTTTGAGGAGGCAACGCTCTGGTAACCTCTCTAATATGCGTAGTCACTGTTGAAGC AAATG |
| 196 | GCTATTGCTGGGATTTTGAGGAGGCAACGCTCTGGTAACCTCTCTAATATGCGTAGTCACTGTTGAAGC AAATG |
| 197 | GCTATTGCTGGGATTTTGAGGAGGCAACGCTCTGGTAACCTCTCTAATTGATAGAAGCATTTGCTTCAAC AGTGACTACG |
| 198 | GCTATTGCTGGGATTTTGAGGAGGCAACGCTCTGGTAACCTCTCTAATTGATAGAAGCATTTGCTTCAAC AGTGACTACG |
| 199 | GCTATTGCTGGGATTTTGAGGAGGCAACGCTCTGGTAACCTCTCTAATTGATAGATTCCAGCGTAGTCA CTGTTGAAGCAAATG |

(continued)

| SEQ ID NO | Sequence |
| --- | --- |
| 200 | GCTATTGCTGGGATTTTGAGGAGGCAACGCTCTGGTAACCTCTCTAATTGATAGATTCCAGCGTAGTCACTGTTGAAGCAAATG |
| 201 | GCTATTGCTGGGATTTTGAGGAGGCAACGCTCTGGTAACCTCTCTAATTGATAGTTCCGATACGTGCAACTTGTCTCGTAGTCACTGTTGAAGCAAATG |
| 202 | GCTATTGCTGGGATTTTGAGGAGGCAACGCTCTGGTAACCTCTCTAATTGATAGTTCCGATACGTGCAACTTGTCTCGTAGTCACTGTTGAAGCAAATG |
| 203 | GCTATTGCTGGGATTTTGAGGATATGTTCCAGTAGACGCGCAACAGGGCTTCTACGGTTCGCCGGTTATTGACTTACTGCACGTTGGGGAGCGGCTTGAATTGAGTCCCAGGCCCGAGTCCGTACCGATGCTCTTAGGCGAGCCACGTTTCTGGACCCACCCCGTGCTACCTATGGCCGTTCTTCGTATCTGTCTCTTAGCGCGCCTCAACTATGGTGTCCTCGCCTAGTAGAGCTCCGTAGACGTCCACCCCTTCGCAGGCAACGCTCTGGTAACCTCTCTACCCGGGAAGGGATTACAGGCTCGATTCCAGTCGCAGATGACACCGCTGTTCTACTCGGCACCTGACTACCTACCAGATGGGCCCGCAACACGTCGTGCACCCGCGGAACCGGTTAAAGAACGTTAGTTC<br><br>CCTGGCCTTGGAGCCTAAACAAACTTACTGAGCCGCACCTTCCGAGTCTCGCTGTACTGTGATCCCCGCTTCCCTGGTACTAGAGGGCAAATCCGACTGGCTATACCGACGTAGTCACTGTTGAAGCAAATG |
| 204 | GCTATTGCTGGGATTTTGAGGATATGTTCCAGTAGACGCGCAACAGGGCTTCTACGGTTCGCCGGTTATTGACTTACTGCACGTTGGGGAGCGGCTTGAATTGAGTCCCAGGCCCGAGTCCGTACCGATGCTCTTAGGCGAGCCACGTTTCTGGACCCACCCCGTGCTACCTATGGCCGTTCTTCGTATCTGTCTCTTAGCGCGCCTCAACTATGGTGTCCTCGCCTAGTAGAGCTCCGTAGACGTCCACCCCTTCGCAGGCAACGCTCTGGTAACCTCTCTACCCGGGAAGGGATTACAGGCTCGATTCCAGTCGCAGATGACACCGCTGTTCTACTCGGCACCTGACTACCTACCAGATGGGCCCGCAACACGTCGTGCACCCGCGGAACCGGTTAAAGAACGTTAGTTCCCTGGCCTTGGAGCCTAAACAAACTTACTGAGCCGCACCTTCCGAGTCTCGCTGTACTGTGATCCCCGCTTCCCTGGTACTAGAGGGCAAATCCGACTGGCTATACCGACGTAGTCACTGTTGAAGCAAATG |
| 205 | GCTATTGCTGGGATTTTGAGGATCTGTATCCCAAGTGTTCAGACCTTCATATTGCATGAAGCCTTCGTCTCACAGCTATTGATAGTTCCGATTGCAACTTGACGTCTAGCATTTGCTTCAACAGTGACTACG |
| 206 | GCTATTGCTGGGATTTTGAGGCCCGCCCCGCCCTGGCAACGCTCTGGTAACCTCTCTAGCCCGCCCCGCCCATTTGCTTCAACAGTGACTACG |
| 207 | GCTATTGCTGGGATTTTGAGGCCCGCCCCGCCCTGGCAACGCTCTGGTAACCTCTCTAGCCCGCCCCGCCCATTTGCTTCAACAGTGACTACG |
| 208 | GCTATTGCTGGGATTTTGAGGCCCGCCCCGCCCTGGCAACGCTCTGGTAACCTCTCTAGCCCGCCCCGTCCCGTAGTCACTGTTGAAGCAAATG |
| 209 | GCTATTGCTGGGATTTTGAGGCCCGCCCCGCCCTGGCAACGCTCTGGTAACCTCTCTAGCCCGCCCCGTCCCGTAGTCACTGTTGAAGCAAATG |
| 210 | GCTATTGCTGGGATTTTGAGGCGGGCGCGCCGCGCGCGACGCGCGTCCCGTCCGGCAACGCTCTGGTAACCTCTCTAACCCGCACGCCGGCGACCGCGCGCCCGGCATTTGCTTCAACAGTGACTACG |
| 211 | GCTATTGCTGGGATTTTGAGGCGGGCGCGCCGCGCGCGACGCGCGTCCCGTCCGGCAACGCTCTGGTAACCTCTCTAACCCGCACGCCGGCGACCGCGCGCCCGGCATTTGCTTCAACAGTGACTACG |
| 212 | GCTATTGCTGGGATTTTGAGGCGGGCGCGCCGCGCGCGACGCGCGTCCCGTCCGGCAACGCTCTGGTAACCTCTCTAACCCGCACGCCGGCGCCGCGCGCCCGAGCGCACGTAGTCACTGTTGAAGCAAATG |
| 213 | GCTATTGCTGGGATTTTGAGGCGGGCGCGCCGCGCGCGACGCGCGTCCCGTCCGGCAACGCTCTGGTAACCTCTCTAACCCGCACGCCGGCGCCGCGCGCCCGAGCGCACGTAGTCACTGTTGAAGCAAATG |
| 214 | GCTATTGCTGGGATTTTGAGGCGGTAATTACTGTTAGACTGGTGGGTATAAACTTCGTTATTTGGATTGGAATTGTTGAGCCCTACCTGACTCTGTATCCCAAGTGTTCTCTGCTTCATATTGGCAACGCTCTGGTAACCTCTCTAAATTGATAGTTCCGATTGCAACTTGACGTCTAGCCCGTATAAATAGCCGGTCTAAACAGCGATGAAATTTCTGTAGAATCAACTAAATTTTCCGTTCAACGGATCCTCATTTGCTTCAACAGTGACTACG |
| 215 | GCTATTGCTGGGATTTTGAGGCGGTAATTACTGTTAGACTGGTGGGTATAAACTTCGTTATTTGGATTGGAATTGTTGAGCCCTACCTGACTCTGTATCCCAAGTGTTCTCTGCTTCATATTGGCAACGCTCTGGTAACCTCTCTAAATTGATAGTTCCGATTGCAACTTGACGTCTAGCCCGTATAAATAGCCGGTCTAAACAGCGATGAAATTTCTGTAGAATCAACTAAATTTTCCGTTCAACGGATCCTCATTTGCTTCAACAGTGACTACG |

(continued)

| SEQ ID NO | Sequence |
|---|---|
| 216 | GCTATTGCTGGGATTTTGAGGCGTGTTGTTTCGATTTAACTTGTCCATGTGTCTCTGCTGCTTTCTTCCTT TCCACTTCACTACTCTTATTCGGGCAACGCTCTGGTAACCTCTCTAATTCTCGTAGTCACTGTTGAAGCA AATG |
| 217 | GCTATTGCTGGGATTTTGAGGCGTTATTTGGATTGGAATTGTTGAGCCCTACCTGACTCTGTATCCCAAG TGTTCTCTGCTTCATATTGGCAACGCTCTGGTAACCTCTCTAAATTGATAGTTCCGATTGCAACTTGACG TCTAGCCGATAAATAGCCGGTCTAAACAGCGATGAAATTTCCGTAGTCACTGTTGAAGCAAATG |
| 218 | GCTATTGCTGGGATTTTGAGGCGTTATTTGGATTGGAATTGTTGAGCCCTACCTGACTCTGTATCCCAAG TGTTCTCTGCTTCATATTGGCAACGCTCTGGTAACCTCTCTAAATTGATAGTTCCGATTGCAACTTGACG TCTAGCCGATAAATAGCCGGTCTAAACAGCGATGAAATTTCCGTAGTCACTGTTGAAGCAAATG |
| 219 | GCTATTGCTGGGATTTTGAGGCTGGAATTTGTGCTCTTAGGTCGTGGGGCTGCTGTTAAGTCGCTCGCT ATCTAAAGTTCAGTCAAGGATGGCAACGCTCTGGTAACCTCTCTAGAAATCGTAGTCACTGTTGAAGCA AATG |
| 220 | GCTATTGCTGGGATTTTGAGGGACCTCGACCGCTGGCAACGCTCTGGTAACCTCTCTATCCTCCCCTCT CCCGTAGTCACTGTTGAAGCAAATG |
| 221 | GCTATTGCTGGGATTTTGAGGGACCTCGACCGCTGGCAACGCTCTGGTAACCTCTCTATCCTCCCCTCT CCCGTAGTCACTGTTGAAGCAAATG |
| 222 | GCTATTGCTGGGATTTTGAGGGCATGTCGGCTCGGTCTGTCTCTTTCCCCTCATCTCTCGGTACACTCCT TACCTCGCCCACCCCGGCAACGCTCTGGTAACCTCTCTATCTGGCCTGTCACGAATCACTGTCCATCTAA CCTCGGAGCCCTGTCACGCGGCGGACTTGGAGACATTTGCTTCAACAGTGACTACG |
| 223 | GCTATTGCTGGGATTTTGAGGGCATGTCGGCTCGGTCTGTCTCTTTCCCCTCATCTCTCGGTACACTCCT TACCTCGCCCACCCCGGCAACGCTCTGGTAACCTCTCTATCTGGCCTGTCACGAATCACTGTCCATCTAA CCTCGGAGCCCTGTCACGCGGCGGACTTGGAGACATTTGCTTCAACAGTGACTACG |
| 224 | GCTATTGCTGGGATTTTGAGGGCCCGCGCGCCGGCGGCGGCGCGGTGGCCGGCGGCAACGCTCTGGT AACCTCTCTAGGCGGCGGCGCCACCGCGCGGGCGGGCGGGCCCGTAGTCACTGTTGAAGCAAATG |
| 225 | GCTATTGCTGGGATTTTGAGGGCCCGCGCGCCGGCGGCGGCGCGGTGGCCGGCGGCAACGCTCTGGT AACCTCTCTAGGCGGCGGCGCCACCGCGCGGGCGGGCGGGCCCGTAGTCACTGTTGAAGCAAATG |
| 226 | GCTATTGCTGGGATTTTGAGGGCCGTGCCGAGGGTCCAGGTCGAGACTCCATCCCGAGGCGTGTGTCC CCATGGCCGTCCTCCAGGCTAGTACTGTGCCCCGTCGCCGTCGCACAAGGCCGGTCGATCGTGGTGGC TGTCAGGCGGGGTGGCAACGCTCTGGTAACCTCTCTACGGCGTAGTAGTTCGTGCCCCTCCCCTTGCG ACCTCCCGCTACCACCCGTCACTCCCCGGTAAGAGGCTCTCACGGACGGCAGAGTCGGTCGCGCGCTC CGGATGTGGTCCCCTCCCAGTCCTCCCCGCGTAGTCACTGTTGAAGCAAATG |
| 227 | GCTATTGCTGGGATTTTGAGGGCCGTGCCGAGGGTCCAGGTCGAGACTCCATCCCGAGGCGTGTGTCC CCATGGCCGTCCTCCAGGCTAGTACTGTGCCCCGTCGCCGTCGCACAAGGCCGGTCGATCGTGGTGGC TGTCAGGCGGGGTGGCAACGCTCTGGTAACCTCTCTACGGCGTAGTAGTTCGTGCCCCTCCCCTTGCG ACCTCCCGCTACCACCCGTCACTCCCCGGTAAGAGGCTCTCACGGACGGCAGAGTCGGTCGCGCGCTC CGGATGTGGTCCCCTCCCAGTCCTCCCCGCGTAGTCACTGTTGAAGCAAATG |
| 228 | GCTATTGCTGGGATTTTGAGGGCGCGGCGGTGGAGCGCTCGCGGTGGTGCGCTGGCAACGCTCTGGT AACCTCTCTATGGCGCGTGGCCACGCTCCCGCGCGACGGCCGCGTAGTCACTGTTGAAGCAAATG |
| 229 | GCTATTGCTGGGATTTTGAGGGCGCGGCGGTGGAGCGCTCGCGGTGGTGCGCTGGCAACGCTCTGGT AACCTCTCTATGGCGCGTGGCCACGCTCCCGCGCGACGGCCGCGTAGTCACTGTTGAAGCAAATG |
| 230 | GCTATTGCTGGGATTTTGAGGGTAAACAGAGCGGAATCACAAATATTTATGCCTACCAAACCGATTTCTC AAAAGTAAAACAAAGTACGTCTCATTAATACTGTGGTGTAAGTATTATCAAAATAAAATAGTGTAACTGT ATGTATGTTGGCAACGCTCTGGTAACCTCTCTAATAAATTGATAAATTACACTGAGTTTGCATAGGAATC GTTATATATCAAAGTATGTTTTCTGACTACTATCAAACGCGCAAGTTACTTACTCTAAAAGTATTTGAGTT TAAGCCATTAGTCACCGATACGTAGTCACTGTTGAAGCAAATG |
| 231 | GCTATTGCTGGGATTTTGAGGTAGCAATATTGAATTCTAGATTATACGAGGCAACGCTCTGGTAACCTCT CTATTATTTAAGCTATCATACTCTAGTGTTTCATTTGCTTCAACAGTGACTACG |
| 232 | GCTATTGCTGGGATTTTGAGGTAGCAATATTGAATTCTAGATTATACGAGGCAACGCTCTGGTAACCTCT CTATTATTTAAGCTATCATACTCTAGTGTTTCATTTGCTTCAACAGTGACTACG |
| 233 | GCTATTGCTGGGATTTTGAGGTATATAAATAAATGGCAACGCTCTGGTAACCTCTCTAAATAAATAAAAT ACGTAGTCACTGTTGAAGCAAATG |

58

| SEQ ID NO | Sequence |
|---|---|
| 234 | GCTATTGCTGGGATTTTGAGGTATATAAATAAATGGCAACGCTCTGGTAACCTCTCTAAATAAATAAAAT ACGTAGTCACTGTTGAAGCAAATG |
| 235 | GCTATTGCTGGGATTTTGAGGTATCCCAAGTGTTCTCTGCTTCATATTGGCAACGCTCTGGTAACCTCTC TAAATTGATAGTTCCGATTGCAACTTGACGTCATTTGCTTCAACAGTGACTACG |
| 236 | GCTATTGCTGGGATTTTGAGGTATTATTATTTTAAATTAATATTATAATTTTAACTTTTATTGATTATATATT AGTCATTATATATAAAGGCAACGCTCTGGTAACCTCTCTATCTTAGTTTTATTAATATAAAATTTATATAAT AATATTTATTAAATAAATTCTATTATATTATTGATTCGTAGTCACTGTTGAAGCAAATG |
| 237 | GCTATTGCTGGGATTTTGAGGTATTATTATTTTAAATTAATATTATAATTTTAACTTTTATTGATTATATATT AGTCATTATATATAAAGGCAACGCTCTGGTAACCTCTCTATCTTAGTTTTATTAATATAAAATTTATATAAT AATATTTATTAAATAAATTCTATTATATTATTGATTCGTAGTCACTGTTGAAGCAAATG |
| 238 | GCTATTGCTGGGATTTTGAGGTCATAGTTCTATATACATTGTCATGACACGAATTGGCTGAAAACGGTTG ATAGAAGATATTGACTATATTCTCTTCCGCTGTATCCCGTTTCTTTTGGAAATTGACCGTATTATGGTCAC CATCAGCCTAAGTGATCTCTGGACCGTCGAGAGACCCCATTGACTTGGTTCTTCGGTTTGATGCACTCA TGTAAAATGTAGTCTCAATCAATACCATCCATTTCTAGCATACGGGTGAGCATGAAGCCTTCGTCTCACA GCTCCGGTACAGGTAATCGAGAGAACACTAAAACAGTCCGACATGAGATTCATTAAAACCTATTTTCACC AATCGGTAGAACGGTTATGCGCAAAATATTTTCGGGGTCCACAGTGCACCTATGTAATCTGTAACATGA AGTTGTACGAAAATAGAGAACCCACCCAGCTTATCTAGGAAATTGATCTCTTCGATTTAAGGATGTGTCG ACACGTATCATGCCAAGTGATCAGAGGCGTAACATTTGCTTCAACAGTGACTACG |
| 239 | GCTATTGCTGGGATTTTGAGGTCATAGTTCTATATACATTGTCATGACACGAATTGGCTGAAAACGGTTG ATAGAAGATATTGACTATATTCTCTTCCGCTGTATCCCGTTTCTTTTGGAAATTGACCGTATTATGGTCAC CATCAGCCTAAGTGATCTCTGGACCGTCGAGAGACCCCATTGACTTGGTTCTTCGGTTTGATGCACTCA TGTAAAATGTAGTCTCAATCAATACCATCCATTTCTAGCATACGGGTGAGCATGAAGCCTTCGTCTCACA GCTCCGGTACAGGTAATCGAGAGAACACTAAAACAGTCCGACATGAGATTCATTAAAACCTATTTTCACC AATCGGTAGAACGGTTATGCGCAAAATATTTTCGGGGTCCACAGTGCACCTATGTAATCTGTAACATGA AGTTGTACGAAAATAGAGAACCCACCCAGCTTATCTAGGAAATTGATCTCTTCGATTTAAGGATGTGTCG ACACGTATCATGCCAAGTGATCAGAGGCGTAACGTAGTCACTGTTGAAGCAAATG |
| 240 | GCTATTGCTGGGATTTTGAGGTCATAGTTCTATATACATTGTCATGACACGAATTGGCTGAAAACGGTTG ATAGAAGATATTGACTATATTCTCTTCCGCTGTATCCCGTTTCTTTTGGAAATTGACCGTATTATGGTCAC CATCAGCCTAAGTGATCTCTGGACCGTCGAGAGACCCCATTGACTTGGTTCTTCGGTTTGATGCACTCA TGTAAAATGTAGTCTCAATCAATACCATCCATTTCTAGCATACGGGTGAGGCAACGCTCTGGTAACCTCT CTACCGGTACAGGTAATCGAGAGAACACTAAAACAGTCCGACATGAGATTCATTAAAACCTATTTTCACC AATCGGTAGAACGGTTATGCGCAAAATATTTTCGGGGTCCACAGTGCACCTATGTAATCTGTAACATGA AGTTGTACGAAAATAGAGAACCCACCCAGCTTATCTAGGAAATTGATCTCTTCGATTTAAGGATGTGTCG ACACGTATCATGCCAAGTGATCAGAGGCGTAACGTAGTCACTGTTGAAGCAAATG |
| 241 | GCTATTGCTGGGATTTTGAGGTCATAGTTCTATATACATTGTCATGACACGAATTGGCTGAAAACGGTTG ATAGAAGATATTGACTATATTCTCTTCCGCTGTATCCCGTTTCTTTTGGAAATTGACCGTATTATGGTCAC CATCAGCCTAAGTGATCTCTGGACCGTCGAGAGACCCCATTGACTTGGTTCTTCGGTTTGATGCACTCA TGTAAAATGTAGTCTCAATCAATACCATCCATTTCTAGCATACGGGTGAGGCAACGCTCTGGTAACCTCT CTACCGGTACAGGTAATCGAGAGAACACTAAAACAGTCCGACATGAGATTCATTAAAACCTATTTTCACC AATCGGTAGAACGGTTATGCGCAAAATATTTTCGGGGTCCACAGTGCACCTATGTAATCTGTAACATGA AGTTGTACGAAAATAGAGAACCCACCCAGCTTATCTAGGAAATTGATCTCTTCGATTTAAGGATGTGTCG ACACGTATCATGCCAAGTGATCAGAGGCGTAACGTAGTCACTGTTGAAGCAAATG |
| 242 | GCTATTGCTGGGATTTTGAGGTCGCTCGCCCCTACTTACACCACCCCTCCCCGTAGTCACTGTTGAAGC AAATG |
| 243 | GCTATTGCTGGGATTTTGAGGTCTGGCATGTCGGCTCGGTCTGTCTCTTTCCCCTCATCTCTCGGTACAC TCCTTACCTCGCCCACCCCGGCAACGCTCTGGTAACCTCTCTATCTGGCCTGTCACGAATCACTGTCCAT CTAACCTCGGAGCCCTGTCACGCGGCGGACTTGGAGACTGTCGTAGTCACTGTTGAAGCAAATG |
| 244 | GCTATTGCTGGGATTTTGAGGTCTGGCATGTCGGCTCGGTCTGTCTCTTTCCCCTCATCTCTCGGTACAC TCCTTACCTCGCCCACCCCGGCAACGCTCTGGTAACCTCTCTATCTGGCCTGTCACGAATCACTGTCCAT CTAACCTCGGAGCCCTGTCACGCGGCGGACTTGGAGACTGTCGTAGTCACTGTTGAAGCAAATG |
| 245 | GCTATTGCTGGGATTTTGAGGTCTGTATCCCAAGTGTTCTCTGCTTCATATTGGCAACGCTCTGGTAACC TCTCTAAATTGATAGTTCCGATTGCAACTTGACGTCTAGCGTAGTCACTGTTGAAGCAAATG |
| 246 | GCTATTGCTGGGATTTTGAGGTCTGTATCCCAAGTGTTCTCTGCTTCATATTGGCAACGCTCTGGTAACC TCTCTAAATTGATAGTTCCGATTGCAACTTGACGTCTAGCGTAGTCACTGTTGAAGCAAATG |

(continued)

| SEQ ID NO | Sequence |
|---|---|
| 247 | GCTATTGCTGGGATTTTGAGGTGAATATTTTATTCCCTAATTTTATTATTATGTTCTAAAAGGTATTTAAAT ACTTTTCATTAATGGCAACGCTCTGGTAACCTCTCTATCATAAATATTTTAAATACTAGAATCTTATTTTAT TCTTTAGAATAGTAGAAATTTAATTAAATCATTTGCTTCAACAGTGACTACG |
| 248 | GCTATTGCTGGGATTTTGAGGTGAATATTTTATTCCCTAATTTTATTATTATGTTCTAAAAGGTATTTAAAT ACTTTTCATTAATGGCAACGCTCTGGTAACCTCTCTATCATAAATATTTTAAATACTAGAATCTTATTTTAT TCTTTAGAATAGTAGAAATTTAATTAAATCATTTGCTTCAACAGTGACTACG |
| 249 | GCTATTGCTGGGATTTTGAGGTGCCGAGGGTCCAGGTCGAGACTCCATCCCGAGGCGTGTGTCCCCAT GGCCGTCCTCCAGGCTAGTACTGTGCCCCGTCGCCGTCGCACAAGGCCGGTCGATCGTGGTGGCTGTC AGGCGGGGTGGCAACGCTCTGGTAACCTCTCTACGGCGTAGTAGTTCGTGCCCCTCCCCTTGCGACCT CCCGCTACCACCCGTCACTCCCCGGTAAGAGGCTCTCACGGACGGCAGAGTCGGTCGCGCGCTCCGGA TGTGGTCCCCTCCCAGTCCTCCATTTGCTTCAACAGTGACTACG |
| 250 | GCTATTGCTGGGATTTTGAGGTGCCGAGGGTCCAGGTCGAGACTCCATCCCGAGGCGTGTGTCCCCAT GGCCGTCCTCCAGGCTAGTACTGTGCCCCGTCGCCGTCGCACAAGGCCGGTCGATCGTGGTGGCTGTC AGGCGGGGTGGCAACGCTCTGGTAACCTCTCTACGGCGTAGTAGTTCGTGCCCCTCCCCTTGCGACCT CCCGCTACCACCCGTCACTCCCCGGTAAGAGGCTCTCACGGACGGCAGAGTCGGTCGCGCGCTCCGGA TGTGGTCCCCTCCCAGTCCTCCATTTGCTTCAACAGTGACTACG |
| 251 | GCTATTGCTGGGATTTTGAGGTGCGTCGATGCTGTGTGAGGTGAAGACCTAGAGGCAACGCTCTGGTA ACCTCTCTACACGCTTAGCAACGCTGCATGTCGAGTCTCCACGTAGTCACTGTTGAAGCAAATG |
| 252 | GCTATTGCTGGGATTTTGAGGTGCGTCGATGCTGTGTGAGGTGAAGACCTAGAGGCAACGCTCTGGTA ACCTCTCTACACGCTTAGCAACGCTGCATGTCGAGTCTCCACGTAGTCACTGTTGAAGCAAATG |
| 253 | GCTATTGCTGGGATTTTGAGGTGCGTCGCGGCTGTGGGAGGTGCGGACCTAGAGGCAACGCTCTGGTA ACCTCTCTACACGCTTAGCGCCGCTGCCTGTCGACCGTCCACGTAGTCACTGTTGAAGCAAATG |
| 254 | GCTATTGCTGGGATTTTGAGGTGCGTCGCGGCTGTGGGAGGTGCGGACCTAGAGGCAACGCTCTGGTA ACCTCTCTACACGCTTAGCGCCGCTGCCTGTCGACCGTCCACGTAGTCACTGTTGAAGCAAATG |
| 255 | GCTATTGCTGGGATTTTGAGGTGGGGCTGGCAGGGGCGGGTGGGGAGGAGGGCGGGGTGGGGTCGG GGCCAAGGGGAGCGGGGAGCGGCGGCAACGCTCTGGTAACCTCTCTAGCCCGTCCGTGCCGTCCGCC GCCTGGGAGCCTCGCTCGGGGACAGCCGGGACTGGGGACGCGGGCCGCCGTAGTCACTGTTGAAGCA AATG |
| 256 | GCTATTGCTGGGATTTTGAGGTGGGGCTGGCAGGGGCGGGTGGGGAGGAGGGCGGGGTGGGGTCGG GGCCAAGGGGAGCGGGGAGCGGCGGCAACGCTCTGGTAACCTCTCTAGCCCGTCCGTGCCGTCCGCC GCCTGGGAGCCTCGCTCGGGGACAGCCGGGACTGGGGACGCGGGCCGCCGTAGTCACTGTTGAAGCA AATG |
| 257 | GCTATTGCTGGGATTTTGAGGTGGTAGATGGCGTTTTGTTTCAGGAGTTTATCATTACCGACTTAAAGCT AACAACGAAACTTATGAAATGGATCTTAGGCAACGCTCTGGTAACCTCTCTAATCGCCGTAGTCACTGTT GAAGCAAATG |
| 258 | GCTATTGCTGGGATTTTGAGGTGTAATATTAACAAGTAATAAAGAAATATATAGCATGAAGCCTTCGTCT CACAGCTTTTATTCAATTTAATGATTACCTTTATTATCTCATTTGCTTCAACAGTGACTACG |
| 259 | GCTATTGCTGGGATTTTGAGGTGTAATATTAACAAGTAATAAAGAAATATATAGCATGAAGCCTTCGTCT CACAGCTTTTATTCAATTTAATGATTACCTTTATTATCTCGTAGTCACTGTTGAAGCAAATG |
| 260 | GCTATTGCTGGGATTTTGAGGTGTAATATTAACAAGTAATAAAGAAATATATAGGCAACGCTCTGGTAAC CTCTCTATTTATTCAATTTAATGATTACCTTTATTATCTCGTAGTCACTGTTGAAGCAAATG |
| 261 | GCTATTGCTGGGATTTTGAGGTGTAATATTAACAAGTAATAAAGAAATATATAGGCAACGCTCTGGTAAC CTCTCTATTTATTCAATTTAATGATTACCTTTATTATCTCGTAGTCACTGTTGAAGCAAATG |
| 262 | GCTATTGCTGGGATTTTGAGGTTGCAACTTGACGTCTCGTAGTCACTGTTGAAGCAAATG |
| 263 | GCTATTGCTGGGATTTTGAGGTTTAATAAATAAATTAAATATTATATAAATTAGGCAACGCTCTGGTAACC TCTCTATATTATATTAAATTATTAAATTAATAATTATACGTAGTCACTGTTGAAGCAAATG |
| 264 | GCTATTGCTGGGATTTTGAGGTTTAATAAATAAATTAAATATTATATAAATTAGGCAACGCTCTGGTAACC TCTCTATATTATATTAAATTATTAAATTAATAATTATACGTAGTCACTGTTGAAGCAAATG |

(continued)

| SEQ ID NO | Sequence |
| --- | --- |
| 265 | GCTATTGCTGGGATTTTGAGGTTTCTGAATATTTTATTCCCTAATTTTATTATTATGTTCTAAAAGGTATTT AAATACTTTTCATTAATGGCAACGCTCTGGTAACCTCTCTATCATAAATATTTTAAATACTAGAATCTTATT TTATTCTTTAGAATAGTAGAAATTTAATTAAATGCACCGTAGTCACTGTTGAAGCAAATG |
| 266 | GCTATTGCTGGGATTTTGAGGTTTCTGAATATTTTATTCCCTAATTTTATTATTATGTTCTAAAAGGTATTT AAATACTTTTCATTAATGGCAACGCTCTGGTAACCTCTCTATCATAAATATTTTAAATACTAGAATCTTATT TTATTCTTTAGAATAGTAGAAATTTAATTAAATGCACCGTAGTCACTGTTGAAGCAAATG |
| 267 | GCTATTGCTGGGATTTTGAGGTTTGTTTTCGTTTCTTTCTCTCTTTCTTATCGTAGTCACTGTTGAAGCAA ATG |
| 268 | GGAGAAAAGCCACATGAATGCAAAACACCAGCAATCTCAAGACCCACCTATAAACATTGGTATGGTTTC TCTCCAG |
| 269 | GGAGAAAAGCCACATGAATGCAAAACACCAGCAATCTCAAGACCCACCTATAAACTGGAGAGAAACCAT ACCAATG |
| 270 | GGAGAAAAGCCACATGAATGCAAAAGAAGAAATAAGATAAAATACAACAATAATCAAAGACACAAAACA AACATAAACACCAGCAATCTCAAGACCCACCTAAGAACATTGGTATGGTTTCTCTCCAG |
| 271 | GGAGAAAAGCCACATGAATGCAAAAGAAGAAATAAGATAAAATACAACAATAATCAAAGACACAAAACA AACATAAACACCAGCAATCTCAAGACCCACCTAAGAACTGGAGAGAAACCATACCAATG |
| 272 | GGAGAAAAGCCACATGAATGCAAAATAAATACTAAACAAAACTAACAACACAAACACCAGCAATCTCAA GACCCACCTATAAACATTGGTATGGTTTCTCTCCAG |
| 273 | GGAGAAAAGCCACATGAATGCAAAATAAATACTAAACAAAACTAACAACACAAACACCAGCAATCTCAA GACCCACCTATAAACTGGAGAGAAACCATACCAATG |
| 274 | GGAGAAAAGCCACATGAATGCATATAATATACTACAATTATTAAAATATGCATGAAGCCTTCGTCTCACA GCTAAGTTCTGGAGAGAAACCATACCAATG |
| 275 | TCTCTGATCGGTCCCTTTACTCGCCTCCCTACTCTTCATTCTATTCTCCTTCTCGTTCTTGTTTCTTCTTTT GTCTCTTTGCTTCCCTCGTATCTGTTCCTTTCCCGTCTCCCCATTCCCCGCCCCACTACCCAACACCCAC CAATCAACCAAAACCTACAACCCATCCACACACCACCTCACTAACTCCTACCTCGCTCCTCTACACTTCA CTGGCAACGCTCTGGTAACCTCTCTATCTCACCCCTTAATTTCCGCACCTATT |
| 276 | TCTCTGATCGGTCCCTTTACTCTCCCAACCCCTCCCTCGCCCATCCCCACTCCGCTCGCTTCCCCTGGCC CTGTCCGCCTCCACCCGTCGTCCTCATCCAGCCGCAAGTTGGCAACGCTCTGGTAACCTCTCTACGCCG CCCCCTTAATTTCCGCACCTATT |
| 277 | TCTCTGATCGGTCCCTTTACTCTCCCTCGCCTCCTTCCCACCCTCTTCCTCACTCACCCCACTTTTCTATC TACTTCACTGGCAACGCTCTGGTAACCTCTCTACCCCTCCCCTTAATTTCCGCACCTATT |
| 278 | TCTCTGATCGGTCCCTTTACTCTGCCTTTTCTCCTTTCTTTCCTTCCTCATCCACTTCCACCCACCTCACTC ACCCTAACCCCGCCCTCCCAACCATCACCAACACCCCTCAAACCTACCTCCTCCGCTCCCCACACTCTCC CTACTCAACTCTACACATGGCAACGCTCTGGTAACCTCTCTATCTCGCCCCTTAATTTCCGCACCTATT |
| 279 | TCTCTGATCGGTCCCTTTACTCTTCTGTCCTTCCTCCTGTATTCGCTTATCTTCCACTTTCCAATTTAACGA TATGACGAGTTTATTCCTGCTTGAGTCTAGTTCCGTTTCAAATACCCCTGCGCCCTTCTTTGTCTTACTTG TTCGGTTCACTTGCTCCTCTACTTCACGGTCTCTTTAACTCAGGCAACGCTCTGGTAACCTCTCTATCACT CCCCTTAATTTCCGCACCTATT |
| 280 | TGCAGAAACACTACCTGGTACAAAACACCAGCAATCTCAAGACCCACCTATAAACACAAACTGGGTGAA CTTGG |
| 281 | TGCAGAAACACTACCTGGTACAAAAGAAGAAATAAGATAAAATACAACAATAATCAAAGACACAAAACAA ACATAAACACCAGCAATCTCAAGACCCACCTAAGAACACAAACTGGGTGAACTTGG |
| 282 | TGCAGAAACACTACCTGGTACAAAATAAATACTAAACAAAACTAACAACACAAACACCAGCAATCTCAAG ACCCACCTATAAACACAAACTGGGTGAACTTGG |
| 283 | CGTAGTCACTGTTGAAGCAAATG |
| 284 | GTGATGTGAAGGATTATGGGGA |
| 285 | CATTGGTATGGTTTCTCTCCAG |
| 286 | ATTCTCCAGACAAGGCACTG |

(continued)

| SEQ ID NO | Sequence |
|---|---|
| 287 | AATAGGTGCGGAAATTAAGGGG |

The invention will now be described further by the following non-limiting Examples.

## Examples

The core technology is a system of at least three natural target or competitor polynucleotides, used in a nucleic acid amplification reaction for evaluation of a certain combination of one or more sequences of interest. As the sequences are replicated, they compete for these shared primers, conferring unique characteristics to the resulting readout. For example, take a set of natural gene transcripts, each paired with an engineered synthetic competitor (Figure 8). An amplification reaction is run with a fixed amount of each competitor and various amounts of each natural target. As the natural sequence in each competitive pair replicates, it produces a green fluorescent signal; each corresponding synthetic sequence produces an orange signal. Since all green signals and all orange signals stack on top of one another, looking at the relative strength of orange and green at the end of the reaction tells you how close, on aggregate, the concentration of all the transcripts are to the concentration of their respective competitors. Each competitor sequence can be designed to reflect the concentration range of interest for each individual natural target; maybe some transcripts have interesting effects within a narrow window whereas others have more gradual impacts as their concentration changes. This principle has many applications, from human diagnostics to bioprocess manufacturing and biomedical research.

The "direct" competitive amplification network described above, comprising multiple pairs of natural and synthetic targets each competing for both primers, constitutes the simplest embodiment of this invention. However, the same competition principle applies to more complex networks. For example, a natural target could share one of its primers with one synthetic target, which in turn shares its other primer with a second synthetic target, making an "indirect" CAN (Figure 9). Primers can be shared between multiple synthetic targets, and fully connected networks can be designed to include multiple natural targets, creating the possibility of performing non-linear operations (Figure 10). A single natural sequence can be independently targeted at multiple locations on the same oligo, creating a "redundant" system with powerful properties (Figure 11).

## Direct Competitive PCR

In competitive PCR, a competitor polynucleotide (REF) is included as a reference alongside the target (denoted in the figures as WT)(Figure 3). This competitor sequence is designed to share the same primer sequences as the WT but contains a different probe sequence. A probe with one fluorophore (e.g., fluorescein, or FAM, which produces a green colour) can be designed to target the WT, while a separate probe with a different fluorophore (e.g., hexachlorofluorescein, or HEX, which produces an orange colour) targets the REF (competitor).

When the target and the competitor are amplified in the same PCR reaction, they compete for the primers. Since primers are consumed by each replication of a target or competitor strand, the amplification of both sequences stops as soon as the primer pool is exhausted. The quantity of each amplification product at the end of the reaction depends on the relative starting quantity of the two targets. This is reflected in the resulting fluorescent signal (Figure 4). For a target and competitor with the same amplification rate (such as the WT and the ISO from Figure 2) that begin at the same concentration, the fluorescent signal derived from each will be the same at the end of the reaction. If there is more WT than REF at the start of the reaction, the WT fluorophore will be more intense at the end, and vice versa. The sharpness of this transition from pure WT signal to pure REF signal can be tuned by adjusting the amplification rate of the competitor.

Figure 4 shows competitive amplification of a WT sequence with various competitors (REFs), demonstrating the breadth of accessible behaviours, from very broad transitions (BP240, GC85) to very sharp (BP30). The midpoint of the response curve can be shifted to higher or lower WT concentrations by adjusting the initial concentration of the REF. Using gel electrophoresis, we can directly measure the final concentration of the amplicons in each reaction, confirming the dynamics observed in the fluorescent signal. In essence, this system is reporting on how close the expression of the gene of interest is to a pre-determined concentration. We can define this concentration, as well as the range over which we are interested, by choosing the appropriate design of the REF and its initial concentration.

## Direct Competitive Amplification Networks

Now, a pair of competing targets is not much of a "network", nor does a single gene target reflect the complexity of gene expression signatures. However, we can combine multiple competitive pairs in the same reaction, each producing HEX and FAM signals that reflect a different RNA transcript. Each competitive pair reports on how close the given gene is to its individual set point, and these signals will all simply stack on top of one another. The result is an aggregate measure of the overall similarity of all genes. Regardless of the number of genes under investigation, the difference between the total HEX intensity and the total FAM intensity integrate the information from the whole system. To illustrate why this is useful, let's look at how we can use such a network to diagnose tuberculosis by mimicking the statistical technique of logistic regression.

### Case Study: Diagnosis tuberculosis with a direct CAN

More people die each year from tuberculosis than from any other infectious disease. 2018 saw 10 million new cases and 1.5 million deaths. Tuberculosis is particularly prevalent (and deadly) among those also infected with HIV, a population particularly difficult to diagnose with current TB tests. A gene expression signature was found in human white blood cells that can be used to diagnose TB. ( (1)

Kaforou, M.; Wright, V. J.; Oni, T.; French, N.; Anderson, S. T.; Bangani, N.; Banwell, C. M.; Brent, A. J.; Crampin, A. C.; Dockrell, H. M.; Eley, B.; Heyderman, R. S.; Hibberd, M. L.; Kern, F.; Langford, P. R.; Ling, L.; Mendelson, M.; Ottenhoff, T. H.; Zgambo, F.; Wilkinson, R. J.; Coin, L. J.; Levin, M. Detection of Tuberculosis in HIV-Infected and -Uninfected African Adults Using Whole Blood RNA Expression Signatures: A Case-Control Study. PLOS Medicine 2013, 10 (10), e1001538. https://doi.org/10.1371/journal.pmed.1001538. (2) Gliddon, H. D.; Kaforou, M.; Alikian, M.; Habgood-Coote, D.; Zhou, C.; Oni, T.; Anderson, S. T.; Brent, A. J.; Crampin, A. C.; Eley, B.; Kern, F.; Langford, P. R.; Ottenhoff, T. H. M.; Hibberd, M. L.; French, N.; Wright, V. J.; Dockrell, H. M.; Coin, L. J.; Wilkinson, R. J.; Levin, M.; Consortium, on behalf of the I. Identification of Reduced Host Transcriptomic Signatures for Tuberculosis and Digital PCR-Based Validation and Quantification. bioRxiv 2019, 583674. https://doi.org/10.1101/583674.)

Crucially, this test performs equally well in patients with and without HIV. However, the technology used to identify this signature - microarrays - is too cumbersome and expensive for use in the rural, poor regions of the world where such a test is needed most. A direct Competitive Amplification Network can evaluate the gene expression signature and translate the test to a rapid, inexpensive, and easy-to-use format.

### Diagnosing with statistics: logistic regression

To understand how we can use a CAN to diagnose TB, we first need to understand the statistical technique we are trying to mimic: logistic regression. Logistic regression models the probability of being in one group (infected with tuberculosis) compared to another (having some other disease, OD) by looking at the individual contributions of various determining factors (expression levels of various genes). It assumes that the *log-odds,* or *relative* probability, is given by a (linear) weighted sum of these factors:

$$\frac{TB}{1-TB} = \beta 1 \cdot [GBP6] + \beta 2 \cdot [TMCC1] + \beta 3 \cdot [ARG1] + \beta 4 \cdot [PRDM1]$$

We can look at the contribution of individual genes to the overall classifier by finding the marginal log-odds for each (Figure 11A); i.e., if all three other genes are at their mean values (providing no information), then how much information is provided by various amounts of this gene? Because log-odds represent *relative* probability, a negative score implies "more likely to be OD" (coded as -1) while a positive score implies "more likely to be TB" (coded as +1). Two scales are shown: marginal log-odds on the left and marginal probability on the right. The grey dots are the gene copy numbers for individual patients, while the dashed line is the regressed log-odds (or probability) of TB indicated by the given gene copy number.

To diagnose a patient based on logistic regression, we just add up the contribution of each individual gene. For example, a patient may have 103 copies of GBP6, contributing a marginal log-odds of +0.25. The same patient might have 104 and 104 copies of ARG1 and TMCC1, respectively, contributing -0.5 and -0.2. The overall log-odds of this patient having TB would be 0.25 - 0.5 - 0.2 = -0.45, so we can conclude that this patient is unlikely to have TB. Repeating this for every patient (Figure 11B), we can see our regression result achieves high accuracy, correctly categorizing 36 out of 40 patients.

### Mimicking the statistics with a direct CAN

We can use a direct CAN to recapitulate this statistical inference on a molecular level by designing a competitor for each of our three gene transcripts (Figure 8A). Since GBP6 is *positively* correlated with TB, we use a HEX-labelled probe for the transcript and a FAM-labelled probe for the competitor; since ARG1 and TMCC1 are *negatively* correlated with TB, the probe labels are swapped. We then choose an appropriate region from the transcript as our natural target, and an appropriate sequence as our synthetic target (described further below), to display amplification behaviour that produces response curves which match the marginal log-odds relationship from logistic regression. By including all components in the same amplification reactions, the total HEX and FAM fluorescence intensities aggregate the independent contributions of individual pairs. The difference between the strength of these two colours acts as a surrogate for the log-odds derived from logistic regression (Figure 8B), providing a probabilistic diagnosis matching that predicted by the statistical results.

In order to choose an appropriate target region and design the synthetic target sequence, we use the results of logistic regression as an "objective function": our goal is to find a pair of sequences that, when amplified together, give us an input-output response curve that approximates this objective. Thus, for each target, we try to approximate a line with the slope derived from the equation above (the respective $\beta$ term) and which intercepts 0 at the mean concentration of that target observed in our data set. Using simulation, we can predict the behaviour of any two sequences amplified together, and so we can use standard curve-fitting algorithms known to the art to find the optimal parameters. In this case, those are the parameters that produce a response curve that matches the line specified above as closely as possible in the range of target concentrations observed in our dataset, then flattens as quickly as possible outside that range (See Figure 7).

Once suitable parameters are found, we then need to select sequences which exhibit them. Using the equations described above in the section "Testing and predicting competitor amplification behavior", we can predict the combinations of length and GC content which provide these parameters. Note that our simulations do not include the drift term ($m$) or plateau term ($K$) found in our regression equations. This is because the simulations represent ideal behavior, and these two parameters describe deviations from that ideal. Thus, in choosing optimal length and GC content, we would seek to minimize drift and maximize the plateau, so that we select sequences as close to the ideal as possible.

It is likely that multiple sets of parameters could give nearly-optimal curves. It may be preferable that a suitable target sequence be identified *a priori* (due to external constraints), its amplification parameters measured, then using the curve-fitting algorithm to select only competitor amplification parameters which produce a nearly-optimal response when simulated along with the measured parameters. The simulation of the amplification behavior is described above; supplied with the suitable equations for simulation, the skilled person would be able to perform any of several optimization techniques and algorithms, including Gradient Descent, Stochastic Gradient Descent, and Quasi-Newton optimization, among others.

## Limitations of direct CANs

The direct networks presented above have two main drawbacks. First, they will get expensive quickly for larger gene signatures since at least one if not two probes need to be designed for each transcript targeted. Economies of scale for DNA sequences are quite favourable for scale-up, but at a development scale each fluorescently-labelled probe costs ~ £200 (for context, each primer costs ~£2 and each synthetic target ~£20). For gene signatures with 20-50 targets iterating on sequence designs becomes prohibitively expensive. Second, direct CANs are somewhat limited in the response curves attainable. To address these issues, indirect CANs provide similar functionality at a more or less fixed cost regardless of the number of genes under investigation. Indirect competition also opens the possibility of higher-order networks capable of complex, non-linear analysis of multiple targets simultaneously. Finally, redundant targeting allows additional flexibility for all CAN architectures.

## Indirect Competitive PCR

Instead of direct competition between a probed target and a probed competitor, an unprobed target can simply *mediate* the competition between competitor polynucleotides. Because both primers are necessary for exponential amplification of a given target, replication can be arrested by depletion of only one primer. So, we can design a synthetic target, REFH, that shares one primer with a natural sequence, WT, and its second primer with a second synthetic target, REFF (Figure 12). If all components have equal amplification rate and the two REFs start at equal concentration, without any WT present the HEX and FAM signals will amplify equally. However, increasing WT begins to outcompete REFH, dampening the HEX signal. This, in turn, creates more room for REFF to grow, leading to a greater FAM signal at the end of the reaction. The result is an S-shaped response curve to various WT concentrations, similar to that observed from direct competition (Figure 9A). This response curve can be tuned by adjusting the amplification rate of any of the targets, the starting concentration of the synthetic targets, the concentration of any of the primers, or the topology of the network itself (Figure 9B,C). The key advantage of this system is that, because we have complete freedom over the "interior" sequence of the synthetic targets, the same two probe sequences can be reused in multiple REFs, minimizing development costs regardless of how many natural targets are utilized or how complex the network is.

**Case Study: Diagnosing cancer with an indirect CAN**

A promising avenue of early cancer diagnosis or monitoring of cancer treatment is through detection of tumor-derived DNA in the bloodstream (circulating tumour DNA, ctDNA), chromosomal fragments shed by the cells as they die. This is distinguishable from the ordinary milieu of cell-free DNA (cfDNA) through specific mutations, such as single nucleotide polymorphisms (SNPs) or insertion-deletions (indels). By detecting known pathogenic mutations, we may be able to diagnose someone before the tumour shows up on a scan. We can also look for ctDNA after or during treatment, to see if the patient is responding or if the cancer has come back. The difficulty is, these variants are much lower in concentration than the corresponding natural sequence. Furthermore, a single base change is hard to differentiate using ordinary PCR (indels are easier, so we'll focus on SNPs with the understanding that whatever works for SNPs will work even better for indels). While in some cases specific mutations can inform treatment decisions (namely targeted treatment susceptibility/resistance), in general the total ctDNA burden is all that is needed even though any of numerous mutations can act as proxies for that total, making this a good application for CANs.

To use CANs for ctDNA detection, we will adapt Blocker Displacement Amplification (Wu et al., 2017), a published approach for preferentially amplifying variant alleles over the corresponding wild-type (Figure 13). In BDA, a short oligo is designed to overlap the SNP site but bind more strongly to the WT sequence. This "blocker" is chemically modified to prevent extension by the polymerase. By selecting a primer site adjacent to the SNP and overlapping with the blocker region, the blocker and primer compete for binding to the WT and SNP targets. This suppresses the amplification rate of the WT, since the blocker binds more strongly than the primer, but allows the SNP to amplify with minimal perturbation since the primer outcompetes the blocker. This system can be coupled into an indirect CAN tuned such that one signal quickly dominates as the SNP concentration increases, even at high variable allele frequency (VAF). Designing one such CAN for several different targets allows for multiplexed surveillance, where the total signal reflects the total mutation burden in the ctDNA.

**Higher-order Competitive Networks**

The flexibility of the indirect CAN allows incorporation of multiple natural targets in a single closed network, enabling non-linear analysis of target combinations. For example, Figure 14 shows CAN motifs that approximate AND, OR, and XOR logic from Boolean logic. Redundant Competitive Networks

The CANs shown above are limited in their response to a given target; the output is always monotonic or at least unimodal with regards to the target concentration. However, we can further exploit the additive nature of fluorescent signals by redundantly targeting a single sequence. Genes transcripts are typically several thousand nucleotides long, while only 50-300 nucleotides are needed for a PCR target. Accordingly, we can design independent CANs each targeting a different region of the same sequence. Their outputs will stack, producing powerful emergent behaviour. From a mathematical point of view, the individual networks become a library of "basis functions" from which theoretically any response relationship can be built, limited only by the number of target regions available within a given sequence.

**Case Study: Dilution-agnostic comparator with a redundant CAN**

Biosensing faces a bit of a paradox: variation in the concentration of a biomolecule is used to infer disease state, yet there are many non-biological reasons a sample could vary in the concentration of targets. The patient could be more or less hydrated than expected, the sample volume could be inaccurate, or simple statistics could lead to variation in the number of cells obtained. A classic approach to accommodate these uncertainties is the use of an internal standard, something innate to the sample that shouldn't vary with disease condition. For analysis of RNA, this internal standard is typically a "housekeeping" gene, a transcript so fundamental to growth of a cell (controlling cytoskeleton or cell membrane metabolism, for example) that its concentration reflects only the number of cells analysed rather than their state. The concentration of truly interesting gene transcripts can be compared to the housekeeping gene(s) to produce a more reliable measure of their deviation from normality. Typically, these are either separate PCR reactions performed in parallel or multiple probes within a single reaction; in either case, this becomes very time-, resource-, and sample-intensive if, say, 16 genes of interest and 5 housekeeping genes are needed, with extensive post-processing required. Redundant targeting of indirect CANs offers a way to perform this calculation explicitly, on the molecular level, so the reported signal reflects the relative concentrations of two genes regardless of their absolute concentrations (Figure 15).

**Further Applications**

Two and a half decades of gene expression analysis have identified dozens or even hundreds of potentially diagnostic expression signatures. RT-PCR, Nanostring, and RNA-seq analyses have similarly produced useful insight. In addition to the signatures described above, the following reports present promising candidates for adaptation of the CAN platform:

- Sepsis antibiotic decision model, 11 Genes
- Breast cancer chemeotherapy decision, 70 Genes
- Breast cancer diagnosis, 21 Genes
- Bloodstream candidiasis, 40 Genes
- Bovine Tuberculosis, 15 Genes
- Bovine Mastitis, 15 Genes

The CAN platform could also solve a problem in bioprocessing, the industrial use of synthetic cells to produce a product such as a drug or to break down a material, such as petrochemicals or greenhouse gases. This involves coordination of several synthetic and natural gene systems and may involve more than one population of engineered cells grown simultaneously. Currently, system performance is verified through RNA-seq or microarrays, which are expensive and time consuming. Alternatively, engineers include genes that produce "reporter" in conjunction with the desired product. However, doing so consumes raw materials that otherwise could be used for production of the desired compound while putting greater stress and uncertainty on the engineered cells. The CAN architecture would provide a way to get a snapshot of the transcriptional activity of all relevant genes simultaneously. A CAN could be designed to produce one colour if all genes are operating within a pre-specified window, but if any gene is above or below that window a different colour is produced.

**Conclusion**

Competitive Amplification Networks offer the potential to perform powerful calculations on a molecular level, explicitly performing analyte pattern recognition within a biosensor architecture. By leveraging the ubiquitous DNA amplification technology PCR, the CAN platform is fast, inexpensive, and, above all, easy to use. The data-driven nature of the technology is both its strength and its weakness: an adequate dataset is all that's necessary to design and test a CAN but acquiring a sufficiently robust dataset may be a lengthy challenge. Fortunately, extensive literature exists on the topic, much with open-access data. The results here only begin to describe the potential of the technology; more work is needed to establish rules and algorithms for network design, target sequence selection, and experimental validation. As it is early stages yet, creating a CAN is a very manual process, but the whole process could become simplified through integration of modelling and automated instrumentation to iterate on the cycle of i) design a network for an application, ii) select competitor and primer sequences, iii) robotically assemble the competitors from building block oligos, iv) run an appropriate number of reactions, v) compare the results against the predicted response, vi) adjust the network or sequence design. Such a close-loop development system will allow rapid deployment of the CAN platform for a wide range of biosensing applications.

**Claims**

1. A method of amplifying at least a first and at least a second target polynucleotide in a sample, wherein the method comprises:
   providing:

   a) a sample potentially comprising the at least a first and at least a second target polynucleotides
   b) a first tuned competitor polynucleotide and a second tuned competitor polynucleotide;
   c) at least a first primer wherein at least the first primer is capable of hybridising to:

   a first target polynucleotide in the sample; and
   the first tuned competitor polynucleotide; and

   initiating a primer extension reaction such that the first and second target polynucleotides (if present in the sample) and the first tuned competitor polynucleotide and the second tuned competitor polynucleotide are amplified,
   wherein amplification results in a first target product, a second target product, a first tuned competitor product and a second tuned competitor product.

2. The method according to claim 1 wherein the method comprises providing a second primer, optionally wherein;

a) the second primer is capable of hybridising to the first target polynucleotide, wherein the first and second primer hybridise on opposite strands of the target so as to result in the production of the first target product, optionally a first target polymerase chain reaction (PCR) product;

b) the second primer is capable of hybridising to the first tuned competitor polynucleotide, wherein the first and second primer hybridise on opposite strands of the first tuned competitor polynucleotide so as to result in the production of the first tuned competitor product, optionally first tuned competitor PCR product;

c) the second primer is:

i) capable of hybridising to the first tuned competitor polynucleotide, wherein the first and second primer hybridise on opposite strands of the first tuned competitor polynucleotide so as to result in the production of the first tuned competitor product, optionally first tuned competitor PCR product; and

ii) is capable of hybridising to the second tuned competitor polynucleotide and initiating a primer extension reaction such that the second tuned competitor polynucleotide is amplified so as to result in the production of the second tuned competitor product, optionally in combination with a further primer wherein the second and further primer hybridise on opposite strands of the second tuned competitor polynucleotide so as to result in the production of the second tuned competitor product, optionally a second tuned competitor PCR product,

optionally wherein the second primer is not capable of hybridising to the first target polynucleotide; and/or

d) the second primer is:

i) capable of hybridising to the first target polynucleotide, wherein the first and second primer hybridise on opposite strands of the target so as to result in the production of the first target product, optionally a first target polymerase chain reaction (PCR) product; and

ii) is not capable of hybridising to the first or second tuned competitor polynucleotide

and wherein the method comprises a third primer capable of hybridising to the first and to the second tuned competitor polynucleotide.

3. The method of any one of claims 1 or 2 wherein the amplification kinetics of the first target polynucleotide are not the same as the amplification kinetics of the first tuned competitor polynucleotide, or are not substantially similar to the amplification kinetics of the first tuned competitor polynucleotide,

optionally wherein the number of target product polynucleotides generated is different to the number of tuned competitor product polynucleotides generated, when the initial number of target polynucleotides and the number of tuned competitor polynucleotides prior to primer extension is the same or is substantially the same.

4. The method according to any one of claims 1-3 wherein:

the first tuned competitor product is:

i) at least 5 nucleotides shorter than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the first target product; or

ii) at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product; and/or

the second tuned competitor product is:

i) at least 5 nucleotides shorter than the second target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the second target product; or

ii) at least 5 nucleotides longer than the second target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the second target product; and/or

the difference in GC content of the first target polynucleotide portion to be amplified and the first competitor polynucleotide to be amplified is at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85% or at least 90% or 95%.

5. The method according to any one of claims 1-4 wherein the first tuned competitor amplification product and the second tuned competitor amplification product are detected.

6. The method according to any one of claims 1-5 wherein the first target amplification product and the second target amplification product are not detected.

7. The method of claim 5 or 6 wherein said detection is performed using a probe labelled with a first label, optionally where the label is a fluorophore.

8. The method of any of claims 5-7 wherein each competitor amplification product is detected using the same probe labelled with a first label, optionally labelled with a fluorophore.

9. The method of any of claims 5-7 wherein each competitor amplification product is detected using a different probe labelled with a first label, optionally different probes labelled with a first fluorophore, optionally wherein each probe is labelled with the same label optionally labelled with the same fluorophore.

10. The method according to any one of claims 1-9 wherein the first target amplification product, the second target amplification product, the first tuned competitor amplification product and the second tuned competitor amplification product are detected.

11. The method according to any one of claims 1-9 wherein the method comprises providing one or more probe groups, wherein each probe group comprises at least one probe polynucleotide labelled with a first label and at least one probe polynucleotide labelled with a second label, and wherein neither probe is capable of hybridising to the first target product.

12. The method according to any one of claims 1-11 wherein the method comprises providing one or more probe polynucleotides, wherein each probe polynucleotide is labelled with a fluorophore.

13. The method according to any of claims 7-12 wherein

each probe that is capable of hybridising to a target polynucleotide product that is associated with a positive predictive relationship of a particular state is labelled with the first label, and the corresponding probe that is capable of hybridising to the tuned competitor polynucleotide product is labelled with the second label; and/or

each probe that is capable of hybridising to a target polynucleotide product that is associated with a negative predictive relationship of the particular state is labelled with the second label, and the corresponding probe that is capable of hybridising to the tuned competitor polynucleotide product is labelled with the first label

optionally where there are no probes capable of hybridising to a target polynucleotide.

14. A method of diagnosis or prognosis of a disease or condition in a subject wherein the method comprises the method of any one of claims 1-13.

15. A kit for carrying out the method of any one of claims 1-13, wherein the kit comprises:

a) at least two primers designed to amplify both a target polynucleotide and a competitor polynucleotide so as to produce a target product and a competitor product; and
b) at least a first tuned competitor polynucleotide, wherein said primers in (a) amplify at least one of the at least two tuned polynucleotides and wherein
the first tuned competitor product is:

i) at least 5 nucleotides shorter than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the first target product; or
ii) at least 5 nucleotides longer than the first target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the first target product; and/or

the second tuned competitor product is:

i) at least 5 nucleotides shorter than the second target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides shorter than the second target product; or
ii) at least 5 nucleotides longer than the second target product, optionally at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290 or at least 330 nucleotides longer than the second target product;
and/or

the difference in GC content of the first target polynucleotide portion to be amplified and the first competitor polynucleotide to be amplified is at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85% or at least 90% or 95%.

## Figure 1 (part 1 of 2)

# Figure 1 (part 2 of 2)

Figure 2 (part 1 of 4)

A

Figure 2 (part 2 of 4)

Figure 2 (part 3 of 4)

Figure 2 (part 4 of 4)

Figure 3

Figure 4 (part 1 of 3 )

Figure 4 (part 2 of 3 )

Figure 4 (part 3 of 3 )

Figure 5

Figure 6 (part 1 of 2)

Figure 6 (part 2 of 2)

Figure 7 (part 1 of 2)

| Dye | Ex. | Em. | Alternative |
|---|---|---|---|
| 6-FAM (NHS Ester) | 496 | 516 | N/A |
| 6-FAM (Fluorescein) | 495 | 520 | N/A |
| Fluorescein dT | 495 | 520 | N/A |
| Cy3TM | 550 | 564 | TYE™ 563 |
| TAMRATM | 559 | 583 | N/A |
| JOE (NHS Ester) | 529 | 555 | MAX (NHS Ester) |
| Cy5TM | 648 | 668 | TYE™ 665 |
| TAMRA (NHS Ester) | 559 | 583 | N/A |
| MAX (NHS Ester) | 531 | 556 | JOE (NHS Ester) |
| TETTM | 522 | 539 | N/A |
| Cy5.5TM | 685 | 706 | TYE 705 |
| ROX (NHS Ester) | 588 | 608 | ATTOTM Rho101 (NHS Ester) |
| TYE™ 563 | 549 | 563 | Cy3TM |
| Yakima Yellow® | 524 | 551 | N/A |
| HEX | 538 | 555 | N/A |
| TEX 615 | 596 | 613 | Texas Red®-X (NHS Ester) |
| TYE™ 665 | 645 | 665 | Cy5TM |
| TYE 705 | 686 | 704 | Cy5.5TM |
| ATTOTM 488 (NHS Ester) | 502 | 522 | Alexa Fluor® 488 (NHS Ester) |
| ATTOTM 532 (NHS Ester) | 534 | 554 | N/A |
| ATTOTM 550 (NHS Ester) | 560 | 575 | N/A |
| ATTOTM 565 (NHS Ester) | 570 | 591 | N/A |
| ATTOTM Rho101 (NHS Ester) | 592 | 609 | ROX (NHS Ester) |

# Figure 7 (part 2 of 2)

| Dye | Ex. | Em. | Alternative |
|---|---|---|---|
| ATTOTM 590 (NHS Ester) | 593 | 622 | Alexa Fluor® 594 (NHS Ester) |
| ATTOTM 633 (NHS Ester) | 635 | 653 | N/A |
| ATTOTM 647N (NHS Ester) | 649 | 662 | Alexa Fluor® 647 (NHS Ester) |
| Alexa Fluor® 488 (NHS Ester) | 492 | 517 | ATTOTM 488 (NHS Ester) |
| Alexa Fluor® 532 (NHS Ester) | 527 | 553 | N/A |
| Alexa Fluor® 546 (NHS Ester) | 556 | 571 | N/A |
| Alexa Fluor® 594 (NHS Ester) | 584 | 616 | ATTOTM 590 (NHS Ester) |
| Alexa Fluor® 647 (NHS Ester) | 650 | 670 | ATTOTM 647N (NHS Ester) |
| Alexa Fluor® 660 (NHS Ester) | 661 | 691 | N/A |
| Alexa Fluor® 750 (NHS Ester) | 753 | 782 | N/A |
| 5' IRDye® 700 | 684 | 702 | N/A |
| 5' IRDye® 800 | 791 | 809 | N/A |
| 5' IRDye® 800CW (NHS Ester) | 767 | 791 | N/A |
| Rhodamine GreenTM-X (NHS Ester) | 504 | 531 | N/A |
| Rhodamine RedTM-X (NHS Ester) | 574 | 594 | N/A |
| 5-TAMRA TM (Azide) | 546 | 579 | N/A |
| 6-FAM (Azide) | 496 | 516 | N/A |
| Texas Red®-X (NHS Ester) | 598 | 617 | TEX 615 |
| Lightcycler® 640 (NHS Ester) | 620 | 635 | N/A |
| Dy 750 (NHS Ester) | 747 | 776 | N/A |

Figure 8 (part 1 of 2)

Figure 8 (part 2 of 2)

Figure 9

# Figure 10 (part 1 of 2)

# Figure 10 (part 2 of 2)

OR MOTIF

FULL
XOR MOTIF

Figure 11 (part 1 of 2)

Figure 11 (part 2 of 2)

Figure 12

Figure 13 (1 of 3)

A

WT

blocker

blocker

SNP

variant
site

REF<sup>H</sup>

HEX ——— Q

REF<sup>F</sup>

FAM ——— Q

Figure 13 (2 of 3)

Figure 13 (3 of 3)

C

# Figure 14 (part 1 of 2)

# Figure 14 (part 2 of 2)

Figure 15 (part 1 of 2)

A

B

Figure 15 (part 2 of 2)

C

D

Figure 16 (part 1 of 8)

Figure 16 (part 2 of 8)

Figure 16 (part 3 of 8)

Figure 16 (part 4 of 8)

Figure 16 (part 5 of 8)

Figure 16 (part 6 of 8)

Figure 16 (part 7 of 8)

Figure 16 (part 8 of 8)

Figure 17 (part 1 of 8)

EP 4 748 945 A2

| Name | F primer | Core | R primer (complement) | Full Seq | GC (core) | BP (core) | GC (global) | BP (global) | Observed amplification rate |
|---|---|---|---|---|---|---|---|---|---|
| **TMCC1 Targets** | | | | | | | | | |
| WT | GCTATTGCTG GGATTTTGAG G (SEQ ID NO: 1) | AGCTTTTCCTAAAAGGA TTGTACACCTTAGAAGT GCTTAAGGAAGAGTGAT GAAGATAG**GCATGAAG CCTTCGTCTCACAGCT** *GCATG* (SEQ ID NO: 2) | CGTAGTCACTGTT GAAGCAAATG (SEQ ID NO: 3) | GCTATTGCTGGGATTTTGAGGAG CTTTTCCTAAAAGGATTGTACACC TTAGAAGTGCTTAAGGAAGAGTGA TGAAGATAGGCATGAAGCCTTCGT CTCACAGCTGCATGCGTAGTCACT GTTGAAGCAAATG (SEQ ID NO: 4) | 43% | 88 | 44% | 132 | |
| ISO | GCTATTGCTG GGATTTTGAG G (SEQ ID NO: 1) | TCTGTATCCCAAGTGTT CAGAGCTTCATATT**GGC AACGCTCTGGTAACCT CTCTA***AATTGATAGTTC CGATTGCAACTTGACGT CTAG* (SEQ ID NO: 5) | CGTAGTCACTGTT GAAGCAAATG (SEQ ID NO: 3) | GCTATTGCTGGGATTTTGAGGTCT GTATCCCAAGTGTTCAGAGCTTCA TATTGGCAACGCTCTGGTAACCTC TCTAAATTGATAGTTCCGATTGCA ACTTGACGTCTAGCGTAGTCACTG TTGAAGCAAATG (SEQ ID NO: 6) | 43% | 88 | 44% | 132 | 0.83 |
| GC15 | GCTATTGCTG GGATTTTGAG G (SEQ ID NO: 1) | TTTAATAAATAAATTAAA TATTATATAAATTA**GGC AACGCTCTGGTAACCT CTCTA***TATTATATTAAA TTATTAAATTAATAATTA TA* (SEQ ID NO: 7) | CGTAGTCACTGTT GAAGCAAATG (SEQ ID NO: 3) | GCTATTGCTGGGATTTTGAGGTTT AATAAATAAATTAAATATTATATAA ATTAGGCAACGCTCTGGTAACCTC TCTATATTATATTAAATTATTAAAT TAATAATTATACGTAGTCACTGTT GAAGCAAATG (SEQ ID NO: 8) | 15% | 88 | 25% | 132 | 0.83 |
| GC25 | GCTATTGCTG GGATTTTGAG G (SEQ ID NO: 1) | TGTAATATTAACAAGTA ATAAAGAAATATATA**GG CAACGCTCTGGTAACC TCTCTA***TTTATTCAATT TAATGATTACCTTTATTA TCT* (SEQ ID NO: 9) | CGTAGTCACTGTT GAAGCAAATG (SEQ ID NO: 3) | GCTATTGCTGGGATTTTGAGGTGT AATATTAACAAGTAATAAAGAAAT ATATAGGCAACGCTCTGGTAACCT CTCTATTTATTCAATTTAATGATTA CCTTTATTATCTCGTAGTCACTGTT GAAGCAAATG (SEQ ID NO: 10) | 25% | 88 | 32% | 132 | 1.00 |
| GC35 | GCTATTGCTG GGATTTTGAG G (SEQ ID NO: 1) | AACGTAGCAATATTGAA TTCTAGATTATACGA**GG CAACGCTCTGGTAACC TCTCTA***TTATTTAAGCT ATCATACTCTAGTGTTT CGTT* (SEQ ID NO: 11) | CGTAGTCACTGTT GAAGCAAATG (SEQ ID NO: 3) | GCTATTGCTGGGATTTTGAGGAAC GTAGCAATATTGAATTCTAGATTA TACGAGGCAACGCTCTGGTAACCT CTCTATTATTTAAGCTATCATACTC TAGTGTTTCGTTCGTAGTCACTGT TGAAGCAAATG (SEQ ID NO: 12) | 35% | 88 | 39% | 132 | 0.93 |

Figure 17 (part 2 of 8)

EP 4 748 945 A2

| Name | F primer | Core | R primer (complement) | Full Seq | GC (core) | BP (core) | GC (global) | BP (global) | Observed amplification rate |
|---|---|---|---|---|---|---|---|---|---|
| **TMCC1 Targets** | | | | | | | | | |
| GC55 | GCTATTGCT GGGATTTTG AGG (SEQ ID NO: 1) | TGCGTCGATGCTGTG TGAGGTGAAGACCTA GAGGCAACGCTCTG GTAACCTCTCTACAC GCTTAGCAACGCTGC ATGTCGAGTCTCCA (SEQ ID NO: 13) | CGTAGTCACTGT TGAAGCAAATG (SEQ ID NO: 3) | GCTATTGCTGGGATTTTGAGGT GCGTCGATGCTGTGTGAGGTG AAGACCTAGAGGCAACGCTCTG GTAACCTCTCTACACGCTTAGC AACGCTGCATGTCGAGTCTCCA CGTAGTCACTGTTGAAGCAAAT G (SEQ ID NO: 14) | 55% | 88 | 52% | 132 | 0.74 |
| GC65 | GCTATTGCT GGGATTTTG AGG (SEQ ID NO: 1) | TGCGTCGCGGCTGTG GGAGGTGCGGACCTA GAGGCAACGCTCTG GTAACCTCTCTACAC GCTTAGCGCCGCTGC CTGTCGACCGTCCA (SEQ ID NO: 15) | CGTAGTCACTGT TGAAGCAAATG (SEQ ID NO: 3) | GCTATTGCTGGGATTTTGAGGT GCGTCGCGGCTGTGGGGAGGTG CGGACCTAGAGGCAACGCTCT GGTAACCTCTCTACACGCTTAG CGCCGCTGCCTGTCGACCGTC CACGTAGTCACTGTTGAAGCAA ATG (SEQ ID NO: 16) | 65% | 88 | 58% | 132 | 0.62 |
| GC75 | GCTATTGCT GGGATTTTG AGG (SEQ ID NO: 1) | GCGCGGCGGTGGAG CGCTCGCGGTGGTGC GCTGGCAACGCTCT GGTAACCTCTCTATG GCGCGTGGCCACGCT CCCGCGCGACGGCCG (SEQ ID NO: 17) | CGTAGTCACTGT TGAAGCAAATG (SEQ ID NO: 3) | GCTATTGCTGGGATTTTGAGGG CGCGGCGGTGGAGCGCTCGCG GTGGTGCGCTGGCAACGCTCT GGTAACCTCTCTATGGCGCGTG GCCACGCTCCCGCGCGACGGC CGCGTAGTCACTGTTGAAGCAA ATG (SEQ ID NO: 18) | 75% | 88 | 65% | 132 | 0.67 |
| GC80 | GCTATTGCT GGGATTTTG AGG (SEQ ID NO: 1) | CGGGCGCGCCGCGC GCGACGCGCGTCCCG TCCGGCAACGCTCT GGTAACCTCTCTAAC CCGCACGCCGGCGCC GCGCGCCCGAGCGCA (SEQ ID NO: 19) | CGTAGTCACTGT TGAAGCAAATG (SEQ ID NO: 3) | GCTATTGCTGGGATTTTGAGGC GGGCGCGCCGCGCGACGC GCGTCCCGTCCGGCAACGCTCT GGTAACCTCTCTAACCCGCACG CCGGCGCCGCGCGCCCGAGCG CACGTAGTCACTGTTGAAGCAA ATG (SEQ ID NO: 20) | 80% | 88 | 68% | 132 | 0.73 |
| GC85 | GCTATTGCT GGGATTTTG AGG (SEQ ID NO: 1) | GCGGCCGCGCGCCCG GCGGCGCGTGGGCC GGCGGCAACGCTCT GGTAACCTCTCTAG GCGGCGGCGCACCGC GCGGCGCGGGCGGC GC (SEQ ID NO: 21) | CGTAGTCACTGT TGAAGCAAATG (SEQ ID NO: 3) | GCTATTGCTGGGATTTTGAGGG CGGCCGCGCGCCCGGCGGCGC GTGGGCCGGCGGCAACGCTCT GGTAACCTCTCTAGGCGGCGG CGCACCGCGCGGCGCGGGCG GCGCCGTAGTCACTGTTGAAGC AAATG (SEQ ID NO: 22) | 85% | 88 | 72% | 132 | |

Figure 17 (part 3 of 8)

EP 4 748 945 A2

| Name | F primer | Core | R primer (complement) | Full Seq | GC (core) | BP (core) | GC (global) | BP (global) | Observed amplification rate |
|---|---|---|---|---|---|---|---|---|---|
| **TMCC1 Targets** | | | | | | | | | |
| BP240 | GCTATTGCTG GGATTTTGAG G (SEQ ID NO: 1) | ACCACGGTAATTACTGT TAGACTGGTGGGTATA AACTTCGTTATTTGGAT TGGAATTGTTGAGCCCT ACCTGACTCTGTATCCC AAGTGTTCTCTGCTTCA TATTGGCAACGCTCTG GTAACCTCTCTAAATTG ATAGTTCCGATTGCAAC TTGACGTCTAGCCCGTA TAAATAGCCGGTCTAAA CAGCGATGAAATTTCTG TAGAATCAACTAAATTT TCCGTTCAACGGATCCT CTGC (SEQ ID NO: 23) | CGTAGTCACTGTT GAAGCAAATG (SEQ ID NO: 3) | GCTATTGCTGGGATTTTGAGGAC CACGGTAATTACTGTTAGACTGGT GGGTATAAACTTCGTTATTTGGAT TGGAATTGTTGAGCCCTACCTGA CTCTGTATCCCAAGTGTTCTCTGC TTCATATTGGCAACGCTCTGGTAA CCTCTCTAAATTGATAGTTCCGAT TGCAACTTGACGTCTAGCCCGTAT AAATAGCCGGTCTAAACAGCGAT GAAATTTCTGTAGAATCAACTAAA TTTTCCGTTCAACGGATCCTCTGC CGTAGTCACTGTTGAAGCAAATG (SEQ ID NO: 24) | 42% | 240 | 43% | 284 | 0.58 |
| BP200 | GCTATTGCTG GGATTTTGAG G (SEQ ID NO: 1) | AAGATCTGTTCATGCGT TCGTTATTTGGATTGGA ATTGTTGAGCCCTACCT GACTCTGTATCCCAAGT GTTCTCTGCTTCATATT **GGCAACGCTCTGGTA ACCTCTCTA***AATTGAT AGTTCCGATTGCAACTT GACGTCTAGCCCGTATA AATAGCCGGTCTAAACA GCGATGAAATTTCGTCC GAACAAGTTTCAACTT* (SEQ ID NO: 25) | CGTAGTCACTGTT GAAGCAAATG (SEQ ID NO: 3) | GCTATTGCTGGGATTTTGAGGAA GATCTGTTCATGCGTTCGTTATTT GGATTGGAATTGTTGAGCCCTAC CTGACTCTGTATCCCAAGTGTTCT CTGCTTCATATTGGCAACGCTCTG GTAACCTCTCTAAATTGATAGTTC CGATTGCAACTTGACGTCTAGCC CGTATAAATAGCCGGTCTAAACA GCGATGAAATTTCGTCCGAACAA GTTTCAACTTCGTAGTCACTGTTG AAGCAAATG (SEQ ID NO:26) | 43% | 200 | 43% | 244 | 0.63 |

110

Figure 17 (part 4 of 8)

| Name | F primer | Core | R primer (complement) | Full Seq | GC (core) | BP (core) | GC (global) | BP (global) | Observed amplification rate |
|---|---|---|---|---|---|---|---|---|---|
| **TMCC1 Targets** | | | | | | | | | |
| BP160 | GCTATTGCTG GGATTTTGAG G (SEQ ID NO: 1) | CGTTATTTGGATTGGAA TTGTTGAGCCCTACCT GACTCTGTATCCCAAGT GTTCTCTGCTTCATATT **GGCAACGCTCTGGTA ACCTCTCTA***AATTGAT AGTTCCGATTGCAACTT GACGTCTAGCCCGTAT AAATAGCCGGTCTAAA CAGCGATGAAATTTC* (SEQ ID NO: 27) | CGTAGTCACTGT TGAAGCAAATG (SEQ ID NO: 3) | GCTATTGCTGGGATTTTGAGGCG TTATTTGGATTGGAATTGTTGAG CCCTACCTGACTCTGTATCCCAA GTGTTCTCTGCTTCATATTGGCAA CGCTCTGGTAACCTCTCTAAATT GATAGTTCCGATTGCAACTTGAC GTCTAGCCCGTATAAATAGCCGG TCTAAACAGCGATGAAATTTCCG TAGTCACTGTTGAAGCAAATG (SEQ ID NO: 28) | 43% | 162 | 44% | 206 | 0.66 |
| BP55 | GCTATTGCTG GGATTTTGAG G (SEQ ID NO: 1) | AGGCAACGCTCTGGTA ACCTCTCTAATTGATAG TTCCGATACGTGCAACT TGTCT (SEQ ID NO: 29) | CGTAGTCACTGT TGAAGCAAATG (SEQ ID NO: 3) | GCTATTGCTGGGATTTTGAGGAG GCAACGCTCTGGTAACCTCTCTA ATTGATAGTTCCGATACGTGCAA CTTGTCTCGTAGTCACTGTTGAA GCAAATG (SEQ ID NO: 30) | 45% | 55 | 45% | 99 | 0.90 |
| BP40 | GCTATTGCTG GGATTTTGAG G (SEQ ID NO: 1) | AGGCAACGCTCTGGTA ACCTCTCTAATTGATAG ATTCCAG (SEQ ID NO: 31) | CGTAGTCACTGT TGAAGCAAATG (SEQ ID NO: 3) | GCTATTGCTGGGATTTTGAGGAG GCAACGCTCTGGTAACCTCTCTA ATTGATAGATTCCAGCGTAGTCA CTGTTGAAGCAAATG (SEQ ID NO: 32) | 45% | 40 | 45% | 84 | 0.95 |
| BP30 | GCTATTGCTG GGATTTTGAG G (SEQ ID NO: 1) | AGGCAACGCTCTGGTA ACCTCTCTAATATG (SEQ ID NO: 33) | CGTAGTCACTGT TGAAGCAAATG (SEQ ID NO: 3) | GCTATTGCTGGGATTTTGAGGAG GCAACGCTCTGGTAACCTCTCTA ATATGCGTAGTCACTGTTGAAGC AAATG (SEQ ID NO: 34) | 47% | 30 | 46% | 74 | 0.97 |
| BP15 | GCTATTGCTG GGATTTTGAG G (SEQ ID NO: 1) | TTGCAACTTGACGTC (SEQ ID NO: 35) | CGTAGTCACTGT TGAAGCAAATG (SEQ ID NO: 3) | GCTATTGCTGGGATTTTGAGGTT GCAACTTGACGTCCGTAGTCACT GTTGAAGCAAATG (SEQ ID NO: 36) | 47% | 15 | 46% | 59 | |

Figure 17 (part 5 of 8)

EP 4 748 945 A2

| Name | F primer | Core | R primer (complement) | Full Seq | GC (core) | BP (core) | GC (global) | BP (global) | Observed amplification rate |
|---|---|---|---|---|---|---|---|---|---|
| **ARG1 Targets** | | | | | | | | | |
| WT | CGCCAAGTC CAGAACCATA G (SEQ ID NO: 37) | GGATTATTGGAGCTCC TTTCTCAAAGGGACAG CCACGAGGAGGGGTG GAAGAAGGCCCTACAG TATT*GAGAAAGGCTG GTCTGCTTGAGAAAC TTAAAGAACAAGAGT* (SEQ ID NO: 38) | GTGATGTGAAGG ATTATGGGGA (SEQ ID NO: 39) | CGCCAAGTCCAGAACCATAGGGA TTATTGGAGCTCCTTTCTCAAAG GGACAGCCACGAGGAGGGGTGG AAGAAGGCCCTACAGTATTGAGA AAGGCTGGTCTGCTTGAGAAACT TAAAGAACAAGAGTGTGATGTGA AGGATTATGGGGA (SEQ ID NO: 40) | 48% | 108 | 49% | 150 | |
| ISO | CGCCAAGTC CAGAACCATA G (SEQ ID NO: 37) | GGATTATTGGAGCTCC TTTCTCAAAGGGACAG CCACGAGGAGGGGTG GAAGAAGGCCCTACAG TATT**GGCAACGCTCTG GTAACCTCTCTA***AATT AAAGAACAAGAGT* (SEQ ID NO: 41) | GTGATGTGAAGG ATTATGGGGA (SEQ ID NO: 39) | CGCCAAGTCCAGAACCATAGGGA TTATTGGAGCTCCTTTCTCAAAG GGACAGCCACGAGGAGGGGTGG AAGAAGGCCCTACAGTATTGGCA ACGCTCTGGTAACCTCTCTAAATT AAAGAACAAGAGTGTGATGTGAA GGATTATGGGGA (SEQ ID NO: 42) | 48% | 108 | 49% | 150 | |
| BP88_GC43 | CGCCAAGTC CAGAACCATA G (SEQ ID NO: 37) | TCTGTATCCCAAGTGTT CAGAGCTTCATATT**GG CAACGCTCTGGTAAC CTCTCTA***AATTGATAG TTCCGATTGCAACTTGA CGTCTAG* (SEQ ID NO: 43) | GTGATGTGAAGG ATTATGGGGA (SEQ ID NO: 39) | CGCCAAGTCCAGAACCATAGTCT GTATCCCAAGTGTTCAGAGCTTC ATATTGGCAACGCTCTGGTAACC TCTCTAAATTGATAGTTCCGATTG CAACTTGACGTCTAGGTGATGTG AAGGATTATGGGGA (SEQ ID NO: 44) | 43% | 88 | 45% | 130 | |
| BP30_GC43 | CGCCAAGTC CAGAACCATA G (SEQ ID NO: 37) | GGCAACGCTCTGGTAA CCTCTCTA*AATTAA* (SEQ ID NO: 45) | GTGATGTGAAGG ATTATGGGGA (SEQ ID NO: 39) | CGCCAAGTCCAGAACCATAGGGC AACGCTCTGGTAACCTCTCTAAAT TAAGTGATGTGAAGGATTATGGG GA (SEQ ID NO: 46) | 43% | 30 | 47% | 72 | |
| BP108_GC25 | CGCCAAGTC CAGAACCATA G (SEQ ID NO: 37) | TAATTATTATAGCTAAT TTCTCAAATTTACAGAA ACGAATAGAAGTTTAA GAATTAAATACAGTATT **GG** (SEQ ID NO: 47) | GTGATGTGAAGG ATTATGGGGA (SEQ ID NO: 39) | CGCCAAGTCCAGAACCATAGTAA TTATTATAGCTAATTTCTCAAATT TACAGAAACGAATAGAAGTTTAA GAATTAAATACAGTATTGGGTGA TGTGAAGGATTATGGGGA (SEQ ID NO: 48) | 22% | 69 | 32% | 111 | |

Figure 17 (part 6 of 8)

EP 4 748 945 A2

| Name | F primer | Core | R primer (complement) | Full Seq | GC (core) | BP (core) | GC (global) | BP (global) | Observed amplification rate |
|---|---|---|---|---|---|---|---|---|---|
| **GBP6 Targets** | | | | | | | | | |
| WT | GCAAGAACCA AGACCCTCAG (SEQ ID NO: 49) | GGAGGGAATCACAGTC AC**TGGGAATCGTCTG GGAACTCTGG**CAGTG ACTTATGTAGAGGCCAT CAACAGTGGAG (SEQ ID NO: 50) | CAGTGCCTTGTCT GGAGAAT (SEQ ID NO: 51) | GCAAGAACCAAGACCCTCAGGGA GGGAATCACAGTCACTGGGAATC GTCTGGGAACTCTGGCAGTGACT TATGTAGAGGCCATCAACAGTGG AGCAGTGCCTTGTCTGGAGAAT (SEQ ID NO: 52) | 53% | 74 | 53% | 114 | |
| ISO | GCAAGAACCA AGACCCTCAG (SEQ ID NO: 49) | GGAGGGAATCACAGTC AC**GCATGAAGCCTTC GTCTCACAGCT**CGTGA CTTATGTAGAGGCCATC AACAGTGGAG (SEQ ID NO: 53) | CAGTGCCTTGTCT GGAGAAT (SEQ ID NO: 51) | GCAAGAACCAAGACCCTCAGGGA GGGAATCACAGTCACGCATGAAG CCTTCGTCTCACAGCTCGTGACTT ATGTAGAGGCCATCAACAGTGGA GCAGTGCCTTGTCTGGAGAAT (SEQ ID NO: 54) | 53% | 74 | 53% | 114 | |
| BP88_GC43 | GCAAGAACCA AGACCCTCAG (SEQ ID NO: 49) | ATCTGTATCCCAAGTGT TCAGAGCTTCATATTGC ATGAAGCCTTCGTCTCA CAGCTATTGATAGTTCC GATTGCAACTTGACGTC TAG (SEQ ID NO: 55) | CAGTGCCTTGTCT GGAGAAT (SEQ ID NO: 51) | GCAAGAACCAAGACCCTCAGATC TGTATCCCAAGTGTTCAGAGCTTC ATATTGCATGAAGCCTTCGTCTCA CAGCTATTGATAGTTCCGATTGCA ACTTGACGTCTAGCAGTGCCTTG TCTGGAGAAT (SEQ ID NO: 56) | 43% | 88 | 46% | 128 | |
| BP30_GC53 | GCAAGAACCA AGACCCTCAG (SEQ ID NO: 49) | GCATGAAGCCTTCGTCT CACAGCT*CGTGAT* (SEQ ID NO: 57) | CAGTGCCTTGTCT GGAGAAT (SEQ ID NO: 51) | GCAAGAACCAAGACCCTCAGGCA TGAAGCCTTCGTCTCACAGCTCG TGATCAGTGCCTTGTCTGGAGAA T (SEQ ID NO: 58) | 53% | 30 | 53% | 70 | |
| **EGFR Targets** | | | | | | | | | |
| WT | GCAGCATGTC AAGATCACAG ATT (SEQ ID NO: 59) | TTGGGCTGGCCAAACT GCTGGGTGCGGAAGAG AAAGAATACCATGCA (SEQ ID NO: 60) | GAAGGAGGCAAA GTAAGGAGGT (SEQ ID NO: 61) | GCAGCATGTCAAGATCACAGATT TTGGGCTGGCCAAACTGCTGGGT GCGGAAGAGAAAGAATACCATGC AGAAGGAGGCAAAGTAAGGAGGT (SEQ ID NO: 62) | 53% | 47 | 50% | 92 | |

Figure 17 (part 7 of 8)

EP 4 748 945 A2

| Name | F primer | Core | R primer (complement) | Full Seq | GC (core) | BP (core) | GC (global) | BP (global) | Observed amplification rate |
|---|---|---|---|---|---|---|---|---|---|
| SNP | GCAGCATGTCAAGATCACAGATT (SEQ ID NO: 59) | TTGGGCGGGCCAAACTGCTGGGTGCGGAAGAGAAAGAATACCATGCA (SEQ ID NO: 63) | GAAGGAGGCAAAGTAAGGAGGT (SEQ ID NO: 61) | GCAGCATGTCAAGATCACAGATTTTTGGGCGGGCCAAACTGCTGGGTGCGGAAGAGAAAGAATACCATGCAGAAGGAGGCAAAGTAAGGAGGT (SEQ ID NO: 64) | 55% | 47 | 51% | 92 | |
| A | CATTTGCTTCAACAGTGACTACG (SEQ ID NO: 65) | AAGATCTGTTCATGCGTTCGTTATTTGGATTGGAATTGTTGAGCCCTACCTGACTCTGTATCCCAAGTGTTCTCTGCTTCATATTGCATGAAGCCTTCGTCTCACAGCTAATTGATAGTTCCGATTGCAACTTGACGTCTAGCCCGTATAAATAGCCGGTCTAAACAGCGATGAAATTTCGTCCGAACAAGTTTCAACTT (SEQ ID NO: 66) | GAAGGAGGCAAAGTAAGGAGGT (SEQ ID NO: 61) | CATTTGCTTCAACAGTGACTACGAAGATCTGTTCATGCGTTCGTTATTTGGATTGGAATTGTTGAGCCCTACCTGACTCTGTATCCCAAGTGTTCTCTGCTTCATATTGCATGAAGCCTTCGTCTCACAGCTAATTGATAGTTCCGATTGCAACTTGACGTCTAGCCCGTATAAATAGCCGGTCTAAACAGCGATGAAATTTCGTCCGAACAAGTTTCAACTTGAAGGAGGCAAAGTAAGGAGGT (SEQ ID NO: 67) | 43% | 200 | 43% | 245 | |
| B | GCTATTGCTGGGATTTTGAGG (SEQ ID NO: 68) | AAGATCTGTTCATGCGTTCGTTATTTGGATTGGAATTGTTGAGCCCTACCTGACTCTGTATCCCAAGTGTTCTCTGCTTCATATTGGCAACGCTCTGGTAACCTCTCTAAATTGATAGTTCCGATTGCAACTTGACGTCTAGCCCGTATAAATAGCCGGTCTAAACAGCGATGAAATTTCGTCCGAACAAGTTTCAACTT (SEQ ID NO: 69) | CGTAGTCACTGTTGAAGCAAATG (SEQ ID NO: 70) | GCTATTGCTGGGATTTTGAGGAGATCTGTTCATGCGTTCGTTATTTGGATTGGAATTGTTGAGCCCTACCTGACTCTGTATCCCAAGTGTTCTCTGCTTCATATTGGCAACGCTCTGGTAACCTCTCTAAATTGATAGTTCCGATTGCAACTTGACGTCTAGCCCGTATAAATAGCCGGTCTAAACAGCGATGAAATTTCGTCCGAACAAGTTTCAACTTCGTAGTCACTGTTGAAGCAAATG (SEQ ID NO: 71) | 43% | 200 | 43% | 244 | |

# Figure 17 (part 8 of 8)

| Sequence | SEQ ID NO |
|---|---|
| **Primers** | |
| GCTATTGCTGGGATTTTGAGG | SEQ ID NO: 1 |
| GCAAGAACCAAGACCCTCAG | SEQ ID NO: 49 |
| ATTCTCCAGACAAGGCACTG | SEQ ID NO: 72 |
| CATTTGCTTCAACAGTGACTACG | SEQ ID NO: 65 |
| CGCCAAGTCCAGAACCATAG | SEQ ID NO: 37 |
| TCCCCATAATCCTTCACATCAC | SEQ ID NO: 73 |
| | |
| GCAGCATGTCAAGATCACAGATT | SEQ ID NO: 59 |
| ACCTCCTTACTTTGCCTCCTTC | SEQ ID NO: 74 |
| CATTTGCTTCAACAGTGACTACG | SEQ ID NO: 65 |
| GCTATTGCTGGGATTTTGAGG | SEQ ID NO: 1 |
| | |
| **Blockers** | |
| GATCACAGATTTTGGGCTGGCCAAACATAA-C3 | SEQ ID NO: 75 |
| GATCACAGATTTTGGGCGGGCCAAACATAA-C3 | SEQ ID NO: 76 |
| | |
| **Probes** | |
| /56-FAM/AGCTGTGAG/Zen/ACGAAGGCTTCATGC/3IABkFQ/ | SEQ ID NO: 77 |
| /5HEX/TAGAGAGGT/ZEN/TACCAGAGCGTTGCC/3IABkFQ/ | SEQ ID NO: 78 |
| /56-FAM/AGTTTCTCA/Zen/AGCAGACCAGCCTTTCTC/3IABkFQ/ | SEQ ID NO: 79 |
| /56-HEX/CCAGAGTTC/Zen/CCAGACGATTCCCA/3IABkFQ/ | SEQ ID NO: 80 |

Figure 18

Figure 19

Figure 20

Figure 21 (part 1 of 3)

# Figure 21 (part 2 of 3)

# Figure 21 (part 3 of 3)

Figure 22 (part 1 of 3)

Figure 22 (part 2 of 3)

Figure 22 (part 3 of 3)

# Figure 23 (part 1 of 9)

| | Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|---|
| 0 | S044.0 | 16 | 0.4375 | dye | 24.65853 | -7.52511 | 0.111405748 | 1.01650026 | 1.393834061 | 3.55E-05 | FP004 | 81 | RP004 | 88 | - | 262 |
| 1 | S044.1 | 30 | 0.266667 | dye | 21.86603 | -7.07723 | 0.096597868 | 1.076087425 | 0.897247676 | 3.67E-05 | FP004 | 81 | RP004 | 88 | - | 267 |
| 2 | S036.3 | 30 | 0.433333 | dye | 26.39468 | -7.08113 | 0.150006115 | 0.891669668 | 1.480879929 | 0.0002941435 | FP002 | 82 | RP002 | 89 | - | 132 |
| 3 | S044.2 | 30 | 0.466667 | dye | 25.96957 | -7.38082 | 0.133982444 | 0.942177802 | 1.757524886 | 0.0003938052 | FP004 | 81 | RP004 | 88 | - | 195 |
| 4 | S044.3 | 30 | 0.666667 | dye | 26.39147 | -5.16687 | 0.246654083 | 0.650432977 | 1.131530443 | 0.0013010422 | FP004 | 81 | RP004 | 88 | - | 242 |
| 5 | S057.6.2 | 33 | 0.393939 | dye | 21.95237 | -3.97314 | 0.290898538 | 0.597302528 | 0.819884373 | 0.0012879 | FP003 | 83 | RP008x | 90 | - | 268 |
| 6 | S057.5.2 | 33 | 0.393939 | dye | 22.55477 | -5.30734 | 0.202436466 | 0.780147017 | 0.722710085 | 0.0013099915 | FP003 | 83 | RP008x | 285 | - | 269 |
| 7 | S057.8.2 | 33 | 0.393939 | dye | 17.66352 | -4.38678 | 0.199598548 | 0.824140979 | 1.219114841 | 0.0010522296 | FP057.1.0 | 84 | RP003x | 91 | - | 280 |
| 8 | S056.2.8 | 36 | 0.444444 | dye | 19.77602 | -4.5139 | 0.222613228 | 0.755430919 | 1.914602158 | 0.0044520444 | FP002 | 82 | RP002x | 284 | - | 105 |
| 9 | S056.4.8 | 36 | 0.444444 | dye | 24.45625 | -6.10608 | 0.177953676 | 0.826851462 | 0.904293765 | 0.0018395755 | FP004 | 81 | RP004x | 283 | - | 197 |
| 10 | S056.2.8 | 36 | 0.444444 | dye | 22.66316 | -5.92217 | 0.168214974 | 0.865508727 | 0.625904081 | 0.0017651144 | FP002 | 82 | RP002x | 284 | - | 106 |
| 11 | S056.1.8 | 36 | 0.444444 | dye | 21.48975 | -6.06302 | 0.143584244 | 0.937543561 | 0.641910939 | 0.0010092144 | FP001 | 85 | RP001x | 92 | - | 159 |
| 12 | S044.4 | 40 | 0.45 | dye | 24.22461 | -6.77879 | 0.141430313 | 0.929251259 | 1.834889896 | 0.0002717417 | FP004 | 81 | RP004 | 88 | - | 199 |
| 13 | S056.2.5 | 49 | 0.755102 | dye | 27.30521 | -3.41213 | 0.385152773 | 0.409740293 | 1.897509728 | 0.0001087655 | FP002 | 82 | RP002x | 284 | - | 112 |
| 14 | S056.2.5 | 49 | 0.755102 | dye | 35.00627 | -6.63325 | 0.246662997 | 0.615971951 | 0.589325634 | 0.0017625466 | FP002 | 82 | RP002x | 284 | - | 113 |
| 15 | S056.4.5 | 49 | 0.755102 | dye | 30.15123 | -5.71757 | 0.247615544 | 0.626085997 | 0.817942093 | 0.0026233644 | FP004 | 81 | RP004x | 283 | - | 206 |
| 16 | S056.1.5 | 49 | 0.755102 | dye | 29.37189 | -5.90212 | 0.231199482 | 0.664757994 | 0.520001152 | 0.0013866687 | FP001 | 85 | RP001x | 92 | - | 171 |
| 17 | S044.5 | 50 | 0.26 | dye | 22.00935 | -6.43317 | 0.129602264 | 0.972425616 | 1.169952759 | 0.0004269522 | FP004 | 81 | RP004 | 88 | - | 233 |
| 18 | S044.6 | 50 | 0.62 | dye | 22.59062 | -5.25477 | 0.202504339 | 0.774854997 | 1.384715516 | 0.0012518322 | FP004 | 81 | RP004 | 88 | - | 220 |
| 19 | S044.7 | 50 | 0.74 | dye | 36.15022 | -7.34075 | 0.217180527 | 0.670944313 | 1.086045823 | 0.0019189333 | FP004 | 81 | RP004 | 88 | - | 208 |
| 20 | S044.8 | 55 | 0.454545 | dye | 25.44055 | -6.89321 | 0.151489726 | 0.894360176 | 2.142305393 | 0.0008199897 | FP004 | 81 | RP004 | 88 | - | 201 |
| 21 | S057.6.4 | 62 | 0.306452 | dye | 21.36097 | -3.875 | 0.292404957 | 0.600297353 | 1.129235053 | 0.0014585833 | FP003 | 83 | RP008x | 90 | - | 272 |

## Figure 23 (part 2 of 9)

| # | Name | BP | GC | Reporter | tau | F0_lg | rho | r | k | m | FP name | FP SEQ ID NO | RP name | RP SEQ ID NO | Probe SEQ ID NO | CO SEQ ID NO |
|---|------|----|----|----------|-----|-------|-----|---|---|---|---------|--------------|---------|--------------|-----------------|--------------|
| 22 | S057.5.4 | 62 | 0.306452 | dye | 22.95594 | -5.28325 | 0.208755846 | 0.764157403 | 0.874556803 | 0.001826124 | FP003 | 83 | RP008 | 285 | - | 273 |
| 23 | S057.8.4 | 62 | 0.306452 | dye | 17.49557 | -4.26076 | 0.207824697 | 0.808213367 | 1.483880261 | 0.00068011 | FP057.1.0 | 84 | RP003x | 91 | - | 282 |
| 24 | S036.5 | 74 | 0.527027 | dye | 21.43727 | -5.32385 | 0.187200967 | 0.823899091 | 2.174642424 | 0.001238399 | FP001 | 85 | RP001 | 286 | - | 181 |
| 25 | S036.6 | 74 | 0.527027 | dye | 22.56923 | -5.75439 | 0.175604818 | 0.845598774 | 2.265008968 | 0.000714334 | FP001 | 85 | RP001 | 286 | - | 178 |
| 26 | S056.2.1 | 80 | 0.3375 | dye | 20.86904 | -5.10064 | 0.194657577 | 0.811020717 | 0.890709561 | 0.001788856 | FP002 | 82 | RP002x | 284 | - | 137 |
| 27 | S056.4.1 | 80 | 0.3375 | dye | 20.83949 | -5.293 | 0.183010427 | 0.841874742 | 1.240115326 | 0.001893667 | FP004 | 81 | RP004x | 283 | - | 231 |
| 28 | S056.1.1 | 80 | 0.3375 | dye | 18.76496 | -5.1847 | 0.156213969 | 0.918520785 | 0.944309514 | 0.000920471 | FP001 | 85 | RP001x | 92 | - | 183 |
| 29 | S056.1.9 | 80 | 0.425 | dye | 19.91795 | -5.19247 | 0.174929881 | 0.865539195 | 0.928979825 | 0.001139497 | FP001 | 85 | RP001x | 92 | - | 184 |
| 30 | S056.2.7 | 84 | 0.797619 | dye | 23.69025 | -4.88981 | 0.240841537 | 0.681055284 | 2.673741214 | 0.006332732 | FP002 | 82 | RP002x | 284 | - | 116 |
| 31 | S056.2.7 | 84 | 0.797619 | dye | 25.30386 | -6.12206 | 0.184821611 | 0.800336352 | 1.072077768 | 0.004693838 | FP002 | 82 | RP002x | 284 | - | 117 |
| 32 | S056.1.7 | 84 | 0.797619 | dye | 24.56026 | -6.07276 | 0.180415875 | 0.817270639 | 1.162031717 | 0.003555065 | FP001 | 85 | RP001x | 92 | - | 172 |
| 33 | S056.4.7 | 84 | 0.797619 | dye | 24.21626 | -6.02593 | 0.177993774 | 0.824488944 | 1.342147049 | 0.002657985 | FP004 | 81 | RP004x | 283 | - | 210 |
| 34 | S057.6.0 | 85 | 0.294118 | dye | 22.60534 | -4.10096 | 0.288044688 | 0.598019927 | 1.114918836 | 0.001426017 | FP003 | 83 | RP008x | 90 | - | 270 |
| 35 | S057.5.0 | 85 | 0.294118 | dye | 24.70262 | -5.75281 | 0.199119896 | 0.772296882 | 1.030933818 | 0.002513671 | FP003 | 83 | RP008 | 285 | - | 271 |
| 36 | S057.8.0 | 85 | 0.294118 | dye | 18.23201 | -4.44436 | 0.204055826 | 0.809919032 | 1.718028195 | 0.000164523 | FP057.1.0 | 84 | RP003x | 91 | - | 281 |
| 37 | S044.9 | 88 | 0.147727 | dye | 25.22 | -5.74767 | 0.204318251 | 0.758259949 | 2.198177864 | 0.006168834 | FP004 | 81 | RP004 | 88 | - | 263 |
| 38 | S044.10 | 88 | 0.25 | dye | 23.8542 | -6.9906 | 0.127439076 | 0.972200018 | 2.248585595 | 0.002201928 | FP004 | 81 | RP004 | 88 | - | 260 |
| 39 | S044.11 | 88 | 0.352273 | dye | 25.01305 | -7.01978 | 0.140757677 | 0.928039748 | 2.470400248 | 0.000702507 | FP004 | 81 | RP004 | 88 | - | 187 |
| 40 | S036.2 | 88 | 0.431818 | dye | 23.74954 | -5.75443 | 0.189834368 | 0.802940098 | 2.229267176 | 0.000445698 | FP002 | 82 | RP002 | 89 | - | 143 |
| 41 | S044.12 | 88 | 0.431818 | dye | 24.67649 | -6.22622 | 0.173048909 | 0.839189749 | 2.360427081 | 0.002745813 | FP004 | 81 | RP004 | 88 | - | 245 |
| 42 | S044.13 | 88 | 0.431818 | dye | 25.49836 | -6.86896 | 0.159112219 | 0.881726622 | 2.289582183 | 0.001036163 | FP004 | 81 | RP004 | 88 | - | 194 |

# Figure 23 (part 3 of 9)

| | Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|---|
| 43 | S036.7 | 88 | 0.431818 | Dye | 21.97007 | -6.03219 | 0.152182728 | 0.912691246 | 2.250461875 | 0.000240107 | FP001 | 85 | RP001 | 286 | - | 163 |
| 44 | S044.14 | 88 | 0.545455 | dye | 25.62734 | -5.85128 | 0.202072092 | 0.759388879 | 2.705744133 | 0.003209506 | FP004 | 81 | RP004 | 88 | - | 251 |
| 45 | S044.15 | 88 | 0.647727 | dye | 24.62975 | -5.60579 | 0.219687109 | 0.737331517 | 2.792340651 | 0.000366468 | FP004 | 81 | RP004 | 88 | - | 253 |
| 46 | S044.16 | 88 | 0.75 | dye | 24.39622 | -5.78804 | 0.206859201 | 0.767703296 | 3.164287663 | 0.001051159 | FP004 | 81 | RP004 | 88 | - | 228 |
| 47 | S044.17 | 88 | 0.795455 | dye | 29.12396 | -7.58454 | 0.163657626 | 0.849334684 | 3.039924378 | 0.000583586 | FP004 | 81 | RP004 | 88 | - | 212 |
| 48 | S044.18 | 88 | 0.852273 | dye | 35.43473 | -9.34891 | 0.150533979 | 0.861390483 | 2.072098945 | 0.001509776 | FP004 | 81 | RP004 | 88 | - | 224 |
| 49 | S036.4 | 108 | 0.25 | dye | 26.93994 | -6.77725 | 0.168150187 | 0.839929004 | 2.318966986 | 0.001821916 | FP002 | 82 | RP002 | 89 | - | 134 |
| 50 | S036.1 | 108 | 0.481481 | dye | 23.86644 | -5.17575 | 0.224701634 | 0.719253584 | 2.560986659 | 0.001311985 | FP002 | 82 | RP002 | 89 | - | 130 |
| 51 | S036.0 | 108 | 0.481481 | dye | 22.184 | -4.83636 | 0.229909485 | 0.721057277 | 2.536634412 | 0.000140665 | FP002 | 82 | RP002 | 89 | - | 129 |
| 52 | S056.2.4 | 152 | 0.203947 | dye | 20.16035 | -3.738 | 0.295392498 | 0.610957513 | 3.176629065 | 0.002160525 | FP002 | 82 | RP002x | 284 | - | 145 |
| 53 | S056.2.4 | 152 | 0.203947 | dye | 22.24042 | -4.52407 | 0.251510891 | 0.671837801 | 1.176502336 | 0.003119346 | FP002 | 82 | RP002x | 284 | - | 146 |
| 54 | S056.4.4 | 152 | 0.203947 | dye | 21.90508 | -4.8608 | 0.223733998 | 0.736704918 | 1.561801378 | 0.004144096 | FP004 | 81 | RP004x | 283 | - | 247 |
| 55 | S056.1.4 | 152 | 0.203947 | dye | 29.82336 | -7.06505 | 0.179963484 | 0.786470883 | 1.239545963 | 0.002684943 | FP001 | 85 | RP001x | 92 | - | 185 |
| 56 | S056.2.6 | 152 | 0.592105 | dye | 21.90466 | -3.10191 | 0.384676959 | 0.4610999 | 3.235507218 | 0.002854266 | FP002 | 82 | RP002x | 284 | - | 124 |
| 57 | S056.4.6 | 152 | 0.592105 | dye | 23.3767 | -3.80598 | 0.324718444 | 0.534171007 | 1.941211973 | 0.005284191 | FP004 | 81 | RP004x | 283 | - | 222 |
| 58 | S056.2.6 | 152 | 0.592105 | dye | 22.17672 | -3.80692 | 0.310703538 | 0.564803551 | 1.570064464 | 0.003277497 | FP002 | 82 | RP002x | 284 | - | 125 |
| 59 | S056.1.6 | 152 | 0.592105 | dye | 21.84216 | -3.83274 | 0.305942105 | 0.577120754 | 1.594118383 | 0.001423933 | FP001 | 85 | RP001x | 92 | - | 176 |
| 60 | S044.19 | 160 | 0.13125 | dye | 22.592 | -5.90165 | 0.166760151 | 0.869381477 | 1.407293615 | 0.003170533 | FP004 | 81 | RP004 | 88 | - | 236 |
| 61 | S044.20 | 160 | 0.21875 | dye | 23.57248 | -6.01541 | 0.171762291 | 0.848361131 | 1.898367777 | 0.002490637 | FP004 | 81 | RP004 | 88 | - | 265 |
| 62 | S044.21 | 160 | 0.43125 | dye | 22.67854 | -4.94514 | 0.236282582 | 0.709571458 | 3.119322832 | 0.000792741 | FP004 | 81 | RP004 | 88 | - | 217 |
| 63 | S044.22 | 160 | 0.5875 | dye | 23.01352 | -4.13034 | 0.288756188 | 0.594390253 | 2.443828524 | 0.003057071 | FP004 | 81 | RP004 | 88 | - | 243 |
| 64 | S044.23 | 160 | 0.78125 | dye | 36.96463 | -10.3064 | 0.129661638 | 0.920068678 | 1.150401227 | 0.000396712 | FP004 | 81 | RP004 | 88 | - | 255 |

# Figure 23 (part 4 of 9)

| | Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|---|
| 65 | S044.24 | 200 | 0.425 | dye | 23.21749 | -4.9249 | 0.24640739 | 0.687218919 | 3.427291284 | 0.003028616 | FP004 | 81 | RP004 | 88 | - | 189 |
| 66 | S056.2.2 | 232 | 0.413793 | dye | 19.34331 | -3.0764 | 0.356469512 | 0.523503692 | 4.060533663 | 0.000143841 | FP002 | 82 | RP002 x | 284 | - | 120 |
| 67 | S056.4.2 | 232 | 0.413793 | dye | 22.10906 | -4.25517 | 0.274790691 | 0.634195122 | 2.092694079 | 0.004796021 | FP004 | 81 | RP004 x | 283 | - | 214 |
| 68 | S056.2.2 | 232 | 0.413793 | dye | 20.11051 | -3.94627 | 0.273706328 | 0.647647543 | 1.784416097 | 0.003729638 | FP002 | 82 | RP002 x | 284 | - | 121 |
| 69 | S056.1.2 | 232 | 0.413793 | dye | 18.66527 | -3.88687 | 0.257449214 | 0.689529129 | 1.894137176 | 0.000189413 | FP001 | 85 | RP001 x | 92 | - | 173 |
| 70 | S044.25 | 240 | 0.420833 | dye | 24.18757 | -5.1029 | 0.249290487 | 0.679081624 | 3.759592547 | 0.001506206 | FP004 | 81 | RP004 | 88 | - | 191 |
| 71 | S056.2.3 | 272 | 0.672794 | dye | 21.40841 | -2.62491 | 0.426887961 | 0.403514758 | 4.583873912 | 6.24E-05 | FP002 | 82 | RP002 x | 284 | - | 149 |
| 72 | S056.1.3 | 272 | 0.672794 | dye | 26.69774 | -3.76202 | 0.350045353 | 0.464459716 | 2.443049432 | 0.003966982 | FP001 | 85 | RP001 x | 92 | - | 186 |
| 73 | S056.4.3 | 272 | 0.672794 | dye | 24.94727 | -3.69985 | 0.349095937 | 0.486360398 | 2.787871987 | 0.006012187 | FP004 | 81 | RP004 x | 283 | - | 249 |
| 74 | S056.2.3 | 272 | 0.672794 | dye | 24.52717 | -3.87393 | 0.329340594 | 0.517652855 | 2.375256188 | 0.006121578 | FP002 | 82 | RP002 x | 284 | - | 150 |
| 75 | S044.27 | 280 | 0.682143 | dye | 29.07709 | -4.50359 | 0.316848782 | 0.509671148 | 3.202463111 | 0.005098751 | FP004 | 81 | RP004 | 88 | - | 226 |
| 76 | S044.28 | 500 | 0.418 | dye | 26.18572 | -2.71316 | 0.495842261 | 0.352729916 | 2.099881709 | 0.000837512 | FP004 | 81 | RP004 | 88 | - | 240 |
| 77 | S044.29 | 500 | 0.57 | dye | 24.49291 | -3.58733 | 0.351766044 | 0.482520733 | 4.597819372 | 4.74E-05 | FP004 | 81 | RP004 | 88 | - | 203 |
| 78 | S036.3 | 30 | 0.433333 | probe | 26.48099 | -6.69646 | 0.168138223 | 0.840482061 | 0.568292491 | 0.007916798 | FP002 | 82 | RP002 | 89 | 94 | 133 |
| 79 | S044.2 | 30 | 0.466667 | probe | 25.50791 | -7.21511 | 0.134472525 | 0.940679357 | 0.805211519 | 0.011628964 | FP004 | 81 | RP004 | 88 | - | 196 |
| 80 | S036.8 | 30 | 0.533333 | probe | 26.97503 | -7.27253 | 0.14696 | 0.896754314 | 1.117398466 | 0.012654387 | FP001 | 85 | RP001 | 286 | 95 | 174 |
| 81 | S036.8 | 30 | 0.533333 | probe | 25.68873 | -7.38753 | 0.129323385 | 0.955515894 | 1.021689767 | 0.017109545 | FP001 | 85 | RP001 | 286 | 95 | 175 |
| 82 | S056.2.8 | 36 | 0.444444 | probe | 21.17774 | -4.94842 | 0.207458875 | 0.775628609 | 0.668860258 | 0.003156745 | FP002 | 82 | RP002 x | 284 | 94 | 107 |
| 83 | S056.2.8 | 36 | 0.444444 | probe | 23.05884 | -5.50564 | 0.194998269 | 0.793641273 | 0.458133411 | 0.007481535 | FP002 | 82 | RP002 x | 284 | 94 | 108 |
| 84 | S056.4.8 | 36 | 0.444444 | probe | 24.79408 | -6.13635 | 0.178948626 | 0.822296183 | 0.580755328 | 0.013714002 | FP004 | 81 | RP004 x | 283 | 94 | 198 |
| 85 | S044.4 | 40 | 0.45 | probe | 24.12529 | -6.6807 | 0.144232575 | 0.920407573 | 0.797028002 | 0.006467551 | FP004 | 81 | RP004 | 88 | 94 | 200 |
| 86 | S057.2.0 | 49 | 0.693878 | probe | 23.39717 | -4.65159 | 0.254713818 | 0.660056936 | 0.591198417 | 0.032559746 | FP001 | 85 | RP001 | 286 | 95 | 158 |

# Figure 23 (part 5 of 9)

| | Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|---|
| 87 | S056.2.5 | 49 | 0.755102 | probe | 28.66048 | -3.44254 | 0.392216173 | 0.394551857 | 0.421544123 | 0.016380531 | FP002 | 82 | RP002x | 284 | 94 | 114 |
| 88 | S056.4.5 | 49 | 0.755102 | probe | 30.56403 | -4.60875 | 0.327337967 | 0.486807345 | 0.329988861 | 0.015298884 | FP004 | 81 | RP004x | 283 | 94 | 207 |
| 89 | S056.2.5 | 49 | 0.755102 | probe | 27.75893 | -4.12237 | 0.332798823 | 0.487304154 | 0.303859889 | 0.016387454 | FP002 | 82 | RP002x | 284 | 94 | 115 |
| 90 | S044.5 | 50 | 0.26 | probe | 24.20062 | -6.99505 | 0.129734871 | 0.959880771 | 0.519288351 | 0.006916408 | FP004 | 81 | RP004 | 88 | 94 | 234 |
| 91 | S044.6 | 50 | 0.62 | probe | 25.49996 | -5.68227 | 0.208926676 | 0.740206722 | 0.516235409 | 0.006113741 | FP004 | 81 | RP004 | 88 | 94 | 221 |
| 92 | S044.7 | 50 | 0.74 | probe | 32.26196 | -4.49848 | 0.332574697 | 0.461121907 | 0.444022174 | 0.010801041 | FP004 | 81 | RP004 | 88 | 94 | 209 |
| 93 | S044.8 | 55 | 0.454545 | probe | 25.32923 | -6.71744 | 0.156658014 | 0.879273712 | 0.782697795 | 0.005797605 | FP004 | 81 | RP004 | 88 | 94 | 202 |
| 94 | S037.08.01 | 57 | 0.280702 | probe | 18.48296 | -5.00458 | 0.164542224 | 0.899980327 | 0.636912889 | 0.022145551 | FP003 | 83 | RP008 | 285 | - | 274 |
| 95 | S036.5 | 74 | 0.527027 | probe | 26.26203 | -6.34788 | 0.182208789 | 0.801963424 | 0.746524387 | 0.005563503 | FP001 | 85 | RP001 | 286 | 97 | 179 |
| 96 | S036.6 | 74 | 0.527027 | probe | 24.18969 | -5.94335 | 0.181599019 | 0.817293793 | 1.065550803 | 0.010041843 | FP001 | 85 | RP001 | 286 | 95 | 177 |
| 97 | S036.5 | 74 | 0.527027 | probe | 23.30964 | -5.88179 | 0.175474436 | 0.838409789 | 0.836697518 | 0.006578007 | FP001 | 85 | RP001 | 286 | 97 | 180 |
| 98 | S037.02.02 | 77 | 0.571429 | probe | 22.90283 | -3.89164 | 0.308148198 | 0.562211782 | 0.776925654 | 0.007073816 | FP001 | 85 | RP005 | 287 | 95 | 170 |
| 99 | S056.2.1 | 80 | 0.3375 | probe | 22.74352 | -5.08721 | 0.219833027 | 0.738019367 | 0.435908816 | 0.00830186 | FP002 | 82 | RP002x | 284 | 94 | 138 |
| 100 | S056.2.1 | 80 | 0.3375 | probe | 21.08062 | -5.22001 | 0.188939118 | 0.819742367 | 0.629962927 | 0.002783262 | FP002 | 82 | RP002x | 284 | 94 | 139 |
| 101 | S056.4.1 | 80 | 0.3375 | probe | 22.50478 | -5.60995 | 0.183353524 | 0.827348014 | 0.532959339 | 0.004992415 | FP004 | 81 | RP004x | 283 | 94 | 232 |
| 102 | S056.2.9 | 80 | 0.425 | probe | 23.50752 | -4.98929 | 0.231813246 | 0.704113193 | 0.604437311 | 0.004123805 | FP002 | 82 | RP002x | 284 | 94 | 140 |
| 103 | S056.2.9 | 80 | 0.425 | probe | 22.57127 | -5.04981 | 0.217532374 | 0.743290446 | 0.396418856 | 0.008839617 | FP002 | 82 | RP002x | 284 | 94 | 141 |
| 104 | S056.4.9 | 80 | 0.425 | probe | 23.22576 | -5.34652 | 0.207578193 | 0.763245507 | 0.508943627 | 0.005700277 | FP004 | 81 | RP004x | 283 | 94 | 235 |
| 105 | S037.02.01 | 81 | 0.543213 | probe | 18.52531 | -3.44871 | 0.292546984 | 0.619296653 | 0.812093458 | 0.006453263 | FP001 | 85 | RP005 | 287 | 95 | 156 |
| 106 | S056.2.7 | 84 | 0.797619 | probe | 29.34834 | -5.50263 | 0.251503525 | 0.621515905 | 0.543362253 | 0.007725275 | FP002 | 82 | RP002x | 284 | 94 | 118 |
| 107 | S056.2.7 | 84 | 0.797619 | probe | 26.86971 | -5.88689 | 0.210123646 | 0.727521509 | 0.375873442 | 0.014974122 | FP002 | 82 | RP002x | 284 | 94 | 119 |
| 108 | S056.4.7 | 84 | 0.797619 | probe | 25.63802 | -6.00963 | 0.192272766 | 0.778610953 | 0.423802364 | 0.018446973 | FP004 | 81 | RP004x | 283 | 94 | 211 |

# Figure 23 (part 6 of 9)

| | Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|---|
| 109 | S057.3.4 | 85 | 0.529412 | probe | 20.62355 | -3.75741 | 0.294011312 | 0.604800678 | 0.873770591 | 0.008062492 | FP001 | 85 | RP005 | 287 | 95 | 154 |
| 110 | S057.4.0 | 87 | 0.551724 | probe | 26.44523 | -4.62199 | 0.284405271 | 0.580610600 | 0.544326581 | 0.008977662 | FP005 | 86 | RP005 | 287 | 94 | 277 |
| 111 | S044.9 | 88 | 0.147727 | probe | 26.73872 | -5.78854 | 0.219019919 | 0.714669697 | 0.907075837 | 0.007907564 | FP004 | 81 | RP004 | 88 | 94 | 264 |
| 112 | S056.2.12 | 88 | 0.25 | probe | 21.69381 | -4.55065 | 0.243901953 | 0.695864779 | 0.614931108 | 0.011405301 | FP002 | 82 | RP002x | 284 | 95 | 153 |
| 113 | S056.4.12 | 88 | 0.25 | probe | 22.96282 | -5.51975 | 0.193529844 | 0.797932405 | 0.790033018 | 0.006928423 | FP004 | 81 | RP004x | 283 | 95 | 258 |
| 114 | S057.9.1 | 88 | 0.25 | probe | 22.31384 | -5.75412 | 0.171230397 | 0.858940406 | 0.632620201 | 0.014247625 | FP004 | 81 | RP004 | 88 | 95 | 259 |
| 115 | S044.10 | 88 | 0.25 | probe | 24.20053 | -7.18732 | 0.123539143 | 0.984259959 | 0.775581024 | 0.006092054 | FP004 | 81 | RP004 | 88 | 94 | 261 |
| 116 | S044.11 | 88 | 0.352273 | probe | 26.02938 | -7.21987 | 0.141719758 | 0.918345732 | 0.812863544 | 0.006057509 | FP004 | 81 | RP004 | 88 | 94 | 188 |
| 117 | S056.2.11 | 88 | 0.431818 | probe | 20.12171 | -4.07979 | 0.260988327 | 0.671106877 | 0.564324019 | 0.011199276 | FP002 | 82 | RP002x | 284 | 95 | 109 |
| 118 | S056.4.11 | 88 | 0.431818 | probe | 24.17466 | -5.17218 | 0.227307618 | 0.710314216 | 0.730087091 | 0.006453876 | FP004 | 81 | RP004x | 283 | 95 | 205 |
| 119 | S036.2 | 88 | 0.431818 | probe | 24.46266 | -5.52661 | 0.208602707 | 0.749972206 | 0.520196891 | 0.007964762 | FP002 | 82 | RP002 | 89 | 94 | 144 |
| 120 | S044.12 | 88 | 0.431818 | probe | 26.13601 | -6.38563 | 0.179734866 | 0.811322373 | 0.676890067 | 0.006267102 | FP004 | 81 | RP004 | 88 | 94 | 246 |
| 121 | S036.7 | 88 | 0.431818 | probe | 25.50829 | -6.40303 | 0.172657456 | 0.833600363 | 1.123309085 | 0.005844295 | FP001 | 85 | RP001 | 286 | 95 | 161 |
| 122 | S044.13 | 88 | 0.431818 | probe | 24.56012 | -6.19236 | 0.173559654 | 0.837658414 | 1.121480684 | 0.013982403 | FP004 | 81 | RP004 | 88 | 95 | 193 |
| 123 | S036.7 | 88 | 0.431818 | probe | 23.53326 | -6.15127 | 0.163739066 | 0.869378456 | 1.050316381 | 0.006711014 | FP001 | 85 | RP001 | 286 | 95 | 162 |
| 124 | S044.14 | 88 | 0.545455 | probe | 26.78579 | -5.86576 | 0.213368797 | 0.725798521 | 0.741799619 | 0.008991522 | FP004 | 81 | RP004 | 88 | 94 | 252 |
| 125 | S044.15 | 88 | 0.647727 | probe | 25.99815 | -5.20042 | 0.252360792 | 0.653579934 | 0.729286967 | 0.010310075 | FP004 | 81 | RP004 | 88 | 94 | 254 |
| 126 | S044.16 | 88 | 0.75 | probe | 26.33469 | -5.28346 | 0.243801226 | 0.663248008 | 0.735305274 | 0.023161874 | FP004 | 81 | RP004 | 88 | 94 | 229 |
| 127 | S044.17 | 88 | 0.795455 | probe | 30.73468 | -6.62085 | 0.214413919 | 0.706794399 | 0.815735988 | 0.016206679 | FP004 | 81 | RP004 | 88 | 94 | 213 |
| 128 | S044.18 | 88 | 0.852273 | probe | 36.60254 | -7.95076 | 0.207798705 | 0.706469096 | 0.564007983 | 0.034961325 | FP004 | 81 | RP004 | 88 | 94 | 225 |
| 129 | S057.3.0 | 90 | 0.455556 | probe | 20.77715 | -3.60809 | 0.307550691 | 0.578205903 | 0.853049672 | 0.008494957 | FP001 | 85 | RP005 | 287 | 95 | 167 |
| 130 | S057.0.0 | 92 | 0.456522 | probe | 19.95252 | -4.07722 | 0.257596128 | 0.677628007 | 0.623372006 | 0.011432039 | FP002 | 82 | RP006 | 93 | 95 | 111 |

# Figure 23 (part 7 of 9)

| | Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|---|
| 131 | S037.02.04 | 92 | 0.554348 | probe | 19.06714 | -3.32695 | 0.31136807 | 0.579044933 | 0.767283289 | 0.006383185 | FP001 | 85 | RP005 | 287 | 95 | 155 |
| 132 | S057.1.0 | 93 | 0.494624 | probe | 22.11012 | -4.23914 | 0.268910048 | 0.636387354 | 0.491098558 | 0.007929297 | FP006 | 87 | RP006 | 93 | 94 | 99 |
| 133 | S037.02.03 | 95 | 0.484211 | probe | 19.94637 | -4.33624 | 0.235562773 | 0.720896455 | 0.842735428 | 0.007092123 | FP001 | 85 | RP005 | 287 | 95 | 160 |
| 134 | S037.06.01 | 98 | 0.479592 | probe | 19.68759 | -4.15438 | 0.247403216 | 0.699198183 | 0.439963833 | 0.011590639 | FP004 | 81 | RP004 | 88 | 94 | 219 |
| 135 | S057.3.1 | 98 | 0.571429 | probe | 22.92024 | -3.61026 | 0.333370417 | 0.522655157 | 0.860449897 | 0.008716287 | FP001 | 85 | RP005 | 287 | 95 | 166 |
| 136 | S037.04.01 | 100 | 0.48 | probe | 20.04926 | -3.07611 | 0.353402344 | 0.507871134 | 1.268198224 | 0.011708888 | FP002 | 82 | RP006 | 93 | 94 | 136 |
| 137 | S057.0.1 | 100 | 0.48 | probe | 21.76216 | -4.22461 | 0.268500061 | 0.644228903 | 0.625954616 | 0.012521077 | FP002 | 82 | RP006 | 93 | 95 | 110 |
| 138 | S037.06.02 | 101 | 0.435644 | probe | 19.59495 | -4.43809 | 0.223447834 | 0.750873876 | 0.484839148 | 0.010407763 | FP004 | 81 | RP004 | 88 | 94 | 216 |
| 139 | S057.0.2 | 103 | 0.446602 | probe | 21.58317 | -4.28648 | 0.260307976 | 0.660291267 | 0.634733968 | 0.012346787 | FP002 | 82 | RP006 | 93 | 95 | 104 |
| 140 | S037.05.01 | 103 | 0.485437 | probe | 19.50329 | -3.92487 | 0.262841473 | 0.667590268 | 0.681614576 | 0.007763109 | FP006 | 87 | RP006 | 93 | 95 | 101 |
| 141 | S037.04.04 | 104 | 0.432692 | probe | 21.12654 | -3.3125 | 0.334494741 | 0.520991183 | 1.281800586 | 0.012337621 | FP002 | 82 | RP006 | 93 | 94 | 103 |
| 142 | S037.06.03 | 106 | 0.405660 | probe | 18.07058 | -4.112 | 0.228556064 | 0.755011175 | 0.488284129 | 0.010093714 | FP004 | 81 | RP004 | 88 | 94 | 257 |
| 143 | S037.05.02 | 106 | 0.443396 | probe | 16.48625 | -3.53658 | 0.253457944 | 0.712180349 | 0.664252253 | 0.008384896 | FP006 | 87 | RP006 | 93 | 95 | 100 |
| 144 | S036.4 | 108 | 0.25 | probe | 30.26318 | -7.27937 | 0.174665597 | 0.799194579 | 0.578393889 | 0.007164336 | FP002 | 82 | RP002 | 89 | 94 | 135 |
| 145 | S036.0 | 108 | 0.481481 | probe | 22.07135 | -4.58777 | 0.241516382 | 0.690036363 | 0.886290889 | 0.014072113 | FP002 | 82 | RP002 | 89 | 96 | 128 |
| 146 | S036.1 | 108 | 0.481481 | probe | 26.52282 | -5.62392 | 0.222755206 | 0.703500938 | 0.506552275 | 0.008196745 | FP002 | 82 | RP002 | 89 | 94 | 131 |
| 147 | S057.1.2 | 111 | 0.459459 | probe | 21.78563 | -4.26135 | 0.264020424 | 0.649927369 | 0.504931285 | 0.007536096 | FP006 | 87 | RP006 | 93 | 94 | 98 |
| 148 | S057.1.1 | 113 | 0.486726 | probe | 21.33173 | -3.75396 | 0.301596631 | 0.583598 | 0.479573381 | 0.008600305 | FP006 | 87 | RP001 | 93 | 94 | 102 |
| 149 | S057.3.2 | 115 | 0.521739 | probe | 22.16016 | -3.46563 | 0.335762955 | 0.519173814 | 0.821141654 | 0.009930111 | FP001 | 85 | RP005 | 287 | 95 | 169 |
| 150 | S037.03.02 | 117 | 0.675214 | probe | 25.12651 | -3.26281 | 0.380987693 | 0.428633168 | 0.465369681 | 0.012629325 | FP005 | 86 | RP005 | 287 | 94 | 276 |
| 151 | S056.2.4 | 152 | 0.203947 | probe | 25.6221 | -4.53018 | 0.281010527 | 0.588375198 | 0.424346271 | 0.010918471 | FP002 | 82 | RP002x | 284 | 94 | 147 |
| 152 | S056.4.4 | 152 | 0.203947 | probe | 24.28297 | -5.36499 | 0.216391573 | 0.734422428 | 0.582597332 | 0.005593292 | FP004 | 81 | RP004x | 283 | 94 | 248 |

# Figure 23 (part 8 of 9)

| Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|
| 153 | S056.2.4 | 152 | 0.203947 | probe | 22.846888 | -5.45512 | 0.195025696 | 0.793600179 | 0.648606012 | 0.00359828 | FP002 | 82 | RP002x | 284 | 94 | 148 |
| 154 | S056.2.6 | 152 | 0.592105 | probe | 26.6266 | -4.08155 | 0.323566796 | 0.508315041 | 0.593766411 | 0.008734151 | FP002 | 82 | RP002x | 284 | 94 | 126 |
| 155 | S056.4.6 | 152 | 0.592105 | probe | 26.574011 | -4.29315 | 0.3075113 | 0.535778509 | 0.488606175 | 0.01013336 | FP004 | 81 | RP004x | 283 | 94 | 223 |
| 156 | S056.2.6 | 152 | 0.592105 | probe | 24.478944 | -4.25859 | 0.291253871 | 0.577169767 | 0.386727034 | 0.011360543 | FP002 | 82 | RP002x | 284 | 94 | 127 |
| 157 | S044.19 | 160 | 0.13125 | probe | 29.1539 | -6.95406 | 0.182352243 | 0.791086759 | 0.400883194 | 0.013259312 | FP004 | 81 | RP004 | 88 | 94 | 237 |
| 158 | S044.20 | 160 | 0.21875 | probe | 29.073666 | -6.86343 | 0.183413028 | 0.784880304 | 0.543915336 | 0.005515097 | FP004 | 81 | RP004 | 88 | 94 | 266 |
| 159 | S044.21 | 160 | 0.43125 | probe | 25.394551 | -5.4802 | 0.229073492 | 0.704544036 | 0.714758766 | 0.006180193 | FP004 | 81 | RP004 | 88 | 94 | 218 |
| 160 | S044.22 | 160 | 0.5875 | probe | 28.835966 | -4.65428 | 0.301217017 | 0.532623827 | 0.493971292 | 0.006858157 | FP004 | 81 | RP004 | 88 | 94 | 244 |
| 161 | S044.23 | 160 | 0.78125 | probe | 35.013122 | -7.28528 | 0.235651486 | 0.664288093 | 0.249378127 | 0.019663297 | FP001 | 85 | RP001 | 286 | 95 | 256 |
| 162 | S037.01.01 | 161 | 0.577648 | probe | 22.969955 | -4.42099 | 0.264420671 | 0.636737821 | 0.578368052 | 0.024718672 | FP005 | 86 | RP005 | 287 | 94 | 182 |
| 163 | S057.4.1 | 167 | 0.550898 | probe | 27.119555 | -3.95639 | 0.340397208 | 0.481655538 | 0.552873188 | 0.010015901 | FP005 | 86 | RP005 | 287 | 94 | 278 |
| 164 | S037.03.01 | 192 | 0.442708 | probe | 21.076888 | -3.88142 | 0.286605378 | 0.607990333 | 0.561223147 | 0.006630308 | FP004 | 81 | RP004 | 88 | 94 | 279 |
| 165 | S044.24 | 200 | 0.425 | probe | 24.963699 | -4.90224 | 0.253970544 | 0.649794717 | 0.694270272 | 0.006281421 | FP005 | 86 | RP005 | 287 | 94 | 190 |
| 166 | S057.4.2 | 221 | 0.502262 | probe | 26.005577 | -3.89277 | 0.334172747 | 0.496332195 | 0.536449239 | 0.010640081 | FP002 | 82 | RP002x | 284 | 94 | 275 |
| 167 | S056.2.2 | 232 | 0.413793 | probe | 23.872599 | -4.25037 | 0.286399861 | 0.590035226 | 0.352201481 | 0.011495697 | FP004 | 81 | RP004x | 283 | 94 | 122 |
| 168 | S056.4.2 | 232 | 0.413793 | probe | 26.207099 | -4.75385 | 0.275566081 | 0.599459081 | 0.454247136 | 0.007861917 | FP002 | 82 | RP002x | 284 | 94 | 215 |
| 169 | S056.2.2 | 232 | 0.413793 | probe | 24.309522 | -4.43922 | 0.276201165 | 0.607952803 | 0.560885163 | 0.006159044 | FP004 | 81 | RP004 | 88 | 94 | 123 |
| 170 | S044.25 | 240 | 0.420833 | probe | 26.577955 | -4.82712 | 0.271727896 | 0.602442729 | 0.654036149 | 0.006190297 | FP002 | 82 | RP002x | 284 | 94 | 192 |
| 171 | S056.2.3 | 272 | 0.672794 | probe | 25.650556 | -3.31433 | 0.379595973 | 0.427033943 | 0.502416111 | 0.011276522 | FP004 | 81 | RP004x | 283 | 94 | 151 |
| 172 | S056.4.3 | 272 | 0.672794 | probe | 28.71674 | -4.08984 | 0.341682263 | 0.469051843 | 0.428286509 | 0.013476094 | FP002 | 82 | RP002x | 284 | 94 | 250 |
| 173 | S056.2.3 | 272 | 0.672794 | probe | 24.33611 | -3.58435 | 0.344712515 | 0.486638463 | 0.351734276 | 0.013534156 | FP004 | 81 | RP004 | 88 | 94 | 152 |
| 174 | S044.26 | 280 | 0.325 | probe | 32.736022 | -6.2065 | 0.245474087 | 0.623384231 | 0.480414553 | 0.004816403 | | | | | | 230 |

# Figure 23 (part 9 of 9)

| | Name | BP | GC | Reporter | tau | F0_lg | rho | r | K | m | FP name | FP SEQ ID NO | RP name | RP SEQ ID NO | Probe SEQ ID NO | CO SEQ ID NO |
|---|---|---|---|---|---|---|---|---|---|---|---|---|---|---|---|---|
| 175 | S044.27 | 280 | 0.682143 | probe | 33.56682 | -4.25399 | 0.359931979 | 0.415689084 | 0.42202557 | 0.015862445 | FP004 | 81 | RP004 | 88 | 94 | 227 |
| 176 | S037.01.03 | 326 | 0.496933 | probe | 22.10237 | -3.81544 | 0.300913657 | 0.573079628 | 0.521672365 | 0.028722555 | FP001 | 85 | RP001 | 286 | 95 | 165 |
| 177 | S057.2.2 | 331 | 0.516616 | probe | 21.77261 | -3.8624 | 0.295817851 | 0.587830722 | 0.509408425 | 0.033804952 | FP001 | 85 | RP001 | 286 | 95 | 157 |
| 178 | S057.2.4 | 380 | 0.486842 | probe | 24.56643 | -4.37521 | 0.282863099 | 0.589989639 | 0.452778158 | 0.063318658 | FP001 | 85 | RP001 | 286 | 95 | 164 |
| 179 | S057.2.1 | 461 | 0.505423 | probe | 24.81827 | -3.88456 | 0.324505763 | 0.518176883 | 0.510809614 | 0.034302904 | FP001 | 85 | RP001 | 286 | 95 | 168 |
| 180 | S044.28 | 500 | 0.418 | probe | 39.04724 | -5.27132 | 0.334212519 | 0.4368245 | 0.404445736 | 0.020426227 | FP004 | 81 | RP004 | 88 | 94 | 241 |
| 181 | S056.2.10 | 500 | 0.418 | probe | 27.31762 | -3.71033 | 0.359028865 | 0.448599573 | 0.474662506 | 0.017211915 | FP002 | 82 | RP002x | 284 | 95 | 142 |
| 182 | S056.4.10 | 500 | 0.418 | probe | 30.60635 | -4.23218 | 0.348665826 | 0.451598844 | 0.561948974 | 0.018471635 | FP004 | 81 | RP004x | 283 | 95 | 238 |
| 183 | S057.9.0 | 500 | 0.418 | probe | 29.21485 | -4.28633 | 0.329756396 | 0.482352588 | 0.508226766 | 0.017387489 | FP004 | 81 | RP004 | 88 | 95 | 239 |
| 184 | S044.29 | 500 | 0.57 | probe | 32.3858 | -4.04319 | 0.364350978 | 0.412115209 | 0.336536686 | 0.022460035 | FP004 | 81 | RP004 | 88 | 94 | 204 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WARSINSKE, H.** ; **VASHISHT, R.** ; **KHATRI, P.** Host-Response-Based Gene Signatures for Tuberculosis Diagnosis: A Systematic Comparison of 16 Signatures. *PLOS Medicine*, 2019, vol. 16 (4), e1002786, https://doi.org/10.1371/journal.pmed.1002786
- **SWEENEY, T. E.** ; **WONG, H. R.** ; **KHATRI, P.** Robust Classification of Bacterial and Viral Infections via Integrated Host Gene Expression Diagnostics. *Science Translational Medicine*, 2016, vol. 8 (346), https://doi.org/10.1126/scitranslmed.aaf7165
- **CARDOSO, F.** ; **VAN'T VEER, L. J.** ; **BOGAERTS, J.** ; **SLAETS, L.** ; **VIALE, G.** ; **DELALOGE, S.** ; **PIERGA, J.-Y.** ; **BRAIN, E.** ; **CAUSERET, S.** ; **DELORENZI, M.** 70-Gene Signature as an Aid to Treatment Decisions in Early-Stage Breast Cancer. *New England Journal of Medicine*, 2016, vol. 375 (8), 717-729, https://doi.org/10.1056/NEJMoa1602253
- **ZAAS, A. K.** ; **AZIZ, H.** ; **LUCAS, J.** ; **PERFECT, J. R.** ; **GINSBURG, G. S.** Blood Gene Expression Signatures Predict Invasive Candidiasis. *Science Translational Medicine*, 2010, vol. 2 (21), https://doi.org/10.1126/scitranslmed.3000715
- **COOMBES et al.** *Clinical Cancer Research*, 2019, vol. 25
- **YU et al.** *PLoS One*, 2015, vol. 10, e0132253
- **KONING et al.** *British Journal of Haematology*, 2016, vol. 178, 983-968
- **REID et al.** *Angewandte Chemie*, 2018, vol. 57, 11856-11866
- **MONOD, JACQUES.** The growth of bacterial cultures. *Annual Review of Microbiology*, 1949, vol. 3, 371-394
- **SPIESS et al.** *BMC Bioinformatics*, 2008, vol. 9 (221)
- **RUTLEDGE.** *NAR*, 2004, vol. 32, e178
- **LIU et al.** *Cell Culture and Tissue Engineering*, 2001, vol. 27, 1407-1414
- **KAFOROU, M.** ; **WRIGHT, V. J.** ; **ONI, T.** ; **FRENCH, N.** ; **ANDERSON, S. T.** ; **BANGANI, N.** ; **BANWELL, C. M.** ; **BRENT, A. J.** ; **CRAMPIN, A. C.** ; **DOCKRELL, H. M.** Detection of Tuberculosis in HIV-Infected and -Uninfected African Adults Using Whole Blood RNA Expression Signatures: A Case-Control Study. *PLOS Medicine*, 2013, vol. 10 (10), e1001538, https://doi.org/10.1371/journal.pmed.1001538
- **GLIDDON, H. D.** ; **KAFOROU, M.** ; **ALIKIAN, M.** ; **HABGOOD-COOTE, D.** ; **ZHOU, C.** ; **ONI, T.** ; **ANDERSON, S. T.** ; **BRENT, A. J.** ; **CRAMPIN, A. C.** ; **ELEY, B.** Consortium, on behalf of the I. Identification of Reduced Host Transcriptomic Signatures for Tuberculosis and Digital PCR-Based Validation and Quantification. *bioRxiv*, 2019, 583674, https://doi.org/10.1101/583674